(19) Europäisches Patentamt
European Patent Office
Office européen des brevets

(11) EP 2 119 771 B1

(12) **EUROPEAN PATENT SPECIFICATION**

(45) Date of publication and mention of the grant of the patent:
**22.08.2018 Bulletin 2018/34**

(51) Int Cl.:
***C12N 9/10*** *(2006.01)*

(21) Application number: **09011127.9**

(22) Date of filing: **23.12.2004**

(54) **Proteins**

Proteine

Protéines

(84) Designated Contracting States:
**AT BE BG CH CY CZ DE DK EE ES FI FR GB GR HU IE IS IT LI LT LU MC NL PL PT RO SE SI SK TR**

(30) Priority: **24.12.2003 GB 0330016**
**15.01.2004 PCT/IB2004/000655**
**16.07.2004 GB 0415999**
**02.08.2004 US 911160**

(43) Date of publication of application:
**18.11.2009 Bulletin 2009/47**

(62) Document number(s) of the earlier application(s) in accordance with Art. 76 EPC:
**04806537.9 / 1 704 236**

(73) Proprietor: **DuPont Nutrition Biosciences ApS**
**1411 Copenhagen K (DK)**

(72) Inventors:
- **Soe, Jorn, Borch**
  **8381 Tilst (DK)**
- **Mikkelsen, Jorn, Dalgaard**
  **2650 Hvidovre (DK)**
- **Lorentsen, Rikke, Hoegh**
  **8900 Randers (DK)**
- **Kellett, Smith, Anja, Hemmingsen**
  **8000 Arhus C (DK)**
- **Brunstedt, Janne**
  **4000 Roskilde (DK)**
- **Sorensen, Jens, Frisbaek**
  **8200 Aarhus (DK)**

(74) Representative: **D Young & Co LLP**
**120 Holborn**
**London EC1N 2DY (GB)**

(56) References cited:
**WO-A-00/05396 WO-A-98/45453**

- **DATABASE UniProt 1 March 2001 (2001-03-01), LACHMANN I. ET AL.: "SubName: Full=Glycerophospholipid-cholesterol acyltransferase" XP002549433 Database accession no. Q9F7Y6**
- **BRUMLIK MICHAEL J ET AL: "Identification of the catalytic triad of the lipase/acyltransferase from Aeromonas hydrophila" JOURNAL OF BACTERIOLOGY, AMERICAN SOCIETY FOR MICROBIOLOGY, US, vol. 178, no. 7, 1 January 1996 (1996-01-01), pages 2060-2064, XP002315734 ISSN: 0021-9193**
- **ROBERTSON D L ET AL: "Influence of Active Site and Tyrosine Modification on the Secretion and Activity of the Aeromonas hydrophila Lipase/Acyltransferase" JOURNAL OF BIOLOGICAL CHEMISTRY, AMERICAN SOCIETY OF BIOLOCHEMICAL BIOLOGISTS, BIRMINGHAM, US, vol. 269, no. 3, 21 January 1994 (1994-01-21), pages 2146-2150, XP002318365 ISSN: 0021-9258**
- **GRAILLE J: "POSSIBLE APPLICATIONS OF ACYLTRANSFERASES IN OLEOTECHNOLOGY" LIPID TECHNOLOGY, BARKING ESSEX, GB, vol. 5, no. 1, 1 January 1993 (1993-01-01), pages 11-16, XP008054101 ISSN: 0956-666X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 119 771 B1

## Description

REFERENCE TO RELATED APPLICATIONS

Reference is made to the following related applications: United States Application Serial Number 09/750,990 filed on 20 July 1999; United States Application Serial Number 10/409,391; United States Application Serial Number 60/489,441 filed on 23 July 2003; United Kingdom Application Number GB 0330016.7 filed on 24 December 2003 and International Patent Application Number PCT/IB2004/000655 filed on 15 January 2004.

FIELD OF INVENTION

The present invention relates to methods of producing variant enzymes. The present invention further relates to novel variant enzymes and to the use of these novel variant enzymes.

TECHNICAL BACKGROUND

Lipid-cholesterol acyltransferase enzymes have been known for some time (see for example Buckley - Biochemistry 1983, 22, 5490-5493). In particular, glycerophospholipid:cholesterol acyl transferases (GCATs) have been found, which like the plant and/or mammalian lecithin:cholesterol acyltransferases (LCATs), will catalyse fatty acid transfer between phosphatidylcholine and cholesterol.
Upton and Buckley (TIBS 20, May 1995, p178-179) and Brumlik and Buckley (J. of Bacteriology Apr, 1996, p2060-2064) teach a lipase/acyltransferase from *Aeromonas hydrophila* which has the ability to carry out acyl transfer to alcohol receptors in aqueous media.
A putative substrate binding domain and active site of the *A. hydrophila* acyltransferase have been identified (see for example Thornton et al 1988 Biochem. et Biophys. Acta, 959, 153-159 and Hilton & Buckley 1991 J. Biol. Chem. 266, 997-1000) for this enzyme.
Buckley et al (J. Bacteriol 1996, 178(7) 2060-4) taught that Ser16, Asp116 and His291 are essential amino acids which must be retained for enzyme activity to be maintained,
**[0007]** Robertson et al (J. Biol. Chem. 1994, 269, 2146-50) taught some specific mutations, namely Y226F, Y230F, Y30F, F13S, S18G, S18V, of the *A. hydrophila* acyltransferase, none of which are encompassed by the present invention.

SUMMARY ASPECTS OF THE PRESENT INVENTION

The present invention is predicated upon the finding of specific variants of a GDSx containing lipid acyltransferase enzyme, which variants have an increased transferase activity compared with a parent enzyme. In particular, the variants described herein have an enhanced transferase activity using galactolipid as an acyl donor as compared with a parent enzyme. These lipid acyltransferases are referred to herein as glycolipid acyltransferases. The variants described herein may additionally have an enhanced ratio of transferase activity using galactolipids as an acyl donor as compared with phospholipid transferase activity (GL:PL ratio) and/or an enhanced ratio of transferse activity using galactolipids as an acyl donor as compared with galactolipid hydrolysis activity (GLt:GLh ratio) compared with a parent enzyme.
The present invention provides a variant lipid acyltransferase having increased phospholipid transferase activity wherein the enzyme comprises the amino acid sequence motif GDSX, wherein X is one or more of the following amino acid residues L,A,V, I, F, Y, H, Q, T, N, M or S, wherein the variant enzyme comprises one or more amino acid modifications compared with a parent sequence at any one or more of the amino acid residues:

S3N, Q, K, R, P or M; or
E309A;

wherein the numbering is that obtained from alignment of the variant sequence with the reference sequence shown as SEQ ID No. 2 and wherein the parent lipid acyltransferase comprises the amino acid sequence shown as SEQ ID No. 28 or an amino acid sequence which has 75% or more identity with SEQ ID No. 28; or wherein the parent lipid acyltransferase is encoded by the nucleotide sequence shown as SEQ ID No. 29, or a nucleotide sequence which has at least 75% or more identity with the sequence shown as SEQ ID No. 29.
Further provided by the present invention is a method of preparing a foodstuff, the method comprising adding a variant lipid acyltransferase enzyme of the present invention to one or more ingredients of the foodstuff.
Further provided by the present invention is a method of preparing a baked product from a dough, the method comprising adding a variant lipid acyltransferase enzyme of the present invention to the dough.
The present invention also provides use of a variant lipid acyltransferase enzyme of the present invention in a process of treating egg or egg-based products to produce lysophospholipids.

Further provided by the present invention is a process of enzymatic degumming of vegetable or edible oils, comprising treating the edible oil or vegetable oil with a variant lipid acyltransferase enzyme of the present invention so as to hydrolyse a major part of the polar lipids (e.g. phospholipid and/or glycolipid).

The present invention also provides use of a variant lipid acyltransferase enzyme of the present invention in a process for reducing the content of a phospholipid in an edible oil, comprising treating the oil with said variant lipid acyltransferase enzyme so as to hydrolyse a major part of the phospholipid, and separating an aqueous phase containing the hydrolysed phospholipid from the oil.

The present invention also provides use of a variant lipid acyltransferase enzyme of the present invention in the bioconversion of polar lipids (preferably glycolipids) to make high value products, such as carbohydrate esters and/or protein esters and/or protein subunit esters and/or a hydroxy acid ester.

Further provided by the present invention is an immobilised variant lipid acyltransferase enzyme of the present invention.

Further described herein is a method of producing a variant glycolipid acyltransferase enzyme comprising; (a) selecting a parent enzyme which is a lipid acyltransferase enzyme characterised in that the enzyme comprises the amino acid sequence motif GDSX, wherein X is one or more of the following amino acid residues L, A, V, I, F, Y, H, Q, T, N, M or S; (b) modifying one or more amino acids to produce a variant lipid acyltransferase; (c) testing the variant lipid acyltransferase for activity on a galactolipid substrate, and optionally a phospholipid substrate and/or optionally a triglyceride substrate; (d) selecting a variant enzyme with an enhanced activity towards galactolipids compared with the parent enzyme; and optionally (e) preparing a quantity of the variant enzyme.

Described herein is a variant glycolipid acyltransferase enzyme characterised in that the enzyme comprises the amino acid sequence motif GDSX, wherein X is one or more of the following amino acid residues L, A, V, I, F, Y, H, Q, T, N, M or S, and wherein the variant enzyme comprises one or more amino acid modifications compared with a parent sequence at any one or more of the amino acid residues defined in set 2 or set 4 or set 6 or set 7 (defined hereinbelow).

Described herein is a variant glycolipid acyltransferase enzyme characterised in that the enzyme comprises the amino acid sequence motif GDSX, wherein X is one or more of the following amino acid residues L, A, V, I, F, Y, H, Q, T, N, M or S, and wherein the variant enzyme comprises one or more amino acid modifications compared with a parent sequence at any one or more of the amino acid residues detailed in set 2 or set 4 or set 6 or set 7 (defined hereinbelow) identified by said parent sequence being structurally aligned with the structural model of P10480 defined herein, which is preferably obtained by structural alignment of P10480 crystal structure coordinates with 1IVN.PDB and/or 1DEO.PDB as taught herein.

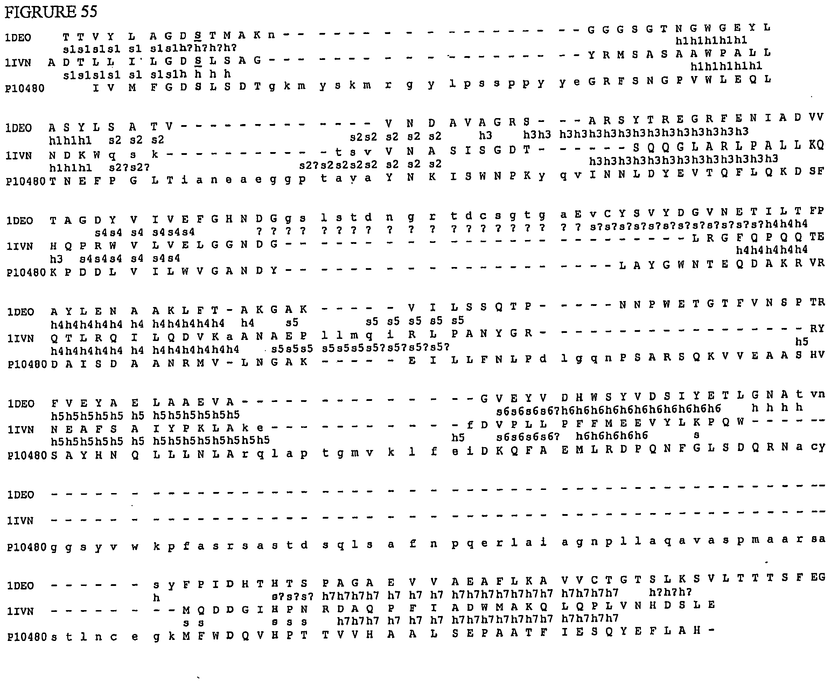

Described herein is a variant glycolipid acyltransferase enzyme characterised in that the enzyme comprises the amino acid sequence motif GDSX, wherein X is one or more of the following amino acid residues L, A, V, I, F, Y, H, Q, T, N, M or S, and wherein the variant enzyme comprises one or more amino acid modifications compared with a parent sequence at any one or more of the amino acid residues taught in set 2 identified when said parent sequence is aligned to the pfam consensus sequence (SEQ ID No. 1) and modified according to a structural model of P10480 to ensure best fit overlap (see Figure 55) as taught

Described herein is a variant glycolipid acyltransferase enzyme wherein the variant enzyme comprises an amino acid sequence, which amino acid sequence is shown as SEQ ID No. 2, SEQ ID No. 3, SEQ ID No. 4, SEQ ID No. 5, SEQ ID No. 6, SEQ ID No. 12, SEQ ID No. 14, SEQ ID No. 16, SEQ ID No. 18, SEQ ID No. 20, SEQ ID No. 22, SEQ ID No. 24, SEQ ID No. 26, SEQ ID No. 28, SEQ ID No. 30, SEQ ID No. 33, SEQ ID No. 34, SEQ ID No. 36, SEQ ID No. 37, SEQ ID No. 39, SEQ ID No. 41, SEQ ID No. 43, or SEQ ID No. 45 except for one or more amino acid modifications at any one or more of the amino acid residues defined in set 2 or set 4 or set 6 or set 7 (hereinafter defined) identified by sequence alignment with SEQ ID No. 2.

Described herein is a variant glycolipid acyltransferase enzyme wherein the variant enzyme comprises an amino acid sequence, which amino acid sequence is shown as SEQ ID No. 2, SEQ ID No. 3, SEQ ID No. 4, SEQ ID No. 5, SEQ ID No. 6, SEQ ID No. 12, SEQ ID No. 14, SEQ ID No. 16, SEQ ID No. 18, SEQ ID No. 20, SEQ ID No. 22, SEQ ID No. 24, SEQ ID No. 26, SEQ ID No. 28, SEQ ID No. 30, SEQ ID No. 33, SEQ ID No. 34, SEQ ID No. 36, SEQ ID No. 37, SEQ ID No. 39, SEQ ID No. 41, SEQ ID No. 43 or SEQ ID No. 45 except for one or more amino acid modifications at any one or more of the amino acid residues defined in set 2 or set 4 or set 6 or set 7 identified by said parent sequence being structurally aligned with the structural model of P10480 defined herein, which is preferably obtained by structural alignment of P10480 crystal structure coordinates with 1IVN.PDB and/or 1DEO.PDB as taught herein.

Described herein is a variant glycolipid acyltransferase enzyme wherein the variant enzyme comprises an amino acid sequence, which amino acid sequence is shown as SEQ ID No. 2, SEQ ID No. 3, SEQ ID No. 4, SEQ ID No. 5, SEQ ID No. 6, SEQ ID No. 12, SEQ ID No. 14, SEQ ID No. 16, SEQ ID No. 18, SEQ ID No. 20, SEQ ID No. 22, SEQ ID No. 24, SEQ ID No. 26, SEQ ID No. 28, SEQ ID No. 30, SEQ ID No. 33, SEQ ID No. 34, SEQ ID No. 36, SEQ ID No. 37, SEQ ID No. 39, SEQ ID No. 41, SEQ ID No. 43 or SEQ ID No. 45 except for one or more amino acid modifications at any one or more of the amino acid residues taught in set 2 identified when said parent sequence is aligned to the pfam consensus sequence (SEQ ID No. 1) and modified according to a structural model of P10480 to ensure best fit overlap (see Figure 55) as taught Described herein is a variant glycolipolytic enzyme according to the present invention or obtained by a method described herein in the manufacture of a substrate (preferably a foodstuff) to prepare a lyso-glycolipid, for example digalactosyl monoglyceride (DGMG) or monogalactosyl monoglyceride (MGMG) by treatment of a glycolipid (e.g. digalactosyl diglyceride (DGDG) or monogalactosyl diglyceride (MGDG)) with the variant lipolytic enzyme described herein to produce the partial hydrolysis product, i.e. the lyso-glycolipid. Further described herein is use of a variant lipolytic enzyme described herein in the manufacture of a substrate (preferably a foodstuff) to prepare a lysophospholipid, for example lysolecithin, by treatment of a phospholipid (e.g. lecithin) with the variant lipolytic enzyme described herein to produce a partial hydrolysis product, i.e a lyso-phospholipid. Described herein is a method of preparing a foodstuff the method comprising adding a variant lipolytic enzyme described herein to one or more ingredients of the foodstuff.

Described herein is a method of preparing a baked product from a dough, the method comprising adding a variant lipolytic enzyme according to the present invention or obtained by a method according to the present invention to the dough.

Also described herein is the use of a variant lipolytic enzyme described herein in the manufacture of an egg-based product for producing lysophospholipids.

Described herein is a method of treating eggs or egg-based products comprising adding a variant lipolytic enzyme described herein to an egg or an egg-based product to produce a lysophospholipid.

The variants described herein may be used in a process of production of a snack food such as instant noodles in analogy with WO02/065854.

The present description relates to the use of the variant lipid acyltransferase described herein to results in a preferred technical effect or combination of technical effects in for example the foodstuff (such as those listed herein under 'Technical Effects').

Further described herein is a process of enzymatic degumming of vegetable or edible oils, comprising treating the edible or vegetable oil with a variant lipolytic enzyme described herein so as to hydrolyse a major part of the polar lipids (e.g. phospholipid and/or glycolipid).

Described herein is a process comprising treating a phospholipid so as to hydrolyse fatty acyl groups, which process comprising admixing said phospholipids with a variant lipolytic enzyme described herein.

Described herein is a process of reducing the content of a phospholipid in an edible oil, comprising treating the oil with a variant lipolytic enzyme described herein so as to hydrolyse a major part of the phospholipid, and separating an aqueous phase containing the hydrolysed phospholipid from the oil.

Described herein is a method of preparing a variant lipolytic enzyme described herein the method comprising transforming a host cell with a recombinant nucleic acid comprising a nucleotide sequence coding for said variant lipolytic enzyme, the host cell being capable of expressing the nucleotide sequence coding for the polypeptide of the lipolytic enzyme, cultivating the transformed host cell under conditions where the nucleic acid is expressed and harvesting the variant lipolytic enzyme. \The present description relates to the use of a variant lipolytic enzyme described herein in the bioconversion of polar lipids (preferably glycolipids) to make high value products, such as carbohydrate esters and/or protein esters and/or protein subunit esters and/or a hydroxy acid ester,

A method of bioconverting polar lipids (preferably glycolipids) to high value products, which method comprises admixing said polar lipid with a variant lipolytic enzyme is described herein which method comprises admixing said polar lipid with a variant lipolytic enzyme described herein.

The present description yet further relates to an immobilised variant lipolytic enzyme described herein,

Other aspects concerning the nucleotide sequences which can be used in the present description include: a construct comprising the sequences as described herein a vector comprising the sequences for use as described herein a plasmid comprising the sequences for use as described herein a transformed cell comprising the sequences for use as described herein a transformed tissue comprising the sequences for use as described herein a transformed organ comprising the sequences for use as described herein a transformed host comprising the sequences for use as described herein a transformed organism comprising the sequences for use as described herein. Also described herein are methods of expressing the nucleotide-sequence for use as described herein using the same, such as expression in a host cell; including methods for transferring same. The present description further encompasses methods of isolating the nucleotide sequence, such as isolating from a host cell.

Other aspects concerning the amino acid sequence for use as described herein include: a construct encoding the amino acid sequences for use as described herein ; a vector encoding the amino acid sequences for use as described herein a plasmid encoding the amino acid sequences for use as described herein a transformed cell expressing the amino acid sequences for use as described herein a transformed tissue expressing the amino acid sequences for use as described herein a transformed organ expressing the amino acid sequences for use as described herein a transformed host expressing the amino acid sequences for use as described herein a transformed organism expressing the amino acid sequences for use as described herein. The present description also encompasses methods of purifying the amino acid sequence for use as described herein using the same, such as expression in a host cell; including methods of transferring same, and then purifying said sequence.

For the ease of reference, these and further aspects described herein are now discussed under appropriate section headings. However, the teachings under each section are not necessarily limited to each particular section.

DEFINITION OF SETS

Amino acid set 1:

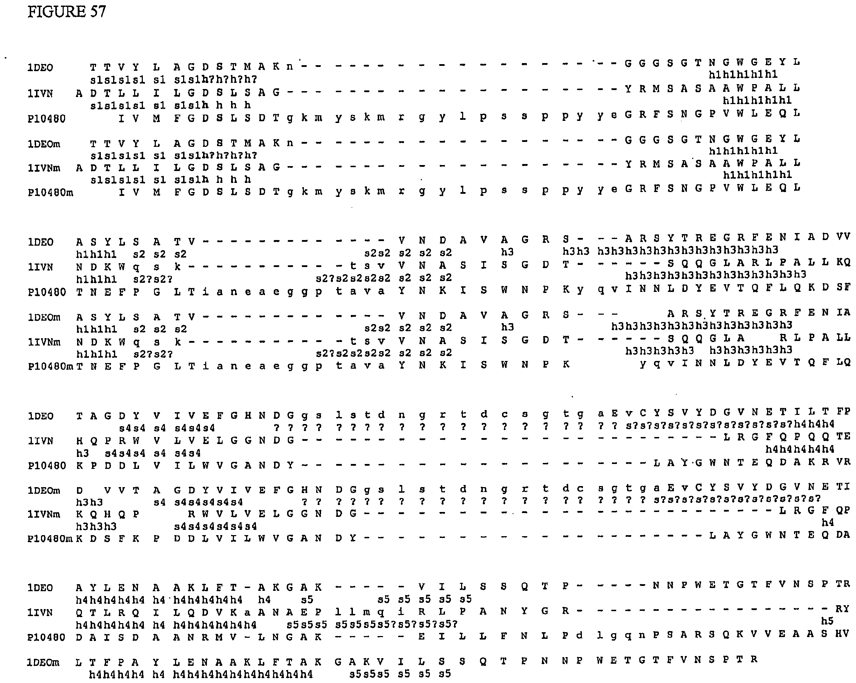

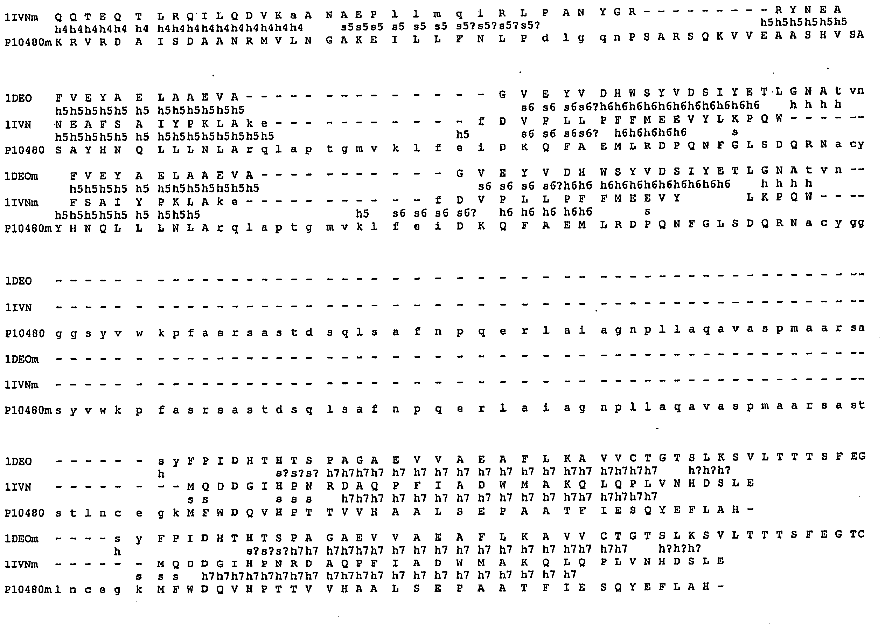

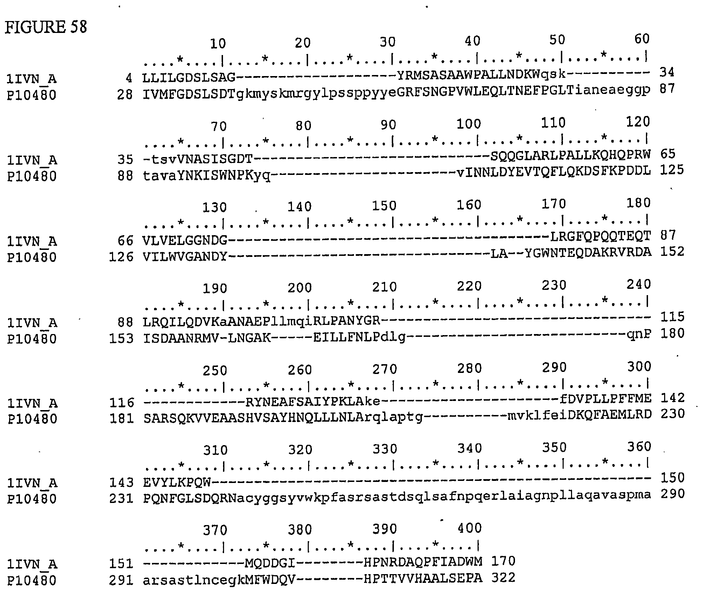

Amino acid set 1 (note that these are amino acids in 1IVN - Figure 57 and Figure 58.) Gly8, Asp9, Ser10, Leu11, Ser12, Tyrl5, Gly44, Asp45, Thr46, Glu69, Leu70, Gly71, Gly72, Asn73, Asp74, Gly75, Leu76, Gln106, Ile107, Arg108, Leu109, Pro110, Tyrll3, Phe121, Phel39, Phe140, Met141, Tyr145, Met151, Asp154, His157, Gly155, Ile156, Pro158

The highly conserved motifs, such as GDSx and catalytic residues, were deselected from set 1 (residues underlined). For the avoidance of doubt, set 1 defines the amino acid residues within 10Å of the central carbon atom of a glycerol in the active site of the 1IVN model.

Amino acid set 2:

Amino acid set 2 (note that the numbering of the amino acids refers to the amino acids in the P 10480 mature sequence) Leul7, Lys22, Met23, Gly40, Asn80, Pro81, Lys82, Asn87, Asn88, Trp111, Val112, Ala114, Tyr117, Leu118, Pro156, Gly159, Gln160, Asn161, Pro162, Ser163, Ala164, Arg165, Ser166, Gln167, Lys168, Val169, Val170, Glu171, Ala172, Tyr179, His180, Asn181, Met209, Leu210, Arg211, Asn215, Lys284, Met285, Gln289 and Val290.

Table of selected residues in Set 1 compared with Set 2:

| IVN model | | | P10480 Mature sequence Residue Number |
|---|---|---|---|
| IVN | A.hyd homologue | | |
| | PFAM | Structure | |
| Gly8 | Gly32 | | |
| Asp9 | Asp33 | | |
| Ser10 | Ser34 | | |
| Leu11 | Leu35 | | Leu17 |
| Ser12 | Ser36 | | Ser18 |
| | | | Lys22 |
| | | | Met23 |
| Tyr15 | Gly58 | | Gly40 |
| Gly44 | Asn98 | | Asn80 |
| Asp45 | Pro99 | | Pro81 |
| Thr46 | Lys100 | | Lys82 |
| | | | Asn87 |
| | | | Asn88 |
| Glu69 | Trp129 | | Trp111 |
| Leu70 | Val130 | | Val112 |
| Gly71 | Gly131 | | |
| Gly72 | Ala132 | | Ala114 |
| Asn73 | Asn133 | | |
| Asp74 | Asp134 | | |
| Gly75 | Tyr135 | | Tyr117 |
| Leu76 | Leu136 | | Leu118 |
| Gln106 | | Pro174 | Pro156 |
| Ile107 | | Gly177 | Gly159 |

(continued)

| IVN model | | | P10480 Mature sequence Residue Number |
|---|---|---|---|
| IVN | A.hyd homologue | | |
| | PFAM | Structure | |
| Arg108<br>Leu109<br>Pro110<br>Tyr113 | | Gln178<br>Asn179<br>180 to 190 | Gln160<br>Asn161<br>Pro162<br>Ser163<br>Ala164<br>Arg165<br>Ser166<br>Gln167<br>Lys168 |
| Phe121<br>Phe139<br>Phe140<br>Met141<br>Tyr145<br>Met151 Asp154<br>Gly155<br>Ile156<br>His157<br>Pro158 | His198<br>Met227<br>Leu228<br>Arg229<br>Asn233<br>Met303 Asp306<br>Gln307<br>Val308<br>His309<br>Pro310 | Tyr197<br>His198<br>Asn199 | Val169<br>Val170<br>Glu171<br>Ala172<br>Tyr179<br>His180<br>Asn181<br>Met209<br>Leu210<br>Arg211<br>Asn215<br>Lys284<br>Met285<br>Gln289<br>Val290 |

Amino acid set 3:

Amino acid set 3 is identical to set 2 but refers to the *Aeromonas salmonicida* (SEQ ID No. 28) coding sequence, i.e. the amino acid residue numbers are 18 higher in set 3 as this reflects the difference between the amino acid numbering in the mature protein (SEQ ID No. 2) compared with the protein including a signal sequence (SEQ ID No. 28).

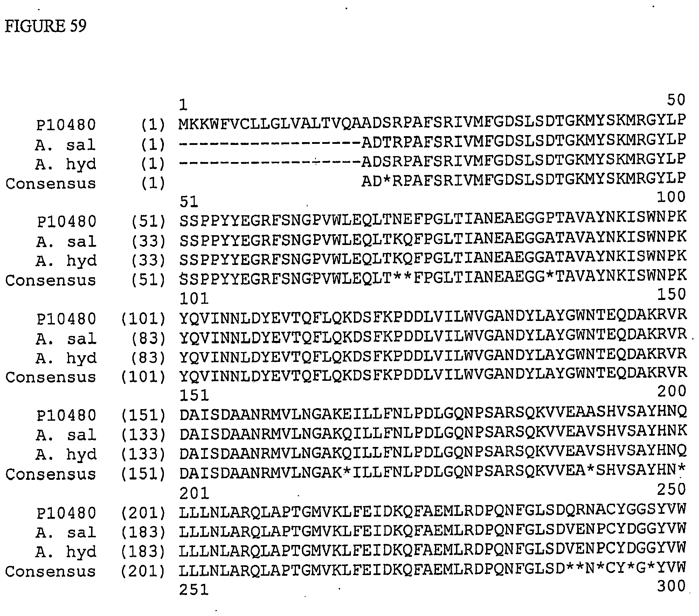

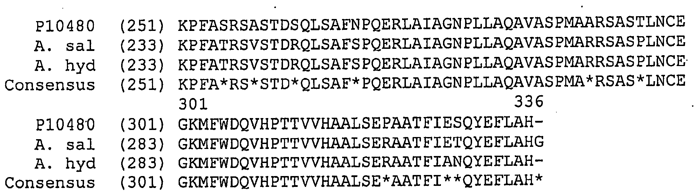

The mature proteins of *Aeromonas salmonicida* GDSX (SEQ ID No. 28) and *Aeromonas hydrophila* GDSX (SEQ ID No. 26) differ in five amino acids. These are Thr3Ser, Gln182Lys, Glu309Ala, Ser310Asn, Gly318-, where the *salmonicida* residue is listed first and the *hydrophila* residue is listed last (FIGURE 59). The *hydrophila* protein is only 317 amino acids long and lacks a residue in position 318. The *Aeromonas salmonicidae* GDSX has considerably high activity on polar lipids such as galactolipid substrates than the *Aeromonas hydrophila* protein. Site scanning was performed on all five amino acid positions.

Amino acid set 4:

Amino acid set 4 is S3, Q182, E309, S310, and -318.

Amino acid set 5:

F13S, D15N, S18G, S18V, Y30F, D116N, D116E, D157N, Y226F, D228N Y230F.

Amino acid set 6:

Amino acid set 6 is Ser3, Leul7, Lys22, Met23, Gly40, Asn80, Pro81, Lys82, Asn 87, Asn88, Trp111, Va1112, Ala114, Tyr117, Leu118, Pro156, Gly159, Gln160, Asn161, Pro162, Ser163, Ala164, Arg165, Ser166, Gln167, Lys168, Val169, Val170, Glu171, Ala172, Tyr179, His180, Asn181, Gln182, Met209, Leu210, Arg211, Asn215, Lys284, Met285, Gln289, Val290, Glu309, Ser310, -318.

The numbering of the amino acids in set 6 refers to the amino acids residues in P10480 (SEQ ID No. 2) - corresponding amino acids in other sequence backbones can be determined by homology alignment and/or structural alignment to P10480 and/or 1IVN.

Amino acid set 7:

Amino acid set 7 is Ser3, Leu17, Lys22, Met23, Gly40, Asn80, Pro81, Lys82, Asn 87, Asn88, Trp111, Val112, Ala114, Tyr117, Leu118, Pro156, Gly159, Gln160, Asn161, Pro162, Ser163, Ala164, Arg165, Ser166, Gln167, Lys168, Val169, Val170, Glu171, Ala172, Tyr179, His180, Asn181, Gln182, Met209, Leu210, Arg211, Asn215, Lys284, Met285, Gln289, Val290, Glu309, Ser310, -318, Y30X (where X is selected from A, C, D, E, G, H, I, K, L, M, N, P, Q, R, S, T, V, or W), Y226X (where X is selected from A, C, D, E, G, H, I, K, L, M, N, P, Q, R, S, T, V, or W), Y230X (where X is selected from A, C, D, E, G, H, I, K, L, M, N, P, Q, R, S, T, V, or W), S18X (where X is selected from A, C, D, E, F, H, I, K, L, M, N, P, Q, R, T, W or Y), D157X (where X is selected from A, C, E, F, G, H, I, K, L, M, P, Q, R, S, T, V, W or Y). The numbering of the amino acids in set 7 refers to the amino acids residues in P10480 (SEQ ID No. 2) - corresponding amino acids in other sequence backbones can be determined by homology alignment and/or structural alignment to P10480 and/or 1IVN).

DETAILED ASPECTS

Preferably, the parent lipid acyltransferase enzyme comprises any one of the following amino acid sequences: SEQ ID No. 2, SEQ ID No. 3, SEQ ID No. 4, SEQ ID No. 5, SEQ ID No. 6, SEQ ID No. 12, SEQ ID No. 14, SEQ ID No. 16, SEQ ID No. 18, SEQ ID No. 20, SEQ ID No. 22, SEQ ID No. 24, SEQ ID No. 26, SEQ ID No. 28, SEQ ID No. 30, SEQ ID No. 33, SEQ ID No. 34, SEQ ID No. 36, SEQ ID No. 37, SEQ ID No. 39, SEQ ID No. 41, SEQ ID No. 43 or SEQ ID No. 45 or an amino acid sequence which has 75% or more identity with any one of the sequences shown as SEQ ID No. 2, SEQ ID No. 3, SEQ ID No. 4, SEQ ID No. 5, SEQ ID No. 6, SEQ ID No. 12, SEQ ID No. 14, SEQ ID No. 16, SEQ ID No. 18, SEQ ID No. 20, SEQ ID No. 22, SEQ ID No. 24, SEQ ID No. 26, SEQ ID No. 28, SEQ ID No. 30, SEQ ID No. 33, SEQ ID No. 34, SEQ ID No. 36, SEQ ID No. 37, SEQ ID No. 39, SEQ ID No. 41, SEQ ID No. 43 or SEQ ID No. 45.

Suitably, the parent lipid acyltransferase enzyme comprises an amino acid sequence which has at least 80%, preferably at least 85%, more preferably at least 90%, more preferably at least 95%, more at least 98% homology with any one of the sequences shown as SEQ ID No. 2, SEQ ID No. 3, SEQ ID No. 4, SEQ ID No. 5, SEQ ID No. 6, SEQ ID No. 12, SEQ ID No. 14, SEQ ID No. 16, SEQ ID No. 18, SEQ ID No. 20, SEQ ID No. 22, SEQ ID No. 24, SEQ ID No. 26, SEQ ID No. 28, SEQ ID No. 30, SEQ ID No. 33, SEQ ID No. 34, SEQ ID No. 36, SEQ ID No. 37, SEQ ID No. 39, SEQ ID No. 41, SEQ ID No. 43 or SEQ ID No. 45.

Suitably, the parent lipid acyltransferase enzyme may be encoded by any one of the following nucleotide sequences: SEQ ID No. 7, SEQ ID No. 8, SEQ ID No. 9, SEQ ID No. 10, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No.27, SEQ ID No. 29, SEQ ID No. 31, SEQ ID No. 32, SEQ ID No. 35, SEQ ID No. 38, SEQ ID No. 40, SEQ ID No. 42, SEQ ID No. 44 or SEQ ID No. 46 or a nucleotide sequence which has at least 75% or more identity with any one of the sequences shown as SEQ ID No. 7, SEQ ID No. 8, SEQ ID No. 9, SEQ ID No. 10, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No.27, SEQ ID No. 29, SEQ ID No. 31, SEQ ID No. 32, SEQ ID No. 35, SEQ ID No. 38, SEQ ID No. 40, SEQ ID No. 42, SEQ ID No. 44 or SEQ ID No. 46.

Suitably, the nucleotide sequence may have 80% or more, preferably 90% or more, more preferably 95% or more, even more preferably 98% or more identity with any one of the sequences shown as SEQ ID No. 7, SEQ ID No. 8, SEQ ID No. 9, SEQ ID No. 10, SEQ ID No. 11, SEQ ID No. 13, SEQ ID No. 15, SEQ ID No. 17, SEQ ID No. 19, SEQ ID No. 21, SEQ ID No. 23, SEQ ID No. 25, SEQ ID No.27, SEQ ID No. 29, SEQ ID No. 31, SEQ ID No. 32, SEQ ID No. 35, SEQ ID No. 38, SEQ ID No. 40, SEQ ID No. 42, SEQ ID No. 44 or SEQ ID No. 46.

Preferably, the parent enzyme is modified at one or more of the amino acid residues defined in set 2 or set 4 or set 6 or set 7 when aligned to the reference sequence (SEQ ID No. 2) or structurally aligned to the structural model of P10480, or aligned to the pfam consensus sequence and modified according to the structural model of P10480,

Suitably the variant enzyme may have an enhanced ratio of activity on galactolipids compared with the activity on either phospholipids and/or triglycerides when compared with the parent enzyme.

Suitably, the method described herein may comprise testing the variant lipid acyltransferase for:

(i) transferase activity from a galactolipid substrate, and
(ii) transferase activity from a phospholipids substrate; and

selecting a variant enzyme, which when compared with the parent enzyme, has an enhanced ratio of transferase activity from galactolipids compared with phospholipids.

Suitably, the ratio of transferase activity from galactolipids compared with phospholipids of the variant enzyme described herein may be at least 1, at least 2, at least 3, at least 4 or at least 5.

Suitably, the method described herein may comprise testing the variant lipid acyltransferase for:

(a) transferase activity from a galactolipid substrate, and
(b) hydrolytic activity on a galactolipid substrate; and

selecting a variant enzyme with an enhanced ratio of transferase activity from galactolipids compared with its hydrolytic activity on glycolipids, compared with the parent enzyme.

Suitably, the ratio of transferase activity on galactolipids compared to hydrolytic activity on galactolipids may be great than 1, at least 1.5, at least 2, at least 4 or at least 5.

An assay for determining the transferase and hydrolytic activities from galactolipids and/or phospholipids is/are taught in Example 8 for example.

The term "enhanced activity towards galactolipids" means the enzyme has an enhanced (i.e. higher) transferase activity when the lipid acyl donor is a galactolipid compared with the parent enzyme (galactolipid transferase activity) and/or has an increased ratio of galactolipid transferase activity when compared with phospholipids transferase activity compared with the parent enzyme (GLt:PLt ratio) and/or has an increased ratio of galactolipid transferase activity when compared with galactolipid hydrolysis activity compared with the parent enzyme (GLt:GLh ratio).

Suitably, the variant enzyme compared with the parent enzyme may have an increased galactolipid transferase activity and either the same or less galactolipid hydrolytic activity. In other words, suitably the variant enzyme may have a higher galactolipid transferase activity compared with its galactolipid hydrolytic activity compared with the parent enzyme. Suitably, the variant enzyme may preferentially transfer an acyl group from a galactolipid to an acyl acceptor rather than simply hydrolysing the galactolipid.

The enzyme described herein may have an increased transferase activity towards phospholipids (i.e an increased phospholipid transferase activity) as compared with the parent enzyme. This increased phospholipid transferase activity may be independent of the enhanced activity towards galactolipids. Suitably, however, the variant enzyme may have an increased galactolipid transferase activity and an increased phospholipid transferase activity.

Described herein is a variant lipid acyltransferase enzyme characterised in that the enzyme comprises the amino acid sequence motif GDSX, wherein X is one or more of the following amino acid residues L, A, V, I, F, Y, H, Q, T, N, M or S, wherein the variant has an enhanced activity towards phospholipids, preferably enhanced phospholipid transferase activity, compared with the parent enzyme and wherein the variant enzyme comprises one or more amino acid modifications compared with a parent sequence at any one or more of the amino acid residues defined in set 2 or set 4 or set 6 or set 7.

The term "modifying" as used herein means adding, substituting and/or deleting. Preferably the term "modifying" means "substituting".

For the avoidance of doubt, when an amino acid is substituted in the parent enzyme it is preferably substituted with an amino acid which is different from that originally found at that position in the parent enzyme thus to produce a variant enzyme. In other words, the term "substitution" is not intended to cover the replacement of an amino acid with the same amino acid.

Preferably, the parent enzyme is an enzyme which comprises the amino acid sequence shown as SEQ ID No. 2 and/or SEQ ID No. 28.

Preferably, the variant enzyme is an enzyme which comprises an amino acid sequence, which amino acid sequence is shown as SEQ ID No. 2 except for one or more amino acid modifications at any one or more of the amino acid residues defined in set 2 or set 4 or set 6 or set 7.

In one embodiment, preferably the variant enzyme comprises one or more amino acid modifications compared with the parent sequence at at least one of the amino acid residues defined in set 4.

Suitably, the variant enzyme comprises one or more of the following amino acid modifications compared with the parent enzyme:

S3E,A,G,K,M, Y,R,P,N, T or G
E309Q, R or A, preferably Q or R
-318Y, H, S or Y, preferably Y.

Preferably, X of the GDSX motif is L. Thus, preferably the parent enzyme comprises the amino acid motif GDSL.

Preferably the method of producing a variant lipid acyltransferase enzyme further comprises one or more of the following steps:

1) structural homology mapping or
2) sequence homology alignment

Suitably, the structural homology mapping may comprise one or more of the following steps:

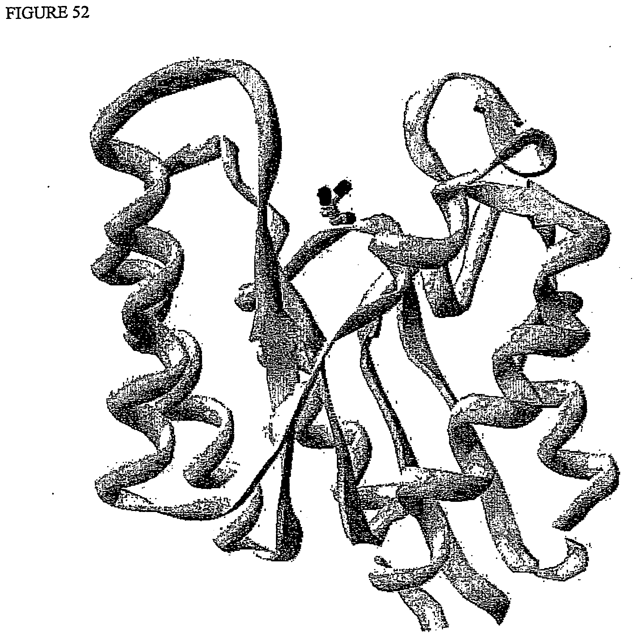

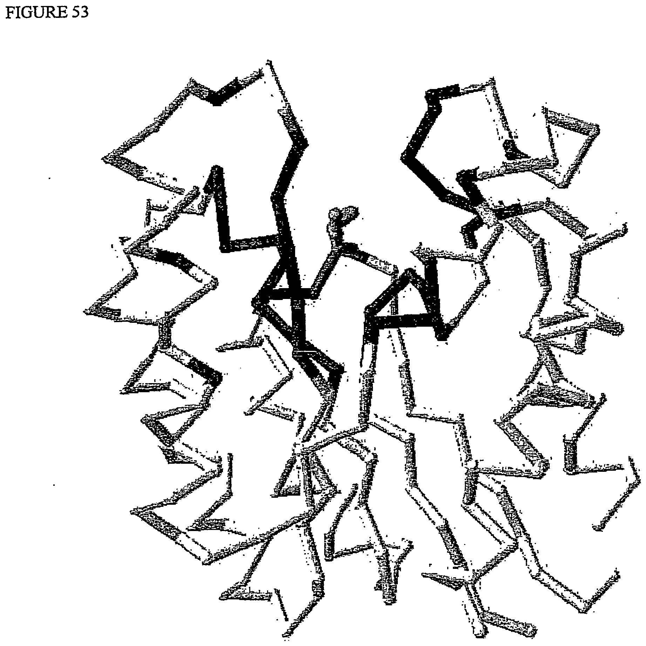

i) aligning a parent sequence with a structural model (1IVN.PDB) shown in Figure 52;
ii) selecting one or more amino acid residue within a 10Å sphere centred on the central carbon atom of the glycerol molecule in the active site (see Figure 53) (such as one or more of the amino acid residues defined in set 1 or set 2); and
iii) modifying one or more amino acids selected in accordance with step (ii) in said parent sequence.

The amino acid residue selected may reside within a 9, preferably within a 8, 7, 6, 5, 4, or 3 Å sphere centred on the central carbon atom of the glycerol molecule in the active site (see Figure 53).

Suitably, the structural homology mapping may comprise one or more of the following steps:

i) aligning a parent sequence with a structural model (1IVN.PDB) shown in Figure 52;
ii) selecting one or more amino acids within a 10Å sphere centred on the central carbon atom of the glycerol molecule in the active site (see Figure 53) (such as one or more of the amino acid residues defined in set 1 or set 2);
iii) determining if one or more amino acid residues selected in accordance with step (ii) are highly conserved (particularly are active site residues and/or part of the GDSx motif and/or part of the GANDY motif); and
iv) modifying one or more amino acids selected in accordance with step (ii), excluding conserved regions identified in accordance with step (iii) in said parent sequence.

The amino acid residue selected may reside within a 9, preferably within a 8, 7, 6, 5, 4, or 3 Å sphere centred on the central carbon atom of the glycerol molecule in the active site (see Figure 53).

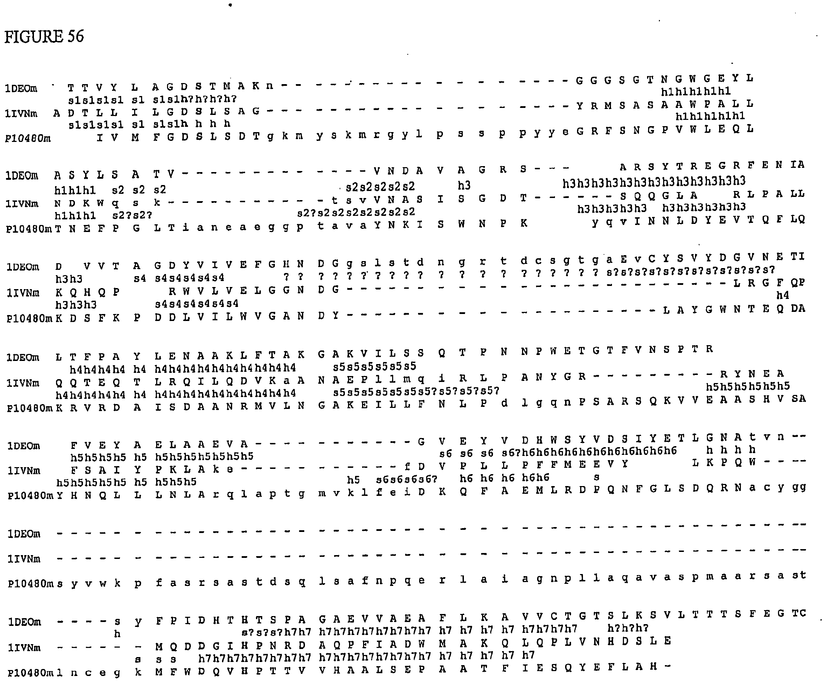

Alternatively to, or in combination with, the structural homology mapping described above, the structureal homology mapping can be performed by selecting specific loop regions (LRs) or intervening regions (IVRs) derived from the pfam alignment (Alignment 2, Figure 56) overlayed with the P10480 model and 1IVN. The loop regions (LRs) or intervening regions (IVRs) are defined in the Table below:

| | P10480 amino acid positions (SEQ ID No 2) |
|---|---|
| IVR1 | 1-19 |
| Loop1 (LR1) | 20-41 |
| IVR2 | 42-76 |
| Loop2 (LR2) | 77-89 |
| IVR3 | 90-117 |
| Loop3 (LR3) | 118-127 |
| IVR4 | 128-145 |
| Loop4 (LR4) | 146-176 |
| IVR5 | 177-207 |
| Loop5 (LR5) | 208-287 |
| IVR6 | 288-317 |

The variant acyltransferase enzyme may not only comprises an amino acid modifications at one or more of the amino acids defined in any one of sets 1-4 and 6-7, but may also comprises at least one amino acid modification in one or more of the above defined intervening regions (IVR1-6) (preferably in one or more of the rVRs 3, 5 and 6, more preferably in IVR 5 or IVR 6) and/or in one or more of the above-defined loop regions (LR1-5) (preferably in one or more of LR1, LR2 or LR5, more preferably in LR5).

The variant acyltransferase described herein or obtained by a method described herein may comprise one or more amino acid modification which is not only defined by one or more of set 2, 4, 6 and 7, but also is within one or more of the IVRs 1-6 (preferably within IVR 3, 5 or 6, more preferably within in IVR 5 or IVR 6) or within one or more of the LRs 1-5 (preferably within LR1, LR2 or LR5, more preferably within LR5). Suitably, the variant acyltransferase described herein may comprise one or more amino acid modification which is not only in set 1 or 2, but also is within IVR 3. Suitably, the variant acyltransferase described herein may comprise one or more amino acid modification which is not only in set 1 or 2, but also is within IVR 5, Suitably, the variant acyltransferase described herein may comprise one or more amino acid modification which is not only in set 1 or 2, but also is within IVR 6. Suitably, the variant acyltransferase described herein may comprise one or more amino acid modification which is not only in set 1 or 2, but also is within LR1. Suitably, the variant acyltransferase described herein may comprise one or more amino acid modification which is not only in set 1 or 2, but also is within LR 2.

Likewise, the variant acyltransferse enzyme may not only comprises an amino acid modification at one or more amino acid residues which reside within a 10, preferably within a 9, 8, 7, 6, 5, 4, or 3, Å sphere centred on the central carbon atom of the glycerol molecule in the active site (see Figure 53), but may also comprises at least one amino acid modification in one or more of the above defined intervening regions (TVR1-6) (preferably in one or more of IVRs 3, 5 and 6, more preferably in IVR 5 or IVR 6) and/or in one or more of the above-defined loop regions (LR1-5) (preferably in one or more of LR1, LR2 or LR5, more preferably in LR5).
Preferably the amino acid modification is at one or more amino acid residues which reside within a 10Å sphere and also within LR5.

Thus, the structural homology mapping may comprise one or more of the following steps:

i) aligning a parent sequence with a structural model (1IVN.PDB) shown in Figure 52;
ii) selecting one or more amino acid residue within a 10Å sphere centred on the central carbon atom of the glycerol molecule in the active site (see Figure 53) (such as one or more of the amino acid residues defined in set 1 or set 2); and/or selecting one or more amino acid residues within IVR1-6) (preferably within IVR 3, 5 or 6, more preferably within in IVR 5 or IVR 6); and/or selecting one or more amino acid residues within LR1-5 (preferably within LR1, LR2 or LR5, more preferably within LR5); and
iii) modifying one or more amino acids selected in accordance with step (ii) in said parent sequence.

The amino acid residue selected may reside within a 9, preferably within a 8, 7, 6, 5, 4, or 3 Å sphere centred on the central carbon atom of the glycerol molecule in the active site (see Figure 53).

Suitably, the structural homology mapping may comprise one or more of the following steps:

i) aligning a parent sequence with a structural model (1IVN.PDB) shown in Figure 52;
ii) selecting one or more amino acids within a 10Å sphere centred on the central carbon atom of the glycerol molecule in the active site (see Figure 53) (such as one or more of the amino acid residues defined in set 1 or set 2); and/or selecting one or more amino acid residues within IVR1-6) (preferably within IVR 3, 5 or 6, more preferably within in IVR 5 or IVR 6); and/or selecting one or more amino acid residues within LR1-5 (preferably within LR1, LR2 or LR5, more preferably within LR5);
iii) determining if one or more amino acid residues selected in accordance with step (ii) are highly conserved (particularly are active site residues and/or part of the GDSx motif and/or part of the GANDY motif); and

modifying one or more amino acids selected in accordance with step (ii), excluding conserved regions identified in accordance with step (iii) in said parent sequence.

Suitably, the one or more amino acids selected in the methods detailed above are not only within a 10Å sphere centred on the central carbon, atom of the glycerol molecule in the active site (see Figure 53) (such as one or more of the amino acid residues defined in set 1 or set 2), but are also within one or more of the IVRs 1-6 (preferably within IVR 3, 5 or 6, more preferably within in IVR 5 or IVR 6) or within one or more of the LRs 1-5 (preferably within LR1, LR2 or LR5, more preferably within LR5),

Preferably the one or more amino acid modifications is/are within LR5. When it is the case that the modifications) is within LR5, the modification is not one which is defined in set 5. Suitably, the one or more amino acid modifications not only fall with the region defined by LR5, but also constitute an amino acid within one or more of set 2, set 4, set 6 or set 7.

Suitably, the sequence homology alignment may comprise one or more of the following steps:

i) selecting a first parent lipid acyltransferase;
ii) identifying a second related lipid acyltransferase having a desirable activity;
iii) aligning said first parent lipid acyltransferase and the second related lipid acyltransferase;
iv) identifying amino acid residues that differ between the two sequences; and
v) modifying one or more of the amino acid residues identified in accordance with step (iv) in said parent lipid acyltransferase.

Suitably, the sequence homology alignment may comprise one or more of the following steps:

i) selecting a first parent lipid acyltransferase;
ii) identifying a second related lipid acyltransferase having a desirable activity;
iii) aligning said first parent lipid acyltransferase and the second related lipid acyltransferase;
iv) identifying amino acid residues that differ between the two sequences;
v) determining if one or more amino acid residues selected in accordance with step (iv) are highly conserved (particularly are active site residues and/or part of the GDSx motif and/or part of the GANDY motif); and
vi) modifying one or more of the amino acid residues identified in accordance with step (iv) excluding conserved regions identified in accordance with step (v) in said parent sequence.

Suitably, said first parent lipid acyltransferase may comprise any one of the following amino acid sequences: SEQ ID No. 2, SEQ ID No. 3, SEQ ID No. 4, SEQ ID No. 5, SEQ ID No. 6, SEQ ID No. 12, SEQ ID No. 14, SEQ ID No. 16, SEQ ID No. 18, SEQ ID No. 20, SEQ ID No. 22, SEQ ID No. 24, SEQ ID No. 26, SEQ ID No. 28, SEQ ID No. 30, SEQ ID No. 33, SEQ ID No. 34, SEQ ID No. 36, SEQ ID No. 37, SEQ ID No. 39, SEQ ID No. 41, SEQ ID No. 43 or SEQ ID No. 45.

Suitably, said second related lipid acyltransferase may comprise any one of the following amino acid sequences: SEQ ID No. 2, SEQ ID No. 3, SEQ ID No. 4, SEQ ID No. 5, SEQ ID No. 6, SEQ ID No. 12, SEQ ID No. 14, SEQ ID No. 16, SEQ ID No. 18, SEQ ID No. 20, SEQ ID No. 22, SEQ ID No. 24, SEQ ID No. 26, SEQ ID No. 28, SEQ ID No. 30, SEQ ID No. 33, SEQ ID No. 34, SEQ ID No. 36, SEQ ID No. 37, SEQ ID No. 39, SEQ ID No. 41, SEQ ID No. 43 or SEQ ID No. 45.

The variant enzyme must comprise at least one amino acid modification compared with the parent enzyme. In some embodiments, the variant enzyme may comprise at least 2, preferably at least 3, preferably at least 4, preferably at least 5, preferably at least 6, preferably at least 7, preferably at least 8, preferably at least 9, preferably at least 10 amino acid modifications compared with the parent enzyme.

Suitably the methods described herein may comprise a further step of formulating the variant enzyme into an enzyme composition and/or a foodstuff composition, such as a bread improving composition.

In order to align a GDSx polypeptide sequence (parent sequence) with SEQ ID No. 2 (P01480), sequence alignment such as pairwise alignment can be used (http://www.ebi.ac.uk/emboss/align/index.html). Thereby, the equivalent amino acids in alternative parental GDSx polypeptides, which correspond to one or more of the amino acids defined in set 2 or set 4 or set 6 or set 7 in respect of SEQ ID No. 2 can be determined and modified. As the skilled person will readily appreciate, when using the emboss pairwise alignment, standard settings usually suffice. Corresponding residues can be identified using "needle" in order to make an alignment that covers the whole length of both sequences. However, it is also possible to find the best region of similarity between two sequences, using "water".

Alternatively, particularly in instances where parent GDSx polypeptides share low homology with SEQ ID No. 2, the corresponding amino acids in alternative parental GDSx polypeptides which correspond to one or more of the amino acids defined in set 2, set 4, set 6 or set 7 in respect of SEQ ID No. 2 can be determined by structural alignment to the structural model of P10480, obtained by comparison of P10480 derived structural model with the structural coordinates of 1IVN.PDB and 1DEO.PDB using the 'Deep View Swiss-PDB viewer' (obtained from www.expasy.org/spdbv/) (Figure 53 and Example 1). Equivalent residues are identified as those overlapping or in closest proximity to the residues in the obtained structural model of P010480, as illustrated in the Table comparing Set 1 and Set 2 (see section entitled "Definition of Sets" hereinabove). In this way other GDSX polypeptides can be compared against the 1IVN.PBD crystal co-ordinates, and equivalent residues to Set 1 determined.

Alternatively, particularly in instances where a parent GDSx polypeptide shares a low homology with SEQ ID No. 2, the equivalent amino acids in alternative parental GDSx polypeptides, which correspond to one or more of the amino acids defined in set 2 or set 4 or set 6 or set 7 in respect of SEQ ID No. 2 can be determined from an alignment obtained from the PFAM database (PFAM consensus) modified based on the structural alignment as shown in Alignment 1 (Figure 55). The modification based on the structural models may be necessary to slightly shift the alignment in order to ensure a best fit overlap. Alignment 1 (Figure 55) provides guidance in this regard.

The variant enzyme described herein preferably does not comprise one or more of the amino acid modifications defined in set 5.

Suitably the variant enzyme may be prepared using site directed mutagenesis.

Alternatively, one can introduce mutations randomly for instance using a commercial kit such as the GeneMorph PCR mutagenesis kit from Stratagene, or the Diversify PCR random mutagenesis kit from Clontech. EP 0 583 265 refers to methods of optimising PCR based mutagenesis, which can also be combined with the use of mutagenic DNA analogues such as those described in EP 0 866 796. Error prone PCR technologies are suitable for the production of variants of lipid acyl transferases with preferred characteristics. WO0206457 refers to molecular evolution of lipases.

A third method to obtain novel sequences is to fragment non-identical nucleotide sequences, either by using any number of restriction enzymes or an enzyme such as Dnase I, and reassembling full nucleotide sequences coding for functional proteins (hereinafter referred to as "shuffling"). Alternatively one can use one or multiple non-identical nucleotide sequences and introduce mutations during the reassembly of the full nucleotide sequence. DNA shuffling and family shuffling technologies are suitable for the production of variants of lipid acyl transferases with preferred characteristics. Suitable methods for performing 'shuffling' can be found in EP0 752 008, EP1 138 763, EP1 103 606. Shuffling can also be combined with other forms of DNA mutagenesis as described in US 6,180,406 and WO 01/34835.

Thus, it is possible to produce numerous site directed or random mutations into a nucleotide sequence, either *in vivo* or *in vitro,* and to subsequently screen for improved functionality of the encoded variant polypeptide by various means.

As a non-limiting example, In addition, mutations or natural variants of a polynucleotide sequence can be recombined with either the wild type or other mutations or natural variants to produce new variants. Such new variants can also be screened for improved functionality of the encoded polypeptide.

The following regions may preferably be selected for localised random mutagenesis and/or shuffling: IVR3, IVR 5, IVR 6, LR1, LR2, and/or LR5, most preferably LR5.

For the production of libraries of variants microbial eukaryotic or prokaryotic expression hosts may be used. In order to ensure uniform expression within a library of variants, low copy number, preferably single event chromosomal expression systems may be preferred. Expression systems with high transformation frequencies are also preferred, particularly for the expression of large variant libraries (>1000 colonies), such as those prepared using random mutagenesis and/or shuffling technologies.

Suitable methods for the use of a eukaryotic expression host, namely yeast, in the production of enzymes are described in EP1131416. Microbial eukaryotic expression hosts, such as yeast, may be preferred for the expression of variant libraries produced using a eukaryotic acyltransferase parent gene.

Suitable methods using *Bacillus,* i.e. *Bacillus subtilis,* as an expression host in the production of enzymes are described in WO02/14490. Microbial prokaryotic expression hosts, such as *Bacillus,* may be preferred for the expression of variant libraries produced using a prokaryotic acyltransferase parent gene, for example the P10480 reference sequence (SEQ ID No 2).

Suitably, the variant lipid acyltransferase described herein retains at least 70%, preferably at least 80%, preferably at least 90%, preferably at least 95%, preferably at least 97%, preferably at least 99% homology with the parent enzyme.

Suitable parent enzymes may include any enzyme with esterase or lipase activity.

Preferably, the parent enzyme aligns to the pfam00657 consensus sequence.

A variant lipid acyltransferase enzyme may retain or incorporates at least one or more of the pfam00657 consensus sequence amino acid residues found in the GDSx, GANDY and HPT blocks.

Enzymes, such as lipases with no or low lipid acyltransferase activity in an aqueous environment may be mutated using molecular evolution tools to introduce or enhance the transferase activity, thereby producing a variant lipid acyltransferase enzyme with significant transferase activity suitable for use in the compositions and methods of the present invention.

Suitably, the lipid acyltransferase for use as described herein may be a variant with enhanced enzyme activity on polar lipids, preferably glycolipids, when compared to the parent enzyme. Preferably, such variants also have low or no activity on lyso polar lipids. The enhanced activity on polar lipids, preferably glycolipids may be the result of hydrolysis and/or transferase activity or a combination of both.

Variant lipid acyltransferases for use as described herein may have decreased activity on triglycerides, and/or monoglycerides and/or diglycerides compared with the parent enzyme.

Suitably the variant enzyme may have no activity on triglycerides and/or monoglycerides and/or diglycerides. Low activity on triglycerides is preferred in variant enzymes which are to be used for bakery applications, for treatment of egg or egg-based products and/or for degumming oils.

Suitably the variant enzyme may have a high activity on diglycerdies and no or low activity on triglycerides.

When referring to specific amino acid residues herein the numbering is that obtained from alignment of the variant sequence with the reference sequence shown as SEQ ID No. 2.

In one aspect preferably the variant enzyme comprises one or more of the following amino acid substitutions:

S3A, C, D, E, F, G, H, I, K, L, M, N, P, Q, R, T, V, W, or Y; and/or
L17A, C, D, E, F, G, H, I, K, M, N, P, Q, R, S, T, V, W, or Y; and/or
S18A, C, D, E, F, H, I, K, L, M, N, P, Q, R, T, W, or Y; and/or
K22A, C, D, E, F, G, H, I, L, M, N, P, Q, R, S, T, V, W, or Y; and/or
M23A, C, D, E, F, G, H, I, K, L, N, P, Q, R, S, T, V, W, or Y; and/or

Y30A, C, D, E, G, H, I, K, L, M, N, P, Q, R, S, T, V, or W; and/or
G40A, C, D, E, F, H, I, K, L, M, N, P, Q, R, S, T, V, W, or Y; and/or
N80A, C, D, E, F, G, H, I, K, L, M, P, Q, R, S, T, V, W, or Y; and/or
P81A, C, D, E, F, G, H, I, K, L, M, N, Q, R, S, T, V, W, or Y; and/or
K82A, C, D, E, F, G, H, I, L, M, N, P, Q, R, S, T, V, W, or Y; and/or
N87A, C, D, E, F, G, H, I, K, L, M, P, Q, R, S, T, V, W, or Y; and/or
N88A, C, D, E, F, G, H, I, K, L, M, P, Q, R, S, T, V, W, or Y; and/or
W111A, C, D, E, F, G, H, I, K, L, M, N, P, Q, R, S, T, V, W or Y; and/or
V112A, C, D, E, F, G, H, I, K, L, M, N, P, Q, R, S, T, W, or Y; and/or
A114C, D, E, F, G, H, I, K, L, M, N, P, Q, R, S, T, V, W, or Y; and/or
Y117A, C, D, E, F, G, H, I, K, L, M, N, P, Q, R, S, T, V, or W; and/or
L118A, C, D, E, F, G, H, I, K, M, N, P, Q, R, S, T, V, W, or Y; and/or
P156A, C, D, E, F, G, H, I, K, L, M, N, Q, R, S, T, V, W, or Y; and/or
D157A, C, E, F, G, H, I, K, L, M, P, Q, R, S, T, V, W, or Y; and/or
G159A, C, D, E, F, H, I, K, L, M, N, P, Q, R, S, T, V, W, or Y; and/or
Q160A, C, D, E, F, G, H, I, K, L, M, N, P, R, S, T, V, W, or Y; and/or
N161A, C, D, E, F, G, H, I, K, L, M P, Q, R, S, T, V, W, or Y; and/or
P162A, C, D, E, F, G, H, I, K, L, M, N, Q, R, S, T, V, W, or Y; and/or
S163A, C, D, E, F, G, H, I, K, L, M, N, P, Q, R, T, V, W, or Y; and/or
A164C, D, E, F, G, H, I, K, L, M, N, P, Q, R, S, T, V, W, or Y; and/or
R165A, C, D, E, F, G, H, I, K, L, M, N, P, Q, S, T, V, W, or Y; and/or
S166A, C, D, E, F, G, H, I, K, L, M, N, P, Q, R, T, V, W, or Y; and/or
Q167A, C, D, E, F, G, H, I, K, L, M, N, P, R, S, T, V, W, or Y; and/or
K168A, C, D, E, F, G, H, I, L, M, N, P, Q, R, S, T, V, W, or Y; and/or
V169A, C, D, E, F, G, H, I, K, L, M, N, P, Q, R, S, T, W, or Y; and/or
V170A, C, D, E, F, G, H, I, K, L, M, N, P, Q, R, S, T, W, or Y; and/or
E171A, C, D, F, G, H, I, K, L, M, N, P, Q, R, S, T, V, W, or Y; and/or
A172C, D, E, F, G, H, I, K, L, M, N, P, Q, R, S, T, V, W, or Y; and/or
Y179A, C, D, E, F, G, H, I, K, L, M, N, P, Q, R, S, T, V, or W; and/or
H180A, C, D, E, F, G, I, K, L, M, P, Q, R, S, T, V, W, or Y; and/or
N181A, C, D, E, F, G, H, I, K, L, M, P, Q, R, S, T, V, W, or Y; and/or
Q182A, C, D, E, F, G, H, I, K, L, M, N, P, R, S, T, V, W, or Y, preferably K; and/or
M209A, C, D, E, F, G, H, I, K, L, N, P, Q, R, S, T, V, W, or Y; and/or
L210 A, C, D, E, F, G, H, I, K, M, N, P, Q, R, S, T, V, W, or Y; and/or
R211 A, C, D, E, F, G, H, I, K, L, M, N, P, Q, R, S, T, V, W, or Y; and/or
N215 A, C, D, E, F, G, H, I, K, L, M, N, P, Q, R, S, T, V, W, or Y; and/or
Y226A, C, D, E, G, H, I, K, L, M, N, P, Q, R, S, T, V, or W; and/or
Y230A, C, D, E, G, H, I, K, L, M, N, P, Q, R, S, T, V or W; and/or
K284A, C, D, E, F, G, H, I, L, M, N, P, Q, R, S, T, V, W, or Y; and/or
M285A, C, D, E, F, G, H, I, K, L, N, P, Q, R, S, T, V, W, or Y; and/or
Q289A, C, D, E, F, G, H, I, K, L, M, N, P, R, S, T, V, W, or Y; and/or
V290A, C, D, E, F, G, H, I, K, L, M, N, P, Q, R, S, T, W, or Y; and/or
E309A, C, D, F, G, H, I, K, L, M, N, P, Q, R, S, T, V, W, or Y; and/or
S310A, C, D, E, F, G, H, I, K, L, M, N, P, Q, R, T, V, W, or Y.

In addition or alternatively thereto there may be one or more C-terminal extensions. Preferably the additional C-terminal extension is comprised of one or more aliphatic amino acids, preferably a non-polar amino acid, more preferably of I, L, V or G. Thus, the present invention further provides for a variant enzyme comprising one or more of the following C-terminal extensions: 318I, 318L, 318V, 318G.

When it is the case that the residues in the parent backbone differ from those in P10480 (SEQ ID No. 2), as determined by homology alignment and/or structural alignment to P10480 and/or 1IVN, it may be desirable to replace the residues which align to any one or more of the following amino acid residues in P10480 (SEQ ID No. 2): Ser3, Leul7, Lys22, Met23, Gly40, Asn80, Pro81, Lys82, Asn 87, Asn88, Trp111, Val112, Ala114, Tyr117, Leu118, Pro156, Gly159, Gln160, Asn161, Pro162, Ser163, Ala164, Arg165, Ser166, Gln167, Lys168, Val169, Val170, Glu171, Ala172, Tyr179, His180, Asn181, Gln182, Met209, Leu210, Arg211, Asn215, Lys284, Met285, Gln289, Val290, Glu309 or Ser310, with the residue found in P10480 respectively.

The following wildtype residues of P10480 have been found to be preferable for retaining good activity, particularly good transferase activity from a galactolipid:, L17, W111, R221, S3, G40, N88, K22, Y117, L118, N181, M209, M285, E309, M23. Thus preferably the variant enzyme comprises the amino acid residue found in P10480 at any one or more of these sites.

Variant enzymes which have an increased hydrolytic activity against a polar lipid may also have an increased transferase activity from a polar lipid.

Variant enzymes which have an increased hydrolytic activity against a phospholipid, such as phosphatidylcholine (PC) may also have an increased transferase activity from a phospholipid.

Variant enzymes which have an increased hydrolytic activity against a galactolipid, such as DGDG, may also have an increased transferase activity from a galactolipid.

Variants enzymes which have an increased transferase activity from a phospholipid, such as phosphatidylcholine (PC), may also have an increased hydrolytic activity against a phospholipid.

Variants enzymes which have an increased transferase activity from a galactolipid, such as DGDG, may also have an increased hydrolytic activity against a galactolipid.

Variants enzymes which have an increased transferase activity from a polar lipid may also have an increased hydrolytic activity against a polar lipid.

Suitably, one or more of the following sites may be involved in substrate binding: Leu17; Ala114; Tyr179; His180; Asu181; Met209; Leu210; Arg211; Asn215; Lys284; Met285; Gln289; Val290.

The variant enzyme in accordance with the present description may have one or more of the following functionalities compared with the parent enzyme:

1) an increased relative transferase activity against galactolipid (DG) compared to PC calculated as % $T_{DG}/T_{PC}$ (as illustrated in Example 8)
2) an increased absolute transferase activity against galactolipid (DG) (as illustrated in Example 8)
3) an increased transferase activity using galactolipid as donor ($T_{DG}$) relative to the hydrolytic activity $H_{DG}$ on galactolipid (DG) (as illustrated in Example 8)
4) an increased absolute transferase activity against PC (as illustrated in Example 8)

Wherein DG is galactolipid (e.g. DGDG) (and may be herein also referred to as GL) and PC is phospholipid (e.g. lecithin). Variants with an increased activity towards galactolipid include variants within categories 1), 2) and 3) above. Variants with an increased activity on galactolipids may also have an increased activity in phospholipids (as per category 4) above).

**1. A modification to one or more of the following residues may result in a variant enzyme having an increased relative transferase activity against DG compared to PC calculated as % $T_{DG}/T_{PC}$:**

-318, N215, L210, S310, E309, H180, N80, V112, Y30X (where X is selected from A, C, D, E, G, H, I, K, L, M, N, P, Q, R, S, T, V, or W), V290, Q289, K22, G40, Y179, M209, L211, K22, P81, N87, Y117, N181, Y230X (where X is selected from A, C, D, E, G, H, I, K, L, M, N, P, Q, R, S, T, V, or W), Q182.

Typically, one or more of the following substitutions may be preferred:

S3A, C, D, E, F, G, H, I, K, L, M, N, P, Q, R, T, V, W or Y, preferably M, R, N, G, T, Q, P, Y, S, L, E, W, most preferably Q
K22A, E, C, F, G, H, I, L, M, N, P, Q, R, S, T, V, W or Y, preferably A, C, E or R
Y30A, C, D, H, K, M, N, P, Q, R, T, V, W, G, I, L, S, M, A, R or E, preferably H, T, W, N, D, C, Q G, I, L, S, M, A, R or E
G40 L, N, T, V or A
N80N, R, D, A, C, E, F, G, H, I, K, L, M, P, Q, S, T, V, W or Y, preferably H, I, Y, C, Q, M, S, W, L, N, R, D or F
P81A, C, D, E, F, G H, I, K, L, M, N, Q, R, S, T, V, W or Y, preferably I, M, F, G, V, Y, D, C or A
K82A, C, D, E, F, G, H, I, L, M, N, P, Q, R, S, T, V, W or Y, preferably H, K, S or R N87A, C, D, E, F, G, H, I, K, L, M, P, Q, R, S, T, V, W or Y, preferably I, Y, M, T, Q, S, W, F, V or P
N88A, C, D, E, F, G, H, I, K, L, M, P, Q, R, S, T, V, W or Y, preferably C, V, A or F V112C
Y117A, C, D, E, F, H, T, G, I, K, L, M, N, P, Q, R, S, V or W, preferably A, N, E, H, T, I, F, C, P or S
L118A, C, D, E, F, G, H, I, K, M, N, P, Q, R, S, T, V, W, or Y, preferably F V112A, D, E, F, G, H, I, K, L, M, N, P, Q, R, S, T, W or Y, preferably I, M, F, Y, N, E, T, Q, H or P
Y179A, C, D, E, F, G, H, I, K, L, M, N, P, Q, R, S, T, V or W, preferably F, C, H, I, L, M, P, V or W
H180K, Q, A, C, D, E, F, G, I, L, M, P, R, S, T, V, W, or Y, preferably M, F, C, K or Q
N181A orV
Q182A, C, D, E, F, G, H, I, K, M, N, P, Q, R, S, T, V, W, or Y, preferably K M209L, K, M, A, C, D, E, F, G, H, I, N, P, Q, R, S, T, V, W, or Y, preferably I, F, T, D, C, H, L, K, M or P
L210 G, I, H, E, M, S, W, V, A, R, N, D, Q ,T, C, F, K, P or Y, preferably G, I, H, E, M, S, W, V, A, R, N, D, Q ,T, Y or F
R211G, Q, K, D, A, C, E, F, H, I, L, M, N, P, R, S, T, V, W or Y, preferably G, Q, K, D, H, I, M, F, P, S, Y, N, C, L or W

N215A, C, D, E, F, G, H, I, K, L, M, N, P, Q, R, S, T, V, W or Y, preferably I, F, P, T, W, H or A
Y230A, C, D, E, G, H, I, K, L, M, N, P, Q, R, S, T, V or W, preferably I, T, G, D, R, E, V, M or S, most preferably I, D, R or E
Q289A, C, D, E, F, G, H, I, K, L, M, N, P, R, S, T, V, W or Y, preferably F, W, H, I, Y, L, D, C, K, V, E, G. R, N or P, more preferably R, T, D, K, N or P
V290A, C, D, E, H, F, G, I, K, L, M, N, P, Q, R, S, T, W or Y;
E309S, Q, R, A, C, D, F, G, H, I, K, L, M, N, P, T, V, W or Y, preferably F, W, N, H, I, M, S, Q, R, A or Y
S310A, P, T, H, M, K, G, C, D, E, F, I, L, N, Q, R, V, W or Y, preferably F, Y, C, L, K, A, P, T, H, M, K or G
-318 A, C, D, E, F, G, I, K, L, M, N, P, Q, R, T, V, W, Y, H or S

Preferably, one or more of the following modifications may result in a variant enzyme having an increased relative transferase activity against DG compared to PC calculated as % $T_{DG}/T_{PC}$
S3A, C, D, E, F, G, H, I, K, L, M, N, P, Q, R, T, V, W or Y, preferably M, R, N, G, T, Q, P, Y, S, L, E, W, most preferably Q
G40 L, N, T, V or A
K82A, C, D, E, F, G, H, I, L, M, N, P, Q, R, S, T, V, W or Y, preferably H, K, S or R N88A, C, D, E, F, G, H, I, K, L, M, P, Q, R, S, T, V, W or Y, preferably C, V, A or F Y230A, C, D, E, G, H, I, K, L, M, N, P, Q, R, S, T, V or W, preferably I, T, G, D, R, E, V, M or S, most preferably D, R or E
Q289A, C, D, E, F, G, H, I, K, L, M, N, P, R, S, T, V, W or Y, preferably F, W, H, I, Y, L, D, C, K, V, E, G or P, more preferably R, T, D, K or P
Modification of one or more of the following modifications results in a variant enzyme having an increased relative transferase activity against DG compared to PC calculated as % $T_{DG}/T_{PC}$:

-318 Y, H or S
N215H
L210G, I, H, E, M, S, W, V, A, R, N, D, Q or T
S310A, P, T, H, M, K orG
E309S, Q, A or R
H180K, T or Q
N80N, R or D
V112C
Y30G, I, L, S, M, A, R or E, more preferably Y30M, A or R
V290R, E, H or A
Q289R, T, D or N
K22E
G40L
Y179V or R
M209L, K or M
L211G, Q, K or D
Y230V
G40Q, L or V
N88W
N87R or D

For some embodiments the following substitutions may also be suitable:

K22A or C
P81G
N87 M
Y117A, N, E, H or T
N181A or V
Y230I
V290H
N87R, D, E or M
Q182T

Preferably, the residues modified in order to increase the ratio of galactolipid transferase compared to phospholipid transferase activity are one or more of the following:: -318, N215, L210, E309, H180, N80.
Typically, one or more of the following substitutions are preferred:

-318 Y, H or S, most preferably Y
N215H
L210D, Q or T
E309Q or R
H180K or Q
N80N, R or D

**2. A modification to one or more of the following residues may result in a variant having an increased absolute transferase activity against DG:**

-318, N215, L210, S310, E309, H180, N80, V112, Y30X (where X is selected from A, C, D, E, G, H, I, K, L, M, N, P, Q, R, S, T, V, or W), V290, Q 289, K22, G40, Y179, M209, L211, K22, P81, N87, Y117, N181, Y230X (where X is selected from A, C, D, E, G, H, I, K, L, M, N, P, Q, R, S, T, V, or W), V290, N87, Q182, S3, S310, K82, A309.

In particular, one or more of the following modifications may result in a variant having an increased absolute transferase activity against DG:

-318Y, H, S, A, C, D, E, F, G, I, K, L, M, N, P, Q, R, T, V or W, preferably Y, H, S or I
N215A, C, D, E, F, G, H, I, K, L, M, N, P, Q, R, S, T, V, W or Y; preferably H, I, F, P, T, W or A, most preferably H, S, L, R, Y
L210G, I, H, E, M, S, W, V, A, R, N, D, Q, T, C, F, K, P or Y, preferably D, Q, T, Y or F
S310A, P, T, H, M, K, G, C, D, E, F, I, L, N, Q, R, V, W or Y. preferably F, Y, C, L, K or P,
E309S, Q, R, A, C, D, F, G, H, I, K, L, M, N, P, S, T, V, W or Y; preferably S, Q, R, F, W, N, H, I, M or Y, most preferably S, Q, R, N, P or A
H180A, C, D, E, F, G, I, K, Q L, M, P, R, S, T, V, W or Y; preferably K, Q, M, F or C, most preferably T, K or Q
N181A or V
N80N, R, D, A, C, E, F, G, H, I, K, L, M, P, Q, S, T, V, W or Y, preferably H, I, Y, C, Q, M, S, W, L, N, R, D or F, most preferably N, R, D, P, V, A or G
V112A, C, D, E, F, G, H, I, K, L, M, N, P, Q, R, S, T, W or Y; preferably I, M, F, Y, N, E, T, Q, H or P
Y30G, I, L, S, A, E, C, D, H, K, M, N, P, Q, R, T, V or W, preferably H, T, W, N, D, C or Q
V290A, C, D, E, F, G, H, I, K, L, M, N, P, Q, R, S, T, W or Y;
Q289A, C, D, E, F, G, H, I, K, L, M, N, P, R, S, T, V, W or Y; preferably R, E, G, P, N or R
K22A, C, E, F, G, H, I, L, M, N, P, Q, R, S, T, V, W or Y, preferably C
Y179A, C, D, E, F, G, H, I, K, L, M, N, P, Q, R, S, T, V or W; preferably F, C, H, I, L, M, P or W, more preferably E, R, N, V, K, S
M209A, C, D, E, F, G, H, I, L, K, M, N, P, Q, R, S, T, V, W or Y; preferably R, N, Y, E or V
R211A, C, E, F, G, H, I, L, M, N, P, Q, K, D, R, S, T, V, W or Y, preferably H, I, M, F, P, S, Y, N, C, L or W, most preferably R
S310 C, D, E, F, I, L, N, Q, R, V, W or Y. preferably F, Y, C, L, K or P
S3A, C, D, E, F, G, H, I, K, L, M, N, P, Q, R, T, V, W or Y, preferably M, R, N, A, G, T, Q, P, Y or S most preferably Q or N
K82A, C, D, E, F, G, H, I, L, M, N, P, Q, R, S, T, V, W or Y, preferably H, K, S, E or R
P81A, C, D, E, F, G,H, I, K, L, M, N, Q, R, S, T, V, W or Y; preferably I, M, F, V, Y, D, C or A
N87A, C, F, G, H, I, K, L, M, P, Q, R, D, E, S, T, V, W or Y; preferably L, G or A Y117A, N, E, H, T, C, D, F, G, I, K, L, M, P, Q, R, S, V or W; preferably I, F, C, P or S
N87A, C, F, G, H, I, K, L, P, Q, S, T, V, W or Y; preferably I, Y, T, Q, S, W, F, V or P Q182A, C, D, E, F, G, H, I, K, L, M, N, P, R, S, T, V, W or Y, preferably D or K Y230A, C, D, E, G, H, I, K, L, M, N, P, Q, R, S, T, V or W, preferably W, H, Q, L, P or C, most preferable T or G
D157A, C, E, F, G, H, I, K, L, M, P, Q, R, S, T, V, W or Y, preferably C
G40L
Y226I

Typically, one or more of the following substitutions are preferred:

-318 Y, H or S
N215H
L210G, I, H, E, M, S, W, V, A, R, N, D, Q or T
S310A, P, T, H, M, K or G
E309S, Q or R
H180K or Q
N80N, R or D
V112C

Y30G, I, L, S, M, A, R or E, more preferably Y30M, A or R
V290R, E, H or A
Q289R or N
K22E
G40L
Y179V
M209L, K or M
L211G, Q, K or D

The following substitutions may also be suitable:

K22A or C
P81G
N87 M
Y117A, N, E, H or T
N181A or V
Y230I
V290H
N87R, D, E or M
Q182T

Preferably, the residues modified in order to increase transferase activity from a galactolipid substrate (DGDG) are one or more of the following:: -318, N215, L210, E309, H180, N80.

Typically, one or more of the following substitutions are preferred:

-318 Y, H or S, most preferably Y
N215H
L210D, Q or T
E309Q or R
H180K or Q
N80N, R or D

**3. A modificiation at one or more of the following residues may result in a variant enzyme having an increased transferase activity $T_{DG}$ relative to the hydrolytic activity $H_{DG}$ on DG:**

Y230, S310, H180, Q289, G40, N88, Y179, N215, L210, N80, Y30X (where X is selected from A, C, D, E, G, H, I, K, L, M, N, P, Q, R, S, T, V, or W), N87, M209, R211, S18X (where X is specifically selected from A, C, D, E, F, H, I, K, L, M, N, P, Q, R, T, W or Y).

Preferably, one or more of the following modifications may result in a variant enzyme having an increased transferase activity $T_{DG}$ relative to the hydrolytic activity $H_{DG}$ on DG:

Y230A, C, D, E, G, H, I, K, L, M, N, P, Q, R, S, T or W, preferably W, H, Q, L, P or C
S310A, C, D, E, F, G, H, I, K, L, M, N, Q, R, T, V, W or Y, preferably F, Y, C, L, K or P
Y179A, C, D, E, F, G, H, I, K, L, M, N, P, Q, S, T, V, or W, preferably F, C, H, I, L, M, P or W
H180A, C, D, E, F, G, I, K, L, M, P, Q, R, S, V, W or Y, preferably M, F or C Q289A, C, E, F, G, H, I, K, L, M, N, P, R, S, V, W or Y; preferably F, W, H, I, Y, L, D, C, K, V, E, G or P
G40A, C, D, E, F, H, I, K, M, N, P, R, S, T, W or Y; preferably I, P, W or Y
N88A, C, D, E, F, G, H, I, K, L, M, P, Q, R, S, T, V or Y; preferably I or H
N87A, C, E, F, G, H, I, K, L, M, P, Q, S, T, V, W or Y; preferably I, Y, T, Q, S, W, F, V or P

Typically, one or more of the following substitutions are preferred (such variant enzymes may have a decreased hydrolytic activity (galactolipid and/or phospholipids) and/or an increased tranferase activity from galactolipid:

Y179 E, R, N or Q
N215G
L210D, H, R, E, A, Q, P, N, K, G, R, T, W, I, V or S
N80G
Y30L

N87G

Typically, one or more of the following substitutions are preferred (such variant enzymes may have a decreased hydrolytic activity (galactolipid and/or phospholipids) whilst retaining significant tranferase activity from galactolipid:

Y179 E, R, N, Q

N215 G

L210 D, H, R, E, A, Q, P, N, K, G, R, T, W, I, V and S

N80 G

Y30 L

N87 G

H180 I, T

M209 Y

R211 D, T and G

S18 G, M and T

G40 R and M

N88 W

N87 C, D, R, E and G

**4. Modification of one or more of the following residues may result in a variant enzyme having an increased absolute transferase activity against phospholipid:**

S3, D157, S310, E309, Y179, N215, K22, Q289, M23, H180, M209, L210, R211, P81, V112, N80, L82, N88; N87
Specific modifications which may provide a variant enzyme having an improved transferase activity from a phospholipid may be selected from one or more of the following:

S3A, C, D, E, F, G, H, I, K, L, M, N, P, Q, R, T, V, W or Y; preferably N, E, K, R, A, P or M, most preferably S3A
D157A, C, E, F, G, H, I, K, L, M, N, P, Q, R, S, T, V, W or Y ; preferably D157S, R, E, N, G, T, V, Q, K or C
S310A, C, D, E, F, G, H, I, K, L, M, N, P, Q, R, T, V, W or Y; preferably S310T -318 E
E309A, C, D, E, F, G, H, I, K, L, M, N, P, Q, R, T, V, W or Y; preferably E309 R, E, L, R or A
Y179A, C, D, E, F, G, H, I, K, L, M, N, P, Q, R, S, T, V or W; preferably Y179 D, T, E, R, N, V, K, Q or S, more preferably E, R, N, V, K or Q
N215A, C, D, E, F, G, H, I, K, L, M, P, Q, R, S, T, V, W or Y; preferably N215 S, L, RorY
K22A, C, D, E, F, G, H, I, L, M, N, P, Q, R, S, T, V, W or Y; preferably K22 E, R, C or A
Q289A, C, D, E, F, G, H, I, K, L, M, N, P, R, S, T, V, W or Y; preferably Q289 R, E, G, P or N
M23A, C, D, E, F, G, H, I, K, L N, P, Q, R, S, T, V, W or Y; preferably M23 K, Q, L, G, T or S
H180A, C, D, E, F, G, I, K, L, M, P, Q, R, S, T, V, W or Y; preferably H180 Q, R or K M209 A, C, D, E, F, G, H, I, K, L, N, P, Q, R, S, T, V, W or Y; preferably M209 Q, S, R, A, N, Y, E, V or L
L210A, C, D, E, F, G, H, I, K, M, N, P, Q, R, S, T, V, W or Y; preferably L210 R, A, V, S, T, I, W or M
R211A, C, D, E, F, G, H, I, K, L, M, N, P, Q, S, T, V, W or Y; preferably R211T P81A, C, D, E, F, G, H, I, K, L, M, N, Q, R, S, T, V, W or Y; preferably P81G
V112A, C, D, E, F, G, H, I, K, L, M, N, P, Q, R, S, T, W or Y; preferably V112C N80A, C, D, E, F, G, H, I, K, L, M, P, Q, R, S, T, V, W or Y; preferably N80 R, G, N, D, P, T, E, V, A or G
L82A, C, D, E, F, G, H, I, M, N, P, Q, R, S, T, V, W or Y; preferably L82N, S or E N88A, C, D, E, F, G, H, I, K, L, M, P, Q, R, S, T, V, W or Y; preferably N88C N87A, C, D, E, F, G, H, I, K, L, M, P, Q, R, S, T, V, W or Y; preferably N87M or G

Modification of one or more of the following residues results in a variant enzyme having an increased absolute transferase activity against phospholipid:

S3 N, R, A, G
M23 K, Q, L, G, T, S
H180 R
L82 G
Y179 E, R, N, V, K or Q
E309 R, S, L or A

**5. Residues the modification of which results in an increased transferase activity from a galactolipid substrate (DGDG) and an increase in ratio of galactolipid transferase compared to phospholipid transferase activity** include one or more of: - 318, N215, L210, S310, E309, H180, N80, V112, Y30X (where X is selected from A, C, D, E, G, H, I, K, L, M, N, P, Q, R, S, T, V, or W), V290, Q 289, K22, G40, Y179, M209, L211, K22, P81, N87, Y117, N181, Y230X (where X is selected from A, C, D, E, G, H, I, K, L, M, N, P, Q, R, S, T, V, or W), Q182.

Typically, one or more of the following substitutions are preferred:

-318 Y, H or S
N215H
L210G, I, H, E, M, S, W, V, A, R, N, D, Q or T
S310A, P, T, H, M, K orG
E309S, A, Q or R
H180K or Q
N80N, R or D
V112C
Y30G, I, L, S, M, A, R or E, more preferably Y30M, A or R
V290R, E, H or A
Q289R or N
K22E
G40L
Y179V
M209L, K or M
L211G, Q, K or D

The following substitutions may also be suitable:

K22A or C
P81G
N87 M
Y117A, N, E, H or T
N181A or V
Y230I
V290H
N87R, D, E or M
Q182T

Preferably, the residues modified in order to increase transferase activity from a galactolipid substrate (DGDG) and/or increase the ratio of galactolipid transferase compared to phospholipid transferase activity are one or more of the following:: -318, N215, L210, E309, H180, N80.

Typically, one or more of the following substitutions are preferred:

-318 Y, H or S, most preferably Y
N215H
L210D, Q or T
E309Q or R
H180K or Q
N80N, R or D

**6. The following wildtype residues of P10480 have been found to be preferable for retaining good activity, particularly good transferase activity from a galactolipid:**

W111, R211, N181, S3, L17, G40, N88, Y117, L118, N181, K22, M209, M285, M23

Preferably, these residues are retained in the variant enzyme.

When making variant GDSx acyl-transferases for increased activity transferase from galactolipid substrates, where the parent enzyme has a residue corresponding to residues of the P10480 sequence at positions W111, R211, N181, S3, L17, G40, N88, Y117, L118, N181, K22, M209, M285, M23 other than the residue found in P10480 the variant may preferably contain a substitution at the corresponding position to include the amino acid residue found in the P10480 sequence.

L17 is preferably a hydrophobic amino acid residue

**7. The following combinations may have an increased transferase activity from a galactolipid substrate (DGDG) and/or an increase in ratio of galactolipid transferase activity compared to phospholipid transferase activity**

N215H & -318Y
N215H & L210D, Q, or T
-318Y & L210,D, Q, or T
N215H & -318Y & L210D, Q, or T

The above combinations may optionally also include a C-terminal amino acid addition, such as -318Y, H or S, or preferably -318Y.

The above combinations may optionally also include the following modification:
Suitably one or more of the following combinations may have an increased transferase activity from a galactolipid substrate (DGDG) and/or an increase in ratio of galactolipid transferase activity compared to phospholipid transferase activity:

E309A, Q or R.
N215H & -318Y, H, or S, preferably Y.
L210D, Q or T & -318Y, H, or S, preferably Y.
N215H & E309A, Q or R
L210D, Q or T & E309A, Q or R
-318Y & E309A, Q or R

The above combinations may optionally also include a substitution at position Q182, preferably Q182K.

The following combinations may have an increased transferase activity from a galactolipid substrate (DGDG) and/or an increase in ratio of galactolipid transferase activity compared to phospholipid transferase activity, and/or an increase in ratio of galactolipid transferase compared to hydrolytic activity:

N215H & N80G
-318Y & N80G
L210D or Q & N80G
N215H & N88N
-318Y & N88N
L210D or Q & N88N
N215H & Y30L
-318Y & Y30L
L210D or Q & Y30L
N215H & N87G
-318Y & N87G
L210D or Q & N87G
N215H & Y179E, R, N or Q
-318Y & Y179 E, R, N or Q
L210D or Q & Y179 E, R, N or Q

As noted above, when referring to specific amino acid residues herein the numbering is that obtained from alignment of the variant sequence with the reference sequence shown as SEQ ID No. 2.

For the avoidance of doubt, when a particular amino acid is taught at a specific site, for instance L118 for instance, this refers to the specific amino acid at residue number 118 in SEQ ID No. 2. However, the amino acid residue at site 118 in a different parent enzyme may be different from leucine.

Thus, when taught to substitute an amino acid at residue 118, although reference may be made to L118 it would be readily understood by the skilled person that when the parent enzyme is other than that shown in SEQ ID No. 2, the amino acid being substituted may not be leucine. It is, therefore, possible that when substituting an amino acid sequence in a parent enzyme which is not the enzyme having the amino acid sequence shown as SEQ ID No. 2, the new (substituting) amino acid may be the same as that taught in SEQ ID No. 2. This may be the case, for instance, where the amino acid at say residue 118 is not leucine and is, therefore different from the amino acid at residue 118 in SEQ ID No. 2. In other words, at residue 118 for example, if the parent enzyme has at that position an amino acid other than leucine, this amino acid may be substituted with leucine

The term "lipid acyltransferase" as used herein means an enzyme which has acyltransferase activity (generally classified as E.C. 2,3.1.x in accordance with the Enzyme Nomenclature Recommendations (1992) of the Nomenclature Committee of the International Union of Biochemistry and Molecular Biology), whereby the enzyme is capable of transferring an acyl group from a lipid to one or more acceptor substrates, such as one or more of the following: a sterol; a stanol; a carbohydrate; a protein; a protein subunit; glycerol.

Preferably the lipid acyltransferase is capable of transferring an acyl group from a lipid to at least a sterol and/or a stanol, for example to cholesterol.

Preferably, the lipid acyltransferase variant described herein and/or for use in the methods and/or uses described herein is capable of transferring an acyl group from a lipid (as defined herein) to one or more of the following acyl acceptor substrates: a sterol, a stanol, a carbohydrate, a protein or subunits thereof, or a glycerol.

For some aspects the "acyl acceptor" described herein may be any compound comprising a hydroxy group (-OH), such as for example, polyvalent alcohols, including glycerol; sterol; stanols; carbohydrates; hydroxy acids including fruit acids, citric acid, tartaric acid, lactic acid and ascorbic acid; proteins or a sub-unit thereof, such as amino acids, protein hydrolysates and peptides (partly hydrolysed protein) for example; and mixtures and derivatives thereof. Preferably, the "acyl acceptor" described herein is not water.

The acyl acceptor may preferably not be a monoglyceride and/or a diglyceride.

In one aspect, preferably the variant enzyme is capable of transferring an acyl group from a lipid to a sterol and/or a stanol.

In one aspect, preferably the variant enzyme is capable of transferring an acyl group from a lipid to a carbohydrate.

In one aspect, preferably the variant enzyme is capable of transferring an acyl group from a lipid to a protein or a subunit thereof. Suitably the protein subunit may be one or more of the following: an amino acid, a protein hydrolysate, a peptide, a dipeptide, an oligopeptide, a polypeptide.

Suitably in the protein or protein subunit the acyl acceptor may be one or more of the following constituents of the protein or protein subunit: a serine, a threonine, a tyrosine, or a cysteine.

When the protein subunit is an amino acid, suitably the amino acid may be any suitable amino acid. Suitably the amino acid may be one or more of a serine, a threonine, a tyrosine, or a cysteine for example.

In one aspect, preferably the variant enzyme is capable of transferring an acyl group from a lipid to glycerol.

In one aspect, preferably the variant enzyme is capable of transferring an acyl group from a lipid to a hydroxy acid.

In one aspect, preferably the variant enzyme is capable of transferring an acyl group from a lipid to a polyvalent alcohol.

In one aspect, the variant lipid acyltransferase may, as well as being able to transfer an acyl group from a lipid to a sterol and/or a stanol, additionally be able to transfer the acyl group from a lipid to one or more of the following: a carbohydrate, a protein, a protein subunit, glycerol.

Preferably, the lipid substrate upon which the variant lipid acyltransferase described herein acts is one or more of the following lipids: a phospholipid, such as a lecithin, e.g. phosphatidylcholine, a triacylglyceride, a cardiolipin, a diglyceride, or a glycolipid, such as digalactosyldiglyceride (DGDG) or monogalactosyldiglyceride (MGDG) for example. More preferably, the variant enzyme described herein acts on one or both of DGDG and MGDG. Preferably, the variant enzyme described herein has no (or has only limited) activity on digalactosylmonoglyceride (DGMG) and monogalactosylmonoglyceride (MGMG). Thus preferably the lipid substrate is not one or both of DGMG or MGMG. This lipid substrate may be referred to herein as the "lipid acyl donor". The term lecithin as used herein encompasses phosphatidylcholine, phosphatidylethanolamine, phosphatidylinositol, phosphatidylserine and phosphatidylglycerol.

The term "galactolipid" as used herein means one or more of DGDG or DGMG.

The term "phospholipid" as used herein, means lecithin, including phosphatidylcholine.

The term "polar lipid" as used herein means a phospholipids and/or a galactolipid, preferably a phospholipids and a galactolipid.

For some aspects, preferably the lipid substrate upon which the variant lipid acyltransferase acts is a phospholipid, such as lecithin, for example phosphatidylcholine.

For some aspects, preferably the lipid substrate is a glycolipid, such as DGDG or MGDG for example.

Preferably the lipid substrate is a food lipid, that is to say a lipid component of a foodstuff.

For some aspects, preferably the variant lipid acyltransferase described herein invention is incapable, or substantially incapable, of acting on a triglyceride and/or a 1-monoglyceride and/or 2-monoglyceride.

Suitably, the lipid substrate or lipid acyl donor may be one or more lipids present in one or more of the following substrates: fats, including lard, tallow and butter fat; oils including oils extracted from or derived from palm oil, sunflower oil, soya bean oil, safflower oil, cotton seed oil, ground nut oil, corn oil, olive oil, peanut oil, coconut oil, and rape seed oil. Lecithin from soya, rape seed or egg yolk is also a suitable lipid substrate. The lipid substrate may be an oat lipid or other plant based material containing galactolipids.

In one aspect the lipid acyl donor is preferably lecithin (such as phosphatidylcholine) in egg yolk.

The lipid may be selected from lipids having a fatty acid chain length of from 8 to 22 carbons.

The lipid may be selected from lipids having a fatty acid chain length of from 16 to 22 carbons, more preferably of from 16 to 20 carbons.

The lipid may be selected from lipids having a fatty acid chain length of no greater than 14 carbons, suitably from lipids having a fatty acid chain length of from 4 to 14 carbons, suitably 4 to 10 carbons, suitably 4 to 8 carbons.

Suitably, the variant lipid acyltransferase described herein may exhibit one or more of the following lipase activities: glycolipase activity (E.C. 3.1.1.26), triacylglycerol lipase activity (E.C. 3.1.1.3), phospholipase A2 activity (E.C. 3.1.1.4) or phospholipase A1 activity (E.C. 3.1.1.32). The term "glycolipase activity" as used herein encompasses "galactolipase activity".

Suitably, the variant lipid acyltransferase described herein may have at least one or more of the following activities: glycolipase activity (E.C. 3.1.1.26) and/or phospholipase A1 activity (E.C. 3.1.1.32) and/or phospholipase A2 activity (E.C. 3.1.1.4).

For some aspects, the variant lipid acyltransferase described herein may have at least glycolipase activity (E.C. 3.1.1.26).

Suitably, for some aspects the variant lipid acyltransferase described herein may be capable of transferring an acyl group from a glycolipid and/or a phospholipid to one or more of the following acceptor substrates: a sterol, a stanol, a carbohydrate, a protein, glycerol.

For some aspects, preferably the variant lipid acyltransferase described herein is capable of transferring an acyl group from a glycolipid and/or a phospholipid to a sterol and/or a stanol to form at least a sterol ester and/or a stanol ester.

For some aspects, preferably the variant lipid acyltransferase described herein is capable of transferring an acyl group from a glycolipid and/or a phospholipid to a carbohydrate to form at least a carbohydrate ester.

For some aspects, preferably the variant lipid acyltransferase described herein is capable of transferring an acyl group from a glycolipid and/or a phospholipid to a protein to form at least protein ester (or a protein fatty acid condensate).

For some aspects, preferably the variant lipid acyltransferase described herein is capable of transferring an acyl group from a glycolipid and/or a phospholipid to glycerol to form at least a diglyceride and/or a monoglyceride.

For some aspects) preferably the variant lipid acyltransferase described herein does not exhibit triacylglycerol lipase activity (E.C. 3.1.1.3).

In some aspects, the variant lipid acyltransferase may be capable of transferring an acyl group from a lipid to a sterol and/or a stanol. Thus, in one embodiment the "acyl acceptor" described herein may be either a sterol or a stanol or a combination of both a sterol and a stanol.

Suitably the sterol and/or stanol may comprise one or more of the following structural features:

i) a 3-beta hydroxy group or a 3 -alpha hydroxy group; and/or
ii) A:B rings in the *cis* position or A:B rings in the *trans* position or $C_5$-$C_6$ is unsaturated,

Suitable sterol acyl acceptors include cholesterol and phytosterols, for example alpha-sitosterol, beta-sitosterol, stigmasterol, ergosterol, campesterol, 5,6-dihydrosterol, brassicasterol, alpha-spinasterol, beta-spinasterol, gamma-spinasterol, deltaspinasterol, fucosterol, dimosterol, ascosterol, serebisterol, episterol, anasterol, hyposterol, chondrillasterol, desmosterol, chalinosterol, poriferasterol, clionasterol, sterol glycosides, and other natural or synthetic isomeric forms and derivatives,

- Suitably more than one sterol and/or stanol may act as the acyl acceptor, suitably more than two sterols and/or stanols may act as the acyl acceptor. In other words, suitably more than one sterol ester and/or stanol ester may be produced. Suitably, when cholesterol is the acyl acceptor one or more further sterols or one or more stanols may also act as the acyl acceptor. Thus, described herein is a method for the *in situ* production of both a cholesterol ester and at least one sterol or stanol ester *in* combination. In other words, the lipid acyltransferase for some aspects of the present description may transfer an acyl group from a lipid to both cholesterol and at least one further sterol and/or at least one stanol.

In one aspect, preferably the sterol acyl acceptor is one or more of the following; alpha-sitosterol, beta-sitosterol, stigmasterol, ergosterol and campesterol.

In one aspect, preferably the sterol acyl acceptor is cholesterol. When it is the case that cholesterol is the acyl acceptor for the variant lipid acyltransferase, the amount of free cholesterol in the foodstuff is reduced as compared with the foodstuff prior to exposure to the variant lipid acyltransferase and/or as compared with an equivalent foodstuff which has not been treated with the variant lipid acyltransferase.

Suitable stanol acyl acceptors include phytostanols, for example beta-sitostanol or ss-sitostanol.

In one aspect, preferably the sterol and/or stanol acyl acceptor is a sterol and/or a stanol other than cholesterol.

In some aspects, the foodstuff prepared in accordance with the present description may be used to reduce blood serum cholesterol and/or to reduce low density lipoprotein. Blood serum cholesterol and low density lipoproteins have both been associated with certain diseases in humans, such as atherosclerosis and/or heart disease for example. Thus, it is envisaged that the foodstuffs prepared in accordance with the present description may be used to reduce the risk of such diseases.

Thus, described herein is the use of a foodstuff described herein for use in the treatment and/or prevention of atherosclerosis and/or heart disease. Thus is one aspect the foodstuff may be considered as a neutraceutical.

Further described herein is a medicament comprising a foodstuff described herein.

Described herein is a method of treating and/or preventing a disease in a human or animal patient which method comprises administering to the patient an effective amount of a foodstuff described herein.

Suitably, the sterol and/or the stanol "acyl acceptor" may be found naturally within the foodstuff. Alternatively, the sterol and/or the stanol may be added to the foodstuff. When it is the case that a sterol and/or a stanol is added to the foodstuff, the sterol and/or stanol may be added before, simultaneously with, and/or after the addition of the lipid acyltransferase described herein

Described herein is the addition of exogenous sterols/stanols, particularly phytosterols/phytostanols, to the foodstuff prior to or simultaneously with the addition of the variant enzyme described herein.

For some aspects, one or more sterols present in the foodstuff may be converted to one or more stanols prior to or at the same time as the variant lipid acyltransferase is added as described herein Any suitable method for converting sterols to stanols may be employed. For example, the conversion may be carried out by chemical hydrogenation for example. The conversion may be conducted prior to the addition of the variant lipid acyltransferase as described herein or simultaneously with the addition of the variant lipid acyltransferase as described herein Suitably enzymes for the conversion of sterol to stanols are taught in WO00/061771.

Suitably the present description may be employed to produce phytostanol esters *in situ* in a foodstuff. Phytostanol esters have increased solubility through lipid membranes, bioavailability and enhanced health benefits (see for example WO92/99640).

The stanol ester and/or the sterol ester may be a flavouring and/or a texturiser. In which instances, the present description encompasses the *in situ* production of flavourings and/or texturisers.

Described herein is a method of producing a plant sterol ester and/or stanol ester and lysolecithin in an edible oil (such as a plant oil, such as soya bean oil for instance) without the formation of free fatty acids by treatment of the oil with a variant enzyme according to the present invention. In such instances the lysolecithin so produced may be removed using a degumming process. Any degumming process may be used, such as one or more of the known degumming processes. Any free fatty acids can be removed by deodorizing if necessary. Notably, any stanol/sterol ester produced in the oil is not removed by the deodorizing process. Thus, the edible oil produced comprises sterol esters and/or stanol esters which may have beneficial nutritional and/or nutriceutical effects, such as lowering blood cholesterol levels.

Suitable oils in which this method could be carried out are those comprising *inter alia* lecithin and a sterol/stanol. Suitably, the oil is a crude oil when treated. Suitably, the edible oil may be one or more of the following: corn germ oil, cotton seed oil, linseed oil, palm oil, peanut oil, rapeseed oil, sesame oil, soybean oil, sunflower oil and wheat germ oil.

The variant lipid acyltransferase described herein may utilise a carbohydrate as the acyl acceptor. The carbohydrate acyl acceptor may be one or more of the following: a monosaccharide, a disaccharide, an oligosaccharide or a polysaccharide. Preferably, the carbohydrate is one or more of the following: glucose, fructose, anhydrofructose, maltose, lactose, sucrose, galactose, xylose, xylooligosacharides, arabinose, maltooligosaccharides, tagatose, microthecin, ascopyrone P, ascopyrone T, cortalcerone.

Suitably, the carbohydrate "acyl acceptor" may be found naturally within the foodstuff. Alternatively, the carbohydrate may be added to the foodstuff. When it is the case that the carbohydrate is added to the foodstuff, the carbohydrate may be added before, simultaneously with, and/or after the addition of the variant lipid acyltransferase described herein.

Carbohydrate esters can function as valuable emulsifiers in foodstuffs. Thus, when it is the case that the enzyme functions to transfer the acyl group to a sugar, the description encompasses the production of a second *in situ* emulsifier in the foodstuff.
The variant lipid acyltransferase may utilise both a sterol and/or stanol and a carbohydrate as an acyl acceptor.

The utilisation of a variant lipid acyltransferase which can transfer the acyl group to a carbohydrate as well as to a sterol and/or a stanol is particularly advantageous for foodstuffs comprising eggs. In particular, the presence of sugars, in particular glucose, in eggs and egg products is often seen as disadvantageous. Egg yolk may comprise up to 1% glucose. Typically, egg or egg based products may be treated with glucose oxidase to remove some or all of this glucose. However, as described herein this unwanted sugar can be readily removed by "esterifying" the sugar to form a sugar ester.
The variant lipid acyltransferase described herein may utilise a protein as the acyl acceptor. Suitably, the protein may be one or more of the proteins found in a food product, for example in a dairy product and/or a meat product. By way of example only, suitable proteins may be those found in curd or whey, such as lactoglobulin. Other suitable proteins include ovalbumin from egg, gliadin, glutenin, puroindoline, lipid transfer proteins from grains, and myosin from meat.

Preferably, the parent lipid acyltransferase enzyme described herein
may be characterised using the following criteria:

(i) the enzyme possesses acyl transferase activity which may be defined as ester transfer activity whereby the acyl part of an original ester bond of a lipid acyl donor is transferred to an acyl acceptor to form a new ester; and
(ii) the enzyme comprises the amino acid sequence motif GDSX, wherein X is one or more of the following amino acid residues L, A, V, I, F, Y, H, Q, T, N, M or S.

Preferably, X of the GDSX motif is L. Thus, preferably the enzyme described herein comprises the amino acid sequence motif GDSL.

The GDSX motif is comprised of four conserved amino acids. Preferably, the serine within the motif is a catalytic serine of the lipid acyltransferase enzyme. Suitably, the serine of the GDSX motif may be in a position corresponding to Ser-16 in *Aeromonas hydrophila* lipolytic enzyme taught in Brumlik & Buckley (Journal of Bacteriology Apr. 1996, Vol. 178, No. 7, p 2060-2064).

To determine if a protein has the GDSX motif according to the present invention, the sequence is preferably compared with the hidden markov model profiles (HMM profiles) of the pfam database.

Pfam is a database of protein domain families. Pfam contains curated multiple sequence alignments for each family as well as profile hidden Markov models (profile HMMs) for identifying these domains in new sequences. An introduction to Pfam can be found in Bateman A et al. (2002) Nucleic Acids Res. 30; 276-280. Hidden Markov models are used in a number of databases that aim at classifying proteins, for review see Bateman A and Haft DH (2002) Brief Bioinform 3; 236-245. http://www.ncbi.nlm.nih pov/entrez/query.fcgi?cmd=Retrieve&db=PubMed&list uids =1223 0032&dopt=Abstract http://www.ncbi.nlm.nih.gov/entrez/query.fcgi?cmd=Retrieve&db=PubMed&list uids =11752314&dopt=Abstract

For a detailed explanation of hidden Markov models and how they are applied in the Pfam database see Durbin R, Eddy S, and Krogh A (1998) Biological sequence analysis; probabilistic models of proteins and nucleic acids. Cambridge University Press, ISBN 0-521-62041-4. The Hammer software package can be obtained from Washington University, St Louis, USA.

Alternatively, the GDSX motif can be identified using the Hammer software package, the instructions are provided in Durbin R, Eddy S, and Krogh A (1998) Biological sequence analysis; probabilistic models of proteins and nucleic acids. Cambridge University Press, ISBN 0-521-62041-4 and the references therein, and the HMMER2 profile provided within this specification.

The PFAM database can be accessed, for example, through several servers which are currently located at the following websites.
http://www.sanger.ac.uk/Software/Pfam/index.shfml
http://pfam.wustl.edu/
http://pfam.jouy.inra.fr/
http://pfam.cgb.ki.se/

The database offers a search facility where one can enter a protein sequence. Using the default parameters of the database the protein sequence will then be analysed for the presence of Pfam domains. The GDSX domain is an established domain in the database and as such its presence in any query sequence will be recognised. The database will return the alignment of the Pfam00657 consensus sequence to the query sequence.

A multiple alignment, including *Aeromonas salmonicida* or *Aeromonas hydrophila* can be obtained by:

a) manual
obtain an alignment of the protein of interest with the Pfam00657 consensus sequence and obtain an alignment of P10480 with the Pfam00657 consensus sequence following the procedure described above;

or

b) through the database
After identification of the Pfam00657 consensus sequence the database offers the option to show an alignment of the query sequence to the seed alignment of the Pfam00657 consensus sequence. P10480 is part of this seed alignment and is indicated by GCAT_AERHY. Both the query sequence and P10480 will be displayed in the same window.

The *Aeromonas hydrophila* reference sequence:
The residues of *Aeromonas hydrophila* GDSX lipase are numbered in the NCBI file P10480, the numbers in this text refer to the numbers given in that file which as described herein, is used to determine specific amino acids residues which, as described herein are present in the lipid acyltransferase enzymes described herein.

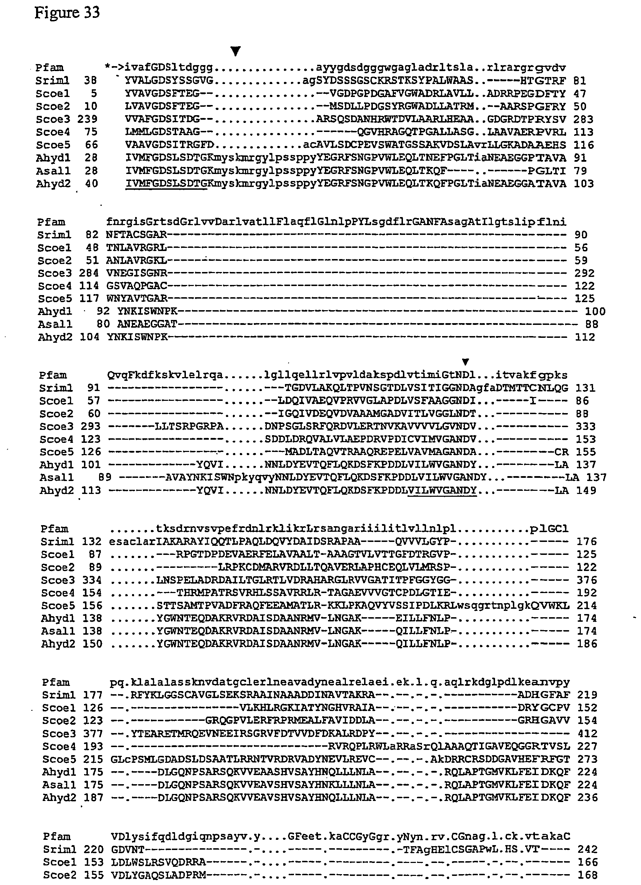

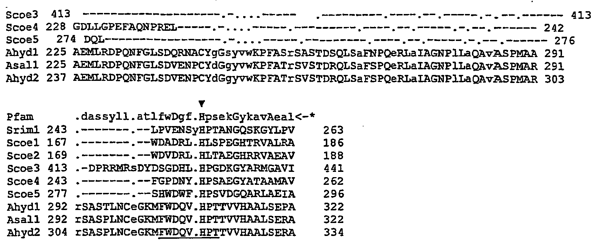

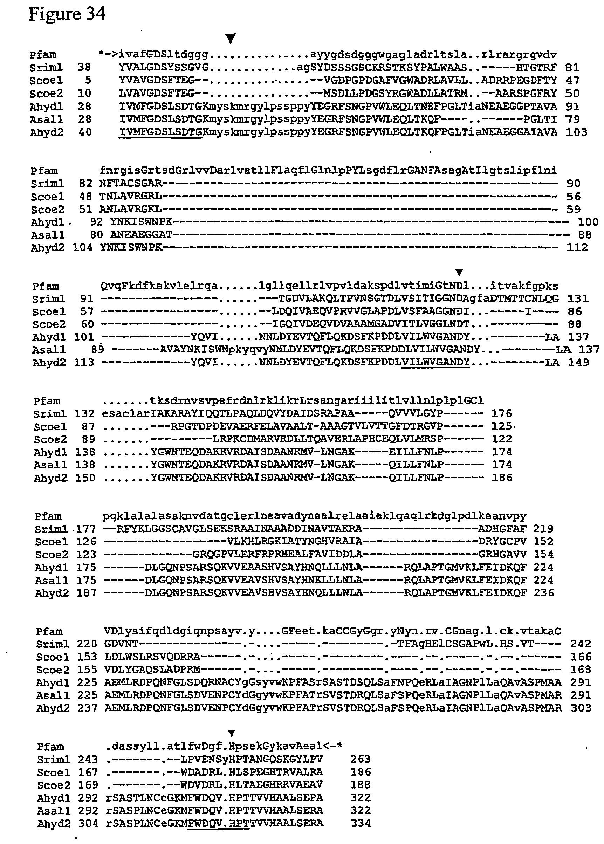

The Pfam alignment was performed (Figure 33 and Figure 34):
The following conserved residues can be recognised and may be present in the variant enzymes for use in the compositions and methods described herein;

Block 1 - GDSX block
hid hid hid hid Gly Asp Ser hid
28 29 30 31 32 33 34 35

Block 2 - GANDY block
hid Gly hid Asn Asp hid
130 131 132 133 134 135

Block 3 - HPT block
His
309

Where 'hid' means a hydrophobic residue selected from Met, Ile, Leu, Val, Ala, Gly, Cys, His, Lys, Trp, Tyr, Phe.

Preferably the parent and/or variant lipid acyltransferase enzyme for use in the compositions/methods described herein be aligned using the Pfam00657 consensus sequence.

Preferably, a positive match with the hidden markov model profile (HMM profile) of the pfam00657 domain family indicates the presence of the GDSL or GDSX domain as described herein.

Preferably when aligned with the Pfam.00657 consensus sequence the parent and/or variant lipid acyltransferase for use in the compositions/methods described herein have at least one, preferably more than one, preferably more than two, of the following, a GDSx block, a GANDY block, a HPT block. Suitably, the parent and/or variant lipid acyltransferase may have a GDSx block and a GANDY block. Alternatively, the parent and/or variant enzyme may have a GDSx block and a HPT block. Preferably the parent and/or variant enzyme comprises at least a GDSx block.

Preferably, when aligned with the Pfam00657 consensus sequence the parent and/or variant enzyme for use in the compositions/methods described herein have at least one, preferably more than one, preferably more than two, preferably more than three, preferably more than four, preferably more than five, preferably more than six, preferably more than seven, preferably more than eight, preferably more than nine, preferably more than ten, preferably more than eleven, preferably more than twelve, preferably more than thirteen, preferably more than fourteen, of the following amino acid residues when compared to the reference *A. hydrophilia* polypeptide sequence, namely SEQ ID No. 26: 28hid, 29bid, 30hid, 31hid, 32gly, 33Asp, 34Ser, 35hid, 130hid, 131Gly, 132Hid, 133Asn, 134Asp, 135hid, 309His

The pfam00657 GDSX domain is a unique identifier which distinguishes proteins possessing this domain from other enzymes.

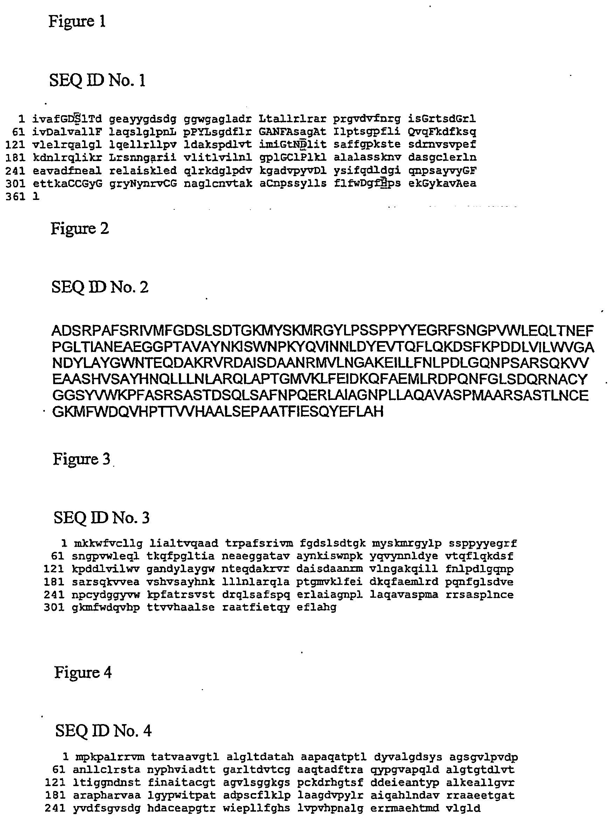

The pfam00657 consensus sequence is presented in Figure 1 as SEQ ID No. 1. This is derived from the identification of the pfam family 00657, database version 6, which may also be referred to as pfam00657.6 herein,

The consensus sequence may be updated by using further releases of the pfam database.

For example, Figures 33 and 34 show the pfam alignment of family 00657, from database version 11, which may also be referred to as pfam00657.11 herein.

The presence of the GDSx, GANDY and HPT blocks are found in the pfam family 00657 from both releases of the database. Future releases of the pfam database can be used to identify the pfam family 00657.

Preferably, the parent lipid acyltransferase enzyme described herein
may be characterised using the following criteria:

(i) the enzyme possesses acyl transferase activity which may be defined as ester transfer activity whereby the acyl part of an original ester bond of a lipid acyl donor is transferred to acyl acceptor to form a new ester;
(ii) the enzyme comprises the amino acid sequence motif GDSX, wherein X is one or more of the following amino acid residues L, A, V, I, F, Y, H, Q, T, N, M or S.;
(iii) the enzyme comprises His-309 or comprises a histidine residue at a position corresponding to His-309 in the *Aeromonas hydrophila* lipolytic enzyme shown in Figure 2 (SEQ ID No. 2 or SEQ ID No. 26).

Preferably, the amino acid residue of the GDSX motif is L.

In SEQ ID No. 26 the first 18 amino acid residues form a signal sequence. His-309 of the full length sequence, that is the protein including the signal sequence, equates to His-291 of the mature part of the protein, i.e. the sequence without the signal sequence.

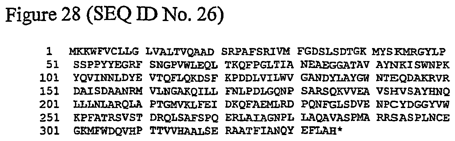

Preferably, the parent lipid acyltransferase enzyme described herein comprises the following catalytic triad: Ser-16, Asp-116 and His-291 or comprises a serine residue, an aspartic acid residue and a histidine residue, respectively, at positions corresponding to Ser-16, Asp-116 and His-291 in the *Aeromonas hydrophila* lipolytic enzyme shown in Figure 2 (SEQ ID No. 2) or at positions corresponding to Ser-34, Asp-134 and His-309 of the full length sequence shown in Figure 28 (SEQ ID No. 26). As stated above, in the sequence shown in SEQ ID No. 26 the first 18 amino acid residues form a signal sequence. Ser-34, Asp-134 and His-309 of the full length sequence, that is the protein including the signal sequence, equate to Ser-16, Asp-116 and His-291 of the mature part of the protein, i.e. the sequence without the signal sequence. In the pfam00657 consensus sequence, as given in Figure 1 (SEQ ID No. 1) the active site residues correspond to Set-7, Asp-157 and His-348.

Preferably, the parent lipid acyltransferase enzyme described herein may be characterised using the following criteria:

(i) the enzyme possesses acyl transferase activity which may be defined as ester transfer activity whereby the acyl part of an original ester bond of a first lipid acyl donor is transferred to an acyl acceptor to form a new ester; and
(ii) the enzyme comprises at least Gly-14, Asp-15, Ser-16, Asp-116 and His-191 at positions corresponding to *Aeromonas hydrophila* enzyme in Figure 2 (SEQ ID No. 2) which is equivalent to positions Gly-32, Asp-33, Ser-34, Asp-134 and His-309, respectively, in Figure 28 (SEQ ID No. 26).

Suitably, the parent lipid acyltransferase enzyme described herein
may be obtainable, preferably obtained, from organisms from one or more of the following genera: *Aeromonas, Corynebacterium, Novosphingobium, Termobifida, Streptomyces, Saccharomyces, Lactococcus, Mycobacterium, Streptococcus, Lactobacillus, Desulfitobacterium, Bacillus, Campylobacter, Vibrionaceae, Xylella, Sulfolobus, Aspergillus, Schizosaccharomyces, Listeria, Neisseria, Mesorhizobium, Ralstonia, Xanthomonas* and *Candida.*

Suitably, the parent lipid acyltransferase enzyme described herein
may be obtainable, preferably obtained, from one or more of the following organisms: *Aeromonas hydrophila, Aeromonas salmonicida, Streptomyces coelicolor, Streptomyces rimosus, Mycobacterium, Streptococcus pyogenes, Lactococcus lactis, Streptococcus pyogenes, Streptococcus thermophilus, Lactobacillus helveticus, Desulfitobacterium dehalogenans, Bacillus sp, Campylobacter jejuni, Vibrionaceae, Xylella fastidiosa, Sulfolobus solfataricus, Saccharomyces cerevisiae, Aspergillus terreus, Schizosaccharomyces pombe, Listeria innocua, Listeria monocytogenes, Neisseria meningitidis, Mesorhizobium loti, Ralstonia solanacearum, Xanthomonas campestris, Xanthomonas axonopodis, Corynebacterium efficens, Novosphingobium aromaticivorans; Termobifida fusca* and *Candida parapsilosis.*

In one aspect, preferably the parent lipid acyltransferase enzyme described herein is obtainable, preferably obtained, from one or more of *Aeromonas hydrophila* or *Aeromonas salmonicida.*

In one aspect, the parent lipid acyltransferase described herein may be a lecithin:cholesterol acyltransferases (LCAT) or variant thereof (for example a variant made by molecular evolution)

Suitable LCATs are known in the art and may be obtainable from one or more of the following organisms for example: mammals, rat, mice, chickens, *Drosophila melanogaster,* plants, including *Arabidopsis* and *Oryza sativa,* nematodes, fungi and yeast.

Preferably, when carrying out a method described herein the product (ie. foodstuff) is produced without increasing or substantially increasing the free fatty acids in the foodstuff.

The term "transferase" as used herein is interchangeable with the term "lipid acyltransferase".

The term "galactolipid transferase activity" as used herein means the ability of the enzyme to catalyse the transfer of an acyl group from a galactolipid donor to an acceptor molecule (other than water), such as glycerol for instance.

Likewise, the term "phospholipids transferase activity" as used herein means the ability of the enzyme to catalyse the transfer of an acyl group from a phospholipids donor to an acceptor molecule (other than water), such as glycerol for instance.

The term "an increased ratio of galactolipase transferase activity compared with phospholipid transferase activity" as used herein means the variant enzyme when compared with the parent enzyme is able to catalyse galactolipid transferase at a higher rate compared with phospholipid transferase. This may mean that both galactolipid transferase activity and phospholipid transferase activity are increased compared with the parent enzyme or that galactolipid transferase activity is increased whilst phospholipid transferase activity is decreased compared with the parent enzyme. It is the final relation between the two activities which is important

Suitably, the lipid acyltransferase as defined herein catalyses one or more of the following reactions: interesterification, transesterification, alcoholysis, hydrolysis.

The term "interesterification" refers to the enzymatic catalysed transfer of acyl groups between a lipid donor and lipid acceptor, wherein the lipid donor is not a free acyl group.

The term "transesterification" as used herein means the enzymatic catalysed transfer of an acyl group from a lipid donor (other than a free fatty acid) to an acyl acceptor (other than water).

As used herein, the term "alcoholysis" refers to the enzymatic cleavage of a covalent bond of an acid derivative by reaction with an alcohol ROH so that one of the products combines with the H of the alcohol and the other product combines with the OR group of the alcohol.

As used herein, the term "alcohol" refers to an alkyl compound containing a hydroxyl group.

As used herein, the term "hydrolysis" refers to the enzymatic catalysed transfer of an acyl group from a lipid to the OH group of a water molecule. Acyl transfer which results from hydrolysis requires the separation of the water molecule.

The term "galactolipid hydrolytic activity" as used herein means the the ability of the enzyme to catalyse the hydrolysis of a galactolipid by transferring an acyl group from the galactolipid to the OH group of a water molecule.

Similarly, the term "phospholipid hydrolytic activity" as used herein means the the ability of the enzyme to catalyse the hydrolysis of a phospholipid by transferring an acyl group from the phospholipid to the OH group of a water molecule.

The term "an increased ratio of galactolipase transferase activity compared with galacolipid hydrolysis activity" as used herein means the variant enzyme when compared with the parent enzyme is able to catalyse galactolipid transferase at a higher rate compared with galactolipid hydrolysis. This may mean that both galactolipid transferase activity and galactolipid hydrolysis activity are increased compared with the parent enzyme or that galactolipid transferase activity is increased whilst galactolipid hydrolysis activity is decreased compared with the parent enzyme. It is the final relation between the two activities which is important.

The term "without increasing or without substantially increasing the free fatty acids" as used herein means that preferably the lipid acyl transferase according to the present invention has 100% transferase activity (i.e. transfers 100% of the acyl groups from an acyl donor onto the acyl acceptor, with no hydrolytic activity); however, the enzyme may transfer less than 100% of the acyl groups present in the lipid acyl donor to the acyl acceptor. In which case, preferably the acyltransferase activity accounts for at least 5%, more preferably at least 10%, more preferably at least 20%, more preferably at least 30%, more preferably at least 40%, more preferably 50%, more preferably at least 60%, more preferably at least 70%, more preferably at least 80%, more preferably at least 90% and more preferably at least 98% of the total enzyme activity. The % transferase activity (i.e. the transferase activity as a percentage of the total enzymatic activity) may be determined by the following protocol:

*Protocol for the determination of % acyltransferase activity:*

A foodstuff to which a lipid acyltransferase described herein has been added may be extracted following the enzymatic reaction with $CHCL_3$:$CH_3OH$ 2:1 and the organic phase containing the lipid material is isolated and analysed by GLC according to the procedure detailed hereinbelow. From the GLC analysis (and if necessary HPLC analysis) the amount of free fatty acids and one or more of sterol/stanol esters; carbohydrate esters, protein esters; diglycerides; or monoglycerides are determined. A control foodstuff to which no enzyme described herein has been added, is analysed in the same way.

*Calculation:*

From the results of the GLC (and optionally HPLC analyses) the increase in free fatty acids and sterol/stanol esters and/or carbohydrate esters and/or protein esters and/or diglycerides and/or monoglycerides can be calculated:

Δ % fatty acid = % Fatty acid(enzyme) - % fatty acid(control); Mv fatty acid = average molecular weight of the fatty acids;
A = Δ % sterol ester/Mv sterol ester (where Δ % sterol ester - % sterol/stanol ester(enzyme) - % sterol/stanol ester(control) and Mv sterol ester = average molecular weight of the sterol/stanol esters) - applicable where the acyl acceptor is a sterol and/or stanol;
B = Δ % carbohydrate ester/Mv carbohydrate ester (where Δ % carbohydrate ester = % carbohydrate ester(enzyme) - % carbohydrate ester(control) and Mv carbohydrate ester - average molecular weight of the carbohydrate ester) - applicable where the acyl acceptor is a carbohydrate;

C = Δ % protein ester/Mv protein ester (where Δ % protein ester = % protein ester(enzyme) - % protein ester(control) and Mv protein ester = average molecular weight of the protein ester) - applicable where the acyl acceptor is a protein; and

D = absolute value of diglyceride and/or monoglyceride/Mv di/monoglyceride (where Δ% diglyceride and/or monoglyceride = % diglyceride and/or monoglyceride (enzyme) - % diglyceride and/or monoglyceride (control) and Mv di/monoglyceride = average molecular weight of the diglyceride and/or monoglyceride) - applicable where the acyl acceptor is glycerol.

The transferase activity is calculated as a percentage of the total enzymatic activity:

$$\text{\% transferase activity} = \frac{A^* + B^* + C^* + D^* \times 100}{A^* + B^* + C^* + D^* + \Delta\ \text{\% fatty acid/(Mv fatty acid)}}.$$

* - delete as appropriate.

The amino acids which fall within the terms "non-polar", "polar - uncharged", "polar - charged" are given in the table below, as are the amino acids falling within the terms "aliphatic" and "aromatic". The term "polar" refers to both "polar - uncharged" and "polar - charged" amino acids.

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

GLC analysis

Perkin Elmer Autosystem 9000 Capillary Gas Chromatograph equipped with WCOT fused silica column 12.5 m x 0.25 mm ID x 0.1 μ film thickness 5% phenyl-methylsilicone (CP Si1 8 CB from Chrompack).

Carrier gas: Helium.

Injector. PSSI cold split injection (initial temp 50°C heated to 385°C), volume 1.0μl Detector FID: 395°C

| | | | |
|---|---|---|---|
| Oven program: | 1 | 2 | 3 |
| Oven temperature, °C. | 90 | 280 | 350 |
| Isothermal, time, min. | 1 | 0 | 10 |
| Temperature rate, °C/min. | 15 | 4 | |

Sample preparation: 30 mg of sample was dissolved in 9 ml Heptane:Pyridin, 2:1 containing internal standard heptadecane, 0.5 mg/ml. 300μl sample solution was transferred to a crimp vial, 300 μl MSTFA (N-Methyl-N-trimethylsilyl-trifluoraceamid) was added and reacted for 20 minutes at 60°C.

Calculation: Response factors for mono-di-triglycerides and free fatty acid were determined from Standard 2 (mono-di-triglyceride), for Cholesterol, Cholesteryl palmitate and Cholesteryl stearate the response factors were determined from pure reference material (weighing for pure material 10mg).

TECHNICAL EFFECTS

Described herein are one or more of the following unexpected technical effects in egg products, particularly mayonnaise: an improved heat stability during pasteurisation; improved organoleptic properties, an improved consistency.

Variant enzymes with increased phospholipid transferase activity, particularly with increased tranferase activity between a phospholipid and a sterol and/or stanol, such as cholesterol, may be particularly useful in methods for producing lysophospholipids and/or for enzymatic degumming of edible oils and/or for the production of egg products with improved emulsification properties and/or health benefits.

For use in methods of enzymatic degumming, variants with an increased absolute phospholipid transferase to sterol activity are preferred.

Suitably, described herein are one or more of the following unexpected technical effects in egg or in egg products: improved stability of emulsion; thermal stability of emulsion; improved flavour; reduced mal-odour; improved thickening properties, improved consistency.

Further described herein are one or more of the following unexpected technical effects in dough and/or baked products: an improved specific volume of either the dough or the baked products (for example of bread and/or of cake); an improved dough stability; an improved crust score (for example a thinner and/or crispier bread crust), an improved crumb score (for example a more homogenous crumb distribution and/or a finer crumb structure and/or a softer crumb); an improved appearance (for example a smooth surface without blisters or holes or substantially without blisters or holes); a reduced staling; an enhanced softness; an improved odour; an improved taste.

Suitably, described herein are one or more of the following unexpected technical effects in a foodstuff: an improved appearance, an improved mouthfeel, an improved stability, in particular an improved thermal stability, an improved taste, an improved softness, an improved resilience, an improved emulsification.

Suitably, described herein are one or more of the following unexpected technical effects in dairy products, such as ice cream for example: an improved mouthfeel (preferably a more creamy mouthfeel); an improved taste; an improved meltdown.

Specific technical effects associated with the use of a lipid acyltransferase as defined herein in the preparation of a foodstuff are listed in the table below:

| | Foodstuff | Effect |
|---|---|---|
| 1 | Bread, Muffins and Doughnuts | Strengthens dough and increases mechanical resistance and increases water absorption capacity and/or increases volume of bakery products and maintains softness of crumb and/or reduces blisters on the bread surface. |
| 2 | Frozen dough | Prevents spoiling during refrigeration |
| 3 | Sponge cake | Makes good cake volume and/or a uniform soft texture |
| 4 | Biscuit, cracker and cookie | Makes stable emulsions of fat and/or prevents stickiness to the machine and/or prevents blooming of high fat products |
| 5 | Batter and breading | Improves texture of fried products. |
| 6 | Noodles | Prevents dough from sticking to the machine and/or increases water content, and/or decreases cooking loss |
| 7 | Instant noodles | Prevent noodles form adhering to each other |
| 8 | Pasta | Dough conditioner prevents adhesion on cooking. |
| 9 | Custard cream | Makes starch paste with a smooth and creamy texture, and/or prevents dehydration. |
| 10 | Coffee whitener | Prevent oil and water separation |
| 11 | Whipping cream | Provides stable emulsion |
| 12 | Chocolate | Prevents or reduced blooming |
| 13 | Caramel, candy and nougat | Improves emulsification of molten sugar and oil and/or prevents separation of oil. |
| 14 | Processed meat, sausages | Improves water holding capacity of sausages and pressed ham, and/or prevents separation of oil phase of pastes and pâté. |

Suitably, described herein are one or more of the following unexpected technical effects in cheese: a decrease in the oiling-off effect in cheese; an increase in cheese yield; an improvement in flavour; a reduced mal-odour; a reduced "soapy" taste.

In one aspect, the present description is based in part on the realisation that yields of foods - such as cheese may be improved by the use of a lipid acyl transferase. In addition or alternatively, the flavour, texture, oxidative stability and/or shelf life of the food may be improved. In addition or alternatively, the food may have a reduced cholesterol level or enhanced content of phytosterol/stanol esters.

Described herein is a food additive composition comprising a lipid acyl transferase as defined herein.

Described herein is a cosmetic composition comprising a lipid acyl transferase as defined herein.

In addition, described herein is the use of an acyltransferase as defined herein to produce a cosmetic composition.

ADVANTAGES

Valiant transferases described herein have one or more of the following advantageous properties compared with the parent enzyme:

i) an increased activity on polar lipids and/or an increased activity on polar lipids compared to triglycerides.

ii) an increased activity on galactolipids (glycolipids), such as one or more of digalactosyl diglyceride (DGDG) and/or monogalactosyl diglyceride (MGDG).

iii) an increased ratio of activity on galactolipids (glycolipids) compared to either phospholipids and/or triglycerides

Preferably variant transferases described herein have increased activity on digalactosyl diglyceride (DGDG) and/or monogalactosyl diglyceride (MGDG).

Preferably variant transferases described herein has increased activity on DGDG and/or MGDG and decreased activity on DGMG and/or MGMG.

The variant transferases described herein may also have an increased activity on triglycerides.

The variant transferases described herein may also have an increased activity on phospholipids, such as lecithin, including phosphatidyl choline.

Variant transferases described herein may have decreased activity on triglycerides, and/or monoglycerides and/or diglycerides.

The term polar lipid refers to the polar lipids usually found in a dough, preferably galactolipids and phospholipids.

When used in preparation of a dough or baked product the variant transferase described herein may result in one or more of the following unexpected technical effects in dough and/or baked products: an improved specific volume of either the dough or the baked products (for example of bread and/or of cake); an improved dough stability; an improved crust score (for example a thinner and/or crispier bread crust), an improved crumb score (for example a more homogenous crumb distribution and/or a finer crumb structure and/or a softer crumb); an improved appearance (for example a smooth surface without blisters or holes or substantially without blisters or holes); a reduced staling; an enhanced softness; an improved odour; an improved taste.

ISOLATED

In one aspect, preferably the polypeptide or protein for use as described herein is in an isolated form. The term "isolated" means that the sequence is at least substantially free from at least one other component with which the sequence is naturally associated in nature and as found in nature.

PURIFIED

In one aspect, preferably the polypeptide or protein for use as described herein is in a purified form. The term "purified" means that the sequence is in a relatively pure state - e.g. at least about 51% pure, or at least about 75%, or at least about 80%, or at least about 90% pure, or at least about 95% pure or at least about 98% pure.

CLONING A NUCLEOTIDE SEQUENCE ENCODING A POLYPEPTIDE DESCRIBED HEREIN

A nucleotide sequence encoding either a polypeptide which has the specific properties as defined herein or a polypeptide which is suitable for modification may be isolated from any cell or organism producing said polypeptide. Various methods are well known within the art for the isolation of nucleotide sequences.

For example, a genomic DNA and/or cDNA library may be constructed using chromosomal DNA or messenger RNA from the organism producing the polypeptide. If the amino acid sequence of the polypeptide is known, labelled oligonucleotide probes may be synthesised and used to identify polypeptide-encoding clones from the genomic library prepared from the organism. Alternatively, a labelled oligonucleotide probe containing sequences homologous to another known polypeptide gene could be used to identify polypeptide-encoding clones. In the latter case, hybridisation and washing conditions of lower stringency are used.

Alternatively, polypeptide-encoding clones could be identified by inserting fragments of genomic DNA into an expression vector, such as a plasmid, transforming enzyme-negative bacteria with the resulting genomic DNA library, and then plating the transformed bacteria onto agar containing an enzyme inhibited by the polypeptide, thereby allowing clones expressing the polypeptide to be identified.

In a yet further alternative, the nucleotide sequence encoding the polypeptide may be prepared synthetically by established standard methods, e.g. the phosphoroamidite method described by Beucage S.L. et al (1981) Tetrahedron Letters 22, p 1859-1869, or the method described by Matthes et al (1984) EMBO J. 3, p 801-805. In the phosphoroamidite method, oligonucleotides are synthesised, e.g. in an automatic DNA synthesiser, purified, annealed, ligated and cloned in appropriate vectors.

The nucleotide sequence may be of mixed genomic and synthetic origin, mixed synthetic and cDNA origin, or mixed genomic and cDNA origin, prepared by ligating fragments of synthetic, genomic or cDNA origin (as appropriate) in accordance with standard techniques. Each ligated fragment corresponds to various parts of the entire nucleotide sequence. The DNA sequence may also be prepared by polymerase chain reaction (PCR) using specific primers, for instance as described in US 4,683,202 or in Saiki R K et al (Science (1988) 239, pp 487-491).

NUCLEOTIDE SEQUENCES

Described herein are nucleotide sequences encoding polypeptides having the specific properties as defined herein. The term "nucleotide sequence" as used herein refers to an oligonucleotide sequence or polynucleotide sequence, and variant, homologues, fragments and derivatives thereof (such as portions thereof). The nucleotide sequence may be of genomic or synthetic or recombinant origin, which may be doublestranded or single-stranded whether representing the sense or antisense strand.

The term "nucleotide sequence" as used herein includes genomic DNA, cDNA, synthetic DNA, and RNA. Preferably it means DNA, more preferably cDNA for the coding sequence.

Preferably the nucleotide sequence *per se* encoding a polypeptide having the specific properties as defined herein does not cover the native nucleotide sequence in its natural environment when it is linked to its naturally associated sequence(s) that is/are also in its/their natural environment. For ease of reference, we shall call this preferred embodiment the "non-native nucleotide sequence". In this regard, the term "native nucleotide sequence" means an entire nucleotide sequence that is in its native environment and when operatively linked to an entire promoter with which it is naturally associated, which promoter is also in its native environment. Thus, the polypeptide of the present invention can be expressed by a nucleotide sequence in its native organism but wherein the nucleotide sequence is not under the control of the promoter with which it is naturally associated within that organism.

Preferably the polypeptide is not a native polypeptide. In this regard, the term "native polypeptide" means an entire polypeptide that is in its native environment and when it has been expressed by its native nucleotide sequence.

Typically, the nucleotide sequence encoding polypeptides having the specific properties as defined herein is prepared using recombinant DNA techniques (i.e. recombinant DNA). However, the nucleotide sequence could be synthesised, in whole or in part, using chemical methods well known in the art (see Caruthers MH et al (1980) Nuc Acids Res Symp Ser 215-23 and Horn T et al (1980) Nuc Acids Res Symp Ser 225-232).

MOLECULAR EVOLUTION

Once an enzyme-encoding nucleotide sequence has been isolated, or a putative enzyme-encoding nucleotide sequence has been identified, it may be desirable to modify the selected nucleotide sequence, for example it may be desirable to mutate the sequence in order to prepare an enzyme described herein

Mutations may be introduced using synthetic oligonucleotides. These oligonucleotides contain nucleotide sequences flanking the desired mutation sites.

A suitable method is disclosed in Morinaga et al (Biotechnology (1984) 2, p646-649). Another method of introducing mutations into enzyme-encoding nucleotide sequences is described in Nelson and Long (Analytical Biochemistry (1989), 180, p 147-151).

Instead of site directed mutagenesis, such as described above, one can introduce mutations randomly for instance using a commercial kit such as the GeneMorph PCR mutagenesis kit from Stratagene, or the Diversify PCR random mutagenesis kit from Clontech. EP 0 583 265 refers to methods of optimising PCR based mutagenesis, which can also be combined with the use of mutagenic DNA analogues such as those described in EP 0 866 796. Error prone PCR technologies are suitable for the production of variants of lipid acyl transferases with preferred characterisitics. WO0206457 refers to molecular evolution of lipases.

A third method to obtain novel sequences is to fragment non-identical nucleotide sequences, either by using any number of restriction enzymes or an enzyme such as Dnase I, and reassembling full nucleotide sequences coding for functional proteins. Alternatively one can use one or multiple non-identical nucleotide sequences and introduce mutations during the reassembly of the full nucleotide sequence. DNA shuffling and family shuffling technologies are suitable for the production of variants of lipid acyl transferases with preferred characteristics. Suitable methods for performing 'shuffling' can be found in EP0 752 008, EP1 138 763, EP1 103 606. Shuffling can also be combined with other forms of DNA mutagenesis as described in US 6,180,406 and WO 01/34835.

Thus, it is possible to produce numerous site directed or random mutations into a nucleotide sequence, either *in vivo* or *in vitro,* and to subsequently screen for improved functionality of the encoded polypeptide by various means. Using in silico and exo mediated recombination methods (see WO 00/58517, US 6,344,328, US 6,361,974), for example, molecular evolution can be performed where the variant produced retains very low homology to known enzymes or proteins. Such variants thereby obtained may have significant structural analogy to known transferase enzymes, but have very low amino acid sequence homology.

As a non-limiting example, In addition, mutations or natural variants of a polynucleotide sequence can be recombined with either the wild type or other mutations or natural variants to produce new variants, Such new variants can also be screened for improved functionality of the encoded polypeptide.

The application, of the above-mentioned and similar molecular evolution methods allows the identification and selection of variants of the enzymes described herein which have preferred characteristics without any prior knowledge of protein structure or function, and allows the production of non-predictable but beneficial mutations or variants. There are numerous examples of the application of molecular evolution in the art for the optimisation or alteration of enzyme activity, such examples include, but are not limited to one or more of the following: optimised expression and/or activity in a host cell or in vitro, increased enzymatic activity, altered substrate and/or product specificity, increased or decreased enzymatic or structural stability, altered enzymatic activity/specificity in preferred environmental conditions, e.g. temperature, pH, substrate

As will be apparent to a person skilled in the art, using molecular evolution tools an enzyme may be altered to improve the functionality of the enzyme.

Suitably, the lipid acyltransferase used as described herein may be a variant, i.e. may contain at least one amino acid substitution, deletion or addition, when compared to a parental enzyme. Variant enzymes retain at least 25%, 30%, 40%, 50 %, 60%, 70%, 80%, 90%, 95%, 97%, 99% homology with the parent enzyme. Suitable parent enzymes may include any enzyme with esterase or lipase activity. Preferably, the parent enzyme aligns to the pfam00657 consensus sequence.

A variant lipid acyltransferase enzyme may retain or incorporates at least one or more of the pfam00657 consensus sequence amino acid residues found in the GDSx, GANDY and HPT blocks.

Enzymes, such as lipases with no or low lipid acyltransferase activity in an aqueous environment may be mutated using molecular evolution tools to introduce or enhance the transferase activity, thereby producing a lipid acyltransferase enzyme with significant transferase activity suitable for use in the compositions and methods described herein.

Suitably, the lipid acyltransferase for use as described herein may be a variant with enhanced enzyme activity on polar lipids, preferably phospholipids and/or glycolipids when compared to the parent enzyme. Preferably, such variants also have low or no activity on lyso polar lipids. The enhanced activity on polar lipids, phospholipids and/or glycolipids may be the result of hydrolysis and/or transferase activity or a combination of both.

Variant lipid acyltransferases for use as described herein may have decreased activity on triglycerides, and/or monoglycerides and/or diglycerides compared with the parent enzyme.

Suitably the variant enzyme may have no activity on triglycerides and/or monoglycerides and/or diglycerides.

Alternatively, the variant enzyme for use as described herein may have increased activity on triglycerides, and/or may also have increased activity on one or more of the following, polar lipids, phospholipids, lecithin, phosphatidylcholine, glycolipids, digalactosyl monoglyceride, monogalactosyl monoglyceride.

Variants of lipid acyltransferases are known, and one or more of such variants may be suitable for use in the methods and uses described herein and/or in the enzyme compositions described herein By way of example only, variants of lipid acyltransferases are described in the following references may be used as described herein : Hilton & Buckley J Biol. Chem. 1991 Jan 15: 266 (2): 997-1000; Robertson et al J. Biol. Chem. 1994 Jan 21; 269(3):2146-50; Brumlik et al J. Bacteriol 1996 Apr; 178 (7): 2060-4; Peelman et al Protein Sci. 1998 Mar; 7(3):587-99.

AMINO ACID SEQUENCES

Also described herein are amino acid sequences of polypeptides having the specific properties as defined herein.

As used herein, the term "amino acid sequence" is synonymous with the term "polypeptide" and/or the term "protein". In some instances, the term "amino acid sequence" is synonymous with the term "peptide".

The amino acid sequence may be prepared/isolated from a suitable source, or it may be made synthetically or it may be prepared by use of recombinant DNA techniques.

Suitably, the amino acid sequences may be obtained from the isolated polypeptides taught herein by standard techniques.

One suitable method for determining amino acid sequences from isolated polypeptides is as follows: Purified polypeptide may be freeze-dried and 100 μg of the freeze-dried material may be dissolved in 50 μl of a mixture of 8 M urea and 0.4 M ammonium hydrogen carbonate, pH 8.4. The dissolved protein may be denatured and reduced for 15 minutes at 50°C following overlay with nitrogen and addition of 5 μl of 45 mM dithiothreitol. After cooling to room temperature, 5 μl of 100 mM iodoacetamide may be added for the cysteine residues to be derivatized for 15 minutes at room temperature in the dark under nitrogen.

135 μl of water and 5 μg of endoproteinase Lys-C in 5 μl of water may be added to the above reaction mixture and the digestion may be carried out at 37°C under nitrogen for 24 hours.

The resulting peptides may be separated by reverse phase HPLC on a VYDAC C18 column (0.46x15cm;10μm; The Separation Group, California, USA) using solvent A: 0,1% TFA in water and solvent B: 0.1% TFA in acetonitrile. Selected peptides may be re-chromatographed on a Develosil C18 column using the same solvent system, prior to N-terminal sequencing. Sequencing may be done using an Applied Biosystems 476A sequencer using pulsed liquid fast cycles according to the manufacturer's instructions (Applied Biosystems, California, USA).

SEQUENCE IDENTITY OR SEQUENCE HOMOLOGY

Also described herein is the use of sequences having a degree of sequence identity or sequence homology with amino acid sequence(s) of a polypeptide having the specific properties defined herein or of any nucleotide sequence encoding such a polypeptide (hereinafter referred to as a "homologous sequence(s)"). Here, the term "homologue" means an entity having a certain homology with the subject amino acid sequences and the subject nucleotide sequences. Here, the term "homology" can be equated with "identity".

The homologous amino acid sequence and/or nucleotide sequence should provide and/or encode a polypeptide which retains the functional activity and/or enhances the activity of the enzyme.

In the present context, a homologous sequence is taken to include an amino acid sequence which may be at least 75, 85 or 90% identical, preferably at least 95 or 98% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, a homologous sequence is taken to include a nucleotide sequence which may be at least 75, 85 or 90% identical, preferably at least 95 or 98% identical to a nucleotide sequence encoding a polypeptide of the present invention (the subject sequence). Typically, the homologues will comprise the same sequences that code for the active sites etc. as the subject sequence. Although homology can also be considered in terms of similarity (i.e. amino acid residues having similar chemical properties/functions), in the context of the present invention it is preferred to express homology in terms of sequence identity.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence is directly compared with the corresponding amino acid in the other sequence, one residue at a time. This is called an "ungapped" alignment Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (Devereux et al 1984 Nuc. Acids Research 12 p387). Examples of other software that can perform sequence comparisons include, but are not limited to, the BLAST package (see Ausubel et al 1999 Short Protocols in Molecular Biology, 4th Ed - Chapter 18), FASTA (Altschul et al 1990 J. Mol. Biol. 403-410) and the GENEWORKS suite of comparison tools. Both BLAST and FASTA are available for offline and online searching (see Ausubel *et al* 1999, pages 7-58 to 7-60). However, for some applications, it is preferred to use the GCG Bestfit program. A new tool, called BLAST 2 Sequences is also available for comparing protein and nucleotide sequence (see FEMS Microbiol Lett 1999 174(2): 247-50; FEMS Microbiol Lett 1999 177(1): 187-8 and tatiana@ncbi.nlm.nih.gov).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). For some applications, it is preferred to use the public default values for the GCG package, or in the case of other software, the default matrix, such as BLOSUM62.

Alternatively, percentage homologies may be calculated using the multiple alignment feature in DNASIS™ (Hitachi Software), based on an algorithm, analogous to CLUSTAL (Higgins DG & Sharp PM (1988), Gene 73(1), 237-244).

Once the software has produced an optimal alignment, it is possible to calculate % homology, preferably % sequence identity. The software typically does this as part of the sequence comparison and generates a numerical result.

The sequences may also have deletions, insertions or substitutions of amino acid residues which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

Also described herein is homologous substitution (substitution and replacement are both used herein to mean the interchange of an existing amino acid residue, with an alternative residue) that may occur i.e. like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by unnatural amino acids.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, involves the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134.

Nucleotide sequences for use as described herein or encoding a polypeptide having the specific properties defined herein may include within them synthetic or modified nucleotides. A number of different types of modification to oligonucleotides are known in the art. These include methylphosphonate and phosphorothioate backbones and/or the addition of acridine or polylysine chains at the 3' and/or 5' ends of the molecule. For the purposes of the present invention, it is to be understood that the nucleotide sequences described herein may be modified by any method available in the art. Such modifications may be carried out in order to enhance the *in vivo* activity or life span of nucleotide sequences.
Also described herein is the use of nucleotide sequences that are complementary to the sequences discussed herein, or any derivative, fragment or derivative thereof. If the sequence is complementary to a fragment thereof then that sequence can be used as a probe to identify similar coding sequences in other organisms etc.
Polynucleotides which are not 100% homologous to the sequences described herein can be obtained in a number of ways. Other variants of the sequences described herein may be obtained for example by probing DNA libraries made from a range of individuals, for example individuals from different populations. In addition, other viral/bacterial, or cellular homologues particularly cellular homologues found in mammalian cells (e.g. rat, mouse, bovine and primate cells), may be obtained and such homologues and fragments thereof in general will be capable of selectively hybridising to the sequences shown in the sequence listing herein. Such sequences may be obtained by probing cDNA libraries made from or genomic DNA libraries from other animal species, and probing such libraries with probes comprising all or part of any one of the sequences in the attached sequence listings under conditions of medium to high stringency. Similar considerations apply to obtaining species homologues and allelic variants of the polypeptide or nucleotide sequences of the invention.
Variants and strain/species homologues may also be obtained using degenerate PCR which will use primers designed to target sequences within the variants and homologues encoding conserved amino acid sequences within the sequences of the present invention. Conserved sequences can be predicted, for example, by aligning the amino acid sequences from several variants/homologues. Sequence alignments can be performed using computer software known in the art. For example the GCG Wisconsin PileUp program is widely used.
The primers used in degenerate PCR will contain one or more degenerate positions and will be used at stringency conditions lower than those used for cloning sequences with single sequence primers against known sequences.
Alternatively, such polynucleotides may be obtained by site directed mutagenesis of characterised sequences. This may be useful where for example silent codon sequence changes are required to optimise codon preferences for a particular host cell in which the polynucleotide sequences are being expressed. Other sequence changes may be desired in order to introduce restriction polypeptide recognition sites, or to alter the property or function of the polypeptides encoded by the polynucleotides.
Polynucleotides (nucleotide sequences) described herein may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 15, preferably at least 20, for example at least 25, 30 or 40 nucleotides in length, and are also encompassed by the term polynucleotides of the invention as used herein.
Polynucleotides such as DNA polynucleotides and probes described herein may be produced recombinantly, synthetically, or by any means available to those of skill in the art. They may also be cloned by standard techniques.
In general, primers will be produced by synthetic means, involving a stepwise manufacture of the desired nucleic acid sequence one nucleotide at a time. Techniques for accomplishing this using automated techniques are readily available in the art.
Longer polynucleotides will generally be produced using recombinant means, for example using a PCR (polymerase chain reaction) cloning techniques. This will involve making a pair of primers (e.g. of about 15 to 30 nucleotides) flanking a region of the lipid targeting sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

## HYBRIDISATION

Described herein are sequences that are complementary to the sequences described herein or sequences that are capable of hybridising either to the sequences described herein or to sequences that are complementary thereto.
The term "hybridisation" as used herein shall include "the process by which a strand of nucleic acid joins with a complementary strand through base pairing" as well as the process of amplification as carried out in polymerase chain reaction (PCR) technologies.
Also described herein is the use of nucleotide sequences that are capable of hybridising to the sequences that are complementary to the subject sequences discussed herein, or any derivative, fragment or derivative thereof.

Also described herein are sequences that are complementary to sequences that are capable of hybridising to the nucleotide sequences discussed herein. Hybridisation conditions are based on the melting temperature (Tm) of the nucleotide binding complex, as taught in Berger and Kimmel (1987, Guide to Molecular Cloning Techniques, Methods in Enzymology, Vol. 152, Academic Press, San Diego CA), and confer a defined "stringency" as explained below. Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridisation can be used to identify or detect identical nucleotide sequences while an intermediate (or low) stringency hybridisation can be used to identify or detect similar or related polynucleotide sequences.

Described herein are sequences that are complementary to sequences that are capable of hybridising under high stringency conditions or intermediate stringency conditions to nucleotide sequences encoding polypeptides having the specific properties as defined herein.

Described herein are sequences that are complementary to sequences that are capable of hybridising under high stringent conditions (e.g. 65°C and 0.1xSSC {1xSSC = 0.15 M NaCl, 0.015 M Na-citrate pH 7.0}) to nucleotide sequences encoding polypeptides having the specific properties as defined herein.

The present description also relates to nucleotide sequences that can hybridise to the nucleotide sequences discussed herein (including complementary sequences of those discussed herein).

The present description also relates to nucleotide sequences that are complementary to sequences that can hybridise to the nucleotide sequences discussed herein (including complementary sequences of those discussed herein).

Also described herein are polynucleotide sequences that are capable of hybridising to the nucleotide sequences discussed herein under conditions of intermediate to maximal stringency.

Described herein are nucleotide sequences that can hybridise to the nucleotide sequences discussed herein, or the complement thereof, under stringent conditions (e.g. 50°C and 0.2xSSC).

Described herein are nucleotide sequences that can hybridise to the nucleotide sequences discussed herein, or the complement thereof, under high stringent conditions (e.g. 65°C and 0.1xSSC).

## EXPRESSION OF POLYPEPTIDES

A nucleotide sequence for use as described herein or for encoding a polypeptide having the specific properties as defined herein can be incorporated into a recombinant replicable vector. The vector may be used to replicate and express the nucleotide sequence, in polypeptide form, in and/or from a compatible host cell. Expression may be controlled using control sequences which include promoters/enhancers and other expression regulation signals. Prokaryotic promoters and promoters functional in eukaryotic cells may be used. Tissue specific or stimuli specific promoters may be used. Chimeric promoters may also be used comprising sequence elements from two or more different promoters described above.

The polypeptide produced by a host recombinant cell by expression of the nucleotide sequence may be secreted or may be contained intracellularly depending on the sequence and/or the vector used. The coding sequences can be designed with signal sequences which direct secretion of the substance coding sequences through a particular prokaryotic or eukaryotic cell membrane.

## EXPRESSION VECTOR

The term "expression vector" means a construct capable of *in vivo* or *in vitro* expression.

Preferably, the expression vector is incorporated in the genome of the organism. The term "incorporated" preferably covers stable incorporation into the genome.

The nucleotide sequence described herein or coding for a polypeptide having the specific properties as defined herein may be present in a vector, in which the nucleotide sequence is operably linked to regulatory sequences such that the regulatory sequences are capable of providing the expression of the nucleotide sequence by a suitable host organism, i.e. the vector is an expression vector.

The vectors described herein may be transformed into a suitable host cell as described below to provide for expression of a polypeptide having the specific properties as defined herein.

The choice of vector, e.g. plasmid, cosmid, virus or phage vector, will often depend on the host cell into which it is to be introduced.

The vectors may contain one or more selectable marker genes - such as a gene which confers antibiotic resistance e.g. ampicillin, kanamycin, chloramphenicol or tetracyclin resistance. Alternatively, the selection may be accomplished by co-transformation (as described in WO91/17243).

Vectors may be used *in vitro,* for example for the production of RNA or used to transfect or transform a host cell.

Thus, also described herein is a method of making nucleotide sequences described herein or nucleotide sequences encoding polypeptides having the specific properties as defined herein by introducing a nucleotide sequence into a replicable vector, introducing the vector into a compatible host cell, and growing the host cell under conditions which bring about replication of the vector.
The vector may further comprise a nucleotide sequence enabling the vector to replicate in the host cell in question. Examples of such sequences are the origins of replication of plasmids pUC19, pACYC177, pUB110, pE194, pAMB1 and pIJ702.

REGULATORY SEQUENCES

In some applications, a nucleotide sequence for use as described herein or a nucleotide sequence encoding a polypeptide having the specific properties as defined herein may be operably linked to a regulatory sequence which is capable of providing for the expression of the nucleotide sequence, such as by the chosen host cell. By way of example, the description covers a vector comprising the nucleotide sequence described herein may be operably linked to such a regulatory sequence, i.e. the vector is an expression vector.
The term "operably linked" refers to a juxtaposition wherein the components described are in a relationship permitting them to function in their intended manner. A regulatory sequence "operably linked" to a coding sequence is ligated in such a way that expression of the coding sequence is achieved under conditions compatible with the control sequences.
The term "regulatory sequences" includes promoters and enhancers and other expression regulation signals.
The term "promoter" is used in the normal sense of the art, e.g. an RNA polymerase binding site.
Enhanced expression of the nucleotide sequence encoding the enzyme having the specific properties as defined herein may also be achieved by the selection of heterologous regulatory regions, e.g. promoter, secretion leader and terminator regions.
Preferably, the nucleotide sequence described herein may be operably linked to at least a promoter.
Examples of suitable promoters for directing the transcription of the nucleotide sequence in a bacterial, fungal or yeast host are well known in the art.

CONSTRUCTS

The term "construct" - which is synonymous with terms such as "conjugate", "cassette" and "hybrid" - includes a nucleotide sequence encoding a polypeptide having the specific properties as defined herein for use as described herein directly or indirectly attached to a promoter. An example of an indirect attachment is the provision of a suitable spacer group such as an intron sequence, such as the Sh1-intron or the ADH intron, intermediate the promoter and the nucleotide sequence described herein The same is true for the term "fused" as used herein which includes direct or indirect attachment. In some cases, the terms do not cover the natural combination of the nucleotide sequence coding for the protein ordinarily associated with the wild type gene promoter and when they are both in their natural environment.
The construct may even contain or express a marker which allows for the selection of the genetic construct.
For some applications, preferably the construct comprises at least a nucleotide sequence described herein or a nucleotide sequence encoding a polypeptide having the specific properties as defined herein operably linked to a promoter.

HOST CELLS

The term "host cell" as used herein includes any cell that comprises either a nucleotide sequence encoding a polypeptide having the specific properties as defined herein or an expression vector as described above and which is used in the recombinant production of a polypeptide having the specific properties as defined herein.
Thus, further described herein are host cells transformed or transfected with a nucleotide sequence described herein or a nucleotide sequence that expresses a polypeptide having the specific properties as defined herein. The cells will be chosen to be compatible with the said vector and may for example be prokaryotic (for example bacterial), fungal, yeast or plant cells. Preferably, the host cells are not human cells.
Examples of suitable bacterial host organisms are gram negative bacterium or gram positive bacteria.
Depending on the nature of the nucleotide sequence encoding a polypeptide having the specific properties as defined herein, and/or the desirability for further processing of the expressed protein, eukaryotic hosts such as yeasts or other fungi may be preferred. In general, yeast cells are preferred over fungal cells because they are easier to manipulate. However, some proteins are either poorly secreted from the yeast cell, or in some cases are not processed properly (e.g. hyperglycosylation in yeast). In these instances, a different fungal host organism should be selected.
The use of suitable host cells, such as yeast, fungal and plant host cells - may provide for post-translational modifications (e.g. myristoylation, glycosylation, truncation, lapidation and tyrosine, serine or threonine phosphorylation) as may be needed to confer optimal biological activity on recombinant expression products of the present invention.

The host cell may be a protease deficient or protease minus strain.

ORGANISM

The term "organism" as used herein includes any organism that could comprise a nucleotide sequence described herein or a nucleotide sequence encoding for a polypeptide having the specific properties as defined herein and/or products obtained therefrom.
Suitable organisms may include a prokaryote, fungus, yeast or a plant.
The term "transgenic organism" as used herein includes any organism that comprises a nucleotide sequence coding for a polypeptide having the specific properties as defined herein and/or the products obtained therefrom, and/or wherein a promoter can allow expression of the nucleotide sequence coding for a polypeptide having the specific properties as defined herein within the organism. Preferably the nucleotide sequence is incorporated in the genome of the organism.
The term "transgenic organism" does not cover native nucleotide coding sequences in their natural environment when they are under the control of their native promoter which is also in its natural environment.
Therefore, the transgenic organism described herein includes an organism comprising any one of, or combinations of, a nucleotide sequence coding for a polypeptide having the specific properties as defined herein, constructs as defined herein, vectors as defined herein, plasmids as defined herein, cells as defined herein, or the products thereof. For example the transgenic organism can also comprise a nucleotide sequence coding for a polypeptide having the specific properties as defined herein under the control of a heterologous promoter.

TRANSFORMATION OF HOST CELLS/ORGANISM

As indicated earlier, the host organism can be a prokaryotic or a eukaryotic organism. Examples of suitable prokaryotic hosts include *E. coli* and *Bacillus subtilis.*
Teachings on the transformation of prokaryotic hosts is well documented in the art, for example see Sambrook et al (Molecular Cloning: A Laboratory Manual, 2nd edition, 1989, Cold Spring Harbor Laboratory Press). If a prokaryotic host is used then the nucleotide sequence may need to be suitably modified before transformation - such as by removal of introns.
The transgenic organism can be a yeast.
Filamentous fungi cells may be transformed using various methods known in the art - such as a process involving protoplast formation and transformation, of the protoplasts followed by regeneration of the cell wall in a manner known. The use of *Aspergillus* as a host microorganism is described in EP 0 238 023.
Another host organism can be a plant. A review of the general techniques used for transforming plants may be found in articles by Potrykus (Annu Rev Plant Physiol Plant Mol Biol [1991] 42:205-225) and Christou (Agro-Food-Industry Hi-Tech March/April 1994 17-27). Further teachings on plant transformation may be found in EP-A-0449375.
General teachings on the transformation of fungi, yeasts and plants are presented in following sections.

TRANSFORMED BACTERIA

A host organism may be a bacterium, such as *Streptomyces, Bacillus subtillis* or *E.coli.* Suitable methods of heterologous expression in *E.coli* are disclosed in WO04/064537. Suitable methods of heterologous expression in *Bacillus* are disclosed in WO02/214490. Examples of suitable bacterial host organisms are gram positive bacterial species such *as Bacillaceae, including Bacillus subtilis, Bacillus. licheniformis, Bacillus lentus, Bacillus brevis, Bacillus stearothermophilus, Bacillus alkalophilus, Bacillus amyloliquefaciens, Bacillus coagulans, Bacillus lautus, Bacillus megaterium and Bacillus thuringiensis, Streptomyces* species, such as *Streptomyces murinus,* lactic acid bacterial species, including *Lactococcus* spp., such as *Lactococcus lactis, Lactobacillus* spp., including *Lactobacillus reuteri,, Leuconostoc spp., Pediococcus* spp. and *Streptococcus spp.* Alternatively, strains of a gram-negative bacterial species belonging to *Enterobacteriaceae,* including *E. coli,* or to *Pseudomonadaceae* can be selected as the host organism.

TRANSFORMED FUNGUS

A host organism may be a fungus - such as a filamentous fungus. Examples of suitable such hosts include any member belonging to the genera Thermomyces, Acremonium, Aspergillus, Penicillium, Mucor, Neurospora, Trichoderma and the like.
Teachings on transforming filamentous fungi are reviewed in US-A-5741665 which states that standard techniques for transformation of filamentous fungi and culturing the fungi are well known in the art. An extensive review of techniques as applied to *N. crassa* is found, for example in Davis and de Serres, Methods Enzymol (1971) 17A: 79-143.

Further teachings on transforming filamentous fungi are reviewed in US-A-5674707.

In one aspect, the host organism can be of the genus *Aspergillus,* such as *Aspergillus niger.*
A transgenic *Aspergillus* described herein can also be prepared by following, for example, the teachings of Turner G. 1994 (Vectors for genetic manipulation. In: Martinelli S.D., Kinghorn J.R.(Editors) Aspergillus: 50 years on. Progress in industrial microbiology vol 29. Elsevier Amsterdam 1994. pp. 641-666). Gene expression in filamentous fungi has been reviewed in Punt et al. (2002) Trends Biotechnol 2002 May;20(5):200-6, Archer & Peberdy Grit Rev Biotechnol (1997) 17(4):273-306.

TRANSFORMED YEAST

The transgenic organism can be a yeast.
A review of the principles of heterologous gene expression in yeast are provided in, for example, Methods Mol Biol (1995), 49:341-54, and Curr Opin Biotechnol (1997) Oct;8(5):554-60
In this regard, yeast - such as the species *Saccharomyces cerevisi or Pichia pastoris* (see FEMS Microbiol Rev (2000 24(1):45-66), may be used as a vehicle for heterologous gene expression.

A review of the principles of heterologous gene expression in *Saccharomyces cerevisiae* and secretion of gene products is given by E Hincheliffe E Kenny (1993, "Yeast as a vehicle for the expression of heterologous genes", Yeasts, Vol 5, Anthony H Rose and J Stuart Harrison, eds, 2nd edition, Academic Press Ltd.).

For the transformation of yeast, several transformation protocols have been developed. For example, a transgenic Saccharomyces as described herein can be prepared by following the teachings of Hinnen et al., (1978, Proceedings of the National Academy of Sciences of the USA 75, 1929); Beggs, J D (1978, Nature, London, 275, 104); and Ito, H et al (1983, J Bacteriology 153,163-168).

The transformed yeast cells may be selected using various selective markers - such as auxotrophic markers dominant antibiotic resistance markers.

A suitable yeast host organism can be selected from the biotechnologically relevant yeasts species such as, but not limited to, yeast species selected from *Pichia* spp., *Hansenula* spp., *Kluyveromyces, Yarrowinia* spp., *Saccharomyces* spp., including *S. cerevisiae,* or *Schizosaccharomyce* spp. including *Schizosaccharomyce pombe.*

A strain of the methylotrophic yeast species *Pichia pastoris* may be used as the host organism.
The host organism may be a *Hansenula* species, such as *H. polymorpha* (as described in WO01/39544).

TRANSFORMED PLANTS/PLANT CELLS

A suitable host organism may be a plant. A review of the general techniques may be found in articles by Potrykus (Annu Rev Plant Physiol Plant Mol Biol [1991] 42:205-225) and Christou (Agro-Food-Industry Hi-Tech March/April 1994 17-27), or in WO01/16308. The transgenic plant may produce enhanced levels of phytosterol esters and phytostanol esters, for example.

Therefore the present description also relates to a method for the production of a transgenic plant with enhanced levels of phytosterol esters and phytostanol esters, comprising the steps of transforming a plant cell with a lipid acyltransferase as defined herein (in particular with an expression vector or construct comprising a lipid acyltransferase as defined herein), and growing a plant from the transformed plant cell.

SECRETION

Often, it is desirable for the polypeptide to be secreted from the expression host into the culture medium from where the enzyme may be more easily recovered.
The secretion leader sequence may be selected on the basis of the desired expression host. Hybrid signal sequences may also be used with the context of the present invention.

Typical examples of heterologous secretion leader sequences are those originating from the fungal amyloglucosidase (AG) gene (*glaA* - both 18 and 24 amino acid versions e.g. from *Aspergillus*)*,* the a-factor gene (yeasts e.g. *Saccharomyces, Kluyveromyces* and *Hallsenula*) or the $\alpha$-amylase gene (*Bacillus*)*.*

DETECTION

A variety of protocols for detecting and measuring the expression of the amino acid sequence are known in the art. Examples include enzyme-linked immunosorbent assay (ELISA), radioimmunoassay (RIA) and fluorescent activated cell sorting (FACS).

A wide variety of labels and conjugation techniques are known by those skilled in the art and can be used in various nucleic and amino acid assays.

A number of companies such as Pharmacia Biotech (Piscataway, NJ), Promega (Madison, WI), and US Biochemical Corp (Cleveland, OH) supply commercial kits and protocols for these procedures.

Suitable reporter molecules or labels include those radionuclides, enzymes, fluorescent, chemiluminescent, or chromogenic agents as well as substrates, cofactors, inhibitors, magnetic particles and the like. Patents teaching the use of such labels include US-A-3,817,837; US-A-3,850,752; US-A-3,939,350; US-A-3,996,345; US-A-4,277,437; US-A-4,275,149 and US-A-4,366,241.

Also, recombinant immunoglobulins may be produced as shown in US-A-4,816,567.

FUSION PROTEINS

A polypeptide having the specific properties as defined herein may be produced as a fusion protein, for example to aid in extraction and purification thereof. Examples of fusion protein partners include glutathione-S-transferase (GST), 6xHis, GAL4 (DNA binding and/or transcriptional activation domains) and β-galactosidase. It may also be convenient to include a proteolytic cleavage site between the fusion protein partner and the protein sequence of interest to allow removal of fusion protein sequences. Preferably the fusion protein will not hinder the activity of the protein sequence.

Gene fusion expression systems in *E. coli* have been reviewed in Curr. Opin. Biotechnol. (1995) 6(5):501-6. The amino acid sequence of a polypeptide having the specific properties as defined herein may be ligated to a heterologous sequence to encode a fusion protein. For example, for screening of peptide libraries for agents capable of affecting the substance activity, it may be useful to encode a chimeric substance expressing a heterologous epitope that is recognised by a commercially available antibody.

The invention will now be described, by way of example only, with reference to the following Figures and Examples.

Figure 1 shows a pfam00657 consensus sequence from database version 6 (SEQ ID No. 1);

Figure 2 shows an amino acid sequence (SEQ ID No. 2) obtained from the organism *Aeromonas hydrophila* (P10480; GI:121051). This amino acid sequence is a reference enzyme, which may be a parent enzyme in accordance with the present invention;

Figure 3 shows an amino acid sequence (SEQ ID No. 3) obtained from the organism *Aeromonas salmonicida* (AAG098404; GI:9964017);

Figure 4 shows an amino acid sequence (SEQ ID No. 4) obtained from the organism *Streptomyces coelicolor* A3(2) (Genbank accession number NP_631558);

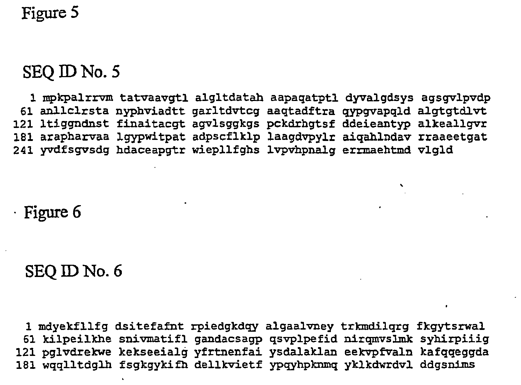

Figure 5 shows an amino acid sequence (SEQ ID No. 5) obtained from the organism *Streptomyces coelicolor* A3(2) (Genbank accession number: CAC42140);

Figure 6 shows an amino acid sequence (SEQ ID No. 6) obtained from the organism *Saccharomyces cerevisiae* (Genbank accession number P41734);

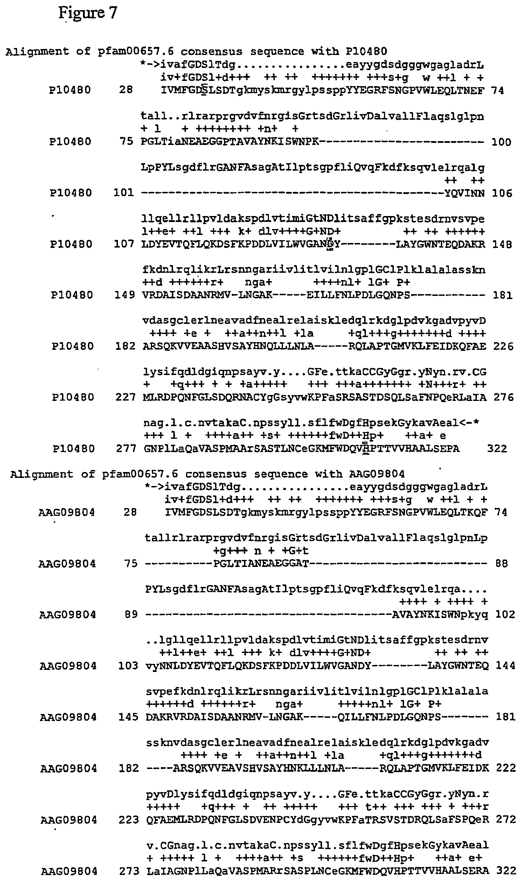

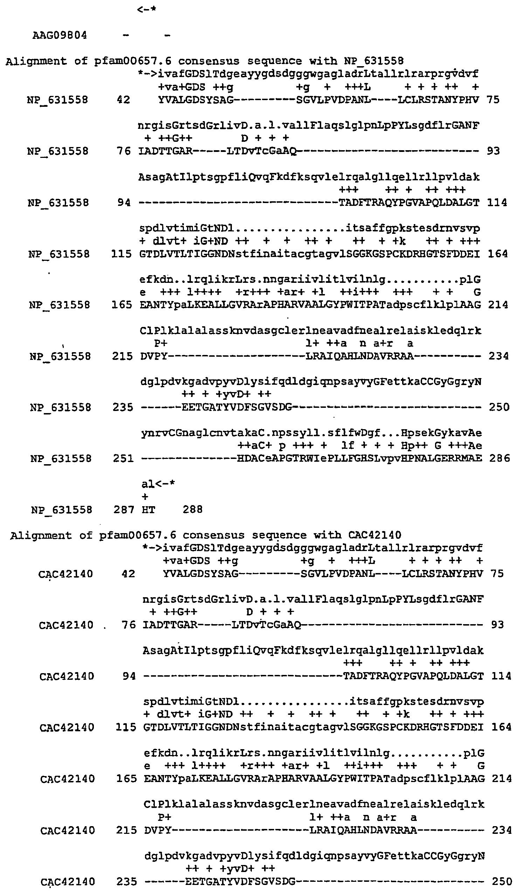

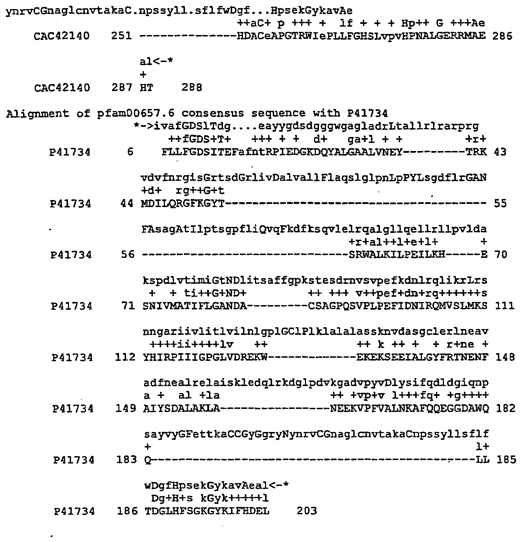

Figure 7 shows an alignment of selected sequences to pfam00657 consensus sequence;

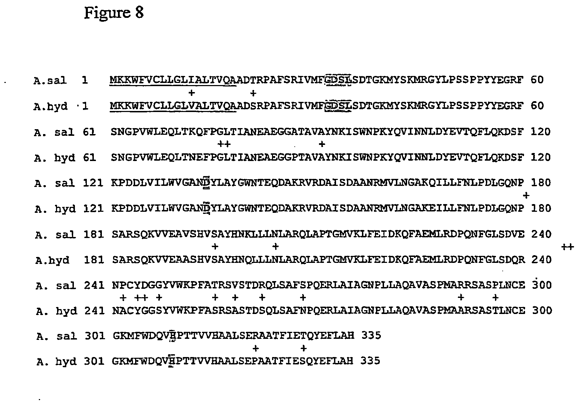

Figure 8 shows a pairwise alignment of SEQ ID No. 3 with SEQ ID No. 2 showing 93% amino acid sequence identity. The signal sequence is underlined. + denotes differences. The GDSX motif containing the active site serine 16, and the active sites aspartic acid 116 and histidine 291 are highlighted (see shaded regions). Numbers after the amino acid is minus the signal sequence;

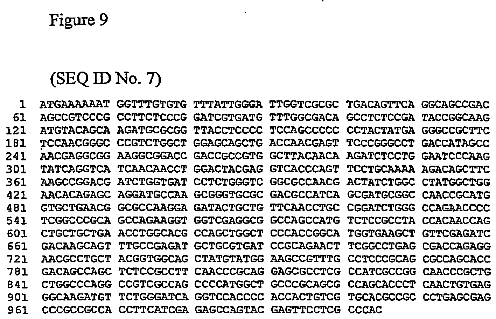

Figure 9 shows a nucleotide sequence (SEQ ID No. 7) encoding a lipid acyl transferase described herein obtained from the organism *Aeromonas hydrophila;*

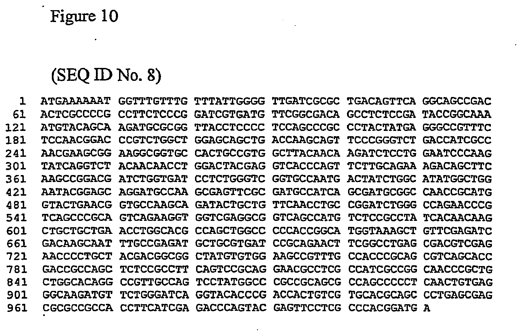

Figure 10 shows a nucleotide sequence (SEQ ID No. 8) encoding a lipid acyl transferase described herein obtained from the organism *Aeromonas salmonicida*;

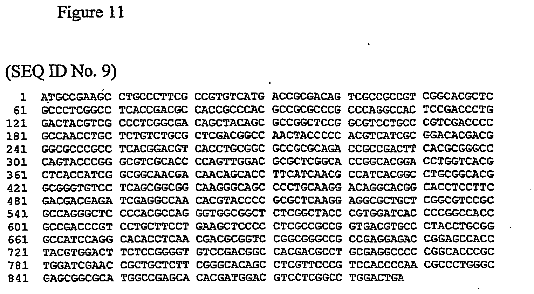

Figure 11 shows a nucleotide sequence (SEQ ID No. 9) encoding a lipid acyl transferase described herein obtained from the organism *Streptomyces coelicolor* A3(2) (Genbank accession number NC_003888.1:8327480..8328367);

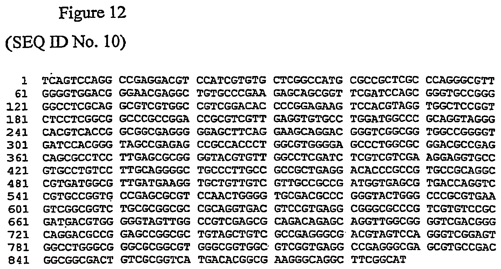

Figure 12 shows a nucleotide sequence (SEQ ID No. 10) encoding a lipid acyl transferase described herein obtained from the organism *Streptomyces coelicolor* A3(2) (Genbank accession number AL939131.1:265480..266367);

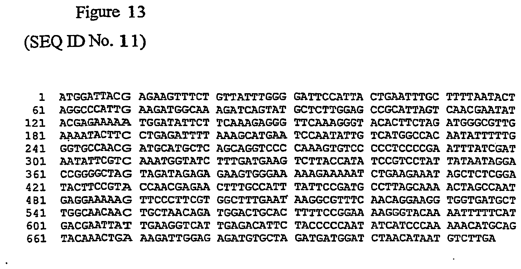

Figure 13 shows a nucleotide sequence (SEQ ID No. 11) encoding a lipid acyl transferase described herein obtained from the organism *Saccharomyces cerevisiae* (Genbank accession number Z75034);

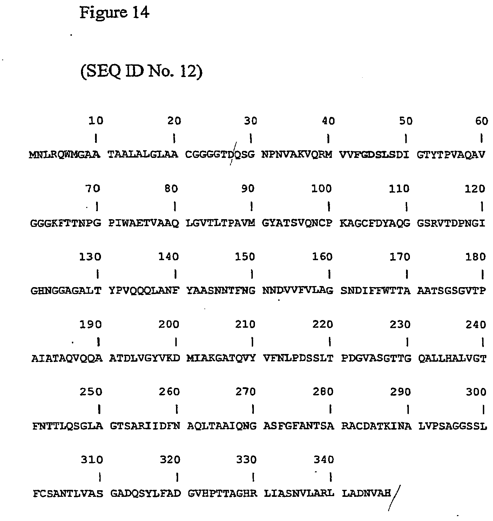

Figure 14 shows an amino acid sequence (SEQ ID No. 12) obtained from the organism *Ralstonia* (Genbank accession number: AL646052);

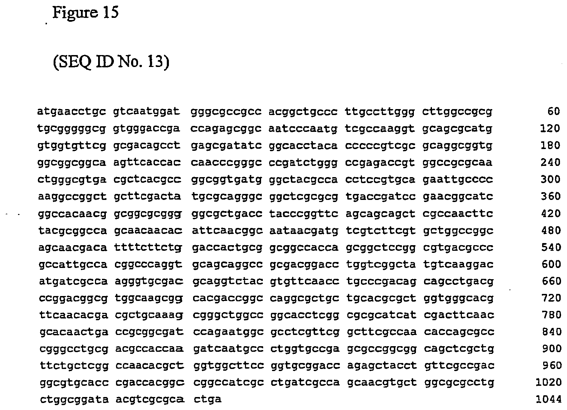

Figure 15 shows a nucleotide sequence (SEQ ID No. 13) encoding a lipid acyl transferase described herein obtained from the organism *Ralstonia*;

Figure 16 shows SEQ ID No. 14. Scoe1 NCBI protein accession code CAB39707.1 GI:4539178 conserved hypothetical protein [Streptomyces coelicolor A3(2)];

Figure 17 shows a nucleotide sequence shown as SEQ ID No. 15 encoding NCBI protein accession code CAB39707.1 01:4539178 conserved hypothetical protein [Streptomyces coelicolor A3(2)];

Figure 18 shows an amino acid shown as SEQ ID No. 16. Scoe2 NCBI protein accession code CAC01477.1 GI:9716139 conserved hypothetical protein [Streptomyces coelicolor A3(2)];

Figure 19 shows a nucleotide sequence shown as SEQ ID No. 17 encoding Scoe2 NCBI protein accession code CAC01477.1 GI:9716139 conserved hypothetical protein [Streptomyces coelicolor A3(2)];

Figure 20 shows an amino acid sequence (SEQ ID No. 18) Scoe3 NCBI protein accession code CAB88833.1 GI:7635996 putative secreted protein. [Streptomyces coelicolor A3(2)];

Figure 21 shows a nucleotide sequence shown as SEQ ID No. 19 encoding Scoe3 NCBI protein accession code CAB88833.1 GI:7635996 putative secreted protein. [Streptomyces coelicolor A3(2)];

Figure 22 shows an amino acid sequence (SEQ ID No. 20) Scoe4 NCBI protein accession code CAB89450.1 GI:7672261 putative secreted protein. [Streptomyces coelicolor A3(2)];

Figure 23 shows an nucleotide sequence shown as SEQ ID No. 21 encoding Scoe4 NCBI protein accession code CAB89450.1 GI:7672261 putative secreted protein. [Streptomyces coelicolor A3(2)];

Figure 24 shows an amino acid sequence (SEQ ID No. 22) Scoe5 NCBI protein accession code CAB62724.1 GI:6562793 putative lipoprotein [Streptomyces coelicolor A3(2)];

Figure 25 shows a nucleotide sequence shown as SEQ ID No. 23, encoding Scoe5 NCBI protein accession code CAB62724.1 GI:6562793 putative lipoprotein [Streptomyces coelicolor A3(2)];

Figure 26 shows an amino acid sequence (SEQ ID No. 24) Srim1 NCBI protein accession code AAK84028.1 GI:15082088 GDSL-lipase [Streptomyces rimosus];

Figure 27 shows a nucleotide sequence shown as SEQ ID No. 25 encoding Srim1 NCBI protein accession code AAK84028.1 GI:15082088 GDSL-lipase [Streptomyces rimosus] ;

Figure 28 shows an amino acid sequence (SEQ ID No. 26) - a lipid acyl transferase from *Aeromonas hydrophila* (ATCC #7965);

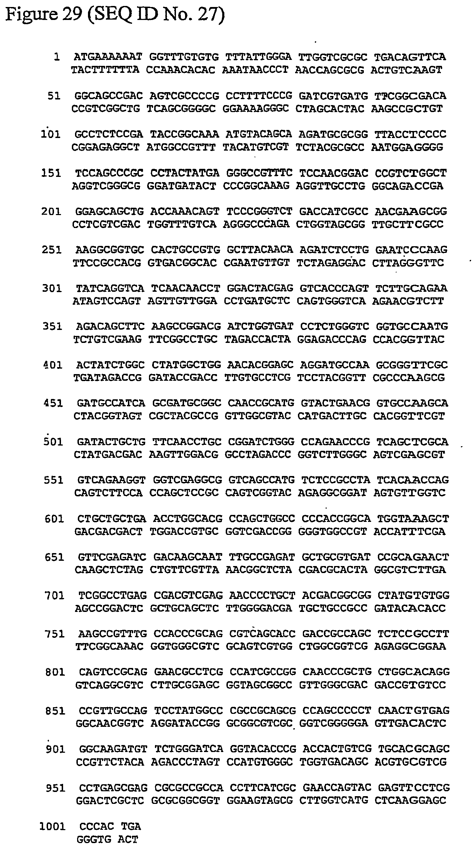

Figure 29 shows a nucleotide sequence (SEQ ID No. 27) encoding a lipid acyltransferase from *Aeromonas hydrophila* (ATCC #7965);

Figure 30 shows an amino acid sequence (SEQ ID No. 28) of a lipid acyltransferase from *Aeromonas salmonicida* subsp. *Salmonicida* (ATCC#14174);

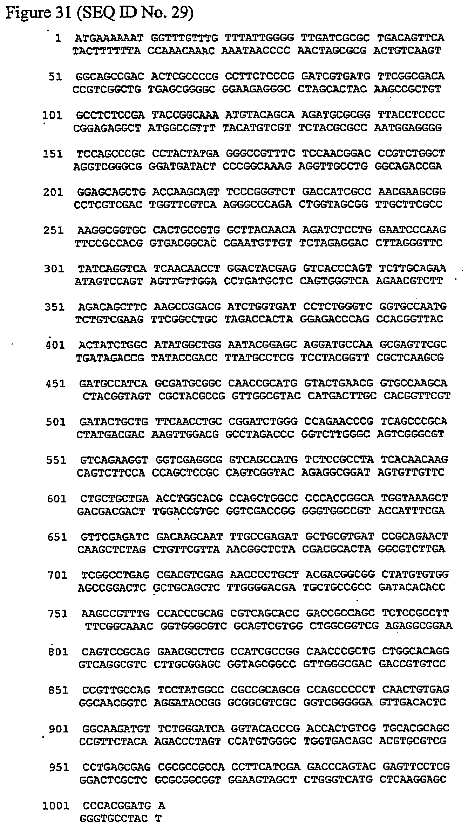

Figure 31 shows a nucleotide sequence (SEQ ID No. 29) encoding a lipid acyltransferase from *Aeromonas salmonicida* subsp. *Salmonicida* (ATCC#14174);

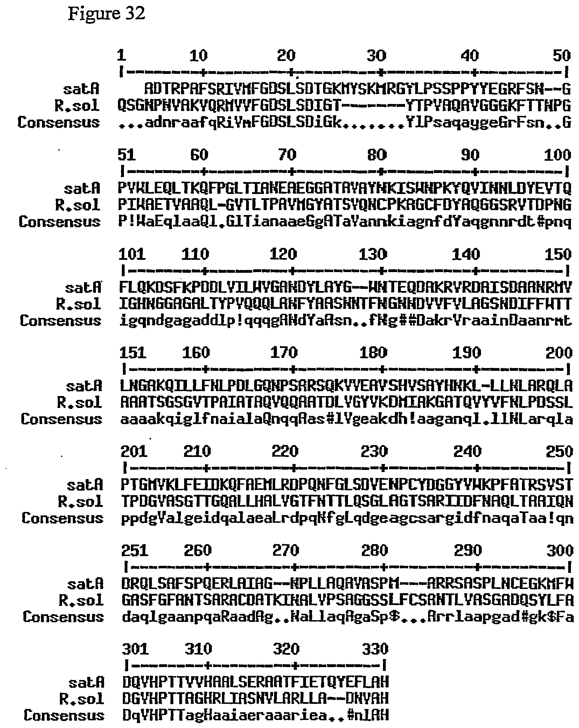

Figure 32 shows that homologues of the *Aeromonas* genes can be identified using the basic local alignment search tool service at the National Center for Biotechnology Information, NIH, MD, USA and the completed genome databases. The GDSX motif was used in the database search and a number of sequences/genes potentially encoding enzymes with lipolytic activity were identified. Genes were identified from the genus Streptomyces, Xanthomonas and Ralstonia. As an example below, the Ralstonia solanacearum was aligned to the Aeromonas salmonicida (satA) gene. Pairwise alignment showed 23% identity. The active site serine is present at the amino terminus and the catalytic residues histidine and aspartic acid can be identified;

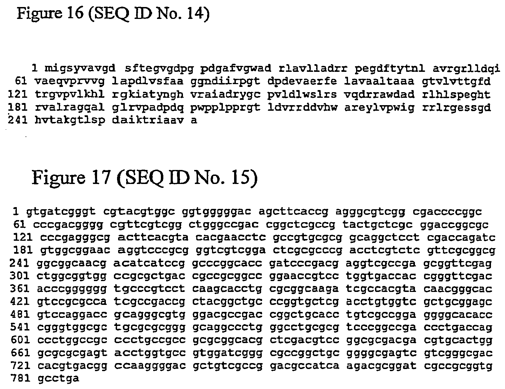

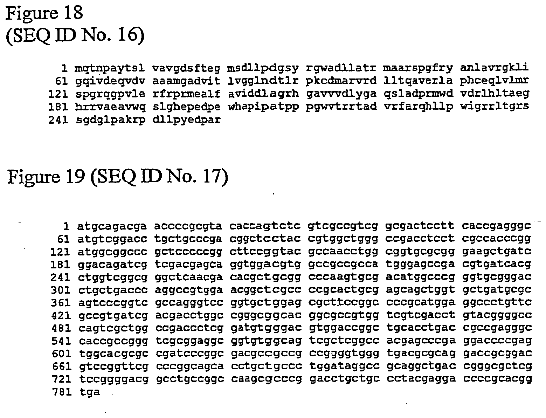

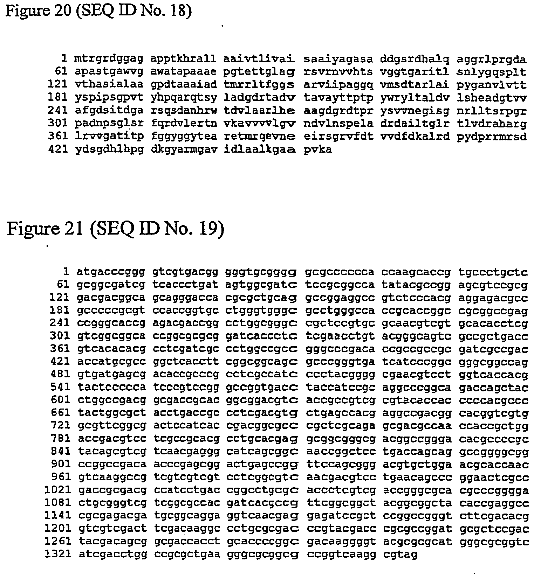

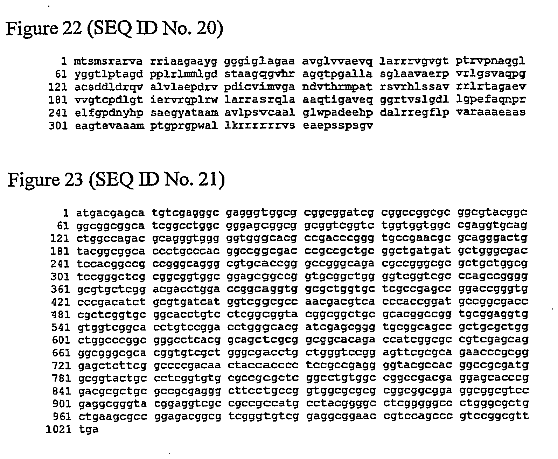

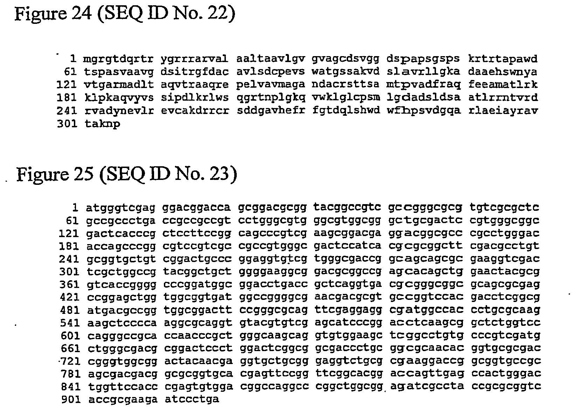

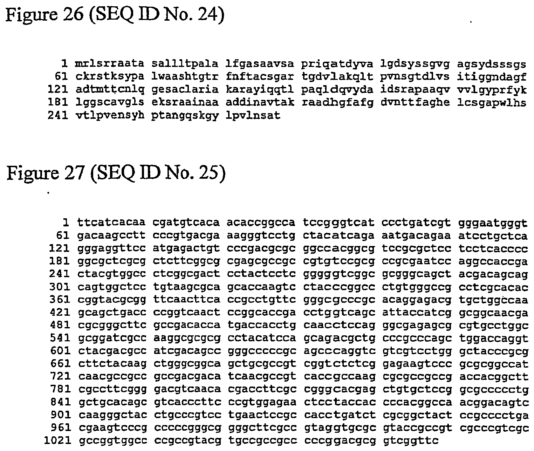

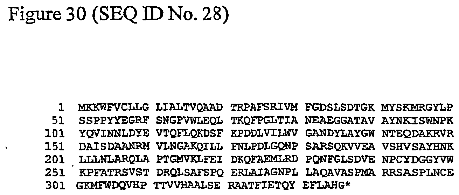

Figure 33 shows the Pfam00657.11 [family 00657, database version 11] consensus sequence (hereafter called Pfam consensus) and the alignment of various sequences to the Pfam consensus sequence. The arrows indicate the active site residues, the underlined boxes indicate three of the homology boxes indicated by [Upton C and Buckley JT (1995) Trends Biochem Sci 20; 179-179]. Capital letters in the Pfam consensus indicate conserved residues in many family members. The - symbol indicates a position where the hidden Markov model of the Pfam consensus expected to find a residue but did not, so a gap is inserted. The . symbol indicates a residue without a corresponding residue in the Pfam consensus. The sequences are the amino acid sequences listed in Figures 16, 18, 20, 22, 24, 26, 28 and 30.

Figure 34 shows the Pfam00657.11 [family 00657, database version 11] consensus sequence (hereafter called Pfam consensus) and the alignment of various sequences to the Pfam consensus sequence. The arrows indicate the active site residues, the underlined boxes indicate three of the homology boxes indicated by [Upton C and Buckley JT (1995) Trends Biochem Sci 20; 179-179]. Capital letters in the Pfam consensus indicate conserved residues in many family members. The - symbol indicates a position where the hidden Markov model of the Pfam consensus expected to find a residue but did not, so a gap is inserted. The . symbol indicates a residue without a corresponding residue in the Pfam consensus. The sequences are the amino acid sequences listed in Figures 2, 16, 18, 20, 26, 28 and 30. All these proteins were found to be active against lipid substrates.

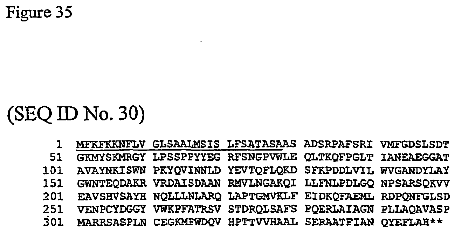

Figure 35 shows an amino acid sequence (SEQ ID No. 30) of the fusion construct used for mutagenesis of the *Aeromonas hydrophila* lipid acyltransferase gene in Example 7. The underlined amino acids is a xylanase signal peptide;

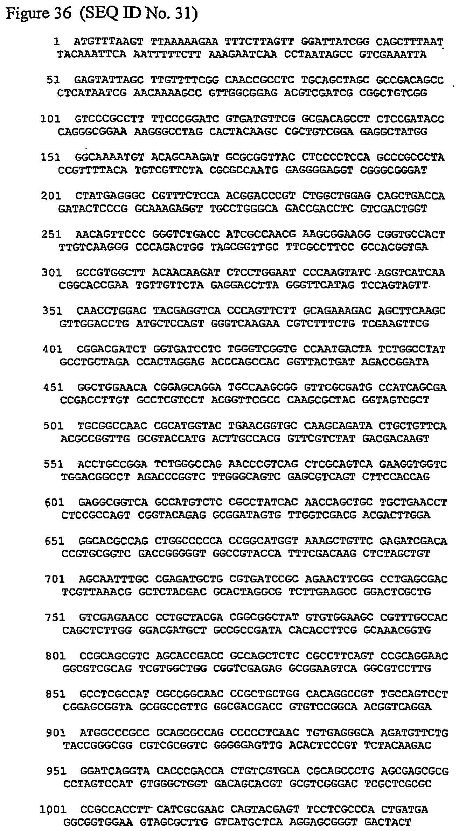

Figure 36 shows a nucleotide sequence (SEQ ID No. 31) encoding a lipid acyltransferase enzyme from *Aeromonas hydrophila* including a xylanase signal peptide;

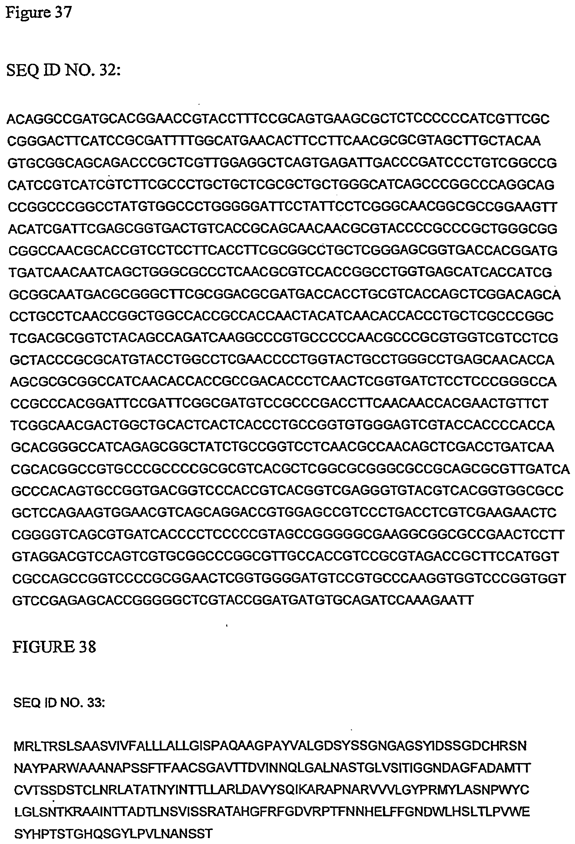

Figure 37 shows a nucleotide sequence encoding a lipid acyltransferase enzyme from *Streptomyces* (SEQ ID No. 32);

Figure 38 shows a polypeptide sequence of a lipid acyltransferase enzyme from *Streptomyces* (SEQ ID No. 33);

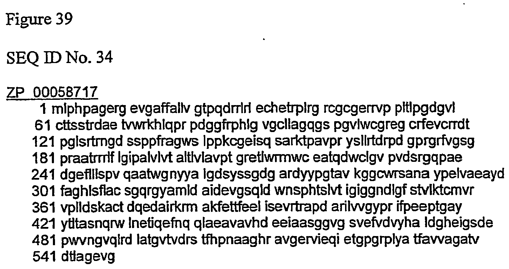

Figure 39 shows a polypeptide sequence of a lipid acyltransferase enzyme from *Termobifida*_(SEQ ID No. 34);

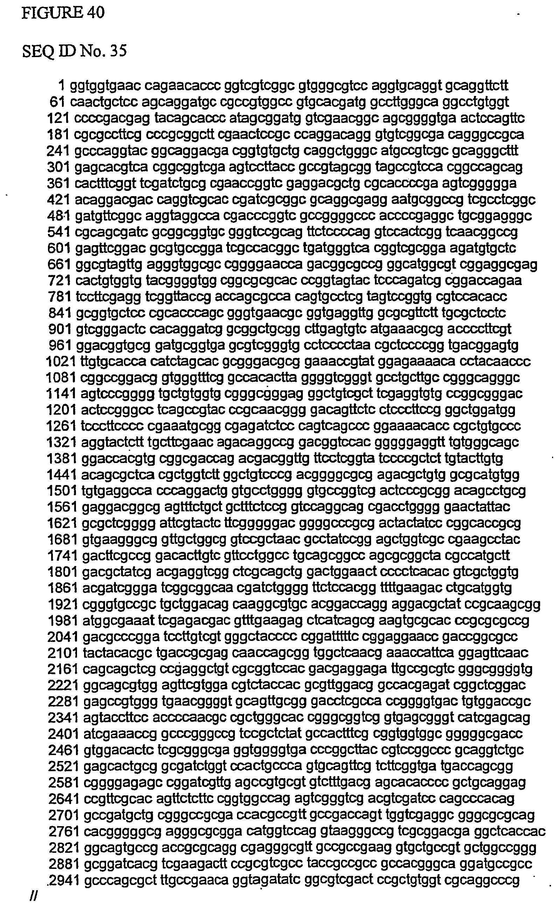

Figure 40 shows a nucleotide sequence encoding a lipid acyltransferase enzyme from *Termobifida*_(SEQ ID No. 35);

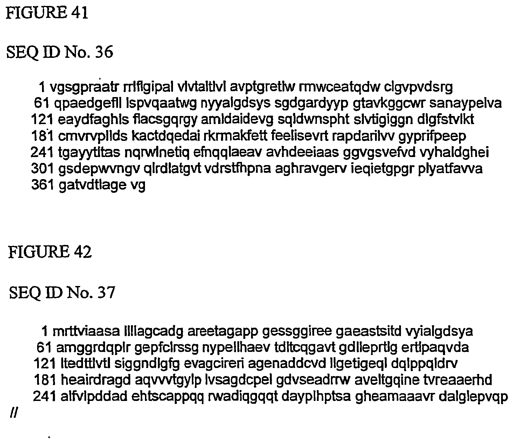

Figure 41 shows a polypeptide sequence of a lipid acyltransferase enzyme from *Termobifida*_(SEQ ID No. 36);

Figure 42 shows a polypeptide of a lipid acyltransferase enzyme from *Corynebacterium\effciens*\ GDSx 300 aa_(SEQ ID No. 37);

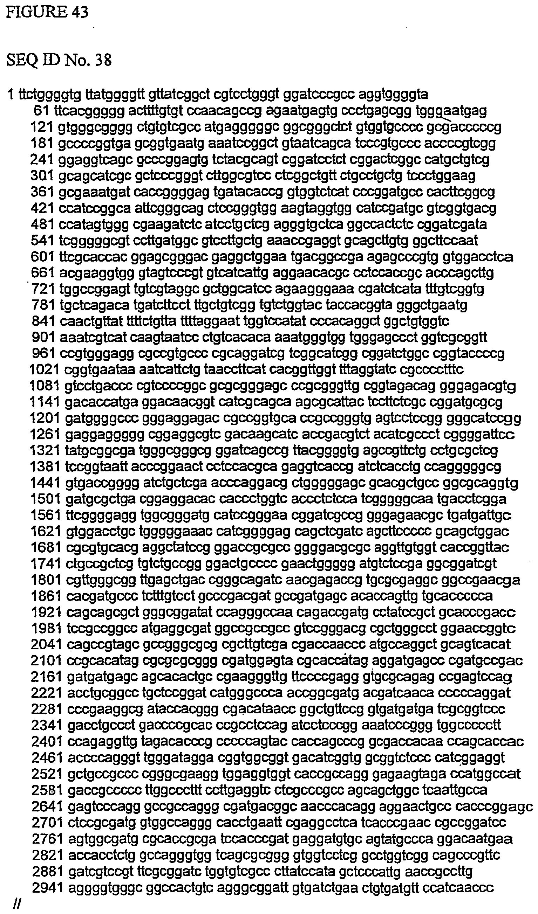

Figure 43 shows a nucleotide sequence encoding a lipid acyltransferase enzyme from *Corynebacterium\effciens*\ GDSx 300 aa_(SEQ ID No. 38);

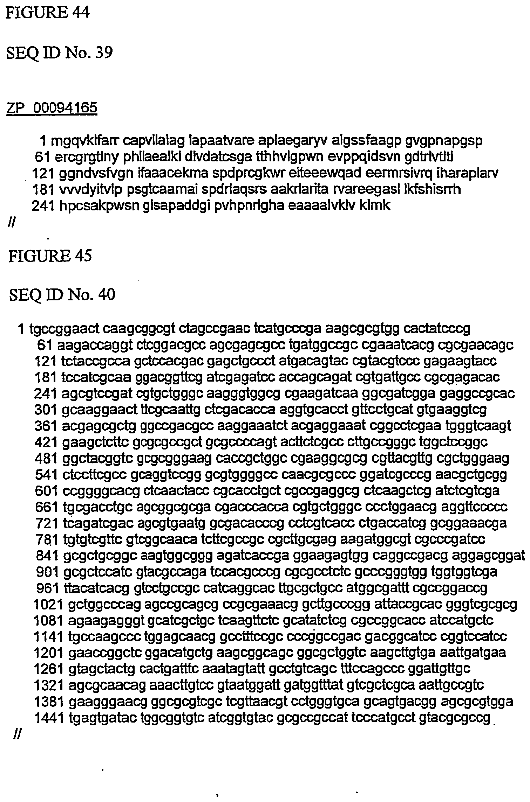

Figure 44 shows a polypeptide of a lipid acyltransferase enzyme from *Novosphingobium\aromaticivorans*\ GDSx 284 aa_(SEQ ID No. 39);

Figure 45 shows a nucleotide sequence encoding a lipid acyltransferase enzyme from *Novosphingobium\aromaticivorans*\ GDSx 284 aa (SEQ ID No. 40);

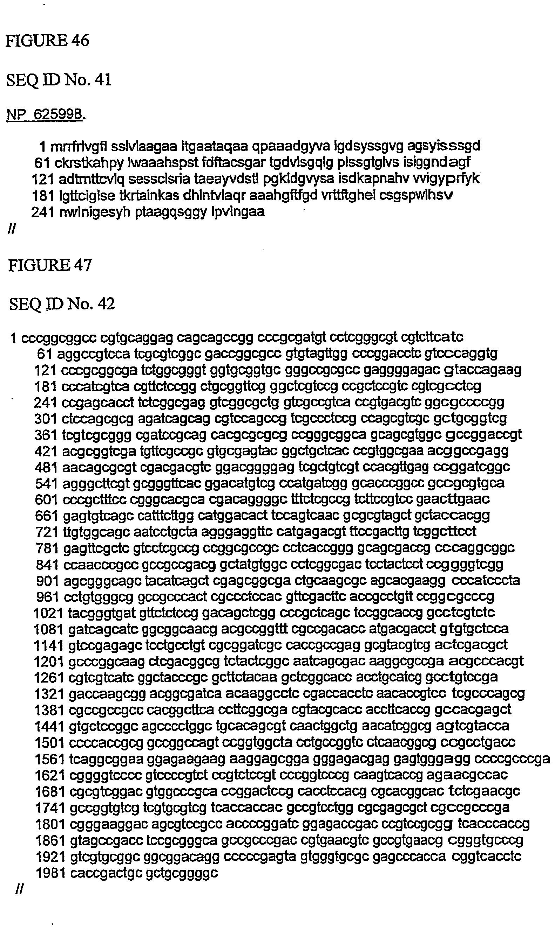

Figure 46 shows a polypeptide of a lipid acyltransferase enzyme from *Streptomyces coelicolor*\ GDSx 268 aa (SEQ ID No. 41);

Figure 47 shows a nucleotide sequence encoding a lipid acyltransferase enzyme from *Streptomyces coelicolor*\ GDSx 268 aa (SEQ ID No. 42);

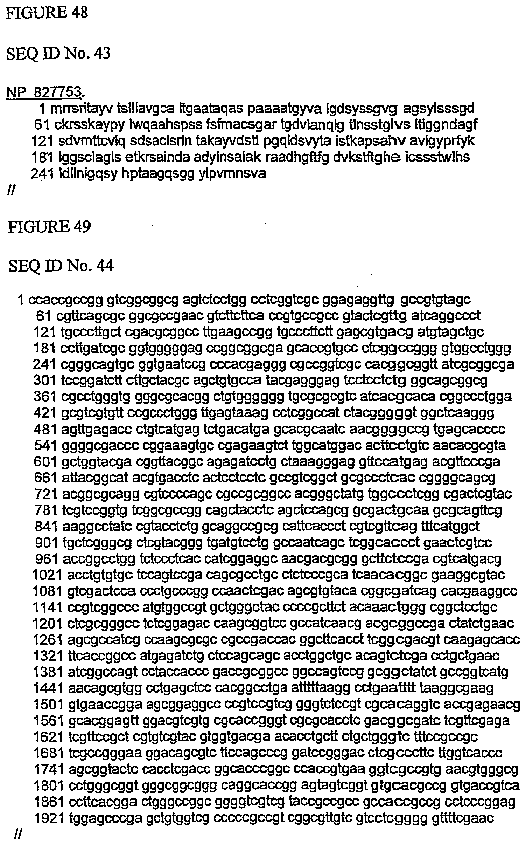

Figure 48 shows a polypeptide of a lipid acyltransferase enzyme from *Streptomyces avermitilis* \ GDSx 269 aa (SEQ ID No. 43);

Figure 49 shows a nucleotide sequence encoding a lipid acyltransferase enzyme from *Streptomyces avermitilis* \ GDSx 269 aa (SEQ ID No. 44);

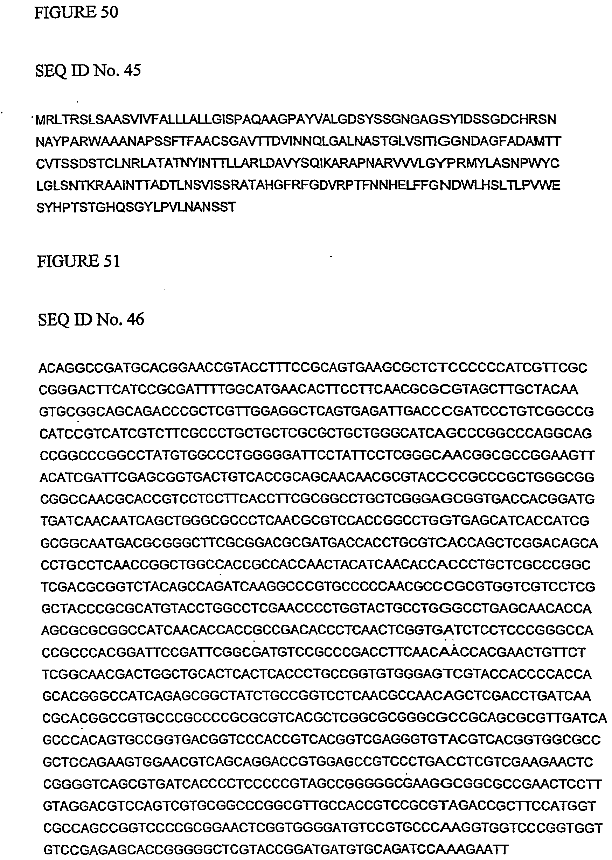

Figure 50 shows a polypeptide of a lipid acyltransferase enzyme from *Streptomyces* (SEQ ID No. 45);

Figure 51 shows a nucleotide sequence encoding a lipid acyltransferase enzyme from *Streptomyces* (SEQ ID No. 46);

Figure 52 shows a ribbon representation of the 1IVN.PDB crystal structure which has glycerol in the active site. The Figure was made using the Deep View Swiss-PDB viewer,

Figure 53 shows 1IVN.PDB Crystal Structure - Side View using Deep View Swiss-PDB viewer, with glycerol in active site - residues within 10Å of active site glycerol are coloured black;

Figure 54 shows 1IVN.PDB Crystal Structure - Top View using Deep View Swiss-PDB viewer, with glycerol in active site - residues within 10Å of active site glycerol are coloured black;

Figure 55 shows alignment 1;

Figure 56 shows alignment 2;

Figures 57 and 58 show an alignment of 1IVN to P10480 (P10480 is the database sequence for *A. hydrophila* enzyme), this alignment was obtained from the PFAM database and used in the model building process;

Figure 59 shows an alignment where P10480 is the database sequence for *Aeromonas hydrophila.* This sequence is used for the model construction and the site selection. Note that the full protein is depicted, the mature protein (equivalent to SEQ ID No. 2) starts at residue 19. A. sal is *Aeromonas salmonicida* (SEQ ID No. 28) GDSX lipase, A. hyd is *Aeromonas hydrophila* (SEQ ID No. 26) GDSX lipase. The consensus sequence contains a * at the position of a difference between the listed sequences;

Figure 60 shows a typical set of 384 clones, the wild type control lies at the intersection of 0.9PC, 0.8DGDG; and

Figure 61 shows three areas of interest. Section 1 contains mutants with an increased ratio R but lower activity towards DGDG. Region 2 contains mutants with an increased ratio R and an increased DGDG activity. Region 3 contains clones with an increased PC or DGDG activity, but no increase in the ratio R.

## EXAMPLE 1

### Modelling of *Aeromonas hydrophila* GDSx lipase on 1IVN

The alignment of the *Aeromonas hydrophila* GDSX lipase amino acid sequence (P10480) to the *Escherichia coli* Tioesterase amino acid sequence (1IVN) and the *Aspergillus aculeatus* rhamnogalacturonan acetylesterase amino acid sequence (1DEO) was obtained from the PFAM database in FASTA format. The alignment of P10480 and 1IVN was fed into an automated 3D structure modeller (SWISS-MODELLER server at www.expasy.org) together with the 1IVN.PDB crystal structure coordinates file FIGURE 52). The obtained model for P10480 was structurally aligned to the crystal structures coordinates of 1IVN.PDB and 1DEO.PDB using the 'Deep View Swiss-PDB viewer (obtained from www.expasy.org/spdbv/) (FIGURE 53). The amino acid alignment obtained from the PFAM database (alignment 1 - (FIGURE 55)) was modified based on the structural alignment of 1DEO.PDB and 1IVN.PDB. This alternative amino acid alignment is called alignment 2 (FIGURE 56).

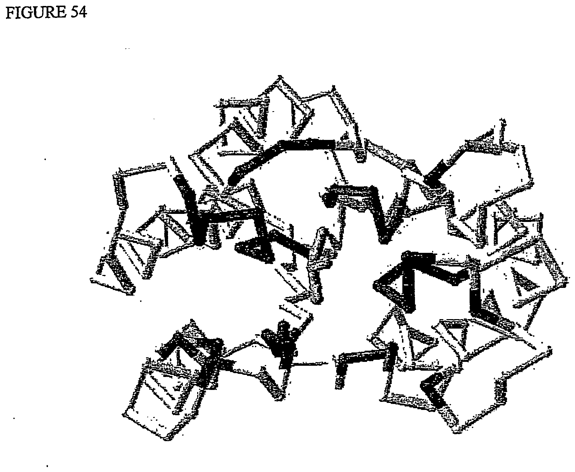

The 1IVN.PDB structure contains a glycerol molecule. This molecule is considered to be in the active site because it is in the vicinity of the catalytic residues. Therefore, a selection can be made of residues that are close to the active site which, due to their vicinity, are likely to have an influence on substrate binding, product release, and/or catalysis. In the 1IVN.PDB structure, all amino acids within a 10 Å sphere centered on the central carbon atom of the glycerol molecule in the active site were selected (amino acid set 1) (See Figure 53 and Figure 54).

The following amino acids were selected from the P10480 sequence; (1) all amino acids in P10480 corresponding to the amino acid set 1 in alignment 1; (2) all amino acids in P10480 corresponding to the amino acid set 1 in alignment 2; (3) from the overlay of the P10480 model and 1IVN all amino acids in the P10480 model within 12Å from the glycerol molecule in 1IVN. All three groups combined give amino acid set 2.

Sequence P10480 was aligned to "AAG09804.1 GI:9964017 glycerophospholipid-cholesterol acyltransferase [Aeromonas salmonicida]" and the residues in AAG09804 corresponding to amino acid set 2 were selected to give amino acid set 3.

Set 1, 2, and 3

Amino acid set 1:

Amino acid set 1 (note that these are amino acids in 1IVN - Figure 57 and Figure 58.) Gly8, Asp9, Leu11, Ser12, Tyr15, Gly44, Asp45, Thr46, Glu69, Leu70, Gly71, Gly72, Asn73, Asp74, Gly75, Leu76, Gln106, Ile107, Arg108, Leu109, Pro110, Tyr113, Phe121, Phe139, Phe140, Met141, Tyr145, Met151, Asp154, His157, Gly155, Ile156, Pro158

The highly conserved motifs, such as GDSx and catalytic residues, were deselected from set 1 (residues underlined). For the avoidance of doubt, set 1 defines the amino acid residues within 10Å of the central carbon atom of a glycerol in the active site of the 1IVN model.

Amino acid set 2:

Amino acid set 2 (note that the numbering of the amino acids refers to the amino acids in the P10480 mature sequence) Leu17, Lys22, Met23, Gly40, Asn80, Pro81, Lys82, Asn87, Asn88, Trp111, Val112, Ala114, Tyr117, Leu118, Pro156, Gly159, Gln160, Asn161, Pro162, Ser163, Ala164, Arg165, Ser166, Gln167, Lys168, Val169, Val170, Glu171, Ala172, Tyr179, His180, Asn181, Met209, Leu210, Arg211, Asn215, Lys284, Met285, Gln289 and Val290.

Table of selected residues in Set 1 compared with Set 2:

| IVN model | | | P10480 Mature sequence residue Number |
|---|---|---|---|
| IVN | A.hyd homologue | | |
| | PFAM | Structure | |
| Gly8 | Gly32 | | |
| Asp9 | Asp33 | | |
| Ser10 | Ser34 | | |
| Leu11 | Leu35 | | Leu17 |
| Ser12 | Ser36 | | Ser18 |
| | | | Lys22 |
| | | | Met23 |
| Tyr15 | Gly58 | | Gly40 |
| Gly44 | Asn98 | | Asn80 |
| Asp45 | Pro99 | | Pro81 |
| Thr46 | Lys100 | | Lys82 |
| | | | Asn87 |
| | | | Asn88 |
| Glu69 | Trp129 | | Trp111 |
| Leu70 | Val130 | | Val112 |
| Gly71 | Gly131 | | |
| Gly72 | Ala132 | | Ala114 |
| Asn73 | Asn133 | | |
| Asp74 | Asp134 | | |
| Gly75 | Tyr135 | | Tyr117 |
| Leu76 | Leu136 | | Leu118 |
| Gln106 | | Pro174 | Pro156 |
| Ile107 | | Gly177 | Gly159 |

(continued)

| IVN model IVN | A.hyd homologue PFAM | Structure | P10480 Mature sequence residue Number |
|---|---|---|---|
| Arg108 | | Gln178 | Gln160 |
| Leu109 | | Asn179 | Asn161 |
| Pro110 | | 180 to 190 | Pro162 |
| Tyr113 | | | Ser163 |
| | | | Ala164 |
| | | | Arg165 |
| | | | Ser166 |
| | | | Gln167 |
| | | | Lys168 |
| | | | Val169 |
| | | | Val170 |
| | | | Glu171 |
| | | | Ala172 |
| Phe121 | His198 | Tyr197 | Tyr179 |
| | | His198 | His180 |
| | | Asn199 | Asn181 |
| Phe139 | Met227 | | Met209 |
| Phel40 | Leu228 | | Leu210 |
| Met141 | Arg229 | | Arg211 |
| Tyr145 | Asn233 | | Asn215 |
| | | | Lys284 |
| Met151 | Met303 | | Met285 |
| Asp154 | Asp306 | | |
| Gly155 | Gln307 | | Gln289 |
| Ile156 | Val308 | | Val290 |
| His157 | His309 | | |
| Pro158 | Pro310 | | |

Amino acid set 3:

Amino acid set 3 is identical to set 2 but refers to the *Aeromonas salmonicida* (SEQ ID No. 28) coding sequence, i.e. the amino acid residue numbers are 18 higher in set 3 as this reflects the difference between the amino acid numbering in the mature protein (SEQ ID No. 2) compared with the protein including a signal sequence (SEQ ID No. 28).

The mature proteins of *Aeromonas salmonicida* GDSX (SEQ ID No. 28) and *Aeromonas hydrophila* GDSX (SEQ ID No. 26) differ in five amino acids. These are Thr3Ser, Gln182Lys, Glu309Ala, Ser310Asn, Gly318-, where the *salmonicida* residue is listed first and the *hydrophila* residue is listed last (FIGURE 59). The *hydrophila* protein is only 317 amino acids long and lacks a residue in position 318. The *Aeromonas salmonicidae* GDSX has considerably high activity on polar lipids such as galactolipid substrates than the *Aeromonas hydrophila* protein. Site scanning was performed on all five amino acid positions.

Amino acid set 4:

Amino acid set 4 is S3, Q182, E309, S310, and -318.

Amino acid set 5:

F13S, D15N, S18G, S18V, Y30F, D116N, D116E, D157N, Y226F, D228N Y230F.

Amino acid set 6:

Amino acid set 6 is Ser3, Leu17, Lys22, Met23, Gly40, Asn80, Pro81, Lys82, Asn 87, Asn88, Trp111, Val112, Ala114, Tyr117, Leu118, Pro156, Gly159, Gln160, Asn161, Pro162, Ser163, Ala164, Arg165, Ser166, Gln167, Lys168, Val169, Val170, Glu171, Ala172, Tyr179, His180, Asn181, Gln182, Met209, Leu210, Arg211, Asn215, Lys284, Met285, Gln289, Val290, Glu309, Ser310, -318.

The numbering of the amino acids in set 6 refers to the amino acids residues in P10480 (SEQ ID No. 2) - corresponding amino acids in other sequence backbones can be determined by homology alignment and/or structural alignment to P10480 and/or 1IVN.

Amino acid set 7:

Amino acid set 7 is Ser3, Leu17, Lys22, Met23, Gly40, Asn80, Pro81, Lys82, Asn 87, Asn88, Trp111, Val112, Ala114, Tyr117, Leu118, Pro156, Gly159, Gln160, Asn161, Pro162, Ser163, Ala164, Arg165, Ser166, Gln167, Lys168, Val169, Val170, Glu171, Ala172, Tyr179, His180, Asn181, Gln182, Met209, Leu210, Arg211, Asn215, Lys284, Met285, Gln289, Val290, Glu309, Ser310, -318, Y30X (where X is selected from A, C, D, E, G, H, I, K, L, M, N, P, Q, R, S, T, V, or W), Y226X (where X is selected from A, C, D, E, G, H, I, K, L, M, N, P, Q, R, S, T, V, or W), Y230X (where X is selected from A, C, D, E, G, H, I, K, L, M, N, P, Q, R, S, T, V, or W), S18X (where X is selected from A, C, D, E, F, H, I, K, L, M, N, P, Q, R, T, W or Y), D157X (where X is selected from A, C, E, F, G, H, I, K, L, M, P, Q, R, S, T, V, W or Y).

The numbering of the amino acids in set 7 refers to the amino acids residues in P10480 (SEQ ID No. 2) - corresponding amino acids in other sequence backbones can be determined by homology alignment and/or structural alignment to P10480 and/or 1IVN).

From the crystal structure one can obtain the secondary structure classification. That means, one can classify each amino acid as being part of an alpha-helix or a beta-sheet. Figure 57 shows the PFAM alignment of 1DEO, 1IVN, and P10480 (the database *Aeromonas hydrophila*). Added below each line of sequence is the structural classification.

The PFAM database contains alignments of proteins with low sequence identity. Therefore, these alignments are not very good. Although the alignment algorithms (HAMMER profiles) are well suited for recognizing conserved motifs the algorithm is not very good on a detailed level. Therefore it is not surprising to find a disparity between the PFAM alignment and a structural alignment. As a skilled person would be readily aware, one can modify the PFAM alignment based on the structural data. Meaning that one can align those structural elements that overlap.

FIGURE 55 shows the original PFAM alignment of 1DEO, 1IVN and P10480. Added to the alignment is the secondary structure information from the crystal structures of 1DEO and 1IVN. Alignment 2 in FIGURE 56 shows a manually modified alignment where the match between the secondary structure elements is improved. Based on conserved residues between either 1DEO and P10480 or between 1IVN and P10480 the alignment was modified for P10480 as well. To easily distinguish the sequence blocks the sequence identifiers in alignment 2 have an extra m (1DEOm, 1IVNm, P10480m).

Alignment 3 is a mix of 1 and 2, it gives the alignment per block.

EXAMPLE 2: Construction of site scan libraries

The Quick Change Multi Site-Directed Mutagenesis Kit from Stratagene was used according to the manufacturers instruction. For each library a degenerate primer with one NNK or NNS (nucleotide abbreviations) codon was designed. Primer design was performed using the tools available on the Stratagene web site. Primer quality control was further confirmed using standard analysis tools which analyze the primer for the potential of forming hairpins or of forming primer-dimers.

The main concepts of the method are as follows; using a non-strand displacing high-fidelity DNA polymerase such as Pfu-Turbo and a single primer one will linearly amplify the DNA template. This is in contrast to the normal exponential amplification process of a PCR reaction. This linear amplification process ensures a low error frequency. The product is single stranded non-methylated DNA and double stranded hemi-methylated DNA. If the template is obtained from a suitable host organism, then the template is double stranded methylated DNA. This means that the template DNA can be digested with Dpn I endonuclease without digesting the product DNA. Therefore upon transformation of the DNA into a suitable host only a very low frequency of the transformants with non-mutagenized plasmid.

EXAMPLE 3: Selection of winners from a site scan library

Two alternative approaches are described; library sequencing followed by analysis of unique amino acids, or library analysis followed by sequencing of the winners.

Selection of winners method 1; library sequencing followed by analysis of unique amino acids.

The transformation/expression shuttle vector used for generation of the site scanning libraries/variants in *E. coli* and expression of the variants in *B. subtilis* was derived from pDP66S, Penninaga et al., (Biochemistry (1995), 3368-3376), by replacement of the selection cassette to a kanamycin selection cassette. The vector used to insert the acyl-transferase variant gene in place of the cgt *gene* down-stream of the P32 promoter. The vector uses the P32 promoter to drive expression of the acyl-transferase variant gene in *B. subtilis.*

The expression vector was transformed into nprE-, aprA- *Bacillus subtillis* DB104 (Kawamura and Doi, J. of Bacteriology Oct 1984, p442-444) using transformation methods, as described in Chapter 3, Molecular Biological Methods for Bacillus (Ed. C.R. Harwood and S.M. Cutting), 1990. John Wiley & Sons Ltd. Chichester, UK).

Site scan libraries were constructed using a degenerate oligo containing one NNK codon, where K stands for G or T and N stands for A, C, G, or T. This means that a set of clones constructed from an amplification reaction using an NNK primer (also known as 'a site scan library') contains in principle 32 unique codons ($4\times4\times2 = 32$ combination options). Assuming no bias due, the number of clones that one needs to pick to have a 95% chance of picking every one of the 32 codons at least once is 95. This can be calculated using the following formula

$$\text{Formula 1;}\quad n = \{\log(1\text{-}c)\} / \{\log(1\text{-}f)\}$$

Where n is the number of clones, c is the fraction value of the confidence interval, for example the 95% confidence interval has a value of 0.95 and the 99% confidence interval has a fraction value of 0.99, and f is the frequency with which each individual codon occurs, which for an NNK primer is 1/32 or 0.03125. Solving the formula for n gives 94.36 or 95 clones. If a 95% confidence interval is deemed to be too low, or if one is unable to avoid bias in one or more steps of the library construction process, one can decide to assay or sequence more clones. For example, in formula 1, if n is set to 384, f to 1/32 or 0.03125 then the confidence interval c is much larger than 99%. Even if 60% of the clones contain the same mutation or the wild type codon, then 363 clones will give a 99% confidence of obtaining all 32 codons. From this one can conclude that, 384 clones will have a 99% confidence of containing each of the 32 codons at least once.

A colony PCR was performed (a PCR reaction on a bacterial colony or on a bacterial liquid culture to amplify a fragment from a plasmid inside a bacterium, and subsequently sequencing that part of the fragment which has been mutagenised is an established procedure. Colony PCR can be routinely performed for sets of 96 due to the availability of prefabricated material (also known as kits) for colony PCR, sequencing, and sequence purification. This entire procedure is offered as a service by several commercial companies such as AGOWA GmbH, Glienicker weg 185, D-12489 Berlin, Germany.

After analysing the 96 sequence reactions, the individual clones were selected representing one for each codon that is available in the set of 96 sequences. Subsequently, for each of the clones representing the mutants, 5 ml of LB broth (Casein enzymatic digest, 10 g/l; low-sodium Yeast extract, 5 g/l; Sodium Chloride, 5 g/l; Inert tableting aids, 2 g/l) supplemented with 50 mg/l kanamycin, was inoculated and incubated at 33 °C for 6 hours at 205 rpm. 0.7 ml of this culture was used to inoculate 50 ml of SAS substrate ($K_2HPO_4$, 10 g/l; MOPS (3-morpholinopropane sulfonic acid), 40 g/l; Sodium Chloride, 5 g/l; Antifoam (Sin 260), 5 drops/l; Soy flour degreased, 20 g/l; Biospringer 106 (100 % dw YE), 20 g/l) supplemented with 50 mg/l kanamycin and a solution of high maltose starch hydrolysates (60 g/l). Incubation was continued for 40 hours at 33 °C and 180 rpm before the culture supernatant was separated by centrifugation at 19000 rpm for 30 min. The supernatant was transferred into a clean tube and directly used for the assay.

Selection of winners method 2; library screening followed by sequencing of the winners

Although one could choose to sequence 384 clones, one may also assay them and select improved variants before sequencing.

A number of issues should be considered when such a number of samples are screened. Without being exhaustive, although it is possible to select variants with altered activity on one substrate, the difference in expression level between 384 cultures can be substantial even if one uses a 384 well microtiter plate, resulting in a high background. Therefore, measuring two activities and selecting winners based on a change in ratio is a preferred method. To illustrate, if two activities have a certain ratio R then regardless of the absolute amount of enzyme present, the ratio between the two activities will always be R. A change in the R value indicates a mutation that changed one activity relative to the second activity.

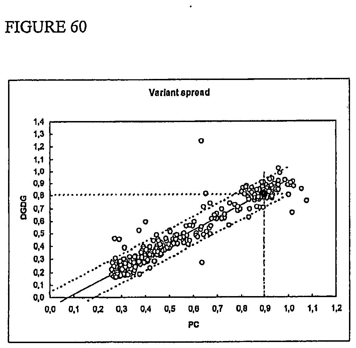

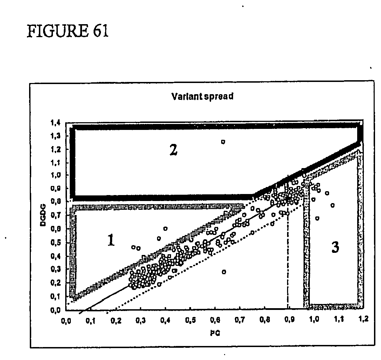

Figure 60 shows a data set obtained from the site scan library. The clones are all tested for activity towards phosphatidyl choline (PC) and digalactosyl diglyceride (DGDG). All clones, which can be mutated or not, that exhibit no change in the R value will lie on a straight line with a certain margin of error. Disregarding these clones three groups of interest appear in Figure 61.

Section 1 in Figure 61 contains all the clones that have a significantly higher R than the wild-type (not mutated) but lower overall DGDG activity. Section 2 contains those clones that have both a higher R value and a higher DGDG activity than the wild type. Section 3 contains clones that do not have a higher R value, but that do have a significantly higher DGDG or PC activity.

If one is interested in variants with an increased activity towards DGDG then section 2 contains the most interesting variants and section 3 contains variants of interest as well. The variants in Section 3 which show a large increase in hydrolytic activity may be accompanied by a decrease in transferase activity.

One thing is worth noticing, if a specificity determining residue is hit, most of the 20 possible amino acids could yield a very different R value. However, if the library contains a large bias towards a single amino acid (for example 60% is Tyrosine) then all those variants will still lie on a straight line.

EXAMPLE 4 : Assays for PC and DGDG activity in a 384 well microtiter plate

Start material

- EM media
- Plate with transformants
- Plate with wild type
- 384 plates
- colony picker
- Waco NEFA-C kit
- PC and DGDG solutions in a 384 plate

Part 1 - picking colonies

- Pick colonies into a 384 plate filled with EM medium
- Skip 4 wells and inoculate those with colonies containing the non-mutated backbone
- Grow o/n at 30°C, 200 rpm shaking speed

Part 2 - Incubation on substrate

- Centrifuge the o/n grown plates; 2500 rpm, 20 min
- Transfer 10 μl supernatant from each well to 2 empty 384 plates
- Add 5 μl 12.5 mM DGDG to one of the plates, add 5 μl 12.5 mM PC to the other plate
- Incubate both plates 2 hrs at 37°C, shake at start to mix then stop the shaking
- Continue with the NEFA C procedure

Part 3 - NEFA-C procedure

- Add 10 μl A solution
- Incubate 10 min 37°C, 300 rpm
- Add 20 μl B solution
- Incubate 10 min 37°C, 300 rpm
- Read the plate at 550 nm

Substrate composition - in mM

25 mM PC eller DGDG
10 mM $CaCl_2$
60 mM Triton X 100
15 mM $NaN_3$
20 mM Briton Robinson pH 5.0

EXAMPLE 5 Selected variants

Determination of enzyme activity

To determine the enzymatic activity towards various substrates 4 μl enzyme solution was incubated with 11 μl substrate for 60 minutes at 37°C. Subsequently the amount of free fatty acids was determined using the WACO NEFA-C kit To the 15 μl enzyme+substrate mix 75 μl NEFA solution A was added and incubated for 15 minutes at 37°C. Subsequently 150 μl NEFA solution B was added and incubated for 15 minutes. Subsequently the optical density (OD) of the sample was measured at 550 nm

As a control, from each variant 4 μl enzyme solution was incubated with 11 μl HEPES buffer for 60 min at 37°C. Subsequently the amount of free fatty acids was determined as described above. The OD values of this control sample was deducted from the observed OD on each substrate to obtain a corrected activity.

Four different substrates were used, the composition was in general 30 mg lipid, 4.75 ml 50 mM HEPES buffer pH 7, 42.5 μl 0.6 M CaCl2, 200 μl 10% Triton X-100 H2O2-free. The 30 mg lipid was either phosphatidyl choline (PC), PC with cholesterol in a 9 to 1 ratio, digalactosyl diglyceride (DGDG), or DGDG with cholesterol in a 9 to 1 ratio.

Selection of improved variants

Variants with improved activity towards PC

Those variants that showed an increase in the OD relative to the wild type enzyme when incubated on PC were selected as variants with improved phospholipase activity.

Variants with improved activity towards DGDG

Those variants that showed an increase in the OD relative to the wild type enzyme when incubated on DGDG were selected as variants with improved activity towards DGDG.

Variants with improved specificity towards DGDG

The specificity towards DGDG is the ratio between the activity towards DGDG and the activity towards phosphatidylcholine (PC). Those variants that showed a higher ratio between DGDG and PC than the wild type were selected as variants with improved specificity towards DGDG.

Variants with improved transferase activity with PC as the acyl donor

The difference in the amount of free fatty acids formed when one incubates an enzyme on PC and on PC with cholesterol is an indication of the amount of transferase activity relative to the amount of hydrolytic activity. Transferase activity will not cause the formation of free fatty acids. The transferase preference is the ratio between the free fatty acids formed when PC is used as a substrate and the free fatty acids formed when PC with cholesterol is used as a substrate. Those variants that show an increase in the transferase preference and show a higher than wild type activity towards PC were selected as having improved transferase activity.

Variants with improved transferase activity with DGDG as the acyl donor

The difference in the amount of free fatty acids formed when one incubates an enzyme on DGDG and on DGDG with cholesterol is an indication of the amount of transferase activity relative to the amount of hydrolytic activity. Transferase activity will not cause the formation of free fatty acids. The transferase preference is the ratio between the free fatty acids formed when DGDG is used as a substrate and the free fatty acids formed when DGDG with cholesterol is used as a substrate. Those variants that show an increase in the transferase preference and show a higher than wild type activity towards DGDG were selected as having improved transferase activity.

Selected variants

For each of the four selection criteria above a number of variants were selected. The "wild type" enzyme in this example is *A. salmonicida* (SEQ ID No. 28). Variants with improved activity towards PC:

| | PC |
|---|---|
| Thr3Asn | 158,0 |
| Thr3Gln | 151,5 |
| Thr3Lys | 141,5 |
| Thr3Arg | 133,0 |
| Glu309Ala | 106,0 |
| Thr3Pro | 101,5 |
| Thr3Met | 96,0 |
| wild-type | 86,5 |

Variants with improved activity towards DGDG:

| | DGDG |
|---|---|
| Gln182Asp | 66,5 |
| Glu309Ala | 60 |
| Tyr230Thr | 59 |
| Tyr230Gly | 57,5 |
| Tyr230Gly | 51 |
| Thr3Gln | 44,5 |
| wild-type | 43,5 |

Variants with improved specificity towards DGDG:

| | $R_{DGDG/PC}$ | PC | DGDG |
|---|---|---|---|
| Gln182Asp | 1,02 | 65,5 | 66,5 |
| Tyr230Gly | 0,79 | 72,5 | 57,5 |
| Tyr230Gly | 0,78 | 65,0 | 51,0 |
| Tyr230Thr | 0,75 | 78,5 | 59,0 |
| Tyr230Val | 0,71 | 58,0 | 41,0 |
| Asp157Cys | 0,69 | 48,0 | 33,0 |
| Glu309Pro | 0,58 | 73,5 | 42,5 |
| Glu309Ala | 0,57 | 106,0 | 60,0 |
| Gly318Ile | 0,53 | 69,5 | 36,5 |
| Tyr230Arg | 0,50 | 63,5 | 32,0 |
| Tyr230Met | 0,50 | 64,5 | 32,5 |
| wild-type | 0,50 | 86,5 | 43,5 |

Variants with improved transferase activity with PC as the acyl donor:

| | $R_{PC+Cho/PC}$ | PC | PC+Cho |
|---|---|---|---|
| Thr3Lys | 0,54 | 142 | 76 |
| Thr3Arg | 0,55 | 133 | 73 |
| Thr3Gln | 0,63 | 152 | 96 |
| Thr3Asn | 0,64 | 158 | 101 |
| Thr3Pro | 0,67 | 102 | 68 |
| Thr3Met | 0,78 | 96 | 75 |
| wild-type | 0,83 | 87 | 72 |

Variants with improved transferase activity with DGDG as the acyl donor:

| | $R_{DGDG+Cho/DG\ DG}$ | DGDG |
|---|---|---|
| Tyr230Thr | 1,10 | 59 |
| Gln182Asp | 1,39 | 67 |
| Tyr230Gly | 1,55 | 58 |
| Glu309Ala | 1,78 | 60 |
| wild-type | 1,78 | 44 |

EXAMPLE 6: Transferase assay Phospholipid:cholesterol

Phospholipid can be replaced by DGDG to provide a transferase assay from a galacolipid. Other acceptors for example, glycerol, glucose, hydroxy acids, proteins or maltose can also be used in the same assay.

300 mg Phosphatidylcholine (Avanti #441601):Cholesterol(Sigma C8503) 9:1 is scaled in a Wheaton glass. 10 ml 50 mM HEPES buffer pH 7.0 is added and stirring at 40 °C disperses the substrate

0,5 ml substrate is transferred to a 4 ml vial and placed in a heating block at 40 °C. 0.050 ml transferase solution is added, also a control with 0.050 ml water is analysed in the same way. The reaction mixture is agitated for 4 hours at 40 °C. The sample is then frozen and lyophilised and analysed by GLC.

Calculation:

From the GLC analysis the content of free fatty acids and cholesterol ester is calculated.

The enzymatic activity is calculated as:

$$\% \text{ Transferase activity} = \frac{\Delta \% \text{ cholesterol ester}/(\text{Mv sterol ester}) \times 100}{\Delta \% \text{ cholesterol ester}/(\text{Mv cholesterol ester}) + \Delta \% \text{ fatty acid}/(\text{Mv fatty acid})}$$

$$\% \text{ Hydrolyse activity} = \frac{\Delta \% \text{ fatty acid}/(\text{Mv fatty acid}) \times 100}{\Delta \% \text{ cholesterol ester}/(\text{Mv cholesterol ester}) + \Delta \% \text{ fatty acid}/(\text{Mv fatty acid})}$$

Ratio Transferase/Hydrolyse =% transferase activity/% Hydrolyse activity Where:

$$\Delta \% \text{ cholesterol ester} = \% \text{ cholesterol ester(sample)} - \% \text{ cholesterol ester(control)}.$$

$$\Delta \% \text{ fatty acid} = \% \text{ fatty acid(sample)} - \% \text{ fatty acid(control)}.$$

Transferase assay Galactolipid: cholesterol.

300 mg Digalactosyldiglyceride (DGDG) (purity >95 galactolipids, the DGDG used is purified from wheat lipid. DGDG from Sigma D4651 is also suitable for use):Cholesterol(Sigma) 9:1 is scaled in a Wheaton glass. 10 ml 50 mM HEPES buffer pH 7.0 is added and stirring at 40 °C disperses the substrate.

0,5 ml substrate is transferred to a 4 ml vial and placed in a heating block at 40 °C. 0.050 ml transferase solution is added, also a control with 0.050 ml water is analysed in the same way. The reaction mixture is agitated for 4 hours at 40 °C. The sample is then frozen and lyophilised and analysed by GLC.

Calculation:

From the GLC analysis the content of free fatty acids and cholesterol ester is calculated.

The enzymatic activity is calculated as:

$$\% \text{ Transferase activity} = \frac{\Delta \% \text{ cholesterol ester}/(\text{Mv sterol ester}) \times 100}{\Delta \% \text{ cholesterol ester}/(\text{Mv cholesterol ester}) + \Delta \% \text{ fatty acid}/(\text{Mv fatty acid})}$$

$$\% \text{ Hydrolyse activity} = \frac{\Delta \% \text{ fatty acid}/(\text{Mv fatty acid}) \times 100}{\Delta \% \text{ cholesterol ester}/(\text{Mv cholesterol ester}) + \Delta \% \text{ fatty acid}/(\text{Mv fatty acid})}$$

$$\text{Ratio Transferase/Hydrolyse} = \% \text{ transferase activity}/\% \text{ Hydrolyse activity}$$

Where:

$\Delta$ % cholesterol ester = % cholesterol ester(sample)-% cholesterol ester(control).

$\Delta$ % fatty acid = % fatty acid(sample) - % fatty acid(control)

EXAMPLE 7: Variants of a lipid acyltransferase for *Aeromonas hydrophila* (SEQ ID No. 26)

Mutations were introduced using the QuikChange™ Multi-Site Directed Mutagenesis kit from Stratagene, La Jolla, CA92037, USA following the instructions provided by Stratagene.

Variants at Tyr256 showed an increased activity towards phospholipids.

Variants at Tyr256 and Tyr260 showed an increased activity towards galactolipids.

Variants at Tyr265 showed an increased transferase activity with galactolipids as the acyl donor.

The numbers indicate positions on the following sequence: An enzyme from *Aeromonas hydrophila* the amino acid sequence of which is shown as SEQ ID No. 26. The nucleotide sequence is as shown as SEQ ID No. 27.

EXAMPLE 8: Screening of mutants of glyrerophospholipid:cholesterol acyltransferase GCAT from *Aeromonas salmonicida*.

Mutants from point mutations of glycerophospholipid:cholesterol acyltransferase GCAT from *Aeromonas salmonicida* were screened for transferase activity using phosphatidylcholine or digalactosyldiglyceride as donor and cholesterol as acceptor with the aim to select mutant with better activity towards digalactocyldiglyceride than phosphatidylcholine.

GCAT mutants were screened for transferase activity using digalactosyldiglyceride(DG) and phosphatidylcholine(PC) as donor and cholesterol as acceptor.

DG (purity >95% digalactosyldiglyceride (DGDG used is purified from wheat lipid. DGDG from Sigma D4651 is also suitable for use from Signma D4651),) and cholesterol (Sigma C8503) was scaled in the ratio 9 :1 and dissolved in chloroform and evaporated to dryness.

The substate was prepared by dispersing of 3% DG:Cholesterol 9:1 in 50 mM HEPES buffer pH 7.

0,250 ml substrate was transferred to a 3 ml glass with screw lid. 0,025 ml supernatant from fermentation of mutant GCAT was added an incubated at 40 °C for 2 hours. A reference sample with water instead of enzyme was also prepared. Heating the reaction mixture in a boiling water bath for 10 minutes stopped the enzyme reaction.

2 ml 99% ethanol was added and submitted to cholesterol analysis as well as free fatty acid analysis.

Cholesterol assay.

100 μl substrate containing:1.4 U/ml Cholesterol oxidase(SERVA Electrophoresis GmbH cat. No 17109), 0,4 mg/ml ABTS (Sigma A-1888), 6 U/ml Peroxidase (Sigma 6782) in 0,1 M TRIS,HCl buffer pH 6.6 + 0,5% Triton X 100(Sigma X-100) was incubated at 37°C for 5 minutes. 5μl cholesterol sample was added and mixed. The reaction mixture was incubated for further 5 minutes and OD 405nm measured. The content of cholesterol was calculated from the analyses of standard solutions of cholesterol containing 0,4mg/ml, 0,3mg/ml, 0,20mg/ml, 0,1mg/ml, 0,05 mg/ml and 0 mg/ml.

Free fatty acid assay.

Free fatty acids in the sample was measured using a NEFA C kit (WAKO Chemicals GmbH) 75 μl NEFA reagent A was incubated for 10 minutes at 37 °C. 15 μl enzyme sample was added and mixed. The reaction mixture was incubated for 10 minutes. 150 μl NEFA reagent B was added, mixed and incubated for further 10 minutes and OD 540 nm was measured. Free fatty acid was calculated from standard solutions of 0,4, 0.3, 0.2, 0.1, 0.05 and 0 mM fatty acid.

Transferase assay using phosphatidylcholine as donor was measured in the same way, but using phosphatidylcholine(Avanti #441601) instead of DG (DGDG).

**Transferase activity was expressed as % cholesterol esterified** calculated from the difference in free cholesterol in the reference sample and free cholesterol in the enzyme sample.

**Hydrolytic activity was expressed as % free fatty acid produced** calculated from the difference in free fatty acid in the enzyme sample and free fatty acid in the reference sample.

The relative Transferase activity against DG and PC was calculated as % $T_{DG}/T_{PC}$. The transferase activity $T_{DG}$ relative to the hydrolytic activity $H_{DG}$ on DG for the mutants were calculated:

$$\frac{0.1 \text{ x } \%TDG/386}{\% HDG/280} = \frac{0.1 \text{ x } \%TDG \text{ x } 280}{\% HDG \text{ x } 386}$$

Where 386 = MW for cholesterol and 280 = MW for fatty acid.

Mutants with $T_{DG}$ >50% and $T_{DG}$($T_{PC}$>3 and $\frac{0.1 \text{ x } \%T_{DG}/386}{\% H_{DG}/280} > 2.5$ were selected as improved mutants.

The data obtained from the above example can be analysed via statistics to identify and prioritise key sites and/or specific amino acid substitutions which provide the desired activity profile, such as increased ration of $T_{DG}$ as compared to $T_{PC}$. For example, the following robust modeling is proposed:

The information regarding $T_{PC}$ and $T_{DG}$ is carried by the censored responses max(0, $T_{PC}$) and max(0, $T_{DG}$).The objective of the study is to identify settings determining $T_{DG}$ >=Tpc, based on the scores for ln(1+ $T_{DG}$)-ln(1+ $T_{PC}$) with positive values as preference, both in absolute scale and in relative scale compared to a control (native). The preferred settings are identified based on a binary response (Event, Non-Event), where Event is defined as a preferred response in relation to the scores. A binomial GLIM model with complementary log-log link, based on the empirical data structure without prior information included, analyses the binary responses. See the following reference for details of how to perform the statistical analysis: proc LOGISTIC in SAS Institute Inc., SAS/STAT® User's Guide, Version 6,4.Ed, Vol.2, Cary, NC: SAS Institute Inc., 1989.

| Variants with increased $T_{DG}/T_{PC}$ | Variants with enhanced DGDG transferase activity $T_{DG}$ | Variants with enhanced $T_{DG}/H_{DG}$ activity |
|---|---|---|
| K22 E, K<br>G40 L<br>N87 R, D, E, M<br>Y117 A, N, E, H, T<br><br>Q182K, T<br>M209 K, M<br>L210 N<br>R211 G<br>N215 H<br>Y230 I<br>-318 Y, H or S<br>N215 H<br>L210 D, Q or T<br>E 309S, Q or R | N80 P, G, or E<br>S310 Q, H or S<br>S3 E, A, G, K, M, Y, R, P, N, T, or Q<br><br>-318 R, S, E, H, Q, N or D<br><br>N215 L, G, V, R or Y<br><br>K82 S | Y179 E, R, N, Q<br>N215 G<br>L210 D, H, R, E, A, Q, P, N, K, G, R, T, W, 1, V or S<br>N80 G<br>Y30L<br>N87 G<br>H180 I, T<br>M209 Y<br>R211 D, T or G<br>S18 G, M or T<br>G40 R or M<br>N88 W<br>N87 C, D, R, E or G |
| H180 K or Q<br>N 80 N, R or D<br>L210 G, I, H, E, M, S, W, V, A, R, N,<br>S310A, P, T, H, M, K, or G<br>V112 C<br>Y30 G, I, L, S, E M, A or R<br>V290 R, E, H or A<br>Q 289 R or N<br>K22 E<br>G40 L<br>Y179 V<br>M209 L, K, M<br>L211 G, Q, K or D<br>Y230 V<br>S310 P<br>Y179 R<br>H180 T<br>Q289 T or D<br>G40 Q, L or V<br>N88 W<br>N87 R or D | | |

EXAMPLE 9: Selection of improved mutants of glycerophospholipid:cholesterol acyltransferase GCAT from *Aeromonas salmonicida.*

The "parent" enzyme in this example is *A. salmonicida* (SEQ ID No. 28).

32 positions of GCAT from *Aeromonas salmonicida* (230 Tyr, 182 Lys, 3 Thr, 157 Asp, 310 Thr, 318 Gly, 309 Glu, 17 Leu, 111 Trp, 117 Tyr, 179 Tyr, 118 Leu, 215 Asn, 22 Lys, 290 Val, 289 Gln, 285 Met, 18 Ser, 23 Met, 180 His, 284 Lys, 181 Asn, 209 Met., 210 Leu, 211 Arg, 40 Gly, 81 Pro,112 Val, 80 Asn, 82 Lys, 88 Asn, 87 Asn were screened according to the experimental outline in Example 8.

Based on the results from the screening and the three selection criteria the following mutants listed in the table 1 were selected.

Table 1

| Position | Amino acid | T,PC | T, DG | T,DG/T,PC | H, PC | H, DG |
|---|---|---|---|---|---|---|
| 210 | GLN | 10,3 | 59,7 | 5,9 | 0,0 | 0,0 |
| 215 | GLY | 15,1 | 55,8 | 4,5 | 3,1 | 1,0 |
| 215 | LEU | 19,4 | 51,9 | 3,3 | 4,3 | 1,1 |
| 215 | TYR | 21,3 | 68,0 | 3,9 | 4,3 | 1,8 |
| 215 | ARG | 16,2 | 62,1 | 4,7 | 5,5 | 2,1 |
| 215 | VAL | 14,7 | 61,6 | 5,2 | 3,5 | 1,7 |
| 215 | HIS | 5,7 | 50,1 | 10,9 | 4,2 | 1,3 |
| 215 | ASN | 9,4 | 47,4 | 6,2 | 4,0 | 1,2 |

EXAMPLE 10: Selection of specific amino acid regions of interest for mutation of the glycerophospholipid:cholesterol acyltransferase GCAT from *Aeromonas salmonicida.*

From the pfam alignment (alignment 2; FIGURE 56) and overlay of the P10480 model and 1IVN all amino acids in regions surrounding the glycerol molecule in the active site of 1IVN were selected and used for defining regions of specific interest (loops). (Numbers refer to the amino acids in the P10480 mature sequence (SEQ ID No. 2)):

Thr 20- Arg 41 (Loop 1, L1)
Ile 77- Leu 89 (Loop 2, L2)
Leu 118 - Asp 127 (Loop 3, L3)
Gly 146- Val 176 (Loop 4, L4)
Glu 208 - Trp 287 (Loop 5, L5)

The intervening regions (IVR) were named accordingly:

Ala 1- Asp 19 (IVR1)

Phe 42 - Lys 76 (IVR2)

Asp 90 - Tyr 117 (IVR3)

Ala 128 - Asn 145 (IVR4)

Ser 177 - Ala 207 (IVR5)

Asp 288 - His 317 (IVR6)

The following table summarizes the allocation of preferred positions for mutation of the glycerophospholipid:cholesterol acyltransferase GCAT from *Aeromonas salmonicida.* The results are based on experimental outlines as set out in Example 8-10.

| | P10480 amino acid positions (SEQ ID No 2) | Preferable sites to produce variants with increased $T_{DG}/T_{PC}$ | 10 Å | Preferred regions for methods of the invention |
|---|---|---|---|---|
| IVR1 | 1-19 | | L17, S18 | |
| Loop1 | 20-41 | K22, G40, Y 30 | K22, M23, G40 | Loop1 |
| IVR2 | 42-76 | | | |

(continued)

| | P10480 amino acid positions (SEQ ID No 2) | Preferable sites to produce variants with increased $T_{DG}/T_{PC}$ | 10 Å | Preferred regions for methods of the invention |
|---|---|---|---|---|
| Loop2 | 77-89 | N80, N87, N88 | N80, P81, K82, N87, N88 | Loop 2 |
| IVR3 | 90-117 | Y117, V112, W111, A114, | W111, V112, A114, Y117 | IVR 3<br>IVR3 & 10A from active site. |
| Loop3 | 118-127 | | L118 | |
| IVR4 | 128-145 | | | |
| Loop4 | 146-176 | | P156 | |
| IVR5 | 177-207 | N181, Q182, H180, Y179 | Y179, H180, N181 | IVR5<br>IVR5 & 10A from active site. |
| Loop5 | 208-287 | M209, L210, R211, N215, Y230 | M209, L210, R211, N215, K284, M285, Q289, V290 | Loop 5<br>Loop 5 & 10A from active site. |
| IVR6 | 288-317 | Q 289, V290, E309 S310, -318 | | IVR 6 |

## Claims

**1.** A variant lipid acyltransferase having increased phospholipid transferase activity wherein the enzyme comprises the amino acid sequence motif GDSX, wherein X is one or more of the following amino acid residues L,A,V, I, F, Y, H, Q, T, N, M or S, wherein the variant enzyme comprises one or more amino acid modifications compared with a parent sequence at any one or more of the amino acid residues:

S3N, Q, K, R, P or M; or
E309A;

wherein the numbering is that obtained from alignment of the variant sequence with the reference sequence shown as SEQ ID No. 2 and wherein the parent lipid acyltransferase comprises the amino acid sequence shown as SEQ ID No. 28 or an amino acid sequence which has 75% or more identity with SEQ ID No. 28; or wherein the parent lipid acyltransferase is encoded by the nucleotide sequence shown as SEQ ID No. 29, or a nucleotide sequence which has at least 75% or more identity with the sequence shown as SEQ ID No. 29.

**2.** A method of preparing a foodstuff, the method comprising adding a variant lipid acyltransferase enzyme according to claim 1 to one or more ingredients of the foodstuff.

**3.** A method of preparing a baked product from a dough, the method comprising adding a variant lipid acyltransferase enzyme according to claim 1 to the dough.

4. Use of a variant lipid acyltransferase enzyme according to claim 1 in a process of treating egg or egg-based products to produce lysophospholipids.

5. A process of enzymatic degumming of vegetable or edible oils, comprising treating the edible oil or vegetable oil with a variant lipid acyltransferase enzyme according claim 1 so as to hydrolyse a major part of the polar lipids (e.g. phospholipid and/or glycolipid).

6. Use of a variant lipid acyltransferase enzyme according to claim 1 in a process for reducing the content of a phospholipid in an edible oil, comprising treating the oil with said variant lipid acyltransferase enzyme so as to hydrolyse a major part of the phospholipid, and separating an aqueous phase containing the hydrolysed phospholipid from the oil.

7. Use of a variant lipid acyltransferase enzyme according to claim 1 in the bioconversion of polar lipids (preferably glycolipids) to make high value products, such as carbohydrate esters and/or protein esters and/or protein subunit esters and/or a hydroxy acid ester.

8. An immobilised variant lipid acyltransferase enzyme according to claim 1.

## Patentansprüche

1. Variante Lipid-Acyltransferase mit erhöhter Phospholipid-Transferaseaktivität, wobei das Enzym das Aminosäuresequenzmotiv GDSX umfasst, wobei X einer oder mehrere der folgenden Aminosäurereste L, A, V, I, F, Y, H, Q, T, N, M oder S ist; wobei das variante Enzym eine oder mehrere Aminosäuremodifikationen verglichen mit einer parentalen Sequenz an einem beliebigen der einen oder mehreren Aminosäurereste umfasst:

   S3N; Q, K, R, P oder M; oder
   E309A;

   wobei die Nummerierung die ist, die erhalten wird von Alignierung der varianten Sequenz mit der als SEQ ID No. 2 dargestellten Referenzsequenz und wobei die parentale Lipid-Acyltransferase die als SEQ ID No. 28 dargestellte Aminosäuresequenz umfasst oder eine Aminosäuresequenz, die 75% oder mehr Identität mit SEQ ID No. 28 aufweist; oder wobei die parentale Lipid-Acyltransferase durch die als SEQ ID No. 29 dargestellte Nucleotidsequenz codiert ist, oder eine Nucleotidsequenz, die mindestens 75% oder mehr Identität mit der als SEQ ID No. 29 dargestellten Sequenz aufweist.

2. Verfahren zur Zubereitung eines Lebensmittels, wobei das Verfahren das Zusetzen einer varianten Lipid-Acyltransferase-Enzyms nach Anspruch 1 zu einem oder mehreren Inhaltsstoffen des Lebensmittels umfasst.

3. Verfahren zur Zubereitung einer Backware aus einem Teig, wobei das Verfahren das Zugeben eines varianten Lipid-Acyltransferase-Enzyms nach Anspruch 1 zu dem Teig umfasst.

4. Verwendung eines varianten Lipid-Acyltransferase-Enzyms nach Anspruch 1 in einem Verfahren zum Behandeln von Ei oder Produkten auf Eierbasis, um Lysophospholipide herzustellen.

5. Verfahren zum enzymatischen Degummieren von Pflanzen- oder Speiseölen, umfassend Behandeln des Speise- oder Pflanzenöls mit einem varianten Glykolipid-Acyltransferase-Enzym nach Anspruch 1, um einen Hauptteil der polaren Lipide (z.B. Phospholipid und/oder Glykolipid) zu hydrolysieren.

6. Verwendung eines Glykolipid-Acyltransferase-Enzyms nach Anspruch 1 in einem Verfahren zum Verringern des Gehalts an einem Phospholipid in einem Speiseöl, umfassend Behandeln des Öls mit dem varianten Lipid-Acyltransferase-Enzym, um einen Hauptteil des Phospholipids zu hydrolysieren, und Abtrennen einer wässrigen Phase, die das hydrolysierte Phospholipid aus dem Öl enthält.

7. Verwendung eines Glykolipid-Acyltransferase-Enzyms nach Anspruch 1 in der Biokonversion von polaren Lipiden (bevorzugt Glykolipiden), um hochwertige Produkte, wie z.B. Kohlenhydratester und/oder Proteinester und/oder Protein-Untereinheit-Ester und/oder einen Hydroxycarbonsäureester, zu erzeugen.

**8.** Immobilisiertes variantes Glykolipid-Acyltransferase-Enzym Anspruch 1.

**Revendications**

**1.** Acyltransférase lipide variante présentant une activité transférase phospholipide accrue dans laquelle l'enzyme comprend le motif de séquences d'acides aminés GDSX, où X est un ou plusieurs des restes ou résidus d'acides aminés suivants L, A, V, I, F, Y, H, Q, T, N, M ou S, dans laquelle l'enzyme variante comprend une ou plusieurs modification(s) d'acides aminés par comparaison avec une séquence parente au niveau de l'un quelconque ou de plusieurs des résidus d'acides aminés :

S3N, Q, K, R, P ou M; ou
E309A;

dans laquelle la numérotation est celle obtenue à partir d'un alignement de la séquence variante avec la séquence de référence présentée sous la forme SEQ ID No. 2 et dans laquelle l'acyltransférase lipide parente comprend la séquence d'acides aminés présentée sous la forme SEQ ID No. 28 ou une séquence d'acides aminés qui présente 75% d'identité ou plus avec SEQ ID No. 28 ; ou dans laquelle l'acyltransférase lipide parente est codée par la séquence de nucléotides présentée sous la forme SEQ ID No. 29, ou une séquence de nucléotides qui présente au moins 75% d'identité ou plus avec la séquence présentée sous la forme SEQ ID No. 29.

**2.** Procédé de préparation d'une denrée alimentaire, le procédé comprenant l'addition d'une enzyme acyltransférase lipide variante selon la revendication 1 à un ou plusieurs ingrédient(s) de la denrée alimentaire.

**3.** Procédé de préparation d'un produit cuit à partir d'une pâte, le procédé comprenant l'addition d'une enzyme acyltransférase lipide variante selon la revendication 1 à la pâte.

**4.** Utilisation d'une enzyme acyltransférase lipide variante selon la revendication 1 dans un processus de traitement d'ovoproduits ou de produits à base d'oeufs afin de produire des lysophospholipides.

**5.** Processus de dégommage enzymatique d'huiles végétales ou comestibles, comprenant le traitement de l'huile comestible ou de l'huile végétale à l'aide d'une enzyme acyltransférase lipide variante selon la revendication 1 de manière à hydrolyser une partie majeure des lipides polaires (par exemple phospholipide et/ou glycolipide).

**6.** Utilisation d'une enzyme acyltransférase lipide variante selon la revendication 1 au niveau d'un processus pour réduire la teneur en un phospholipide dans une huile comestible, comprenant le traitement de l'huile à l'aide de ladite enzyme acyltransférase lipide variante de manière à hydrolyser une partie majeure du phospholipide, et la séparation d'une phase aqueuse qui contient le phospholipide hydrolysé à partir de huile.

**7.** Utilisation d'une enzyme acyltransférase lipide variante selon la revendication 1 dans la bioconversion de lipides polaires (de préférence des glycolipides) afin de fabriquer des produits de haute valeur tels que des esters d'hydrates de carbone et/ou des esters de protéines et/ou des esters de sous-unités de protéines et/ou un ester d'hydroxy acide.

**8.** Enzyme acyltransférase lipide variante immobilisée selon la revendication 1.

Figure 1

SEQ ID No. 1

```
  1 ivafGDSlTd geayygdsdg ggwgagladr Ltallrlrar prgvdvfnrg isGrtsdGrl
 61 ivDalvallF laqslglpnL pPYLsgdflr GANFAsagAt Ilptsgpfli QvqFkdfksq
121 vlelrqalgl lqellrllpv ldakspdlvt imiGtNDlit saffgpkste sdrnvsvpef
181 kdnlrqlikr Lrsnngarii vlitlvilnl gplGClPlkl alalassknv dasgclerln
241 eavadfneal relaiskled qlrkdglpdv kgadvpyvDl ysifqdldgi qnpsayvyGF
301 ettkaCCGyG gryNynrvCG naglcnvtak aCnpssylls flfwDgfHps ekGykavAea
361 l
```

Figure 2

SEQ ID No. 2

ADSRPAFSRIVMFGDSLSDTGKMYSKMRGYLPSSPPYYEGRFSNGPVWLEQLTNEF
PGLTIANEAEGGPTAVAYNKISWNPKYQVINNLDYEVTQFLQKDSFKPDDLVILWVGA
NDYLAYGWNTEQDAKRVRDAISDAANRMVLNGAKEILLFNLPDLGQNPSARSQKVV
EAASHVSAYHNQLLLNLARQLAPTGMVKLFEIDKQFAEMLRDPQNFGLSDQRNACY
GGSYVWKPFASRSASTDSQLSAFNPQERLAIAGNPLLAQAVASPMAARSASTLNCE
GKMFWDQVHPTTVVHAALSEPAATFIESQYEFLAH

Figure 3

SEQ ID No. 3

```
  1 mkkwfvcllg lialtvqaad trpafsrivm fgdslsdtgk myskmrgylp ssppyyegrf
 61 sngpvwleql tkqfpgltia neaeggatav aynkiswnpk yqvynnldye vtqflqkdsf
121 kpddlvilwv gandylaygw nteqdakrvr daisdaanrm vlngakqill fnlpdlgqnp
181 sarsqkvvea vshvsayhnk lllnlarqla ptgmvklfei dkqfaemlrd pqnfglsdve
241 npcydggyvw kpfatrsvst drqlsafspq erlaiagnpl laqavaspma rrsasplnce
301 gkmfwdqvhp ttvvhaalse raatfietqy eflahg
```

Figure 4

SEQ ID No. 4

```
  1 mpkpalrrvm tatvaavgtl algltdatah aapaqatptl dyvalgdsys agsgvlpvdp
 61 anllclrsta nyphviadtt garltdvtcg aaqtadftra qypgvapqld algtgtdlvt
121 ltiggndnst finaitacgt agvlsggkgs pckdrhgtsf ddeieantyp alkeallgvr
181 arapharvaa lgypwitpat adpscflklp laagdvpylr aiqahlndav rraaeetgat
241 yvdfsgvsdg hdaceapgtr wiepllfghs lvpvhpnalg errmaehtmd vlgld
```

Figure 5

SEQ ID No. 5

```
  1 mpkpalrrvm tatvaavgtl algltdatah aapaqatptl dyvalgdsys agsgvlpvdp
 61 anllclrsta nyphviadtt garltdvtcg aaqtadftra qypgvapqld algtgtdlvt
121 ltiggndnst finaitacgt agvlsggkgs pckdrhgtsf ddeieantyp alkeallgvr
181 arapharvaa lgypwitpat adpscflklp laagdvpylr aiqahlndav rraaeetgat
241 yvdfsgvsdg hdaceapgtr wiepllfghs lvpvhpnalg errmaehtmd vlgld
```

Figure 6

SEQ ID No. 6

```
  1 mdyekfllfg dsitefafnt rpiedgkdqy algaalvney trkmdilqrg fkgytsrwal
 61 kilpeilkhe snivmatifl gandacsagp qsvplpefid nirqmvslmk syhirpiiig
121 pglvdrekwe kekseeialg yfrtnenfai ysdalaklan eekvpfvaln kafqqeggda
181 wqqlltdglh fsgkgykifh dellkvietf ypqyhpknmq yklkdwrdvl ddgsnims
```

Figure 7

```
Alignment of pfam00657.6 consensus sequence with P10480
                      *->ivafGDSlTdg................eayygdsdgggwgagladrL
                            iv+fGDSl+d+++  ++ ++  +++++++ +++s+g  w ++l + +
    P10480      28     IVMFGDSLSDTgkmyskmrgylpssppYYEGRFSNGPVWLEQLTNEF 74

                       tall..rlrarprgvdvfnrgisGrtsdGrlivDalvallFlaqslglpn
                       + l    + ++++++++ +n+  +
    P10480      75 PGLTiaNEAEGGPTAVAYNKISWNPK------------------------ 100

                       LpPYLsgdflrGANFAsagAtIlptsgpfliQvqFkdfksqvlelrqalg
                                                                ++  ++
    P10480     101 --------------------------------------------YQVINN 106

                       llqellrllpvldakspdlvtimiGtNDlitsaffgpkstesdrnvsvpe
                       l++e+ ++l +++ k+ dlv++++G+ND+          ++ ++ ++++++
    P10480     107 LDYEVTQFLQKDSFKPDDLVILWVGANDY--------LAYGWNTEQDAKR 148

                       fkdnlrqlikrLrsnngariivlitlvilnlgplGClPlklalalasskn
                       ++d +++++++r+   nga+      ++++nl+ lG+ P+
    P10480     149 VRDAISDAANRMV-LNGAK-----EILLFNLPDLGQNPS----------- 181

                       vdasgclerlneavadfnealrelaiskledqlrkdglpdvkgadvpyvD
                        ++++ +e +  ++a++n++l +la     +ql+++g+++++++d ++++
    P10480     182 ARSQKVVEAASHVSAYHNQLLLNLA-----RQLAPTGMVKLFEIDKQFAE 226

                       lysifqdldgiqnpsayv.y....GFe.ttkaCCGyGgr.yNyn.rv.CG
                       +   +q+++ + + +a+++++   +++ +++a+++++++ +N+++r+ ++
    P10480     227 MLRDPQNFGLSDQRNACYgGsyvwKPFaSRSASTDSQLSaFNPQeRLaIA 276

                       nag.l.c.nvtakaC.npssyll.sflfwDgfHpsekGykavAeal<-*
                       +++ l +  ++++a++ +s+ ++++++fwD++Hp+   ++a+ e
    P10480     277 GNPlLaQaVASPMAArSASTLNCeGKMFWDQVHPTTVVHAALSEPA    322

 Alignment of pfam00657.6 consensus sequence with AAG09804
                       *->ivafGDSlTdg................eayygdsdgggwgagladrL
                             iv+fGDSl+d+++  ++ ++  +++++++ +++s+g  w ++l + +
  AAG09804      28      IVMFGDSLSDTgkmyskmrgylpssppYYEGRFSNGPVWLEQLTKQF 74

                       tallrlrarprgvdvfnrgisGrtsdGrlivDalvallFlaqslglpnLp
                                 +g+++ n + +G+t
  AAG09804      75 ----------PGLTIANEAEGGAT-------------------------- 88

                       PYLsgdflrGANFAsagAtIlptsgpfliQvqFkdfksqvlelrqa....
                                                           ++++ + ++++ +
  AAG09804      89 ------------------------------------AVAYNKISWNpkyq 102

                       ..lgllqellrllpvldakspdlvtimiGtNDlitsaffgpkstesdrnv
                         ++l++e+ ++l +++ k+ dlv++++G+ND+          ++ ++ ++
  AAG09804     103 vyNNLDYEVTQFLQKDSFKPDDLVILWVGANDY--------LAYGWNTEQ 144

                       svpefkdnlrqlikrLrsnngariivlitlvilnlgplGClPlklalala
                       ++++++d +++++++r+   nga+      +++++nl+ lG+ P+
  AAG09804     145 DAKRVRDAISDAANRMV-LNGAK-----QILLFNLPDLGQNPS------- 181

                       ssknvdasgclerlneavadfnealrelaiskledqlrkdglpdvkgadv
                           ++++ +e +  ++a++n++l +la     +ql+++g+++++++d
  AAG09804     182 ----ARSQKVVEAVSHVSAYHNKLLLNLA-----RQLAPTGMVKLFEIDK 222

                       pyvDlysifqdldgiqnpsayv.y....GFe.ttkaCCGyGgr.yNyn.r
                       +++++   +q+++ +  ++ +++++   +++ t++ +++ +++ + +++r
  AAG09804     223 QFAEMLRDPQNFGLSDVENPCYdGgyvwKPFaTRSVSTDRQLSaFSPQeR 272

                       v.CGnag.l.c.nvtakaC.npssyll.sflfwDgfHpsekGykavAeal
                       + +++++ l +  ++++a++ +s  ++++++fwD++Hp+   ++a+ e+
  AAG09804     273 LaIAGNPlLaQaVASPMARrSASPLNCeGKMFWDQVHPTTVVHAALSERA 322
```

```
                     <-*

AAG09804             -     -

Alignment of pfam00657.6 consensus sequence with NP_631558
                     *->ivafGDSlTdgeayygdsdgggwgagladrLtallrlrarprgvdvf
                        +va+GDS ++g          +g  +  +++L     + + + ++  +
   NP_631558    42      YVALGDSYSAG---------SGVLPVDPANL----LCLRSTANYPHV 75

                     nrgisGrtsdGrlivD.a.l.vallFlaqslglpnLpPYLsgdflrGANF
                      + ++G++       D + + +
   NP_631558    76   IADTTGAR-----LTDvTcGaAQ--------------------------- 93

                     AsagAtIlptsgpfliQvqFkdfksqvlelrqalgllqellrllpvldak
                                                +++    ++ +  ++ +++
   NP_631558    94   ----------------------------TADFTRAQYPGVAPQLDALGT 114

                     spdlvtimiGtNDl................itsaffgpkstesdrnvsvp
                     + dlvt+ iG+ND ++  +  +  ++ +   ++  + +k   ++ + +++
   NP_631558   115   GTDLVTLTIGGNDNstfinaitacgtagvlSGGKGSPCKDRHGTSFDDEI 164

                     efkdn..lrqlikrLrs.nngariivlitlvilnlg...........plG
                     e  +++ l++++  +r+++ +ar+ +l  ++i+++  +++   + +   G
   NP_631558   165   EANTYpaLKEALLGVRArAPHARVAALGYPWITPATadpscflklplAAG 214

                     ClPlklalalassknvdasgclerlneavadfnealrelaiskledqlrk
                      P+                    l+ ++a  n a+r  a
   NP_631558   215   DVPY-------------------LRAIQAHLNDAVRRAA---------- 234

                     dglpdvkgadvpyvDlysifqdldgiqnpsayvyGFettkaCCGyGgryN
                           ++ + +yvD+ ++
   NP_631558   235   ------EETGATYVDFSGVSDG--------------------------- 250

                     ynrvCGnaglcnvtakaC.npssyll.sflfwDgf...HpsekGykavAe
                                   ++aC+ p +++ +  lf + + + Hp++ G +++Ae
   NP_631558   251   --------------HDACeAPGTRWIePLLFGHSLvpvHPNALGERRMAE 286

                     al<-*
                     +
   NP_631558   287   HT    288

Alignment of pfam00657.6 consensus sequence with CAC42140
                     *->ivafGDSlTdgeayygdsdgggwgagladrLtallrlrarprgvdvf
                        +va+GDS ++g          +g  +  +++L     + + + ++  +
   CAC42140     42      YVALGDSYSAG---------SGVLPVDPANL----LCLRSTANYPHV 75

                     nrgisGrtsdGrlivD.a.l.vallFlaqslglpnLpPYLsgdflrGANF
                      + ++G++       D + + +
   CAC42140  .  76   IADTTGAR-----LTDvTcGaAQ--------------------------- 93

                     AsagAtIlptsgpfliQvqFkdfksqvlelrqalgllqellrllpvldak
                                                +++    ++ +  ++ +++
   CAC42140     94   ----------------------------TADFTRAQYPGVAPQLDALGT 114

                     spdlvtimiGtNDl................itsaffgpkstesdrnvsvp
                     + dlvt+ iG+ND ++  +  +  ++ +   ++  + +k   ++ + +++
   CAC42140    115   GTDLVTLTIGGNDNstfinaitacgtagvlSGGKGSPCKDRHGTSFDDEI 164

                     efkdn..lrqlikrLrs.nngariivlitlvilnlg...........plG
                     e  +++ l++++  +r+++ +ar+ +l  ++i+++  +++   + +   G
   CAC42140    165   EANTYpaLKEALLGVRArAPHARVAALGYPWITPATadpscflklplAAG 214

                     ClPlklalalassknvdasgclerlneavadfnealrelaiskledqlrk
                      P+                    l+ ++a  n a+r  a
   CAC42140    215   DVPY-------------------LRAIQAHLNDAVRRAA---------- 234

                     dglpdvkgadvpyvDlysifqdldgiqnpsayvyGFettkaCCGyGgryN
                           ++ + +yvD+ ++
   CAC42140    235   ------EETGATYVDFSGVSDG--------------------------- 250
```

```
ynrvCGnaglcnvtakaC.npssyll.sflfwDgf...HpsekGykavAe
                                 ++aC+ p +++ +  lf + + + Hp++ G +++Ae
    CAC42140   251 --------------HDACeAPGTRWIePLLFGHSLvpvHPNALGERRMAE 286

                   al<-*
                   +
    CAC42140   287 HT    288

Alignment of pfam00657.6 consensus sequence with P41734
                  *->ivafGDSlTdg....eayygdsdgggwgagladrLtallrlrarprg
                        ++fGDS+T+   +++ + +  d+   ga+l + +          +r+
      P41734     6    FLLFGDSITEFafntRPIEDGKDQYALGAALVNEY---------TRK 43

                   vdvfnrgisGrtsdGrlivDalvallFlaqslglpnLpPYLsgdflrGAN
                   +d+  rg++G+t
      P41734    44 MDILQRGFKGYT-------------------------------------- 55

                   FAsagAtIlptsgpfliQvqFkdfksqvlelrqalgllqellrllpvlda
                                                +r+al++l+e+l+      +
      P41734    56 ------------------------------SRWALKILPEILKH-----E 70

                   kspdlvtimiGtNDlitsaffgpkstesdrnvsvpefkdnlrqlikrLrs
                    +  + ti++G+ND+         ++ +++ v++pef+dn+rq++++++s
      P41734    71 SNIVMATIFLGANDA---------CSAGPQSVPLPEFIDNIRQMVSLMKS 111

                   nngariivlitlvilnlgplGClPlklalalassknvdasgclerlneav
                    ++++ii++++lv   ++               ++ k ++ +  + r+ne +
      P41734   112 YHIRPIIIGPGLVDREKW-------------EKEKSEEIALGYFRTNENF 148

                   adfnealrelaiskledqlrkdglpdvkgadvpyvDlysifqdldgiqnp
                   a +  al +la                 ++ +vp+v l+++fq+ +g++++
      P41734   149 AIYSDALAKLA----------------NEEKVPFVALNKAFQQEGGDAWQ 182

                   sayvyGFettkaCCGyGgryNynrvCGnaglcnvtakaCnpssyllsflf
                   +                                                l+
      P41734   183 Q------------------------------------------------LL 185

                   wDgfHpsekGykavAeal<-*
                    Dg+H+s kGyk+++++l
      P41734   186 TDGLHFSGKGYKIFHDEL    203
```

Figure 8

```
A.sal   1  MKKWFVCLLGLIALTVQAADTRPAFSRIVMFGDSLSDTGKMYSKMRGYLPSSPPYYEGRF 60
                      +        +
A.hyd   1  MKKWFVCLLGLVALTVQAADSRPAFSRIVMFGDSLSDTGKMYSKMRGYLPSSPPYYEGRF 60

A. sal 61  SNGPVWLEQLTKQFPGLTIANEAEGGATAVAYNKISWNPKYQVINNLDYEVTQFLQKDSF 120
                      ++             +
A. hyd 61  SNGPVWLEQLTNEFPGLTIANEAEGGPTAVAYNKISWNPKYQVINNLDYEVTQFLQKDSF 120

A. sal 121 KPDDLVILWVGANDYLAYGWNTEQDAKRVRDAISDAANRMVLNGAKQILLFNLPDLGQNP 180
                                                                      +
A. hyd 121 KPDDLVILWVGANDYLAYGWNTEQDAKRVRDAISDAANRMVLNGAKEILLFNLPDLGQNP 180

A. sal 181 SARSQKVVEAVSHVSAYHNKLLLNLARQLAPTGMVKLFEIDKQFAEMLRDPQNFGLSDVE 240
                     +        +                                              ++
A.hyd  181 SARSQKVVEAASHVSAYHNQLLLNLARQLAPTGMVKLFEIDKQFAEMLRDPQNFGLSDQR 240

A. sal 241 NPCYDGGYVWKPFATRSVSTDRQLSAFSPQERLAIAGNPLLAQAVASPMARRSASPLNCE 300
            + ++ +       +  +   +     +                      +    +
A. hyd 241 NACYGGSYVWKPFASRSASTDSQLSAFNPQERLAIAGNPLLAQAVASPMAARSASTLNCE 300

A. sal 301 GKMFWDQVHPTTVVHAALSERAATFIETQYEFLAH 335
                               +      +
A. hyd 301 GKMFWDQVHPTTVVHAALSEPAATFIESQYEFLAH 335
```

Figure 9

(SEQ ID No. 7)

```
  1  ATGAAAAAAT GGTTTGTGTG TTTATTGGGA TTGGTCGCGC TGACAGTTCA GGCAGCCGAC
 61  AGCCGTCCCG CCTTCTCCCG GATCGTGATG TTTGGCGACA GCCTCTCCGA TACCGGCAAG
121  ATGTACAGCA AGATGCGCGG TTACCTCCCC TCCAGCCCCC CCTACTATGA GGGCCGCTTC
181  TCCAACGGGC CCGTCTGGCT GGAGCAGCTG ACCAACGAGT TCCCGGGCCT GACCATAGCC
241  AACGAGGCGG AAGGCGGACC GACCGCCGTG GCTTACAACA AGATCTCCTG GAATCCCAAG
301  TATCAGGTCA TCAACAACCT GGACTACGAG GTCACCCAGT TCCTGCAAAA AGACAGCTTC
361  AAGCCGGACG ATCTGGTGAT CCTCTGGGTC GGCGCCAACG ACTATCTGGC CTATGGCTGG
421  AACACAGAGC AGGATGCCAA GCGGGTGCGC GACGCCATCA GCGATGCGGC CAACCGCATG
481  GTGCTGAACG GCGCCAAGGA GATACTGCTG TTCAACCTGC CGGATCTGGG CCAGAACCCC
541  TCGGCCCGCA GCCAGAAGGT GGTCGAGGCG GCCAGCCATG TCTCCGCCTA CCACAACCAG
601  CTGCTGCTGA ACCTGGCACG CCAGCTGGCT CCCACCGGCA TGGTGAAGCT GTTCGAGATC
661  GACAAGCAGT TTGCCGAGAT GCTGCGTGAT CCGCAGAACT TCGGCCTGAG CGACCAGAGG
721  AACGCCTGCT ACGGTGGCAG CTATGTATGG AAGCCGTTTG CCTCCCGCAG CGCCAGCACC
781  GACAGCCAGC TCTCCGCCTT CAACCCGCAG GAGCGCCTCG CCATCGCCGG CAACCCGCTG
841  CTGGCCCAGG CCGTCGCCAG CCCCATGGCT GCCCGCAGCG CCAGCACCCT CAACTGTGAG
901  GGCAAGATGT TCTGGGATCA GGTCCACCCC ACCACTGTCG TGCACGCCGC CCTGAGCGAG
961  CCCGCCGCCA CCTTCATCGA GAGCCAGTAC GAGTTCCTCG CCCAC
```

Figure 10

(SEQ ID No. 8)

```
  1  ATGAAAAAAT GGTTTGTTTG TTTATTGGGG TTGATCGCGC TGACAGTTCA GGCAGCCGAC
 61  ACTCGCCCCG CCTTCTCCCG GATCGTGATG TTCGGCGACA GCCTCTCCGA TACCGGCAAA
121  ATGTACAGCA AGATGCGCGG TTACCTCCCC TCCAGCCCGC CCTACTATGA GGGCCGTTTC
181  TCCAACGGAC CCGTCTGGCT GGAGCAGCTG ACCAAGCAGT TCCCGGGTCT GACCATCGCC
241  AACGAAGCGG AAGGCGGTGC CACTGCCGTG GCTTACAACA AGATCTCCTG GAATCCCAAG
301  TATCAGGTCT ACAACAACCT GGACTACGAG GTCACCCAGT TCTTGCAGAA AGACAGCTTC
361  AAGCCGGACG ATCTGGTGAT CCTCTGGGTC GGTGCCAATG ACTATCTGGC ATATGGCTGG
421  AATACGGAGC AGGATGCCAA GCGAGTTCGC GATGCCATCA GCGATGCGGC CAACCGCATG
481  GTACTGAACG GTGCCAAGCA GATACTGCTG TTCAACCTGC CGGATCTGGG CCAGAACCCG
541  TCAGCCCGCA GTCAGAAGGT GGTCGAGGCG GTCAGCCATG TCTCCGCCTA TCACAACAAG
601  CTGCTGCTGA ACCTGGCACG CCAGCTGGCC CCCACCGGCA TGGTAAAGCT GTTCGAGATC
661  GACAAGCAAT TTGCCGAGAT GCTGCGTGAT CCGCAGAACT TCGGCCTGAG CGACGTCGAG
721  AACCCCTGCT ACGACGGCGG CTATGTGTGG AAGCCGTTTG CCACCCGCAG CGTCAGCACC
781  GACCGCCAGC TCTCCGCCTT CAGTCCGCAG GAACGCCTCG CCATCGCCGG CAACCCGCTG
841  CTGGCACAGG CCGTTGCCAG TCCTATGGCC CGCCGCAGCG CCAGCCCCCT CAACTGTGAG
901  GGCAAGATGT TCTGGGATCA GGTACACCCG ACCACTGTCG TGCACGCAGC CCTGAGCGAG
961  CGCGCCGCCA CCTTCATCGA GACCCAGTAC GAGTTCCTCG CCCACGGATG A
```

Figure 11

(SEQ ID No. 9)

```
  1  ATGCCGAAGC CTGCCCTTCG CCGTGTCATG ACCGCGACAG TCGCCGCCGT CGGCACGCTC
 61  GCCCTCGGCC TCACCGACGC CACCGCCCAC GCCGCGCCCG CCCAGGCCAC TCCGACCCTG
121  GACTACGTCG CCCTCGGCGA CAGCTACAGC GCCGGCTCCG GCGTCCTGCC CGTCGACCCC
181  GCCAACCTGC TCTGTCTGCG CTCGACGGCC AACTACCCCC ACGTCATCGC GGACACGACG
241  GGCGCCCGCC TCACGGACGT CACCTGCGGC GCCGCGCAGA CCGCCGACTT CACGCGGGCC
301  CAGTACCCGG GCGTCGCACC CCAGTTGGAC GCGCTCGGCA CCGGCACGGA CCTGGTCACG
361  CTCACCATCG GCGGCAACGA CAACAGCACC TTCATCAACG CCATCACGGC CTGCGGCACG
421  GCGGGTGTCC TCAGCGGCGG CAAGGGCAGC CCCTGCAAGG ACAGGCACGG CACCTCCTTC
481  GACGACGAGA TCGAGGCCAA CACGTACCCC GCGCTCAAGG AGGCGCTGCT CGGCGTCCGC
541  GCCAGGGCTC CCCACGCCAG GGTGGCGGCT CTCGGCTACC CGTGGATCAC CCCGGCCACC
601  GCCGACCCGT CCTGCTTCCT GAAGCTCCCC CTCGCCGCCG GTGACGTGCC CTACCTGCGG
661  GCCATCCAGG CACACCTCAA CGACGCGGTC CGGCGGGCCG CCGAGGAGAC CGGAGCCACC
721  TACGTGGACT TCTCCGGGGT GTCCGACGGC CACGACGCCT GCGAGGCCCC CGGCACCCGC
781  TGGATCGAAC CGCTGCTCTT CGGGCACAGC CTCGTTCCCG TCCACCCCAA CGCCCTGGGC
841  GAGCGGCGCA TGGCCGAGCA CACGATGGAC GTCCTCGGCC TGGACTGA
```

Figure 12

(SEQ ID No. 10)

```
  1  TCAGTCCAGG CCGAGGACGT CCATCGTGTG CTCGGCCATG CGCCGCTCGC CCAGGGCGTT
 61  GGGGTGGACG GGAACGAGGC TGTGCCCGAA GAGCAGCGGT TCGATCCAGC GGGTGCCGGG
121  GGCCTCGCAG GCGTCGTGGC CGTCGGACAC CCCGGAGAAG TCCACGTAGG TGGCTCCGGT
181  CTCCTCGGCG GCCCGCCGGA CCGCGTCGTT GAGGTGTGCC TGGATGGCCC GCAGGTAGGG
241  CACGTCACCG GCGGCGAGGG GGAGCTTCAG GAAGCAGGAC GGGTCGGCGG TGGCCGGGGT
301  GATCCACGGG TAGCCGAGAG CCGCCACCCT GGCGTGGGGA GCCCTGGCGC GGACGCCGAG
361  CAGCGCCTCC TTGAGCGCGG GGTACGTGTT GGCCTCGATC TCGTCGTCGA AGGAGGTGCC
421  GTGCCTGTCC TTGCAGGGGC TGCCCTTGCC GCCGCTGAGG ACACCCGCCG TGCCGCAGGC
481  CGTGATGGCG TTGATGAAGG TGCTGTTGTC GTTGCCGCCG ATGGTGAGCG TGACCAGGTC
541  CGTGCCGGTG CCGAGCGCGT CCAACTGGGG TGCGACGCCC GGGTACTGGG CCCGCGTGAA
601  GTCGGCGGTC TGCGCGGCGC CGCAGGTGAC GTCCGTGAGG CGGGCGCCCG TCGTGTCCGC
661  GATGACGTGG GGGTAGTTGG CCGTCGAGCG CAGACAGAGC AGGTTGGCGG GGTCGACGGG
721  CAGGACGCCG GAGCCGGCGC TGTAGCTGTC GCCGAGGGCG ACGTAGTCCA GGGTCGGAGT
781  GGCCTGGGCG GGCGCGGCGT GGGCGGTGGC GTCGGTGAGG CCGAGGGCGA GCGTGCCGAC
841  GGCGGCGACT GTCGCGGTCA TGACACGGCG AAGGGCAGGC TTCGGCAT
```

Figure 13

(SEQ ID No. 11)

```
  1 ATGGATTACG AGAAGTTTCT GTTATTTGGG GATTCCATTA CTGAATTTGC TTTTAATACT
 61 AGGCCCATTG AAGATGGCAA AGATCAGTAT GCTCTTGGAG CCGCATTAGT CAACGAATAT
121 ACGAGAAAAA TGGATATTCT TCAAAGAGGG TTCAAAGGGT ACACTTCTAG ATGGGCGTTG
181 AAAATACTTC CTGAGATTTT AAAGCATGAA TCCAATATTG TCATGGCCAC AATATTTTTG
241 GGTGCCAACG ATGCATGCTC AGCAGGTCCC CAAAGTGTCC CCCTCCCCGA ATTTATCGAT
301 AATATTCGTC AAATGGTATC TTTGATGAAG TCTTACCATA TCCGTCCTAT TATAATAGGA
361 CCGGGGCTAG TAGATAGAGA GAAGTGGGAA AAAGAAAAAT CTGAAGAAAT AGCTCTCGGA
421 TACTTCCGTA CCAACGAGAA CTTTGCCATT TATTCCGATG CCTTAGCAAA ACTAGCCAAT
481 GAGGAAAAAG TTCCCTTCGT GGCTTTGAAT AAGGCGTTTC AACAGGAAGG TGGTGATGCT
541 TGGCAACAAC TGCTAACAGA TGGACTGCAC TTTTCCGGAA AAGGGTACAA AATTTTTCAT
601 GACGAATTAT TGAAGGTCAT TGAGACATTC TACCCCCAAT ATCATCCCAA AAACATGCAG
661 TACAAACTGA AAGATTGGAG AGATGTGCTA GATGATGGAT CTAACATAAT GTCTTGA
```

Figure 14

(SEQ ID No. 12)

```
        10         20         30         40         50         60
         |          |          |          |          |          |
MNLRQWMGAA TAALALGLAA CGGGGTDQSG NPNVAKVQRM VVFGDSLSDI GTYTPVAQAV

        70         80         90        100        110        120
         |          |          |          |          |          |
GGGKFTTNPG PIWAETVAAQ LGVTLTPAVM GYATSVQNCP KAGCFDYAQG GSRVTDPNGI

       130        140        150        160        170        180
         |          |          |          |          |          |
GHNGGAGALT YPVQQQLANF YAASNNTFNG NNDVVFVLAG SNDIFFWTTA AATSGSGVTP

       190        200        210        220        230        240
         |          |          |          |          |          |
AIATAQVQQA ATDLVGYVKD MIAKGATQVY VFNLPDSSLT PDGVASGTTG QALLHALVGT

       250        260        270        280        290        300
         |          |          |          |          |          |
FNTTLQSGLA GTSARIIDFN AQLTAAIQNG ASFGFANTSA RACDATKINA LVPSAGGSSL

       310        320        330        340
         |          |          |          |
FCSANTLVAS GADQSYLFAD GVHPTTAGHR LIASNVLARL LADNVAH
```

Figure 15

(SEQ ID No. 13)

```
atgaacctgc gtcaatggat gggcgccgcc acggctgccc ttgccttggg cttggccgcg      60
tgcgggggcg gtgggaccga ccagagcggc aatcccaatg tcgccaaggt gcagcgcatg     120
gtggtgttcg gcgacagcct gagcgatatc ggcacctaca cccccgtcgc gcaggcggtg     180
ggcggcggca agttcaccac caacccgggc ccgatctggg ccgagaccgt ggccgcgcaa     240
ctgggcgtga cgctcacgcc ggcggtgatg ggctacgcca cctccgtgca gaattgcccc     300
aaggccggct gcttcgacta tgcgcagggc ggctcgcgcg tgaccgatcc gaacggcatc     360
ggccacaacg gcggcgcggg ggcgctgacc tacccggttc agcagcagct cgccaacttc     420
tacgcggcca gcaacaacac attcaacggc aataacgatg tcgtcttcgt gctggccggc     480
agcaacgaca ttttcttctg gaccactgcg gcggccacca gcggctccgg cgtgacgccc     540
gccattgcca cggcccaggt gcagcaggcc gcgacggacc tggtcggcta tgtcaaggac     600
atgatcgcca agggtgcgac gcaggtctac gtgttcaacc tgcccgacag cagcctgacg     660
ccggacggcg tggcaagcgg cacgaccggc caggcgctgc tgcacgcgct ggtgggcacg     720
ttcaacacga cgctgcaaag cgggctggcc ggcacctcgg cgcgcatcat cgacttcaac     780
gcacaactga ccgcggcgat ccagaatggc gcctcgttcg gcttcgccaa caccagcgcc     840
cgggcctgcg acgccaccaa gatcaatgcc ctggtgccga gcgccggcgg cagctcgctg     900
ttctgctcgg ccaacacgct ggtggcttcc ggtgcggacc agagctacct gttcgccgac     960
ggcgtgcacc cgaccacggc cggccatcgc ctgatcgcca gcaacgtgct ggcgcgcctg    1020
ctggcggata acgtcgcgca ctga                                          1044
```

Figure 16 (SEQ ID No. 14)

```
  1 migsyvavgd sftegvgdpg pdgafvgwad rlavlladrr pegdftytnl avrgrlldqi
 61 vaeqvprvvg lapdlvsfaa ggndiirpgt dpdevaerfe lavaaltaaa gtvlvttgfd
121 trgvpvlkhl rgkiatyngh vraiadrygc pvldlwslrs vqdrrawdad rlhlspeght
181 rvalragqal glrvpadpdq pwpplpprgt ldvrrddvhw areylvpwig rrlrgessgd
241 hvtakgtlsp daiktriaav a
```

Figure 17 (SEQ ID No. 15)

```
  1 gtgatcgggt cgtacgtggc ggtgggggac agcttcaccg agggcgtcgg cgaccccggc
 61 cccgacgggg cgttcgtcgg ctgggccgac cggctcgccg tactgctcgc ggaccggcgc
121 cccgagggcg acttcacgta cacgaacctc gccgtgcgcg gcaggctcct cgaccagatc
181 gtggcggaac aggtcccgcg ggtcgtcgga ctcgcgcccg acctcgtctc gttcgcggcg
241 ggcggcaacg acatcatccg gcccggcacc gatcccgacg aggtcgccga gcggttcgag
301 ctggcggtgg ccgcgctgac cgccgcggcc ggaaccgtcc tggtgaccac cgggttcgac
361 acccgggggg tgcccgtcct caagcacctg cgcggcaaga tcgccacgta caacgggcac
421 gtccgcgcca tcgccgaccg ctacggctgc ccggtgctcg acctgtggtc gctgcggagc
481 gtccaggacc gcagggcgtg ggacgccgac cggctgcacc tgtcgccgga ggggcacacc
541 cgggtggcgc tgcgcgcggg gcaggccctg ggcctgcgcg tcccggccga ccctgaccag
601 ccctggccgc ccctgccgcc gcgcggcacg ctcgacgtcc ggcgcgacga cgtgcactgg
661 gcgcgcgagt acctggtgcc gtggatcggg cgccggctgc ggggcgagtc gtcgggcgac
721 cacgtgacgg ccaaggggac gctgtcgccg gacgccatca agacgcggat cgccgcggtg
781 gcctga
```

Figure 18
(SEQ ID No. 16)

```
  1 mqtnpaytsl vavgdsfteg msdllpdgsy rgwadllatr maarspgfry anlavrgkli
 61 gqivdeqvdv aaamgadvit lvgglndtlr pkcdmarvrd lltqaverla phceqlvlmr
121 spgrqgpvle rfrprmealf aviddlagrh gavvvdlyga qsladprmwd vdrlhltaeg
181 hrrvaeavwq slghepedpe whapipatpp pgwvtrrtad vrfarqhllp wigrrltgrs
241 sgdglpakrp dllpyedpar
```

Figure 19 (SEQ ID No. 17)

```
  1 atgcagacga accccgcgta caccagtctc gtcgccgtcg gcgactcctt caccgagggc
 61 atgtcggacc tgctgcccga cggctcctac cgtggctggg ccgacctcct cgccacccgg
121 atggcggccc gctcccccgg cttccggtac gccaacctgg cggtgcgcgg gaagctgatc
181 ggacagatcg tcgacgagca ggtggacgtg gccgccgcca tgggagccga cgtgatcacg
241 ctggtcggcg ggctcaacga cacgctgcgg cccaagtgcg acatggcccg ggtgcgggac
301 ctgctgaccc aggccgtgga acggctcgcc ccgcactgcg agcagctggt gctgatgcgc
361 agtcccggtc gccagggtcc ggtgctggag cgcttccggc cccgcatgga ggccctgttc
421 gccgtgatcg acgacctggc cgggcggcac ggcgccgtgg tcgtcgacct gtacggggcc
481 cagtcgctgg ccgaccctcg gatgtgggac gtggaccggc tgcacctgac cgccgagggc
541 caccgccggg tcgcggaggc ggtgtggcag tcgctcggcc acgagcccga ggaccccgag
601 tggcacgcgc cgatcccggc gacgccgccg ccggggtggg tgacgcgcag gaccgcggac
661 gtccggttcg cccggcagca cctgctgccc tggataggcc gcaggctgac cgggcgctcg
721 tccggggacg gcctgccggc caagcgcccg gacctgctgc cctacgagga ccccgcacgg
781 tga
```

Figure 20 (SEQ ID No. 18)

```
  1 mtrgrdggag apptkhrall aaivtlivai saaiyagasa ddgsrdhalq aggrlprgda
 61 apastgawvg awatapaaae pgtettglag rsvrnvvhts vggtgaritl snlygqsplt
121 vthasialaa gpdtaaaiad tmrrltfggs arviipaggq vmsdtarlai pyganvlvtt
181 yspipsgpvt yhpqarqtsy ladgdrtadv tavayttptp ywryltaldv lsheadgtvv
241 afgdsitdga rsqsdanhrw tdvlaarlhe aagdgrdtpr ysvvnegisg nrlltsrpgr
301 padnpsglsr fqrdvlertn vkavvvvlgv ndvlnspela drdailtglr tlvdraharg
361 lrvvgatitp fggyggytea retmrqevne eirsgrvfdt vvdfdkalrd pydprrmrsd
421 ydsgdhlhpg dkgyarmgav idlaalkgaa pvka
```

Figure 21 (SEQ ID No. 19)

```
   1 atgacccggg gtcgtgacgg gggtgcgggg gcgcccccca ccaagcaccg tgccctgctc
  61 gcggcgatcg tcaccctgat agtggcgatc tccgcggcca tatacgccgg agcgtccgcg
 121 gacgacggca gcagggacca cgcgctgcag gccggaggcc gtctcccacg aggagacgcc
 181 gccccgcgt ccaccggtgc ctgggtgggc gcctgggcca ccgcaccggc cgcggccgag
 241 ccgggcaccg agacgaccgg cctggcgggc cgctccgtgc gcaacgtcgt gcacacctcg
 301 gtcggcggca ccggcgcgcg gatcaccctc tcgaacctgt acgggcagtc gccgctgacc
 361 gtcacacacg cctcgatcgc cctggccgcc gggcccgaca ccgccgccgc gatcgccgac
 421 accatgcgcc ggctcacctt cggcggcagc gcccgggtga tcatcccggc gggcggccag
 481 gtgatgagcg acaccgcccg cctcgccatc ccctacgggg cgaacgtcct ggtcaccacg
 541 tactccccca tcccgtccgg gccggtgacc taccatccgc aggcccggca gaccagctac
 601 ctggccgacg gcgaccgcac ggcggacgtc accgccgtcg cgtacaccac ccccacgccc
 661 tactggcgct acctgaccgc cctcgacgtg ctgagccacg aggccgacgg cacggtcgtg
 721 gcgttcggcg actccatcac cgacggcgcc cgctcgcaga gcgacgccaa ccaccgctgg
 781 accgacgtcc tcgccgcacg cctgcacgag gcggcgggcg acggccggga cacgccccgc
 841 tacagcgtcg tcaacgaggg catcagcggc aaccggctcc tgaccagcag gccggggcgg
 901 ccggccgaca acccgagcgg actgagccgg ttccagcggg acgtgctgga acgcaccaac
 961 gtcaaggccg tcgtcgtcgt cctcggcgtc aacgacgtcc tgaacagccc ggaactcgcc
1021 gaccgcgacg ccatcctgac cggcctgcgc accctcgtcg accgggcgca cgcccgggga
1081 ctgcgggtcg tcggcgccac gatcacgccg ttcggcggct acggcggcta caccgaggcc
1141 cgcgagacga tgcggcagga ggtcaacgag gagatccgct ccggccgggt cttcgacacg
1201 gtcgtcgact tcgacaaggc cctgcgcgac ccgtacgacc cgcgccggat gcgctccgac
1261 tacgacagcg gcgaccacct gcaccccggc gacaaggggt acgcgcgcat gggcgcggtc
1321 atcgacctgg ccgcgctgaa gggcgcggcg ccggtcaagg cgtag
```

## Figure 22 (SEQ ID No. 20)

```
  1 mtsmsrarva rriaagaayg gggiglagaa avglvvaevq larrrvgvgt ptrvpnaggl
 61 yggtlptagd pplrlmmlgd staagqgvhr agqtpgalla sglaavaerp vrlgsvaqpg
121 acsddldrqv alvlaepdrv pdicvimvga ndvthrmpat rsvrhlssav rrlrtagaev
181 vvgtcpdlgt iervrqplrw larrasrqla aaqtigaveq ggrtvslgdl lgpefaqnpr
241 elfgpdnyhp saegyataam avlpsvcaal glwpadeehp dalrregflp varaaaeaas
301 eagtevaaam ptgprgpwal lkrrrrrrvs eaepsspsgv
```

## Figure 23 (SEQ ID No. 21)

```
   1 atgacgagca tgtcgagggc gagggtggcg cggcggatcg cggccggcgc ggcgtacggc
  61 ggcggcggca tcggcctggc gggagcggcg gcggtcggtc tggtggtggc cgaggtgcag
 121 ctggccagac gcagggtggg ggtgggcacg ccgacccggg tgccgaacgc gcagggactg
 181 tacggcggca ccctgcccac ggccggcgac ccgccgctgc ggctgatgat gctgggcgac
 241 tccacggccg ccgggcaggg cgtgcaccgg gccgggcaga cgccgggcgc gctgctggcg
 301 tccgggctcg cggcggtggc ggagcggccg gtgcggctgg ggtcggtcgc ccagccgggg
 361 gcgtgctcgg acgacctgga ccggcaggtg gcgctggtgc tcgccgagcc ggaccgggtg
 421 cccgacatct gcgtgatcat ggtcggcgcc aacgacgtca cccaccggat gccggcgacc
 481 cgctcggtgc ggcacctgtc ctcggcggta cggcggctgc gcacggccgg tgcggaggtg
 541 gtggtcggca cctgtccgga cctgggcacg atcgagcggg tgcggcagcc gctgcgctgg
 601 ctggcccggc gggcctcacg gcagctcgcg gcggcacaga ccatcggcgc cgtcgagcag
 661 ggcgggcgca cggtgtcgct gggcgacctg ctgggtccgg agttcgcgca gaacccgcgg
 721 gagctcttcg gccccgacaa ctaccacccc tccgccgagg ggtacgccac ggccgcgatg
 781 gcggtactgc cctcggtgtg cgccgcgctc ggcctgtggc cggccgacga ggagcacccg
 841 gacgcgctgc gccgcgaggg cttcctgccg gtggcgcgcg cggcggcgga ggcggcgtcc
 901 gaggcgggta cggaggtcgc cgccgccatg cctacggggc ctcgggggcc ctgggcgctg
 961 ctgaagcgcc ggagacggcg tcgggtgtcg gaggcggaac cgtccagccc gtccggcgtt
1021 tga
```

Figure 24 (SEQ ID No. 22)

```
  1 mgrgtdqrtr ygrrrarval aaltaavlgv gvagcdsvgg dspapsgsps krtrtapawd
 61 tspasvaavg dsitrgfdac avlsdcpevs watgssakvd slavrllgka daaehswnya
121 vtgarmadlt aqvtraaqre pelvavmaga ndacrsttsa mtpvadfraq feeamatlrk
181 klpkaqvyvs sipdlkrlws qgrtnplgkq vwklglcpsm lgdadsldsa atlrrntvrd
241 rvadynevlr evcakdrrcr sddgavhefr fgtdqlshwd wfhpsvdgqa rlaeiayrav
301 taknp
```

Figure 25 (SEQ ID No. 23)

```
  1 atgggtcgag ggacggacca gcggacgcgg tacggccgtc gccgggcgcg tgtcgcgctc
 61 gccgccctga ccgccgccgt cctgggcgtg ggcgtggcgg gctgcgactc cgtgggcggc
121 gactcacccg ctccttccgg cagcccgtcg aagcggacga ggacggcgcc cgcctgggac
181 accagcccgg cgtccgtcgc cgccgtgggc gactccatca cgcgcggctt cgacgcctgt
241 gcggtgctgt cggactgccc ggaggtgtcg tgggcgaccg gcagcagcgc gaaggtcgac
301 tcgctggccg tacggctgct ggggaaggcg gacgcggccg agcacagctg gaactacgcg
361 gtcaccgggg cccggatggc ggacctgacc gctcaggtga cgcgggcggc gcagcgcgag
421 ccggagctgg tggcggtgat ggccggggcg aacgacgcgt gccggtccac gacctcggcg
481 atgacgccgg tggcggactt ccgggcgcag ttcgaggagg cgatggccac cctgcgcaag
541 aagctcccca aggcgcaggt gtacgtgtcg agcatcccgg acctcaagcg gctctggtcc
601 cagggccgca ccaacccgct gggcaagcag gtgtggaagc tcggcctgtg cccgtcgatg
661 ctgggcgacg cggactccct ggactcggcg gcgaccctgc ggcgcaacac ggtgcgcgac
721 cgggtggcgg actacaacga ggtgctgcgg gaggtctgcg cgaaggaccg gcggtgccgc
781 agcgacgacg gcgcggtgca cgagttccgg ttcggcacgg accagttgag ccactgggac
841 tggttccacc cgagtgtgga cggccaggcc cggctggcgg agatcgccta ccgcgcggtc
901 accgcgaaga atccctga
```

Figure 26 (SEQ ID No. 24)

```
  1 mrlsrraata sallltpala lfgasaavsa priqatdyva lgdsyssgvg agsydsssgs
 61 ckrstksypa lwaashtgtr fnftacsgar tgdvlakqlt pvnsgtdlvs itiggndagf
121 adtmttcnlq gesaclaria karayiqqtl paqldqvyda idsrapaaqv vvlgyprfyk
181 lggscavgls eksraainaa addinavtak raadhgfafg dvnttfaghe lcsgapwlhs
241 vtlpvensyh ptangqskgy lpvlnsat
```

Figure 27 (SEQ ID No. 25)

```
   1 ttcatcacaa cgatgtcaca acaccggcca tccgggtcat ccctgatcgt gggaatgggt
  61 gacaagcctt cccgtgacga aagggtcctg ctacatcaga aatgacagaa atcctgctca
 121 gggaggttcc atgagactgt cccgacgcgc ggccacggcg tccgcgctcc tcctcacccc
 181 ggcgctcgcg ctcttcggcg cgagcgccgc cgtgtccgcg ccgcgaatcc aggccaccga
 241 ctacgtggcc ctcggcgact cctactcctc ggggggtcggc gcgggcagct acgacagcag
 301 cagtggctcc tgtaagcgca gcaccaagtc ctacccggcc ctgtgggccg cctcgcacac
 361 cggtacgcgg ttcaacttca ccgcctgttc gggcgcccgc acaggagacg tgctggccaa
 421 gcagctgacc ccggtcaact ccggcaccga cctggtcagc attaccatcg gcggcaacga
 481 cgcgggcttc gccgacacca tgaccacctg caacctccag ggcgagagcg cgtgcctggc
 541 gcggatcgcc aaggcgcgcg cctacatcca gcagacgctg cccgcccagc tggaccaggt
 601 ctacgacgcc atcgacagcc gggcccccgc agcccaggtc gtcgtcctgg gctacccgcg
 661 cttctacaag ctgggcggca gctgcgccgt cggtctctcg gagaagtccc gcgcggccat
 721 caacgccgcc gccgacgaca tcaacgccgt caccgccaag cgcgccgccg accacggctt
 781 cgccttcggg gacgtcaaca cgaccttcgc cgggcacgag ctgtgctccg gcgcccccctg
 841 gctgcacagc gtcacccttc ccgtggagaa ctcctaccac ccccacggcca acggacagtc
 901 caagggctac ctgcccgtcc tgaactccgc cacctgatct cgcggctact ccgcccctga
 961 cgaagtcccg cccccgggcg gggcttcgcc gtaggtgcgc gtaccgccgt cgcccgtcgc
1021 gccggtggcc ccgccgtacg tgccgccgcc cccggacgcg gtcggttc
```

# Figure 28 (SEQ ID No. 26)

```
  1  MKKWFVCLLG LVALTVQAAD SRPAFSRIVM FGDSLSDTGK MYSKMRGYLP
 51  SSPPYYEGRF SNGPVWLEQL TKQFPGLTIA NEAEGGATAV AYNKISWNPK
101  YQVINNLDYE VTQFLQKDSF KPDDLVILWV GANDYLAYGW NTEQDAKRVR
151  DAISDAANRM VLNGAKQILL FNLPDLGQNP SARSQKVVEA VSHVSAYHNQ
201  LLLNLARQLA PTGMVKLFEI DKQFAEMLRD PQNFGLSDVE NPCYDGGYVW
251  KPFATRSVST DRQLSAFSPQ ERLAIAGNPL LAQAVASPMA RRSASPLNCE
301  GKMFWDQVHP TTVVHAALSE RAATFIANQY EFLAH*
```

# Figure 29 (SEQ ID No. 27)

```
1    ATGAAAAAAT GGTTTGTGTG TTTATTGGGA TTGGTCGCGC TGACAGTTCA
     TACTTTTTTA CCAAACACAC AAATAACCCT AACCAGCGCG ACTGTCAAGT

51   GGCAGCCGAC AGTCGCCCCG CCTTTTCCCG GATCGTGATG TTCGGCGACA
     CCGTCGGCTG TCAGCGGGGC GGAAAAGGGC CTAGCACTAC AAGCCGCTGT

101  GCCTCTCCGA TACCGGCAAA ATGTACAGCA AGATGCGCGG TTACCTCCCC
     CGGAGAGGCT ATGGCCGTTT TACATGTCGT TCTACGCGCC AATGGAGGGG

151  TCCAGCCCGC CCTACTATGA GGGCCGTTTC TCCAACGGAC CCGTCTGGCT
     AGGTCGGGCG GGATGATACT CCCGGCAAAG AGGTTGCCTG GGCAGACCGA

201  GGAGCAGCTG ACCAAACAGT TCCCGGGTCT GACCATCGCC AACGAAGCGG
     CCTCGTCGAC TGGTTTGTCA AGGGCCCAGA CTGGTAGCGG TTGCTTCGCC

251  AAGGCGGTGC CACTGCCGTG GCTTACAACA AGATCTCCTG GAATCCCAAG
     TTCCGCCACG GTGACGGCAC CGAATGTTGT TCTAGAGGAC CTTAGGGTTC

301  TATCAGGTCA TCAACAACCT GGACTACGAG GTCACCCAGT TCTTGCAGAA
     ATAGTCCAGT AGTTGTTGGA CCTGATGCTC CAGTGGGTCA AGAACGTCTT

351  AGACAGCTTC AAGCCGGACG ATCTGGTGAT CCTCTGGGTC GGTGCCAATG
     TCTGTCGAAG TTCGGCCTGC TAGACCACTA GGAGACCCAG CCACGGTTAC

401  ACTATCTGGC CTATGGCTGG AACACGGAGC AGGATGCCAA GCGGGTTCGC
     TGATAGACCG GATACCGACC TTGTGCCTCG TCCTACGGTT CGCCCAAGCG

451  GATGCCATCA GCGATGCGGC CAACCGCATG GTACTGAACG GTGCCAAGCA
     CTACGGTAGT CGCTACGCCG GTTGGCGTAC CATGACTTGC CACGGTTCGT

501  GATACTGCTG TTCAACCTGC CGGATCTGGG CCAGAACCCG TCAGCTCGCA
     CTATGACGAC AAGTTGGACG GCCTAGACCC GGTCTTGGGC AGTCGAGCGT

551  GTCAGAAGGT GGTCGAGGCG GTCAGCCATG TCTCCGCCTA TCACAACCAG
     CAGTCTTCCA CCAGCTCCGC CAGTCGGTAC AGAGGCGGAT AGTGTTGGTC

601  CTGCTGCTGA ACCTGGCACG CCAGCTGGCC CCCACCGGCA TGGTAAAGCT
     GACGACGACT TGGACCGTGC GGTCGACCGG GGGTGGCCGT ACCATTTCGA

651  GTTCGAGATC GACAAGCAAT TTGCCGAGAT GCTGCGTGAT CCGCAGAACT
     CAAGCTCTAG CTGTTCGTTA AACGGCTCTA CGACGCACTA GGCGTCTTGA

701  TCGGCCTGAG CGACGTCGAG AACCCCTGCT ACGACGGCGG CTATGTGTGG
     AGCCGGACTC GCTGCAGCTC TTGGGGACGA TGCTGCCGCC GATACACACC

751  AAGCCGTTTG CCACCCGCAG CGTCAGCACC GACCGCCAGC TCTCCGCCTT
     TTCGGCAAAC GGTGGGCGTC GCAGTCGTGG CTGGCGGTCG AGAGGCGGAA

801  CAGTCCGCAG GAACGCCTCG CCATCGCCGG CAACCCGCTG CTGGCACAGG
     GTCAGGCGTC CTTGCGGAGC GGTAGCGGCC GTTGGGCGAC GACCGTGTCC

851  CCGTTGCCAG TCCTATGGCC CGCCGCAGCG CCAGCCCCCT CAACTGTGAG
     GGCAACGGTC AGGATACCGG GCGGCGTCGC GGTCGGGGGA GTTGACACTC

901  GGCAAGATGT TCTGGGATCA GGTACACCCG ACCACTGTCG TGCACGCAGC
     CCGTTCTACA AGACCCTAGT CCATGTGGGC TGGTGACAGC ACGTGCGTCG

951  CCTGAGCGAG CGCGCCGCCA CCTTCATCGC GAACCAGTAC GAGTTCCTCG
     GGACTCGCTC GCGCGGCGGT GGAAGTAGCG CTTGGTCATG CTCAAGGAGC

1001 CCCAC TGA
     GGGTG ACT
```

Figure 30 (SEQ ID No. 28)

```
  1  MKKWFVCLLG LIALTVQAAD TRPAFSRIVM FGDSLSDTGK MYSKMRGYLP
 51  SSPPYYEGRF SNGPVWLEQL TKQFPGLTIA NEAEGGATAV AYNKISWNPK
101  YQVINNLDYE VTQFLQKDSF KPDDLVILWV GANDYLAYGW NTEQDAKRVR
151  DAISDAANRM VLNGAKQILL FNLPDLGQNP SARSQKVVEA VSHVSAYHNK
201  LLLNLARQLA PTGMVKLFEI DKQFAEMLRD PQNFGLSDVE NPCYDGGYVW
251  KPFATRSVST DRQLSAFSPQ ERLAIAGNPL LAQAVASPMA RRSASPLNCE
301  GKMFWDQVHP TTVVHAALSE RAATFIETQY EFLAHG*
```

Figure 31 (SEQ ID No. 29)

```
1    ATGAAAAAAT GGTTTGTTTG TTTATTGGGG TTGATCGCGC TGACAGTTCA
     TACTTTTTTA CCAAACAAAC AAATAACCCC AACTAGCGCG ACTGTCAAGT

51   GGCAGCCGAC ACTCGCCCCG CCTTCTCCCG GATCGTGATG TTCGGCGACA
     CCGTCGGCTG TGAGCGGGGC GGAAGAGGGC CTAGCACTAC AAGCCGCTGT

101  GCCTCTCCGA TACCGGCAAA ATGTACAGCA AGATGCGCGG TTACCTCCCC
     CGGAGAGGCT ATGGCCGTTT TACATGTCGT TCTACGCGCC AATGGAGGGG

151  TCCAGCCCGC CCTACTATGA GGGCCGTTTC TCCAACGGAC CCGTCTGGCT
     AGGTCGGGCG GGATGATACT CCCGGCAAAG AGGTTGCCTG GGCAGACCGA

201  GGAGCAGCTG ACCAAGCAGT TCCCGGGTCT GACCATCGCC AACGAAGCGG
     CCTCGTCGAC TGGTTCGTCA AGGGCCCAGA CTGGTAGCGG TTGCTTCGCC

251  AAGGCGGTGC CACTGCCGTG GCTTACAACA AGATCTCCTG GAATCCCAAG
     TTCCGCCACG GTGACGGCAC CGAATGTTGT TCTAGAGGAC CTTAGGGTTC

301  TATCAGGTCA TCAACAACCT GGACTACGAG GTCACCCAGT TCTTGCAGAA
     ATAGTCCAGT AGTTGTTGGA CCTGATGCTC CAGTGGGTCA AGAACGTCTT

351  AGACAGCTTC AAGCCGGACG ATCTGGTGAT CCTCTGGGTC GGTGCCAATG
     TCTGTCGAAG TTCGGCCTGC TAGACCACTA GGAGACCCAG CCACGGTTAC

401  ACTATCTGGC ATATGGCTGG AATACGGAGC AGGATGCCAA GCGAGTTCGC
     TGATAGACCG TATACCGACC TTATGCCTCG TCCTACGGTT CGCTCAAGCG

451  GATGCCATCA GCGATGCGGC CAACCGCATG GTACTGAACG GTGCCAAGCA
     CTACGGTAGT CGCTACGCCG GTTGGCGTAC CATGACTTGC CACGGTTCGT

501  GATACTGCTG TTCAACCTGC CGGATCTGGG CCAGAACCCG TCAGCCCGCA
     CTATGACGAC AAGTTGGACG GCCTAGACCC GGTCTTGGGC AGTCGGGCGT

551  GTCAGAAGGT GGTCGAGGCG GTCAGCCATG TCTCCGCCTA TCACAACAAG
     CAGTCTTCCA CCAGCTCCGC CAGTCGGTAC AGAGGCGGAT AGTGTTGTTC

601  CTGCTGCTGA ACCTGGCACG CCAGCTGGCC CCCACCGGCA TGGTAAAGCT
     GACGACGACT TGGACCGTGC GGTCGACCGG GGGTGGCCGT ACCATTTCGA

651  GTTCGAGATC GACAAGCAAT TTGCCGAGAT GCTGCGTGAT CCGCAGAACT
     CAAGCTCTAG CTGTTCGTTA AACGGCTCTA CGACGCACTA GGCGTCTTGA

701  TCGGCCTGAG CGACGTCGAG AACCCCTGCT ACGACGGCGG CTATGTGTGG
     AGCCGGACTC GCTGCAGCTC TTGGGGACGA TGCTGCCGCC GATACACACC

751  AAGCCGTTTG CCACCCGCAG CGTCAGCACC GACCGCCAGC TCTCCGCCTT
     TTCGGCAAAC GGTGGGCGTC GCAGTCGTGG CTGGCGGTCG AGAGGCGGAA

801  CAGTCCGCAG GAACGCCTCG CCATCGCCGG CAACCCGCTG CTGGCACAGG
     GTCAGGCGTC CTTGCGGAGC GGTAGCGGCC GTTGGGCGAC GACCGTGTCC

851  CCGTTGCCAG TCCTATGGCC CGCCGCAGCG CCAGCCCCCT CAACTGTGAG
     GGCAACGGTC AGGATACCGG GCGGCGTCGC GGTCGGGGGA GTTGACACTC

901  GGCAAGATGT TCTGGGATCA GGTACACCCG ACCACTGTCG TGCACGCAGC
     CCGTTCTACA AGACCCTAGT CCATGTGGGC TGGTGACAGC ACGTGCGTCG

951  CCTGAGCGAG CGCGCCGCCA CCTTCATCGA GACCCAGTAC GAGTTCCTCG
     GGACTCGCTC GCGCGGCGGT GGAAGTAGCT CTGGGTCATG CTCAAGGAGC

1001 CCCACGGATG A
     GGGTGCCTAC T
```

Figure 32

```
           1       10        20        30        40        50
           |--------+---------+---------+---------+---------|
     satA     ADTRPAFSRIVMFGDSLSDTGKMYSKMRGYLPSSPPYYEGRFSN--G
    R.sol  QSGNPNVAKVQRMVVFGDSLSDIGT-------YTPVAQAVGGGKFTTNPG
Consensus  ...adnraafqRiVmFGDSLSDiGk.......YlPsaqaygeGrFsn..G

           51      60        70        80        90       100
           |--------+---------+---------+---------+---------|
     satA  PYWLEQLTKQFPGLTIANEAEGGATAVAYNKISWNPKYQVINNLDYEVTQ
    R.sol  PIWAETVAAQL-GVTLTPAVMGYATSVQNCPKAGCFDYAQGGSRVTDPNG
Consensus  P!WaEqlaaQl.GlTianaaeGgATaVannkiagnfdYaqgnnrdt#pnq

           101     110       120       130       140       150
           |--------+---------+---------+---------+---------|
     satA  FLQKDSFKPDDLVILWVGANDYLAYG--WNTEQDAKRVRDAISDAANRMV
    R.sol  IGHNGGAGALTYPVQQQLANFYAASNNTFNGNNDVVFVLAGSNDIFFWTT
Consensus  igqndgagaddlp!qqqgANdYaAsn..fNg##DakrVraainDaanrmt

           151     160       170       180       190       200
           |--------+---------+---------+---------+---------|
     satA  LNGAKQILLFNLPDLGQNPSARSQKVVEAVSHVSAYHNKL-LLNLARQLA
    R.sol  AAATSGSGVTPAIATAQVQQAATDLVGYVKDMIAKGATQVYVFNLPDSSL
Consensus  aaaakqiglfnaialaQnqqAas#lVgeakdh!aaganql.llNLarqla

           201     210       220       230       240       250
           |--------+---------+---------+---------+---------|
     satA  PTGMVKLFEIDKQFAEMLRDPQNFGLSDVENPCYDGGYVWKPFATRSVST
    R.sol  TPDGVASGTTGQALLHALVGTFNTTLQSGLAGTSARIIDFNAQLTAAIQN
Consensus  ppdgValgeidqalaeaLrdpqNfgLqdgeagcsargidfnaqaTaa!qn

           251     260       270       280       290       300
           |--------+---------+---------+---------+---------|
     satA  DRQLSAFSPQERLAIAG--NPLLAQAVASPM---ARRSASPLNCEGKMFW
    R.sol  GASFGFANTSARACDATKINALVPSAGGSSLFCSANTLVASGADQSYLFA
Consensus  daqlgaanpqaRaadAg..NaLlaqAgaSp$...Arrlaapgad#gk$Fa

           301     310       320       330
           |--------+---------+---------|
     satA  DQVHPTTVVHAALSERAATFIETQYEFLAH
    R.sol  DGVHPTTAGHRLIASNVLARLLA--DNVAH
Consensus  DqVHPTTagHaaiaeraaariea..#nlAH
```

Figure 33

```
                         ▼
Pfam        *->ivafGDSltdggg..............ayygdsdgggwgagladrltsla..rlrargrgvdv
Sriml   38     YVALGDSYSSGVG............agSYDSSSGSCKRSTKSYPALWAAS..-----HTGTRF 81
Scoe1    5     YVAVGDSFTEG--..............--VGDPGPDGAFVGWADRLAVLL..ADRRPEGDFTY 47
Scoe2   10     LVAVGDSFTEG--..............--MSDLLPDGSYRGWADLLATRM..--AARSPGFRY 50
Scoe3  239     VVAFGDSITDG--..............ARSQSDANHRWTDVLAARLHEAA..GDGRDTPRYSV 283
Scoe4   75     LMMLGDSTAAG--..............------QGVHRAGQTPGALLASG..LAAVAERPVRL 113
Scoe5   66     VAAVGDSITRGFD............acAVLSDCPEVSWATGSSAKVDSLAvrLLGKADAAEHS 116
Ahyd1   28     IVMFGDSLSDTGKmyskmrgylpssppyYEGRFSNGPVWLEQLTNEFPGLTiaNEAEGGPTAVA 91
Asal1   28     IVMFGDSLSDTGKmyskmrgylpssppyYEGRFSNGPVWLEQLTKQF----..------PGLTI 79
Ahyd2   40     IVMFGDSLSDTGKmyskmrgylpssppyYEGRFSNGPVWLEQLTKQFPGLTiaNEAEGGATAVA 103

Pfam          fnrgisGrtsdGrlvvDarlvatllFlaqflGlnlpPYLsgdflrGANFAsagAtIlgtslipflni
Sriml   82 NFTACSGAR---------------------------------------------------------- 90
Scoe1   48 TNLAVRGRL---------------------------------------------------------- 56
Scoe2   51 ANLAVRGKL---------------------------------------------------------- 59
Scoe3  284 VNEGISGNR---------------------------------------------------------- 292
Scoe4  114 GSVAQPGAC---------------------------------------------------------- 122
Scoe5  117 WNYAVTGAR---------------------------------------------------------- 125
Ahyd1     92 YNKISWNPK---------------------------------------------------------- 100
Asal1     80 ANEAEGGAT---------------------------------------------------------- 88
Ahyd2  104 YNKISWNPK---------------------------------------------------------- 112

                                                           ▼
Pfam        QvqFkdfkskvlelrqa.....lgllqellrlvpvldakspdlvtimiGtNDl...itvakfgpks
Sriml   91 -----------------.....---TGDVLAKQLTPVNSGTDLVSITIGGNDAgfaDTMTTCNLQG 131
Scoe1   57 -----------------.....--LDQIVAEQVPRVVGLAPDLVSFAAGGNDI...-----I---- 86
Scoe2   60 -----------------.....--IGQIVDEQVDVAAAMGADVITLVGGLNDT...---------- 88
Scoe3  293 -------LLTSRPGRPA.....DNPSGLSRFQRDVLERTNVKAVVVVLGVNDV...---------- 333
Scoe4  123 -----------------.....SDDLDRQVALVLAEPDRVPDICVIMVGANDV...---------- 153
Scoe5  126 -----------------.....---MADLTAQVTRAAQREPELVAVMAGANDA...--------CR 155
Ahyd1  101 -------------YQVI.....NNLDYEVTQFLQKDSFKPDDLVILWVGANDY...--------LA 137
Asal1     89 -------AVAYNKISWNpkyqvyNNLDYEVTQFLQKDSFKPDDLVILWVGANDY...---------LA 137
Ahyd2  113 -------------YQVI.....NNLDYEVTQFLQKDSFKPDDLVILWVGANDY...--------LA 149

Pfam        ......tksdrnvsvpefrdnlrklikrLrsangariiilitlvllnlpl..........plGCl
Sriml  132 esaclarIAKARAYIQQTLPAQLDQVYDAIDSRAPAA-----QVVVLGYP-..........----- 176
Scoe1   87 ......---RPGTDPDEVAERFELAVAALT-AAAGTVLVTTGFDTRGVP-..........----- 125
Scoe2   89 ......---------LRPKCDMARVRDLLTQAVERLAPHCEQLVLMRSP-..........----- 122
Scoe3  334 ......LNSPELADRDAILTGLRTLVDRAHARGLRVVGATITPFGGYGG-..........----- 376
Scoe4  154 ......---THRMPATRSVRHLSSAVRRLR-TAGAEVVVGTCPDLGTIE-..........----- 192
Scoe5  156 ......STTSAMTPVADFRAQFEEAMATLR-KKLPKAQVYVSSIPDLKRLwsqgrtnplgkQVWKL 214
Ahyd1  138 ......YGWNTEQDAKRVRDAISDAANRMV-LNGAK-----EILLFNLP-..........----- 174
Asal1  138 ......YGWNTEQDAKRVRDAISDAANRMV-LNGAK-----QILLFNLP-..........----- 174
Ahyd2  150 ......YGWNTEQDAKRVRDAISDAANRMV-LNGAK-----QILLFNLP-..........----- 186

Pfam        pq.klalalassknvdatgclerlneavadynealrelaei.ek.l.q.aqlrkdglpdlkeanvpy
Sriml  177 --.RFYKLGGSCAVGLSEKSRAAINAAADDINAVTAKRA--.--.-.-.----------ADHGFAF 219
Scoe1  126 --.----------------VLKHLRGKIATYNGHVRAIA--.--.-.-.----------DRYGCPV 152
Scoe2  123 --.-----------GRQGPVLERFRPRMEALFAVIDDLA--.--.-.-.----------GRHGAVV 154
Scoe3  377 --.YTEARETMRQEVNEEIRSGRVFDTVVDFDKALRDPY--.--.-.-.----------------- 412
Scoe4  193 --.----------------------------RVRQPLRWLaRRaSrQlAAAQTIGAVEQGGRTVSL 227
Scoe5  215 GLcPSMLGDADSLDSAATLRRNTVRDRVADYNEVLREVC--.--.-.AkDRRCRSDDGAVHEFRFGT 273
Ahyd1  175 --.----DLGQNPSARSQKVVEAASHVSAYHNQLLLNLA--.--.-.-.RQLAPTGMVKLFEIDKQF 224
Asal1  175 --.----DLGQNPSARSQKVVEAVSHVSAYHNKLLLNLA--.--.-.-.RQLAPTGMVKLFEIDKQF 224
Ahyd2  187 --.----DLGQNPSARSQKVVEAVSHVSAYHNQLLLNLA--.--.-.-.RQLAPTGMVKLFEIDKQF 236

Pfam        VDlysifqdldgiqnpsayv.y....GFeet.kaCCGyGgr.yNyn.rv.CGnag.l.ck.vtakaC
Sriml  220 GDVNT---------------.-...-----.---------.-TFAgHElCSGAPwL.HS.VT---- 242
Scoe1  153 LDLWSLRSVQDRRA------.-...-----.---------.----.--.-----.-.--.------- 166
Scoe2  155 VDLYGAQSLADPRM------.-...-----.---------.----.--.-----.-.--.------- 168
```

```
Scoe3  413 ------------------.-...-----   .---------.----.--.-----.-.--.------ 413
Scoe4 228 GDLLGPEFAQNPREL-----.-....-----.---------.----.--.-----.-.--.------ 242
Scoe5   274 DQL---------------.-...-----.---------.----.--.-----.-.--.------ 276
Ahyd1 225 AEMLRDPQNFGLSDQRNACYgGsyvwKPFASrSASTDSQLSaFNPQeRLaIAGNPlLaQAvASPMAA 291
Asal1 225 AEMLRDPQNFGLSDVENPCYdGgyvwKPFATrSVSTDRQLSaFSPQeRLaIAGNPlLaQAvASPMAR 291
Ahyd2 237 AEMLRDPQNFGLSDVENPCYdGgyvwKPFATrSVSTDRQLSaFSPQeRLaIAGNPlLaQAvASPMAR 303

                            ▼
Pfam       .dassyll.atlfwDgf.HpsekGykavAeal<-*
Sriml 243 .-------.--LPVENSyHPTANGQSKGYLPV    263
Scoe1 167 .-------.--WDADRL.HLSPEGHTRVALRA    186
Scoe2 169 .-------.--WDVDRL.HLTAEGHRRVAEAV    188
Scoe3 413 .-DPRRMRsDYDSGDHL.HPGDKGYARMGAVI    441
Scoe4 243 .-------.--FGPDNY.HPSAEGYATAAMAV    262
Scoe5 277 .-------.--SHWDWF.HPSVDGQARLAEIA    296
Ahyd1 292 rSASTLNCeGKMFWDQV.HPTTVVHAALSEPA    322
Asal1 292 rSASPLNCeGKMFWDQV.HPTTVVHAALSERA    322
Ahyd2 304 rSASPLNCeGKMFWDQV.HPTTVVHAALSERA    334
```

Figure 34

```
                    ▼
Pfam          *->ivafGDSltdggg..............ayygdsdgggwgagladrltsla..rlrargrgvdv
Sriml  38      YVALGDSYSSGVG............agSYDSSSGSCKRSTKSYPALWAAS..-----HTGTRF 81
Scoe1   5      YVAVGDSFTEG--..............--VGDPGPDGAFVGWADRLAVLL..ADRRPEGDFTY 47
Scoe2  10      LVAVGDSFTEG--..............--MSDLLPDGSYRGWADLLATRM..--AARSPGFRY 50
Ahyd1  28      IVMFGDSLSDTGKmyskmrgylpssppyYEGRFSNGPVWLEQLTNEFPGLTiaNEAEGGPTAVA 91
Asal1  28      IVMFGDSLSDTGKmyskmrgylpssppyYEGRFSNGPVWLEQLTKQF----..------PGLTI 79
Ahyd2  40      IVMFGDSLSDTGKmyskmrgylpssppyYEGRFSNGPVWLEQLTKQFPGLTiaNEAEGGATAVA 103

Pfam          fnrgisGrtsdGrlvvDarlvatllFlaqflGlnlpPYLsgdflrGANFAsagAtIlgtslipflni
Sriml  82 NFTACSGAR---------------------------------------------------------- 90
Scoe1  48 TNLAVRGRL---------------------------------------------------------- 56
Scoe2  51 ANLAVRGKL---------------------------------------------------------- 59
Ahyd1  92 YNKISWNPK----------------------------------------------------------- 100
Asal1  80 ANEAEGGAT----------------------------------------------------------- 88
Ahyd2 104 YNKISWNPK---------------------------------------------------------- 112

                                                            ▼
Pfam          QvqFkdfkskvlelrqa......lgllqellrlvpvldakspdlvtimiGtNDl...itvakfgpks
Sriml  91 ------------------......---TGDVLAKQLTPVNSGTDLVSITIGGNDAgfaDTMTTCNLQG 131
Scoe1  57 ------------------......--LDQIVAEQVPRVVGLAPDLVSFAAGGNDI...-----I---- 86
Scoe2  60 ------------------......--IGQIVDEQVDVAAAMGADVITLVGGLNDT...---------- 88
Ahyd1 101 --------------YQVI......NNLDYEVTQFLQKDSFKPDDLVILWVGANDY...---------LA 137
Asal1  89 -------AVAYNKISWNpkyqvyNNLDYEVTQFLQKDSFKPDDLVILWVGANDY...---------LA 137
Ahyd2 113 --------------YQVI......NNLDYEVTQFLQKDSFKPDDLVILWVGANDY...---------LA 149

Pfam          .......tksdrnvsvpefrdnlrklikrLrsangariiilitlvllnlplplGCl
Sriml 132 esaclarIAKARAYIQQTLPAQLDQVYDAIDSRAPAA-----QVVVLGYP------ 176
Scoe1  87 .......---RPGTDPDEVAERFELAVAALT-AAAGTVLVTTGFDTRGVP------ 125
Scoe2  89 .......----------LRPKCDMARVRDLLTQAVERLAPHCEQLVLMRSP------ 122
Ahyd1 138 .......YGWNTEQDAKRVRDAISDAANRMV-LNGAK-----EILLFNLP------ 174
Asal1 138 .......YGWNTEQDAKRVRDAISDAANRMV-LNGAK-----QILLFNLP------ 174
Ahyd2 150 .......YGWNTEQDAKRVRDAISDAANRMV-LNGAK-----QILLFNLP------ 186

Pfam          pqklalalassknvdatgclerlneavadynealrelaeieklqaqlrkdglpdlkeanvpy
Sriml 177 --RFYKLGGSCAVGLSEKSRAAINAAADDINAVTAKRA-----------------ADHGFAF 219
Scoe1 126 ------------------VLKHLRGKIATYNGHVRAIA-----------------DRYGCPV 152
Scoe2 123 --------------GRQGPVLERFRPRMEALFAVIDDLA-----------------GRHGAVV 154
Ahyd1 175 ------DLGQNPSARSQKVVEAASHVSAYHNQLLLNLA------RQLAPTGMVKLFEIDKQF 224
Asal1 175 ------DLGQNPSARSQKVVEAVSHVSAYHNKLLLNLA------RQLAPTGMVKLFEIDKQF 224
Ahyd2 187 ------DLGQNPSARSQKVVEAVSHVSAYHNQLLLNLA------RQLAPTGMVKLFEIDKQF 236

Pfam          VDlysifqdldgiqnpsayv.y....GFeet.kaCCGyGgr.yNyn.rv.CGnag.l.ck.vtakaC
Sriml 220 GDVNT---------------.-....-----.---------.-TFAgHElCSGAPwL.HS.VT---- 242
Scoe1 153 LDLWSLRSVQDRRA------.-....-----.---------.----.--.-----.-.--.------ 166
Scoe2 155 VDLYGAQSLADPRM------.-....-----.---------.----.--.-----.-.--.------ 168
Ahyd1 225 AEMLRDPQNFGLSDQRNACYgGsyvwKPFASrSASTDSQLSaFNPQeRLaIAGNPlLaQAvASPMAA 291
Asal1 225 AEMLRDPQNFGLSDVENPCYdGgyvwKPFATrSVSTDRQLSaFSPQeRLaIAGNPlLaQAvASPMAR 291
Ahyd2 237 AEMLRDPQNFGLSDVENPCYdGgyvwKPFATrSVSTDRQLSaFSPQeRLaIAGNPlLaQAvASPMAR 303

                            ▼
Pfam          .dassyll.atlfwDgf.HpsekGykavAeal<-*
Sriml 243 .-------.--LPVENSyHPTANGQSKGYLPV    263
Scoe1 167 .-------.--WDADRL.HLSPEGHTRVALRA    186
Scoe2 169 .-------.--WDVDRL.HLTAEGHRRVAEAV    188
Ahyd1 292 rSASTLNCeGKMFWDQV.HPTTVVHAALSEPA    322
Asal1 292 rSASPLNCeGKMFWDQV.HPTTVVHAALSERA    322
Ahyd2 304 rSASPLNCeGKMFWDQV.HPTTVVHAALSERA    334
```

Figure 35

(SEQ ID No. 30)

```
  1 MFKFKKNFLV GLSAALMSIS LFSATASAAS ADSRPAFSRI VMFGDSLSDT
 51 GKMYSKMRGY LPSSPPYYEG RFSNGPVWLE QLTKQFPGLT IANEAEGGAT
101 AVAYNKISWN PKYQVINNLD YEVTQFLQKD SFKPDDLVIL WVGANDYLAY
151 GWNTEQDAKR VRDAISDAAN RMVLNGAKQI LLFNLPDLGQ NPSARSQKVV
201 EAVSHVSAYH NQLLLNLARQ LAPTGMVKLF EIDKQFAEML RDPQNFGLSD
251 VENPCYDGGY VWKPFATRSV STDRQLSAFS PQERLAIAGN PLLAQAVASP
301 MARRSASPLN CEGKMFWDQV HPTTVVHAAL SERAATFIAN QYEFLAH**
```

Figure 36 (SEQ ID No. 31)

```
1    ATGTTTAAGT TTAAAAAGAA TTTCTTAGTT GGATTATCGG CAGCTTTAAT
     TACAAATTCA AATTTTTCTT AAAGAATCAA CCTAATAGCC GTCGAAATTA

51   GAGTATTAGC TTGTTTTCGG CAACCGCCTC TGCAGCTAGC GCCGACAGCC
     CTCATAATCG AACAAAAGCC GTTGGCGGAG ACGTCGATCG CGGCTGTCGG

101  GTCCCGCCTT TTCCCGGATC GTGATGTTCG GCGACAGCCT CTCCGATACC
     CAGGGCGGAA AAGGGCCTAG CACTACAAGC CGCTGTCGGA GAGGCTATGG

151  GGCAAAATGT ACAGCAAGAT GCGCGGTTAC CTCCCCTCCA GCCCGCCCTA
     CCGTTTTACA TGTCGTTCTA CGCGCCAATG GAGGGGAGGT CGGGCGGGAT

201  CTATGAGGGC CGTTTCTCCA ACGGACCCGT CTGGCTGGAG CAGCTGACCA
     GATACTCCCG GCAAAGAGGT TGCCTGGGCA GACCGACCTC GTCGACTGGT

251  AACAGTTCCC GGGTCTGACC ATCGCCAACG AAGCGGAAGG CGGTGCCACT
     TTGTCAAGGG CCCAGACTGG TAGCGGTTGC TTCGCCTTCC GCCACGGTGA

301  GCCGTGGCTT ACAACAAGAT CTCCTGGAAT CCCAAGTATC AGGTCATCAA
     CGGCACCGAA TGTTGTTCTA GAGGACCTTA GGGTTCATAG TCCAGTAGTT

351  CAACCTGGAC TACGAGGTCA CCCAGTTCTT GCAGAAAGAC AGCTTCAAGC
     GTTGGACCTG ATGCTCCAGT GGGTCAAGAA CGTCTTTCTG TCGAAGTTCG

401  CGGACGATCT GGTGATCCTC TGGGTCGGTG CCAATGACTA TCTGGCCTAT
     GCCTGCTAGA CCACTAGGAG ACCCAGCCAC GGTTACTGAT AGACCGGATA

451  GGCTGGAACA CGGAGCAGGA TGCCAAGCGG GTTCGCGATG CCATCAGCGA
     CCGACCTTGT GCCTCGTCCT ACGGTTCGCC CAAGCGCTAC GGTAGTCGCT

501  TGCGGCCAAC CGCATGGTAC TGAACGGTGC CAAGCAGATA CTGCTGTTCA
     ACGCCGGTTG GCGTACCATG ACTTGCCACG GTTCGTCTAT GACGACAAGT

551  ACCTGCCGGA TCTGGGCCAG AACCCGTCAG CTCGCAGTCA GAAGGTGGTC
     TGGACGGCCT AGACCCGGTC TTGGGCAGTC GAGCGTCAGT CTTCCACCAG

601  GAGGCGGTCA GCCATGTCTC CGCCTATCAC AACCAGCTGC TGCTGAACCT
     CTCCGCCAGT CGGTACAGAG GCGGATAGTG TTGGTCGACG ACGACTTGGA

651  GGCACGCCAG CTGGCCCCCA CCGGCATGGT AAAGCTGTTC GAGATCGACA
     CCGTGCGGTC GACCGGGGGT GGCCGTACCA TTTCGACAAG CTCTAGCTGT

701  AGCAATTTGC CGAGATGCTG CGTGATCCGC AGAACTTCGG CCTGAGCGAC
     TCGTTAAACG GCTCTACGAC GCACTAGGCG TCTTGAAGCC GGACTCGCTG

751  GTCGAGAACC CCTGCTACGA CGGCGGCTAT GTGTGGAAGC CGTTTGCCAC
     CAGCTCTTGG GGACGATGCT GCCGCCGATA CACACCTTCG GCAAACGGTG

801  CCGCAGCGTC AGCACCGACC GCCAGCTCTC CGCCTTCAGT CCGCAGGAAC
     GGCGTCGCAG TCGTGGCTGG CGGTCGAGAG GCGGAAGTCA GGCGTCCTTG

851  GCCTCGCCAT CGCCGGCAAC CCGCTGCTGG CACAGGCCGT TGCCAGTCCT
     CGGAGCGGTA GCGGCCGTTG GGCGACGACC GTGTCCGGCA ACGGTCAGGA

901  ATGGCCCGCC GCAGCGCCAG CCCCCTCAAC TGTGAGGGCA AGATGTTCTG
     TACCGGGCGG CGTCGCGGTC GGGGGAGTTG ACACTCCCGT TCTACAAGAC

951  GGATCAGGTA CACCCGACCA CTGTCGTGCA CGCAGCCCTG AGCGAGCGCG
     CCTAGTCCAT GTGGGCTGGT GACAGCACGT GCGTCGGGAC TCGCTCGCGC

1001 CCGCCACCTT CATCGCGAAC CAGTACGAGT TCCTCGCCCA CTGATGA
     GGCGGTGGAA GTAGCGCTTG GTCATGCTCA AGGAGCGGGT GACTACT
```

Figure 37

SEQ ID NO. 32:

ACAGGCCGATGCACGGAACCGTACCTTTCCGCAGTGAAGCGCTCTCCCCCCATCGTTCGC
CGGGACTTCATCCGCGATTTTGGCATGAACACTTCCTTCAACGCGCGTAGCTTGCTACAA
GTGCGGCAGCAGACCCGCTCGTTGGAGGCTCAGTGAGATTGACCCGATCCCTGTCGGCCG
CATCCGTCATCGTCTTCGCCCTGCTGCTCGCGCTGCTGGGCATCAGCCCGGCCCAGGCAG
CCGGCCCGGCCTATGTGGCCCTGGGGGATTCCTATTCCTCGGGCAACGGCGCCGGAAGTT
ACATCGATTCGAGCGGTGACTGTCACCGCAGCAACAACGCGTACCCCGCCCGCTGGGCGG
CGGCCAACGCACCGTCCTCCTTCACCTTCGCGGCCTGCTCGGGAGCGGTGACCACGGATG
TGATCAACAATCAGCTGGGCGCCCTCAACGCGTCCACCGGCCTGGTGAGCATCACCATCG
GCGGCAATGACGCGGGCTTCGCGGACGCGATGACCACCTGCGTCACCAGCTCGGACAGCA
CCTGCCTCAACCGGCTGGCCACCGCCACCAACTACATCAACACCACCCTGCTCGCCCGGC
TCGACGCGGTCTACAGCCAGATCAAGGCCCGTGCCCCCAACGCCCGCGTGGTCGTCCTCG
GCTACCCGCGCATGTACCTGGCCTCGAACCCCTGGTACTGCCTGGGCCTGAGCAACACCA
AGCGCGCGGCCATCAACACCACCGCCGACACCCTCAACTCGGTGATCTCCTCCCGGGCCA
CCGCCCACGGATTCCGATTCGGCGATGTCCGCCCGACCTTCAACAACCACGAACTGTTCT
TCGGCAACGACTGGCTGCACTCACTCACCCTGCCGGTGTGGGAGTCGTACCACCCCACCA
GCACGGGCCATCAGAGCGGCTATCTGCCGGTCCTCAACGCCAACAGCTCGACCTGATCAA
CGCACGGCCGTGCCCGCCCCGCGCGTCACGCTCGGCGCGGGCGCCGCAGCGCGTTGATCA
GCCCACAGTGCCGGTGACGGTCCCACCGTCACGGTCGAGGGTGTACGTCACGGTGGCGCC
GCTCCAGAAGTGGAACGTCAGCAGGACCGTGGAGCCGTCCCTGACCTCGTCGAAGAACTC
CGGGGTCAGCGTGATCACCCCTCCCCCGTAGCCGGGGGCGAAGGCGGCGCCGAACTCCTT
GTAGGACGTCCAGTCGTGCGGCCCGGCGTTGCCACCGTCCGCGTAGACCGCTTCCATGGT
CGCCAGCCGGTCCCCGCGGAACTCGGTGGGGATGTCCGTGCCCAAGGTGGTCCCGGTGGT
GTCCGAGAGCACCGGGGGCTCGTACCGGATGATGTGCAGATCCAAAGAATT

FIGURE 38

SEQ ID NO. 33:

MRLTRSLSAASVIVFALLLALLGISPAQAAGPAYVALGDSYSSGNGAGSYIDSSGDCHRSN
NAYPARWAAANAPSSFTFAACSGAVTTDVINNQLGALNASTGLVSITIGGNDAGFADAMTT
CVTSSDSTCLNRLATATNYINTTLLARLDAVYSQIKARAPNARVVVLGYPRMYLASNPWYC
LGLSNTKRAAINTTADTLNSVISSRATAHGFRFGDVRPTFNNHELFFGNDWLHSLTLPVWE
SYHPTSTGHQSGYLPVLNANSST

Figure 39

SEQ ID No. 34

ZP_00058717

1 mlphpagerg evgaffallv gtpqdrrlrl echetrplrg rcgcgerrvp pltlpgdgvl
61 cttsstrdae tvwrkhlqpr pdggfrphlg vgcllagqgs pgvlwcgreg crfevcrrdt
121 pglsrtrngd ssppfragws lppkcgeisq sarktpavpr ysllrtdrpd gprgrfvgsg
181 praatrrrlf lgipalvlvt altlvlavpt gretlwrrnwc eatqdwclgv pvdsrgqpae
241 dgeflllspv qaatwgnyya lgdsyssgdg ardyypgtav kggcwrsana ypelvaeayd
301 faghlsflac sgqrgyamld aidevgsqld wnsphtslvt igiggndlgf stvlktcmvr
361 vplldskact dqedairkrm akfettfeel isevrtrapd arilvvgypr ifpeeptgay
421 ytltasnqrw lnetiqefnq qlaeavavhd eeiaasggvg svefvdvyha ldgheigsde
481 pwvngvqlrd latgvtvdrs tfhpnaaghr avgervieqi etgpgrplya tfavvagatv
541 dtlagevg

FIGURE 40

SEQ ID No. 35

1 ggtggtgaac cagaacaccc ggtcgtcggc gtgggcgtcc aggtgcaggt gcaggttctt
61 caactgctcc agcaggatgc cgccgtggcc gtgcacgatg gccttgggca ggcctgtggt
121 ccccgacgag tacagcaccc atagcggatg gtcgaacggc agcggggtga actccagttc
181 cgcgccttcg cccgcggctt cgaactccgc ccaggacagg gtgtcggcga cagggccgca
241 gcccaggtac ggcaggacga cggtgtgctg caggctgggc atgccgtcgc gcagggcttt
301 gagcacgtca cggcggtcga agtccttacc gccgtagcgg tagccgtcca cggccagcag
361 cactttcggt tcgatctgcg cgaaccggtc gaggacgctg cgcaccccga agtcggggga
421 acaggacgac caggtcgcac cgatcgcggc gcaggcgagg aatgcggccg tcgcctcggc
481 gatgttcggc aggtaggcca cgacccggtc gccggggccc accccgaggc tgcggagggc
541 cgcagcgatc gcggcggtgc gggtccgcag ttctccccag gtccactcgg tcaacggccg
601 gagttcggac gcgtgccgga tcgccacggc tgatgggtca cggtcgcgga agatgtgctc
661 ggcgtagttg agggtggcgc cggggaacca gacggcgccg ggcatggcgt cggaggcgag
721 cactgtggtg tacggggtgg cggcgcgcac ccggtagtac tcccagatcg cggaccagaa
781 tccttcgagg tcggttaccg accagcgcca cagtgcctcg tagtccggtg cgtccacacc
841 gcggtgctcc cgcacccagc gggtgaacgc ggtgaggttg gcgcgttctt tgcgctcctc
901 gtcgggactc cacaggatcg gcggctgcgg cttgagtgtc atgaaacgcg acccccttcgt
961 ggacggtgcg gatgcggtga gcgtcgggtg cctcccctaa cgctccccgg tgacggagtg
1021 ttgtgcacca catctagcac gcgggacgcg gaaaccgtat ggagaaaaca cctacaaccc
1081 cggccggacg gtgggtttcg gccacactta ggggtcgggt gcctgcttgc cgggcagggc
1141 agtcccgggg tgctgtggtg cgggcgggag ggctgtcgct tcgaggtgtg ccggcgggac
1201 actccgggcc tcagccgtac ccgcaacggg gacagttctc ctcccttccg ggctggatgg
1261 tcccttcccc cgaaatgcgg cgagatctcc cagtcagccc ggaaaacacc cgctgtgccc
1321 aggtactctt tgcttcgaac agacaggccg gacggtccac gggggaggtt tgtggcagc
1381 ggaccacgtg cggcgaccag acgacggttg ttcctcggta tccccgctct tgtacttgtg
1441 acagcgctca cgctggtctt ggctgtcccg acggggcgcg agacgctgtg gcgcatgtgg
1501 tgtgaggcca cccaggactg gtgcctgggg gtgccggtcg actcccgcgg acagcctgcg
1561 gaggacggcg agtttctgct gctttctccg gtccaggcag cgacctgggg gaactattac
1621 gcgctcgggg attcgtactc ttcgggggac ggggcccgcg actactatcc cggcaccgcg
1681 gtgaagggcg gttgctggcg gtccgctaac gcctatccgg agctggtcgc cgaagcctac
1741 gacttcgccg gacacttgtc gttcctggcc tgcagcggcc agcgcggcta cgccatgctt
1801 gacgctatcg acgaggtcgg ctcgcagctg gactggaact cccctcacac gtcgctggtg
1861 acgatcggga tcggcggcaa cgatctgggg ttctccacgg ttttgaagac ctgcatggtg
1921 cgggtgccgc tgctggacag caaggcgtgc acggaccagg aggacgctat ccgcaagcgg
1981 atggcgaaat tcgagacgac gtttgaagag ctcatcagcg aagtgcgcac ccgcgcgccg
2041 gacgcccgga tccttgtcgt gggctacccc cggatttttc cggaggaacc gaccggcgcc
2101 tactacacgc tgaccgcgag caaccagcgg tggctcaacg aaaccattca ggagttcaac
2161 cagcagctcg ccgaggctgt cgcggtccac gacgaggaga ttgccgcgtc gggcggggtg
2221 ggcagcgtgg agttcgtgga cgtctaccac gcgttggacg gccacgagat cggctcggac
2281 gagccgtggg tgaacggggt gcagttgcgg gacctcgcca ccggggtgac tgtggaccgc
2341 agtaccttcc accccaacgc cgctgggcac cgggcggtcg gtgagcgggt catcgagcag
2401 atcgaaaccg gcccgggccg tccgctctat gccactttcg cggtggtggc gggggcgacc
2461 gtggacactc tcgcgggcga ggtggggtga cccggcttac cgtccggccc gcaggtctgc
2521 gagcactgcg gcgatctggt ccactgccca gtgcagttcg tcttcggtga tgaccagcgg
2581 cggggagagc cggatcgttg agccgtgcgt gtctttgacg agcacacccc gctgcaggag
2641 ccgttcgcac agttctcttc cggtggccag agtcgggtcg acgtcgatcc cagcccacag
2701 gccgatgctg cgggccgcga ccacgccgtt gccgaccagt tggtcgaggc gggcgcgcag
2761 cacgggggcg agggcgcgga catggtccag gtaagggccg tcgcggacga ggctcaccac
2821 ggcagtgccg accgcgcagg cgagggcgtt gccgccgaag gtgctgccgt gctggccggg
2881 gcggatcacg tcgaagactt ccgcgtcgcc taccgccgcc gccacgggca ggatgccgcc
2941 gcccagcgct ttgccgaaca ggtagatatc ggcgtcgact ccgctgtggt cgcaggcccg
//

FIGURE 41

SEQ ID No. 36

1 vgsgpraatr rrlflgipal vlvtaltlvl avptgretlw rmwceatqdw clgvpvdsrg
61 qpaedgefll lspvqaatwg nyyalgdsys sgdgardyyp gtavkggcwr sanaypelva
121 eaydfaghls flacsgqrgy amldaidevg sqldwnspht slvtigiggn dlgfstvlkt
181 cmvrvpllds kactdqedai rkrmakfett feelisevrt rapdarilvv gyprifpeep
241 tgayytltas nqrwlnetiq efnqqlaeav avhdeeiaas ggvgsvefvd vyhaldghei
301 gsdepwvngv qlrdlatgvt vdrstfhpna aghravgerv ieqietgpgr plyatfavva
361 gatvdtlage vg

FIGURE 42

SEQ ID No. 37

1 mrttviaasa llllagcadg areetagapp gessggiree gaeastsitd vyialgdsya
61 amggrdqplr gepfclrssg nypellhaev tdltcqgavt gdlleprtlg ertlpaqvda
121 ltedttlvtl siggndlgfg evagcireri agenaddcvd llgetigeql dqlppqldrv
181 heairdragd aqvvvtgylp lvsagdcpel gdvseadrrw aveltgqine tvreaaerhd
241 alfvlpddad ehtscappqq rwadiqgqqt dayplhptsa gheamaaavr dalglepvqp
//

FIGURE 43

SEQ ID No. 38

1 ttctggggtg ttatggggtt gttatcggct cgtcctgggt ggatcccgcc aggtggggta
61 ttcacggggg acttttgtgt ccaacagccg agaatgagtg ccctgagcgg tgggaatgag
121 gtgggcgggg ctgtgtcgcc atgagggggc ggcgggctct gtggtgcccc gcgaccccg
181 gccccggtga gcggtgaatg aaatccggct gtaatcagca tcccgtgccc accccgtcgg
241 ggaggtcagc gcccggagtg tctacgcagt cggatcctct cggactcggc catgctgtcg
301 gcagcatcgc gctcccgggt cttggcgtcc ctcggctgtt ctgcctgctg tccctggaag
361 gcgaaatgat caccggggag tgatacaccg gtggtctcat cccggatgcc cacttcggcg
421 ccatccggca attcgggcag ctccgggtgg aagtaggtgg catccgatgc gtcggtgacg
481 ccatagtggg cgaagatctc atcctgctcg agggtgctca ggccactctc cggatcgata
541 tcgggggcgt ccttgatggc gtccttgctg aaaccgaggt gcagcttgtg ggcttccaat
601 ttcgcaccac ggagcgggac gaggctggaa tgacggccga agagcccgtg gtggacctca
661 acgaaggtgg gtagtcccgt gtcatcattg aggaacacgc cctccaccgc acccagcttg
721 tggccggagt tgtcgtaggc gctggcatcc agaagggaaa cgatctcata tttgtcggtg
781 tgctcagaca tgatcttcct ttgctgtcgg tgtctggtac taccacggta gggctgaatg
841 caactgttat ttttctgtta ttttaggaat tggtccatat cccacaggct ggctgtggtc
901 aaatcgtcat caagtaatcc ctgtcacaca aaatgggtgg tgggagccct ggtcgcggtt
961 ccgtgggagg cgccgtgccc cgcaggatcg tcggcatcgg cggatctggc cggtaccccg
1021 cggtgaataa aatcattctg taaccttcat cacggttggt tttaggtatc cgcccctttc
1081 gtcctgaccc cgtccccggc gcgcgggagc ccgcgggttg cggtagacag gggagacgtg
1141 gacaccatga ggacaacggt catcgcagca agcgcattac tccttctcgc cggatgcgcg
1201 gatggggccc gggaggagac cgccggtgca ccgccgggtg agtcctccgg gggcatccgg
1261 gaggaggggg cggaggcgtc gacaagcatc accgacgtct acatcgccct cggggattcc
1321 tatgcggcga tgggcgggcg ggatcagccg ttacggggtg agccgttctg cctgcgctcg
1381 tccggtaatt acccggaact cctccacgca gaggtcaccg atctcacctg ccagggggcg
1441 gtgaccgggg atctgctcga acccaggacg ctgggggagc gcacgctgcc ggcgcaggtg
1501 gatgcgctga cggaggacac caccctggtc accctctcca tcgggggcaa tgacctcgga
1561 ttcggggagg tggcgggatg catccgggaa cggatcgccg ggggagaacgc tgatgattgc
1621 gtggacctgc tgggggaaac catcggggag cagctcgatc agcttccccc gcagctggac
1681 cgcgtgcacg aggctatccg ggaccgcgcc ggggacgcgc aggttgtggt caccggttac
1741 ctgccgctcg tgtctgccgg ggactgcccc gaactggggg atgtctccga ggcggatcgt
1801 cgttgggcgg ttgagctgac cggcagatc aacgagaccg tgcgcgaggc ggccgaacga
1861 cacgatgccc tctttgtcct gcccgacgat gccgatgagc acaccagttg tgcacccca
1921 cagcagcgct gggcggatat ccagggccaa cagaccgatg cctatccgct gcacccgacc
1981 tccgccggcc atgaggcgat ggccgccgcc gtccgggacg cgctgggcct ggaaccggtc
2041 cagccgtagc gccgggcgcg cgcttgtcga cgaccaaccc atgccaggct gcagtcacat
2101 ccgcacatag cgcgcgcggg cgatggagta cgcaccatag aggatgagcc cgatgccgac
2161 gatgatgagc agcacactgc cgaagggttg ttccccgagg gtgcgcagag ccgagtccag
2221 acctgcggcc tgctccggat catgggccca accggcgatg acgatcaaca cccccaggat
2281 cccgaaggcg ataccacggg cgacataacc ggctgttccg gtgatgatga tcgcggtccc
2341 gacctgccct gaccccgcac ccgcctccag atcctcccgg aaatcccggg tggccccctt
2401 ccagaggttg tagacacccg cccccagtac caccagcccg gcgaccacaa ccagcaccac
2461 accccagggt tgggatagga cggtggcggt gacatcggtg gcggtctccc catcggaggt
2521 gctgccgccc cgggcgaagg tggaggtggt caccgccagg gagaagtaga ccatggccat
2581 gaccgccccc ttggcccttt ccttgaggtc ctcgcccgcc agcagctggc tcaattgcca
2641 gagtcccagg gccgccaggg cgatgacggc aacccacagg aggaactgcc cacccggagc
2701 ctccgcgatg gtggccaggg cacctgaatt cgaggcctca tcacccgaac cgccggatcc
2761 agtggcgatg cgcaccgcga tccacccgat gaggatgtgc agtatgccca ggacaatgaa
2821 accacctctg gccagggtgg tcagcgcggg gtggtcctcg gcctggtcgg cagcccgttc
2881 gatcgtccgt ttcgcggatc tggtgtcgcc cttatccata gctcccattg aaccgccttg
2941 aggggtgggc ggccactgtc agggcggatt gtgatctgaa ctgtgatgtt ccatcaaccc
//

FIGURE 44

SEQ ID No. 39

ZP_00094165

1 mgqvklfarr capvllalag lapaatvare aplaegaryv algssfaagp gvgpnapgsp
61 ercgrgtlny phllaealkl dlvdatcsga tthhvlgpwn evppqidsvn gdtrlvtlti
121 ggndvsfvgn ifaaacekma spdprcgkwr eiteeewqad eermrsivrq iharaplarv
181 vvvdyitvlp psgtcaamai spdrlaqsrs aakrlarita rvareegasl lkfshisrrh
241 hpcsakpwsn glsapaddgi pvhpnrlgha eaaaalvklv klmk

//

FIGURE 45

SEQ ID No. 40

1 tgccggaact caagcggcgt ctagccgaac tcatgcccga aagcgcgtgg cactatcccg
61 aagaccaggt ctcggacgcc agcgagcgcc tgatggccgc cgaaatcacg cgcgaacagc
121 tctaccgcca gctccacgac gagctgccct atgacagtac cgtacgtccc gagaagtacc
181 tccatcgcaa ggacggttcg atcgagatcc accagcagat cgtgattgcc cgcgagacac
241 agcgtccgat cgtgctgggc aagggtggcg cgaagatcaa ggcgatcgga gaggccgcac
301 gcaaggaact ttcgcaattg ctcgacacca aggtgcacct gttcctgcat gtgaaggtcg
361 acgagcgctg ggccgacgcc aaggaaatct acgaggaaat cggcctcgaa tgggtcaagt
421 gaagctcttc gcgcgccgct gcgccccagt acttctcgcc cttgccgggc tggctccggc
481 ggctacggtc gcgcgggaag caccgctggc cgaaggcgcg cgttacgttg cgctgggaag
541 ctccttcgcc gcaggtccgg gcgtggggcc caacgcgccc ggatcgcccg aacgctgcgg
601 ccgggggcacg ctcaactacc cgcacctgct cgccgaggcg ctcaagctcg atctcgtcga
661 tgcgacctgc agcggcgcga cgacccacca cgtgctgggc ccctggaacg aggttccccc
721 tcagatcgac agcgtgaatg gcgacacccg cctcgtcacc ctgaccatcg gcggaaacga
781 tgtgtcgttc gtcggcaaca tcttcgccgc cgcttgcgag aagatggcgt cgcccgatcc
841 gcgctgcggc aagtggcggg agatcaccga ggaagagtgg caggccgacg aggagcggat
901 gcgctccatc gtacgccaga tccacgcccg cgcgcctctc gcccgggtgg tggtggtcga
961 ttacatcacg gtcctgccgc catcaggcac ttgcgctgcc atggcgattt cgccggaccg
1021 gctggcccag agccgcagcg ccgcgaaacg gcttgcccgg attaccgcac gggtcgcgcg
1081 agaagagggt gcatcgctgc tcaagttctc gcatatctcg cgccggcacc atccatgctc
1141 tgccaagccc tggagcaacg gcctttccgc cccggccgac gacggcatcc cggtccatcc
1201 gaaccggctc ggacatgctg aagcggcagc ggcgctggtc aagcttgtga aattgatgaa
1261 gtagctactg cactgatttc aaatagtatt gcctgtcagc tttccagccc ggattgttgc
1321 agcgcaacag aaacttgtcc gtaatggatt gatggtttat gtcgctcgca aattgccgtc
1381 gaagggaacg ggcgcgtcgc tcgttaacgt cctgggtgca gcagtgacgg agcgcgtgga
1441 tgagtgatac tggcggtgtc atcggtgtac gcgccgccat tcccatgcct gtacgcgccg

//

FIGURE 46

SEQ ID No. 41

NP_625998.

1 mrrfrlvgfl sslvlaagaa ltgaataqaa qpaaadgyva lgdsyssgvg agsyisssgd
61 ckrstkahpy lwaaahspst fdftacsgar tgdvlsgqlg plssgtglvs isiggndagf
121 adtmttcvlq sessclsria taeayvdstl pgkldgvysa isdkapnahv vvigyprfyk
181 lgttciglse tkrtainkas dhlntvlaqr aaahgftfgd vrttftghel csgspwlhsv
241 nwlnigesyh ptaagqsggy lpvlngaa
//

FIGURE 47

SEQ ID No. 42

1 cccggcggcc cgtgcaggag cagcagccgg cccgcgatgt cctcgggcgt cgtcttcatc
61 aggccgtcca tcgcgtcggc gaccggcgcc gtgtagttgg cccggacctc gtcccaggtg
121 cccgcggcga tctggcgggt ggtgcggtgc gggccgcgcc gaggggagac gtaccagaag
181 cccatcgtca cgttctccgg ctgcggttcg ggctcgtccg ccgctccgtc cgtcgcctcg
241 ccgagcacct tctcggcgag gtcggcgctg gtcgccgtca ccgtgacgtc ggcgccccgg
301 ctccagcgcg agatcagcag cgtccagccg tcgccctccg ccagcgtcgc gctgcggtcg
361 tcgtcgcggg cgatccgcag cacgcgcgcg ccgggcggca gcagcgtggc gccggaccgt
421 acgcggtcga tgttcgccgc gtgcgagtac ggctgctcac ccgtggcgaa acggccgagg
481 aacagcgcgt cgacgacgtc ggacggggag tcgctgtcgt ccacgttgag ccggatcggc
541 agggcttcgt gcgggttcac ggacatgtcg ccatgatcgg gcacccggcc gccgcgtgca
601 cccgctttcc cgggcacgca cgacaggggc tttctcgccg tcttccgtcc gaacttgaac
661 gagtgtcagc catttcttgg catggacact tccagtcaac gcgcgtagct gctaccacgg
721 ttgtggcagc aatcctgcta agggaggttc catgagacgt ttccgacttg tcggcttcct
781 gagttcgctc gtcctcgccg ccggcgccgc cctcaccggg gcagcgaccg cccaggcggc
841 ccaacccgcc gccgccgacg gctatgtggc cctcggcgac tcctactcct ccgggtcgg
901 agcgggcagc tacatcagct cgagcggcga ctgcaagcgc agcacgaagg cccatcccta
961 cctgtgggcg gccgcccact cgccctccac gttcgacttc accgcctgtt ccggcgcccg
1021 tacgggtgat gttctctccg gacagctcgg cccgctcagc tccggcaccg gcctcgtctc
1081 gatcagcatc ggcggcaacg acgccggttt cgccgacacc atgacgacct gtgtgctcca
1141 gtccgagagc tcctgcctgt cgcggatcgc caccgccgag gcgtacgtcg actcgacgct
1201 gcccggcaag ctcgacggcg tctactcggc aatcagcgac aaggcgccga acgcccacgt
1261 cgtcgtcatc ggctacccgc gcttctacaa gctcggcacc acctgcatcg gcctgtccga
1321 gaccaagcgg acggcgatca acaaggcctc cgaccacctc aacaccgtcc tcgcccagcg
1381 cgccgccgcc cacggcttca ccttcggcga cgtacgcacc accttcaccg gccacgagct
1441 gtgctccggc agcccctggc tgcacagcgt caactggctg aacatcggcg agtcgtacca
1501 ccccaccgcg gccggccagt ccggtggcta cctgccggtc ctcaacggcg ccgcctgacc
1561 tcaggcggaa ggagaagaag aaggagcgga gggagacgag gagtgggagg ccccgcccga
1621 cggggtcccc gtccccgtct ccgtctccgt cccggtcccg caagtcaccg agaacgccac
1681 cgcgtcggac gtggcccgca ccggactccg cacctccacg cgcacggcac tctcgaacgc
1741 gccggtgtcg tcgtgcgtcg tcaccaccac gccgtcctgg cgcgagcgct cgccgcccga
1801 cgggaaggac agcgtccgcc accccggatc ggagaccgac ccgtccgcgg tcacccaccg
1861 gtagccgacc tccgcgggca gccgcccgac cgtgaacgtc gccgtgaacg cgggtgcccg
1921 gtcgtgcggc ggcggacagg cccccgagta gtgggtgcgc gagcccacca cggtcacctc
1981 caccgactgc gctgcggggc
//

FIGURE 48

SEQ ID No. 43

NP 827753.
1 mrrsritayv tslllavgca ltgaataqas paaaatgyva lgdsyssgvg agsylsssgd
61 ckrsskaypy lwqaahspss fsfmacsgar tgdvlanqlg tlnsstglvs ltiggndagf
121 sdvmttcvlq sdsaclsrin takayvdstl pgqldsvyta istkapsahv avlgyprfyk
181 lggsclagls etkrsainda adylnsaiak raadhgftfg dvkstftghe icssstwlhs
241 ldllnigqsy hptaagqsgg ylpvmnsva
//

FIGURE 49

SEQ ID No. 44

1 ccaccgccgg gtcggcggcg agtctcctgg cctcggtcgc ggagaggttg gccgtgtagc
61 cgttcagcgc ggcgccgaac gtcttcttca ccgtgccgcc gtactcgttg atcaggccct
121 tgcccttgct cgacgcggcc ttgaagccgg tgcccttctt gagcgtgacg atgtagctgc
181 ccttgatcgc ggtgggggag ccggcggcga gcaccgtgcc ctcggccggg gtggcctggg
241 cgggcagtgc ggtgaatccg cccacgaggg cgccggtcgc cacggcggtt atcgcggcga
301 tccggatctt cttgctacgc agctgtgcca tacgagggag tcctcctctg ggcagcggcg
361 cgcctgggtg gggcgcacgg ctgtgggggg tgcgcgcgtc atcacgcaca cggccctgga
421 gcgtcgtgtt ccgccctggg ttgagtaaag cctcggccat ctacgggggt ggctcaaggg
481 agttgagacc ctgtcatgag tctgacatga gcacgcaatc aacggggccg tgagcacccc
541 ggggcgaccc cggaaagtgc cgagaagtct tggcatggac acttcctgtc aacacgcgta
601 gctggtacga cggttacggc agagatcctg ctaaagggag gttccatgag acgttcccga
661 attacggcat acgtgacctc actcctcctc gccgtcggct gcgccctcac cggggcagcg
721 acggcgcagg cgtccccagc cgccgcggcc acgggctatg tggccctcgg cgactcgtac
781 tcgtccggtg tcggcgccgg cagctacctc agctccagcg gcgactgcaa gcgcagttcg
841 aaggcctatc cgtacctctg gcaggccgcg cattcaccct cgtcgttcag tttcatggct
901 tgctcgggcg ctcgtacggg tgatgtcctg gccaatcagc tcggcaccct gaactcgtcc
961 accggcctgg tctccctcac catcggaggc aacgacgcgg gcttctccga cgtcatgacg
1021 acctgtgtgc tccagtccga cagcgcctgc ctctcccgca tcaacacggc gaaggcgtac
1081 gtcgactcca ccctgcccgg ccaactcgac agcgtgtaca cggcgatcag cacgaaggcc
1141 ccgtcggccc atgtggccgt gctgggctac ccccgcttct acaaactggg cggctcctgc
1201 ctcgcgggcc tctcggagac caagcggtcc gccatcaacg acgcggccga ctatctgaac
1261 agcgccatcg ccaagcgcgc cgccgaccac ggcttcacct tcggcgacgt caagagcacc
1321 ttcaccggcc atgagatctg ctccagcagc acctggctgc acagtctcga cctgctgaac
1381 atcggccagt cctaccaccc gaccgcggcc ggccagtccg gcggctatct gccggtcatg
1441 aacagcgtgg cctgagctcc cacggcctga atttttaagg cctgaatttt taaggcgaag
1501 gtgaaccgga agcggaggcc ccgtccgtcg ggtctccgt cgcacaggtc accgagaacg
1561 gcacggagtt ggacgtcgtg cgcaccgggt cgcgcacctc gacggcgatc tcgttcgaga
1621 tcgttccgct cgtgtcgtac gtggtgacga acacctgctt ctgctgggtc tttccgccgc
1681 tcgccgggaa ggacagcgtc ttccagcccg gatccgggac ctcgcccttc ttggtcaccc
1741 agcggtactc cacctcgacc ggcacccggc ccaccgtgaa ggtcgccgtg aacgtgggcg
1801 cctgggcggt gggcggcggg caggcaccgg agtagtcggt gtgcacgccg gtgaccgtca
1861 ccttcacgga ctgggccggc ggggtcgtcg taccgccgcc gccaccgccg cctcccggag
1921 tggagcccga gctgtggtcg cccccgccgt cggcgttgtc gtcctcgggg gttttcgaac
//

FIGURE 50

SEQ ID No. 45

MRLTRSLSAASVIVFALLLALLGISPAQAAGPAYVALGDSYSSGNGAGSYIDSSGDCHRSN
NAYPARWAAANAPSSFTFAACSGAVTTDVINNQLGALNASTGLVSITIGGNDAGFADAMTT
CVTSSDSTCLNRLATATNYINTTLLARLDAVYSQIKARAPNARVVVLGYPRMYLASNPWYC
LGLSNTKRAAINTTADTLNSVISSRATAHGFRFGDVRPTFNNHELFFGNDWLHSLTLPVWE
SYHPTSTGHQSGYLPVLNANSST

FIGURE 51

SEQ ID No. 46

ACAGGCCGATGCACGGAACCGTACCTTTCCGCAGTGAAGCGCTCTCCCCCCATCGTTCGC
CGGGACTTCATCCGCGATTTTGGCATGAACACTTCCTTCAACGCGCGTAGCTTGCTACAA
GTGCGGCAGCAGACCCGCTCGTTGGAGGCTCAGTGAGATTGACCCGATCCCTGTCGGCCG
CATCCGTCATCGTCTTCGCCCTGCTGCTCGCGCTGCTGGGCATCAGCCCGGCCCAGGCAG
CCGGCCCGGCCTATGTGGCCCTGGGGGATTCCTATTCCTCGGGCAACGGCGCCGGAAGTT
ACATCGATTCGAGCGGTGACTGTCACCGCAGCAACAACGCGTACCCCGCCCGCTGGGCGG
CGGCCAACGCACCGTCCTCCTTCACCTTCGCGGCCTGCTCGGGAGCGGTGACCACGGATG
TGATCAACAATCAGCTGGGCGCCCTCAACGCGTCCACCGGCCTGGTGAGCATCACCATCG
GCGGCAATGACGCGGGCTTCGCGGACGCGATGACCACCTGCGTCACCAGCTCGGACAGCA
CCTGCCTCAACCGGCTGGCCACCGCCACCAACTACATCAACACCACCCTGCTCGCCCGGC
TCGACGCGGTCTACAGCCAGATCAAGGCCCGTGCCCCCAACGCCCGCGTGGTCGTCCTCG
GCTACCCGCGCATGTACCTGGCCTCGAACCCCTGGTACTGCCTGGGCCTGAGCAACACCA
AGCGCGCGGCCATCAACACCACCGCCGACACCCTCAACTCGGTGATCTCCTCCCGGGCCA
CCGCCCACGGATTCCGATTCGGCGATGTCCGCCCGACCTTCAACAACCACGAACTGTTCT
TCGGCAACGACTGGCTGCACTCACTCACCCTGCCGGTGTGGGAGTCGTACCACCCCACCA
GCACGGGCCATCAGAGCGGCTATCTGCCGGTCCTCAACGCCAACAGCTCGACCTGATCAA
CGCACGGCCGTGCCCGCCCCGCGCGTCACGCTCGGCGCGGGCGCCGCAGCGCGTTGATCA
GCCCACAGTGCCGGTGACGGTCCCACCGTCACGGTCGAGGGTGTACGTCACGGTGGCGCC
GCTCCAGAAGTGGAACGTCAGCAGGACCGTGGAGCCGTCCCTGACCTCGTCGAAGAACTC
CGGGGTCAGCGTGATCACCCCTCCCCCGTAGCCGGGGGCGAAGGCGGCGCCGAACTCCTT
GTAGGACGTCCAGTCGTGCGGCCCGGCGTTGCCACCGTCCGCGTAGACCGCTTCCATGGT
CGCCAGCCGGTCCCCGCGGAACTCGGTGGGGATGTCCGTGCCCAAGGTGGTCCCGGTGGT
GTCCGAGAGCACCGGGGGCTCGTACCGGATGATGTGCAGATCCAAAGAATT

FIGURE 52

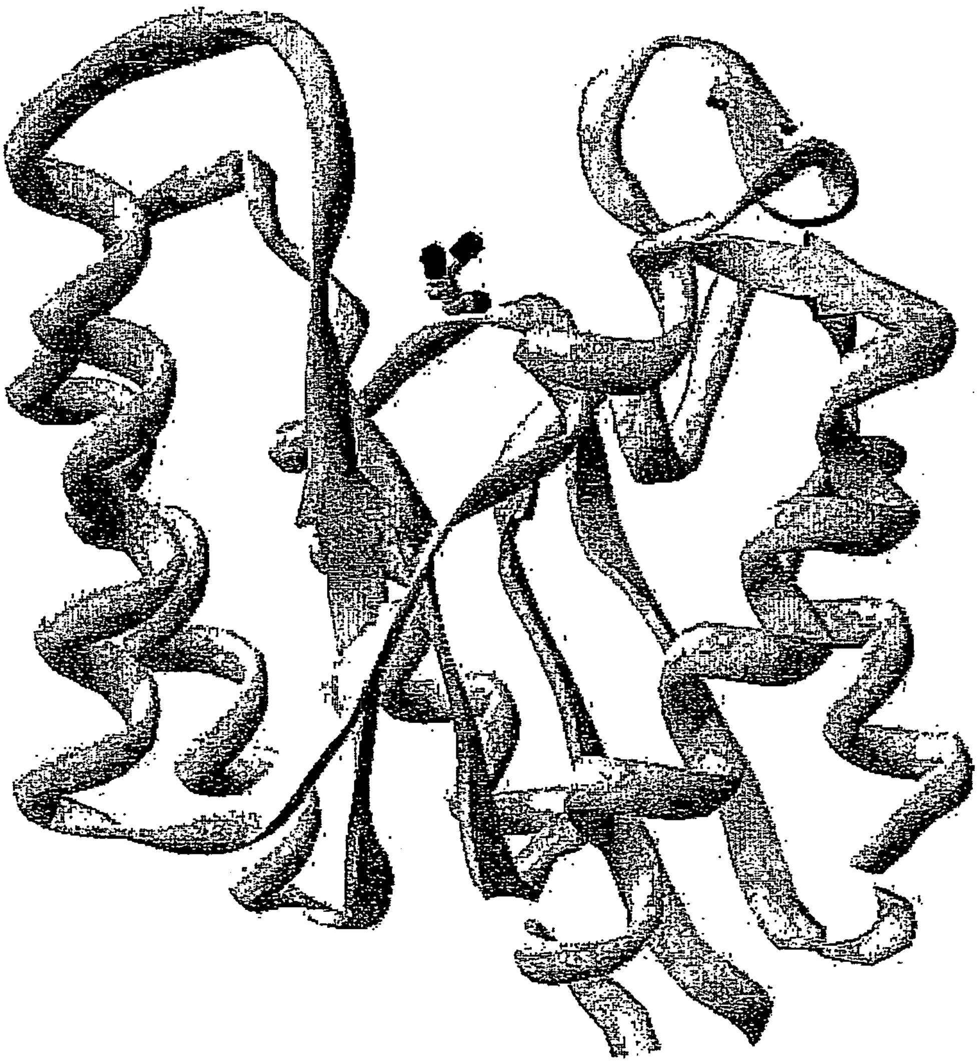

FIGURE 53

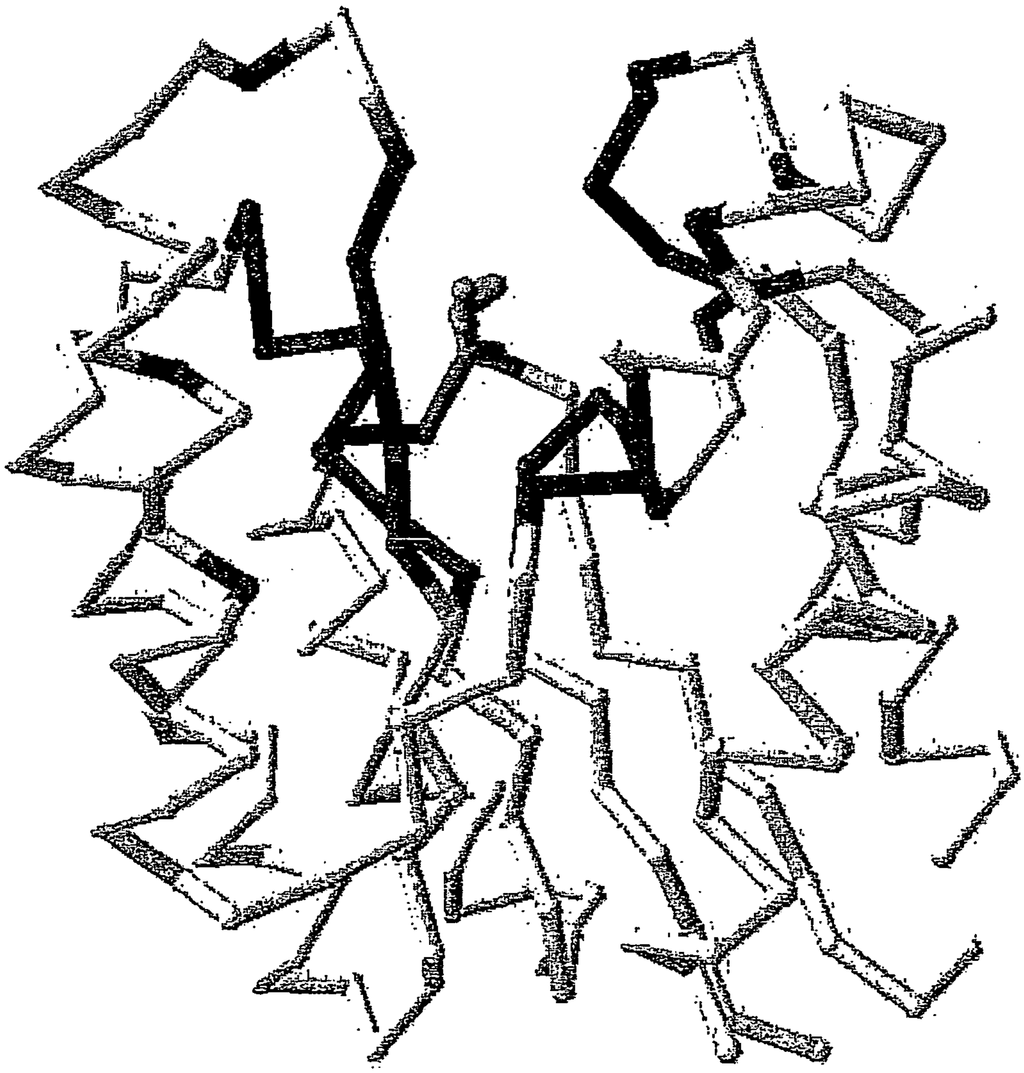

FIGURE 54

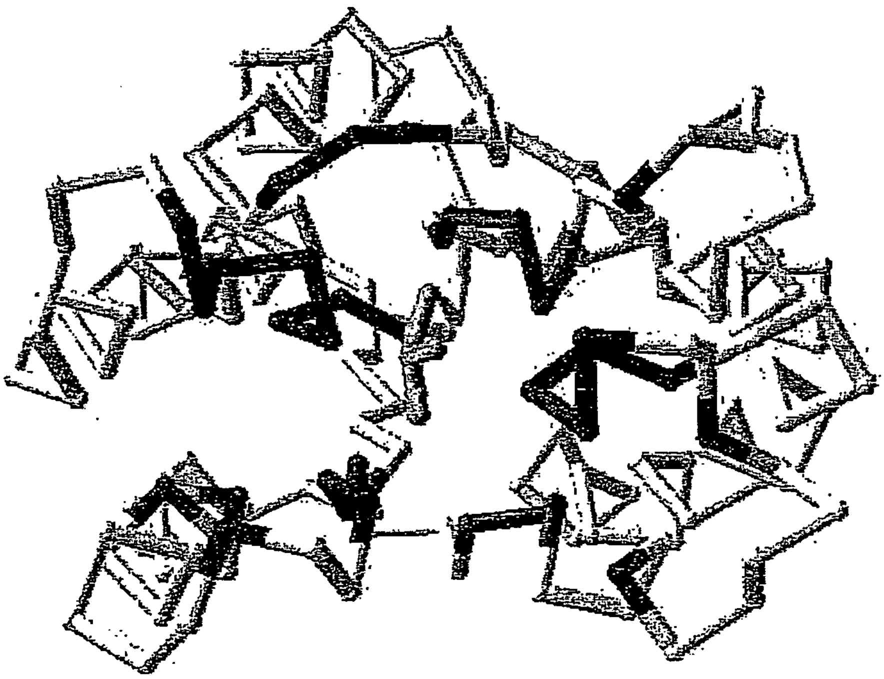

FIGRURE 55

```
1DEO      T T V Y L A G D S T M A K n - - - - - - - - - - - - - - - - - - - - - G G G S G T N G W G E Y L
            s1s1s1s1 s1 s1s1h?h?h?h?                                              h1h1h1h1h1
1IVN    A D T L L I L G D S L S A G - - - - - - - - - - - - - - - - - - - - - - Y R M S A S A A W P A L L
            s1s1s1s1 s1 s1s1h h h h                                                 h1h1h1h1h1
P10480        I V M F G D S L S D T g k m y s k m r g y l p s s p p y y e G R F S N G P V W L E Q L

1DEO    A S Y L S A T V - - - - - - - - - - - - - - - V N D A V A G R S - - - A R S Y T R E G R F E N I A D VV
        h1h1h1  s2 s2 s2                           s2s2 s2 s2 s2   h3  h3h3 h3h3h3h3h3h3h3h3h3h3h3h3h3
1IVN    N D K W q s k - - - - - - - - - - - t s v V N A S I S G D T - - - - - - S Q Q G L A R L P A L L KQ
        h1h1h1  s2?s2?                  s2?s2s2s2s2 s2 s2 s2                 h3h3h3h3h3h3h3h3h3h3h3h3h3
P10480  T N E F P G L T i a n e a e g g p t a y a Y N K I S W N P K y q v I N N L D Y E V T Q F L Q K D SF

1DEO    T A G D Y V I V E F G H N D G g s l s t d n g r t d c s g t g a E v C Y S V Y D G V N E T I L T FP
            s4s4 s4 s4s4s4     ? ? ? ? ? ? ? ? ? ? ? ? ? ? ? ? ? ? ? ? s?s?s?s?s?s?s?s?s?s?s?s?h4h4h4
1IVN    H Q P R W V L V E L G G N D G - - - - - - - - - - - - - - - - - - - - - - - - - - L R G F Q P Q Q TE
        h3  s4s4s4 s4 s4s4                                                                h4h4h4h4h4
P10480  K P D D L V I L W V G A N D Y - - - - - - - - - - - - - - - - - - - - - L A Y G W N T E Q D A K R VR

1DEO    A Y L E N A A K L F T - A K G A K - - - - - V I L S S Q T P - - - - - N N P W E T G T F V N S P TR
        h4h4h4h4h4 h4 h4h4h4h4h4  h4   s5      s5 s5 s5 s5 s5
1IVN    Q T L R Q I L Q D V K a A N A E P l l m q i R L P A N Y G R - - - - - - - - - - - - - - - - - - - RY
        h4h4h4h4h4 h4 h4h4h4h4h4h4  s5s5s5 s5s5s5s5?s5?s5?s5?                                            h5
P10480  D A I S D A A N R M V - L N G A K - - - - - E I L L F N L P d l g q n P S A R S Q K V V E A A S HV

1DEO    F V E Y A E L A A E V A - - - - - - - - - - - - - - - G V E Y V D H W S Y V D S I Y E T L G N A t vn
        h5h5h5h5h5 h5 h5h5h5h5h5h5                          s6s6s6s6?h6h6h6h6h6h6h6h6h6h6h6  h h h h
1IVN    N E A F S A I Y P K L A k e - - - - - - - - - - - - f D V P L L P F F M E E V Y L K P Q W - - - - - --
        h5h5h5h5h5 h5 h5h5h5h5h5h5h5h5            h5   s6s6s6s6?  h6h6h6h6h6    s
P10480  S A Y H N Q L L L N L A r q l a p t g m v k l f e i D K Q F A E M L R D P Q N F G L S D Q R N a cy

1DEO    - - - - - - - - - - - - - - - - - - - - - - - - - - - - - - - - - - - - - - - - - - - - - - - - --
1IVN    - - - - - - - - - - - - - - - - - - - - - - - - - - - - - - - - - - - - - - - - - - - - - - - - --
P10480  g g s y v w k p f a s r s a s t d s q l s a f n p q e r l a i a g n p l l a q a v a s p m a a r sa

1DEO    - - - - - - s y F P I D H T H T S P A G A E V V A E A F L K A V V C T G T S L K S V L T T T S F EG
                    h           s?s?s? h7h7h7h7 h7 h7 h7 h7h7h7h7h7h7h7 h7h7h7h7   h?h?h?
1IVN    - - - - - - - - M Q D D G I H P N R D A Q P F I A D W M A K Q L Q P L V N H D S L E
                      s s       s s s  h7h7h7 h7 h7 h7 h7h7h7h7h7h7h7 h7h7h7h7
P10480  s t l n c e g k M F W D Q V H P T T V V H A A L S E P A A T F I E S Q Y E F L A H -
```

FIGURE 56

```
1DEOm     T T V Y L A G D S T M A K n - - - - - - - - - - - - - - - - - - - G G G S G T N G W G E Y L
          s1s1s1s1 s1 s1s1h?h?h?h?                                                h1h1h1h1h1
1IVNm   A D T L L I L G D S L S A G - - - - - - - - - - - - - - - - - - - - Y R M S A S A A W P A L L
          s1s1s1s1 s1 s1s1h h h h                                                  h1h1h1h1h1
P10480m     I V M F G D S L S D T g k m y s k m r g y l p s s p p y y e G R F S N G P V W L E Q L

1DEOm   A S Y L S A T V - - - - - - - - - - - - - - V N D A V A G R S - - -   A R S Y T R E G R F E N IA
        h1h1h1 s2 s2 s2                       s2s2s2s2s2   h3        h3h3h3h3h3h3h3h3h3h3h3h3h3
1IVNm   N D K W q s k - - - - - - - - - - - - - t s v V N A S I S G D T - - - - - - S Q Q G L A   R L P A LL
        h1h1h1 s2?s2?                     s2?s2s2s2s2s2s2s2                h3h3h3h3h3 h3h3h3h3h3h3
P10480m T N E F P G L T i a n e a e g g p t a v a Y N K I S W N P K   y q v I N N L D Y E V T Q F LQ

1DEOm   D V V T A G D Y V I V E F G H N D G g s l s t d n g r t d c s g t g a E v C Y S V Y D G V N E TI
        h3h3     s4 s4s4s4s4s4         ? ? ? ? ? ? ? ? ? ? ? ? ? ? ? ? ? ? ? ? s?s?s?s?s?s?s?s?s?s?s?s?
1IVNm   K Q H Q P     R W V L V E L G G N D G - - - - - - - - - - - - - - - - - - - - - - - - - L R G F QP
        h3h3h3     s4s4s4s4s4s4                                                                  h4
P10480m K D S F K P D D L V I L W V G A N D Y - - - - - - - - - - - - - - - - - - - - - L A Y G W N T E Q DA

1DEOm   L T F P A Y L E N A A K L F T A K G A K V I L S S Q T P N N P W E T G T F V N S P T R
          h4h4h4h4 h4 h4h4h4h4h4h4h4h4h4h4h4    s5s5s5s5s5s5
1IVNm   Q Q T E Q T L R Q I L Q D V K a A N A E P l l m q i R L P A N Y G R - - - - - - - - - - R Y N E A
        h4h4h4h4h4 h4 h4h4h4h4h4h4h4h4h4h4h4    s5s5s5s5s5s5s5s5?s5?s5?s5?                 h5h5h5h5h5h5
P10480m K R V R D A I S D A A N R M V L N G A K E I L L F N L P d l g q n P S A R S Q K V V E A A S H V SA

1DEOm     F V E Y A E L A A E V A - - - - - - - - - - - - G V E Y V D H W S Y V D S I Y E T L G N A t v n --
          h5h5h5h5 h5 h5h5h5h5h5h5h5                   s6 s6 s6 s6?h6h6h6h6h6h6h6h6h6h6h6   h h h h
1IVNm     F S A I Y P K L A k e - - - - - - - - - - - f D V P L L P F F M E E V Y        L K P Q W - - --
        h5h5h5h5h5 h5 h5h5h5                  h5  s6s6s6s6?  h6 h6 h6 h6h6     s
P10480m Y H N Q L L L N L A r q l a p t g m v k l f e i D K Q F A E M L R D P Q N F G L S D Q R N a c y gg

1DEOm   - - - - - - - - - - - - - - - - - - - - - - - - - - - - - - - - - - - - - - - - - - - - - - --

1IVNm   - - - - - - - - - - - - - - - - - - - - - - - - - - - - - - - - - - - - - - - - - - - - - - --

P10480m s y v w k p f a s r s a s t d s q l s a f n p q e r l a i a g n p l l a q a v a s p m a a r s a st

1DEOm   - - - - s y F P I D H T H T S P A G A E V V A E A F L K A V V C T G T S L K S V L T T T S F E G TC
                h          s?s?s?h7h7 h7h7h7h7h7h7h7h7 h7 h7 h7 h7 h7h7h7h7   h?h?h?
1IVNm   - - - - - - M Q D D G I H P N R D A Q P F I A D W M A K Q L Q P L V N H D S L E
                s s s h7h7h7h7h7h7h7h7 h7h7h7h7h7h7h7h7 h7 h7 h7 h7 h7
P10480m l n c e g k M F W D Q V H P T T V V H A A L S E P A A T F I E S Q Y E F L A H -
```

FIGURE 57

```
1DEO      T T V Y  L  A G D S T M A K n - - - - - - - - - - - - - - - - - - - - - - G G G S G T N G W G E Y L
            s1s1s1s1 s1 s1s1h?h?h?h?                                                  h1h1h1h1h1
1IVN    A D T L L  I  L G D S L S A G - - - - - - - - - - - - - - - - - - - - - - - Y R M S A S A A W P A L L
            s1s1s1s1 s1 s1s1h h h h                                                       h1h1h1h1h1
P10480      I V  M  F G D S L S D T g k m  y s k m  r  g  y  l  p  s  s  p  p y  y e G R F S N G P V W L E Q L

1DEOm     T T V Y  L  A G D S T M A K n - - - - - - - - - - - - - - - - - - - - - - G G G S G T N G W G E Y L
            s1s1s1s1 s1 s1s1h?h?h?h?                                                  h1h1h1h1h1
1IVNm   A D T L L  I  L G D S L S A G - - - - - - - - - - - - - - - - - - - - - - - Y R M S A S A A W P A L L
            s1s1s1s1 s1 s1s1h h h h                                                       h1h1h1h1h1
P10480m     I V  M  F G D S L S D T g k m  y s k m  r  g  y  l  p  s  s  p  p y  y e G R F S N G P V W L E Q L


1DEO    A S Y L S  A  T V - - - - - - - - - - - - - - -  V  N  D  A  V  A  G  R  S -  - - A R S Y T R E G R F E N I A D VV
        h1h1h1  s2 s2 s2                        s2s2 s2 s2 s2        h3       h3h3 h3h3h3h3h3h3h3h3h3h3h3h3h3
1IVN    N D K W q  s  k - - - - - - - - - - - - t s v  V  N  A  S  I  S  G  D  T -  - - - - - S Q Q G L A R L P A L L KQ
        h1h1h1  s2?s2?                    s2?s2s2s2s2 s2 s2 s2                      h3h3h3h3h3h3h3h3h3h3h3h3h3
P10480  T N E F P  G  L T i a n e a e g g p  t a v a  Y  N  K  I  S  W  N  P  K y  q v I N N L D Y E V T Q F L Q K D SF

1DEOm   A S Y L S  A  T V - - - - - - - - - - - - - - -  V  N  D  A  V  A  G  R  S -  - -       A R S Y T R E G R F E N IA
        h1h1h1  s2 s2 s2                        s2s2 s2 s2 s2        h3                  h3h3h3h3h3h3h3h3h3h3h3h3
1IVNm   N D K W q  s  k - - - - - - - - - - - - t s v  V  N  A  S  I  S  G  D  T -  - - - - - S Q Q G L A     R L P A LL
        h1h1h1  s2?s2?                    s2?s2s2s2s2 s2 s2 s2                      h3h3h3h3h3   h3h3h3h3h3h3
P10480m T N E F P  G  L T i a n e a e g g p  t a v a  Y  N  K  I  S  W  N  P  K             y q v I N N L D Y E V T Q F LQ


1DEO    T A G D Y  V  I V E F G H N D G g s  l s t d  n  g  r  t  d  c  s  g  t g  a E v C Y S V Y D G V N E T I L T FP
              s4s4 s4 s4s4s4          ? ? ? ?  ? ? ? ?  ?  ?  ?  ?  ?  ?  ?  ?  ? ?  ? ? s?s?s?s?s?s?s?s?s?s?s?s?h4h4h4
1IVN    H Q P R W  V  L V E L G G N D G - - - - - - - - - - - - - - - - - - - - - - - - - - - - - - - L R G F Q P Q Q TE
        h3  s4s4s4 s4 s4s4                                                                       h4h4h4h4h4
P10480  K P D D L  V  I L W V G A N D Y - - - - - - - - - - - - - - - - - - - - - - - - - - - L A Y G W N T E Q D A K R VR

1DEOm   D   V V T  A  G D Y V I V E F G H N  D G g s  l  s  t  d  n  g  r  t  d c  s g t g a E v C Y S V Y D G V N E TI
        h3h3      s4  s4s4s4s4s4            ? ?  ? ? ? ?  ?  ?  ?  ?  ?  ?  ?  ?  ? ?  ? ? ? ? s?s?s?s?s?s?s?s?s?s?s?s?
1IVNm   K Q H Q P         R W V L V E L G G N  D G - - - - - - - - - - - - - - - - - - - - - - - - - - - - - L R G F QP
        h3h3h3            s4s4s4s4s4s4                                                                           h4
P10480m K D S F K  P  D D L V I L W V G A N  D Y - - - - - - - - - - - - - - - - - - - - - - - - - - - L A Y G W N T E Q DA


1DEO    A Y L E N  A  A K L F T - A K G A K  - - - -  -  V  I  L  S  S  Q  T  P -  - - - - N N P W E T G T F V N S P TR
        h4h4h4h4h4 h4 h4h4h4h4h4  h4     s5              s5 s5 s5 s5 s5
1IVN    Q T L R Q  I  L Q D V K a A N A E P  l l m q  i  R  L  P  A  N  Y  G  R -  - - - - - - - - - - - - - - - - - - - RY
        h4h4h4h4h4 h4 h4h4h4h4h4h4    s5s5s5  s5s5s5s5?s5?s5?s5?                                                  h5
P10480  D A I S D  A  A N R M V - L N G A K  - - - -  -  E  I  L  L  F  N  L  P d  l g q n P S A R S Q K V V E A A S HV

1DEOm   L T F P A  Y  L E N A A K L F T A K  G A K V  I  L  S  S  Q  T  P  N  N P  W E T G T F V N S P T R
          h4h4h4h4 h4 h4h4h4h4h4h4h4h4h4h4       s5s5s5 s5 s5 s5
```

```
1IVNm    Q Q T E Q  T  L R Q I L Q D V K a A  N A E P  l  l  m  q  i  R  L  P  A N  Y G R - - - - - - - - - - R Y N E A
         h4h4h4h4h4 h4 h4h4h4h4h4h4h4h4h4h4     s5s5s5 s5 s5 s5 s5?s5?s5?s5?                            h5h5h5h5h5h5
P10480mK R V R D    A  I S D A A N R M V L N  G A K E  I  L  L  F  N  L  P  d  l  g  q n P S A R S Q K V V E A A S H V SA

1DEO      F V E Y A  E  L A A E V A - - - - - - - - - - - - - - - - - G  V  E  Y V  D H W S Y V D S I Y E T L G N A t vn
          h5h5h5h5h5 h5 h5h5h5h5h5h5                                   s6 s6 s6s6?h6h6h6h6h6h6h6h6h6h6h6   h h h h
1IVN      N E A F S  A  I Y P K L A k e - - - - - - - - - - - -  f  D  V  P  L L  P F F M E E V Y L K P Q W - - - - - --
          h5h5h5h5h5 h5 h5h5h5h5h5h5h5h5                   h5       s6 s6 s6s6?  h6h6h6h6h6      s
P10480    S A Y H N  Q  L L L N L A r q l a p  t g m v  k  l  f  e  i  D  K  Q  F A  E M L R D P Q N F G L S D Q R N a cy

1DEOm       F V E Y  A  E L A A E V A - - - - - - - - - - - - - G  V  E  Y  V  D H  W S Y V D S I Y E T L G N A t v n --
            h5h5h5h5 h5 h5h5h5h5h5h5h5                          s6 s6 s6 s6?h6h6 h6h6h6h6h6h6h6h6h6   h h h h
1IVNm       F S A I  Y  P K L A k e - - - - - - - - - - - - f  D  V  P  L  L  P F  F M E E V Y      L K P Q W - - - --
          h5h5h5h5h5 h5 h5h5h5                  h5   s6 s6 s6 s6?  h6 h6 h6 h6h6     s
P10480mY H N Q L    L  L N L A r q l a p t g  m v k l  f  e  i  D  K  Q  F  A  E M  L R D P Q N F G L S D Q R N a c y gg

1DEO      - - - - -  -  - - - - - - - - - - - - - - - - - - - - - - - - - - - - - - - - - - - - - - - - - - - - - --

1IVN      - - - - -  -  - - - - - - - - - - - - - - - - - - - - - - - - - - - - - - - - - - - - - - - - - - - - - --

P10480    g g s y v  w  k p f a s r s a s t d  s q l s  a  f  n  p  q  e  r  l  a i  a g n p l l a q a v a s p m a a r sa

1DEOm     - - - - -  -  - - - - - - - - - - - - - - - - - - - - - - - - - - - - - - - - - - - - - - - - - - - - - --

1IVNm     - - - - -  -  - - - - - - - - - - - - - - - - - - - - - - - - - - - - - - - - - - - - - - - - - - - - - --

P10480ms y v w k    p  f a s r s a s t d s q  l s a f  n  p  q  e  r  l  a  i  a g  n p l l a q a v a s p m a a r s a st

1DEO      - - - - -  -  s y F P I D H T H T S  P A G A  E  V  V  A  E  A  F  L  K A  V V C T G T S L K S V L T T T S F EG
                        h              s?s?s?  h7h7h7h7 h7 h7 h7 h7 h7 h7 h7 h7 h7h7 h7h7h7h7    h?h?h?
1IVN      - - - - -  -  - - M Q D D G I H P N  R D A Q  P  F  I  A  D  W  M  A  KQ  L Q P L V N H D S L E
                            s s       s s s      h7h7h7 h7 h7 h7 h7 h7 h7 h7 h7 h7h7 h7h7h7h7
P10480    s t l n c  e  g k M F W D Q V H P T  T V V H  A  A  L  S  E  P  A  A  T F  I E S Q Y E F L A H -

1DEOm     - - - - s  y  F P I D H T H T S P A  G A E V  V  A  E  A  F  L  K  A  V V  C T G T S L K S V L T T T S F E G TC
                  h               s?s?s?h7h7 h7h7h7h7 h7 h7 h7 h7 h7 h7 h7 h7 h7h7 h7h7    h?h?h?
1IVNm     - - - - -  -  M Q D D G I H P N R D  A Q P F  I  A  D  W  M  A  K  Q  L Q  P L V N H D S L E
                     s  s s  h7h7h7h7h7h7h7h7h7 h7h7h7h7 h7 h7 h7 h7 h7 h7 h7 h7 h7
P10480ml n c e g    k  M F W D Q V H P T T V  V H A A  L  S  E  P  A  A  T  F  I E  S Q Y E F L A H -
```

FIGURE 58

```
                        10        20        30        40        50        60
                ....*....|....*....|....*....|....*....|....*....|....*....|
1IVN_A        4 LLILGDSLSAG-------------------YRMSASAAWPALLNDKWqsk---------- 34
P10480       28 IVMFGDSLSDTgkmyskmrgylpssppyyeGRFSNGPVWLEQLTNEFPGLTianeaeggp 87

                        70        80        90       100       110       120
                ....*....|....*....|....*....|....*....|....*....|....*....|
1IVN_A       35 -tsvVNASISGDT----------------------------SQQGLARLPALLKQHQPRW 65
P10480       88 tavaYNKISWNPKyq----------------------vINNLDYEVTQFLQKDSFKPDDL 125

                       130       140       150       160       170       180
                ....*....|....*....|....*....|....*....|....*....|....*....|
1IVN_A       66 VLVELGGNDG--------------------------------------LRGFQPQQTEQT 87
P10480      126 VILWVGANDY-------------------------------LA--YGWNTEQDAKRVRDA 152

                       190       200       210       220       230       240
                ....*....|....*....|....*....|....*....|....*....|....*....|
1IVN_A       88 LRQILQDVKaANAEPllmqiRLPANYGR-------------------------------- 115
P10480      153 ISDAANRMV-LNGAK-----EILLFNLPdlg--------------------------qnP 180

                       250       260       270       280       290       300
                ....*....|....*....|....*....|....*....|....*....|....*....|
1IVN_A      116 ------------RYNEAFSAIYPKLAke---------------------fDVPLLPFFME 142
P10480      181 SARSQKVVEAASHVSAYHNQLLLNLArqlaptg----------mvklfeiDKQFAEMLRD 230

                       310       320       330       340       350       360
                ....*....|....*....|....*....|....*....|....*....|....*....|
1IVN_A      143 EVYLKPQW---------------------------------------------------- 150
P10480      231 PQNFGLSDQRNacyggsyvwkpfasrsastdsqlsafnpqerlaiagnpllaqavaspma 290

                       370       380       390       400
                ....*....|....*....|....*....|....*....|
1IVN_A      151 ------------MQDDGI--------HPNRDAQPFIADWM 170
P10480      291 arsastlncegkMFWDQV--------HPTTVVHAALSEPA 322
```

FIGURE 59

```
                        1                                                  50
   P10480      (1) MKKWFVCLLGLVALTVQAADSRPAFSRIVMFGDSLSDTGKMYSKMRGYLP
   A. sal      (1) ------------------ADTRPAFSRIVMFGDSLSDTGKMYSKMRGYLP
   A. hyd      (1) ------------------ADSRPAFSRIVMFGDSLSDTGKMYSKMRGYLP
Consensus      (1)                   AD*RPAFSRIVMFGDSLSDTGKMYSKMRGYLP
                   51                                                100
   P10480     (51) SSPPYYEGRFSNGPVWLEQLTNEFPGLTIANEAEGGPTAVAYNKISWNPK
   A. sal     (33) SSPPYYEGRFSNGPVWLEQLTKQFPGLTIANEAEGGATAVAYNKISWNPK
   A. hyd     (33) SSPPYYEGRFSNGPVWLEQLTKQFPGLTIANEAEGGATAVAYNKISWNPK
Consensus     (51) SSPPYYEGRFSNGPVWLEQLT**FPGLTIANEAEGG*TAVAYNKISWNPK
                   101                                               150
   P10480    (101) YQVINNLDYEVTQFLQKDSFKPDDLVILWVGANDYLAYGWNTEQDAKRVR
   A. sal     (83) YQVINNLDYEVTQFLQKDSFKPDDLVILWVGANDYLAYGWNTEQDAKRVR
   A. hyd     (83) YQVINNLDYEVTQFLQKDSFKPDDLVILWVGANDYLAYGWNTEQDAKRVR
Consensus    (101) YQVINNLDYEVTQFLQKDSFKPDDLVILWVGANDYLAYGWNTEQDAKRVR
                   151                                               200
   P10480    (151) DAISDAANRMVLNGAKEILLFNLPDLGQNPSARSQKVVEAASHVSAYHNQ
   A. sal    (133) DAISDAANRMVLNGAKQILLFNLPDLGQNPSARSQKVVEAVSHVSAYHNK
   A. hyd    (133) DAISDAANRMVLNGAKQILLFNLPDLGQNPSARSQKVVEAVSHVSAYHNQ
Consensus    (151) DAISDAANRMVLNGAK*ILLFNLPDLGQNPSARSQKVVEA*SHVSAYHN*
                   201                                               250
   P10480    (201) LLLNLARQLAPTGMVKLFEIDKQFAEMLRDPQNFGLSDQRNACYGGSYVW
   A. sal    (183) LLLNLARQLAPTGMVKLFEIDKQFAEMLRDPQNFGLSDVENPCYDGGYVW
   A. hyd    (183) LLLNLARQLAPTGMVKLFEIDKQFAEMLRDPQNFGLSDVENPCYDGGYVW
Consensus    (201) LLLNLARQLAPTGMVKLFEIDKQFAEMLRDPQNFGLSD**N*CY*G*YVW
                   251                                               300
```

```
P10480     (251) KPFASRSASTDSQLSAFNPQERLAIAGNPLLAQAVASPMAARSASTLNCE
A. sal     (233) KPFATRSVSTDRQLSAFSPQERLAIAGNPLLAQAVASPMARRSASPLNCE
A. hyd     (233) KPFATRSVSTDRQLSAFSPQERLAIAGNPLLAQAVASPMARRSASPLNCE
Consensus  (251) KPFA*RS*STD*QLSAF*PQERLAIAGNPLLAQAVASPMA*RSAS*LNCE
                 301                                 336
P10480     (301) GKMFWDQVHPTTVVHAALSEPAATFIESQYEFLAH-
A. sal     (283) GKMFWDQVHPTTVVHAALSERAATFIETQYEFLAHG
A. hyd     (283) GKMFWDQVHPTTVVHAALSERAATFIANQYEFLAH-
Consensus  (301) GKMFWDQVHPTTVVHAALSE*AATFI**QYEFLAH*
```

FIGURE 60

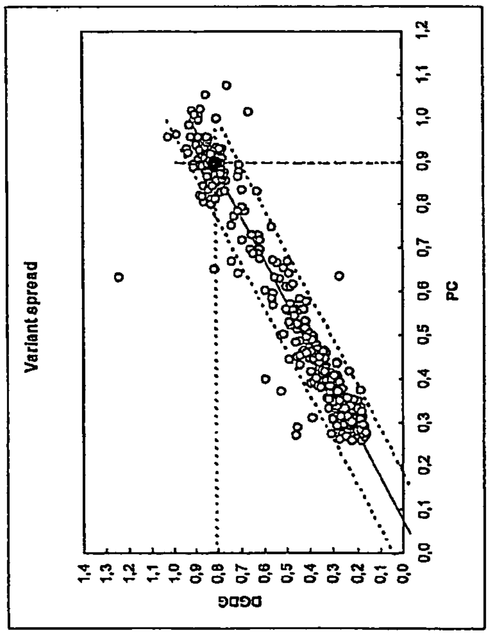
Variant spread
DGDG
PC
1,4
1,3
1,2
1,1
1,0
0,9
0,8
0,7
0,6
0,5
0,4
0,3
0,2
0,1
0,0
0,0 0,1 0,2 0,3 0,4 0,5 0,6 0,7 0,8 0,9 1,0 1,1 1,2

FIGURE 61

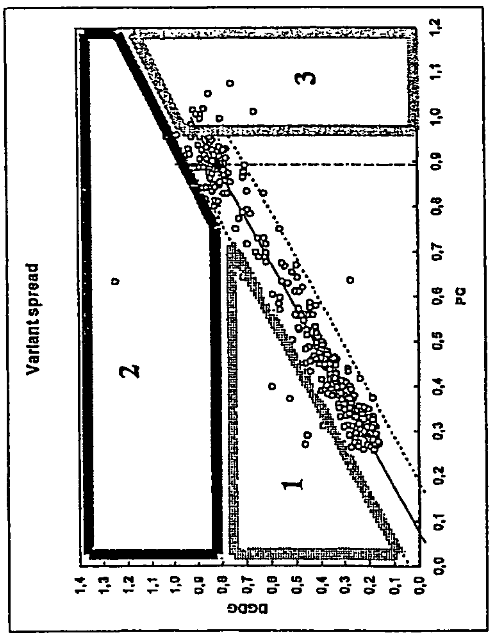

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 09750990 B
- US 10409391 B
- US 60489441 B
- GB 0330016 A
- WO 2004000655 W
- WO 02065854 A
- EP 0583265 A
- EP 0866796 A
- WO 0206457 A
- EP 0752008 A
- EP 1138763 A
- EP 1103606 A
- US 6180406 B
- WO 0134835 A
- EP 1131416 A
- WO 0214490 A
- WO 00061771 A
- WO 9299640 A
- US 4683202 A
- WO 0058517 A
- US 6344328 B
- US 6361974 B
- WO 9117243 A
- EP 0238023 A
- EP 0449375 A
- WO 04064537 A
- WO 02214490 A
- US 5741665 A
- US 5674707 A
- WO 0139544 A
- WO 0116308 A
- US 3817837 A
- US 3850752 A
- US 3939350 A
- US 3996345 A
- US 4277437 A
- US 4275149 A
- US 4366241 A
- US 4816567 A
- WO 04000655 W
- GB 0415999 A
- US 10911160 A

### Non-patent literature cited in the description


- **BUCKLEY.** *Biochemistry,* 1983, vol. 22, 5490-5493
- **UPTON ; BUCKLEY.** *TIBS,* 20 May 1995, 178-179
- **BRUMLIK ; BUCKLEY.** *J. of Bacteriology,* April 1996, 2060-2064
- **THORNTON et al.** *Biochem. et Biophys. Acta,* 1988, vol. 959, 153-159
- **HILTON ; BUCKLEY.** *J. Biol. Chem.,* 1991, vol. 266, 997-1000
- **BUCKLEY et al.** *J. Bacteriol,* 1996, vol. 178 (7), 2060-4
- **ROBERTSON et al.** *J. Biol. Chem.,* 1994, vol. 269, 2146-50
- Nomenclature Committee of the International Union of Biochemistry and Molecular Biology. *Enzyme Nomenclature Recommendations,* 1992
- **BRUMLIK ; BUCKLEY.** *Journal of Bacteriology,* April 1996, vol. 178 (7), 2060-2064
- **BATEMAN A et al.** *Nucleic Acids Res.,* 2002, vol. 30, 276-280
- **BATEMAN A ; HAFT DH.** *Brief Bioinform,* 2002, vol. 3, 236-245, http://www.ncbi.nlm.nih pov/entrez/query.fcgi?cmd=Retrieve&db=PubMed&list uids =12230032&dopt=Abstract http://www.ncbi.nlm.nih.gov/entrez/query.fcgi?cmd=Retrieve&db=PubMed&list uids =11752314&dopt=Abstract
- probabilistic models of proteins and nucleic acids. **DURBIN R ; EDDY S ; KROGH A.** Biological sequence analysis. Cambridge University Press, 1998
- **BEUCAGE S.L. et al.** *Tetrahedron Letters,* 1981, vol. 22, 1859-1869
- **MATTHES et al.** *EMBO J.,* 1984, vol. 3, 801-805
- **SAIKI R K et al.** *Science,* 1988, vol. 239, 487-491
- **CARUTHERS MH et al.** *Nuc Acids Res Symp Ser,* 1980, 215-23
- **HORN T et al.** *Nuc Acids Res Symp Ser,* 1980, 225-232
- **MORINAGA et al.** *Biotechnology,* 1984, vol. 2, 646-649

- **NELSON ; LONG.** *Analytical Biochemistry,* 1989, vol. 180, 147-151
- **HILTON ; BUCKLEY.** *J Biol. Chem.,* 15 January 1991, vol. 266 (2), 997-1000
- **ROBERTSON et al.** *J. Biol. Chem.,* 21 January 1994, vol. 269 (3), 2146-50
- **BRUMLIK et al.** *J. Bacteriol,* April 1996, vol. 178 (7), 2060-4
- **PEELMAN et al.** *Protein Sci.,* March 1998, vol. 7 (3), 587-99
- **DEVEREUX et al.** *Nuc. Acids Research,* 1984, vol. 12, 387
- **AUSUBEL et al.** Short Protocols in Molecular Biology. 1999
- **ALTSCHUL et al.** *J. Mol. Biol.,* 1990, 403-410
- *FEMS Microbiol Lett,* 1999, vol. 174 (2), 247-50
- *FEMS Microbiol Lett,* 1999, vol. 177 (1), 187-8
- **HIGGINS DG ; SHARP PM.** *Gene,* 1988, vol. 73 (1), 237-244
- **SIMON RJ et al.** *PNAS,* 1992, vol. 89 (20), 9367-9371
- **HORWELL DC.** *Trends Biotechnol.,* 1995, vol. 13 (4), 132-134
- Guide to Molecular Cloning Techniques. **BERGER ; KIMMEL.** Methods in Enzymology. Academic Press, 1987, vol. 152
- **SAMBROOK et al.** Molecular Cloning: A Laboratory Manual. Cold Spring Harbor Laboratory Press, 1989
- **POTRYKUS.** *Annu Rev Plant Physiol Plant Mol Biol,* 1991, vol. 42, 205-225
- **CHRISTOU.** *Agro-Food-Industry Hi-Tech,* March 1994, 17-27
- **DAVIS ; SERRES.** *Methods Enzymol,* 1971, vol. 17A, 79-143
- Aspergillus: 50 years on. Progress in industrial microbiology. **TURNER G.** Vectors for genetic manipulation. Elsevier Amsterdam, 1994, vol. 29, 641-666
- **PUNT et al.** *Trends Biotechnol,* May 2002, vol. 20 (5), 200-6
- **ARCHER ; PEBERDY.** *Grit Rev Biotechnol,* 1997, vol. 17 (4), 273-306
- *Methods Mol Biol,* 1995, vol. 49, 341-54
- *Curr Opin Biotechnol,* October 1997, vol. 8 (5), 554-60
- *FEMS Microbiol Rev,* 2000, vol. 24 (1), 45-66
- Yeast as a vehicle for the expression of heterologous genes. **E HINCHELIFFE ; E KENNY.** Yeasts. Academic Press Ltd, 1993, vol. 5
- **HINNEN et al.** *Proceedings of the National Academy of Sciences of the USA,* 1978, vol. 75, 1929
- **BEGGS, J D.** *Nature,* 1978, vol. 275, 104
- **ITO, H et al.** *J Bacteriology,* 1983, vol. 153, 163-168
- *Curr. Opin. Biotechnol.,* 1995, vol. 6 (5), 501-6
- **UPTON C ; BUCKLEY JT.** *Trends Biochem Sci,* 1995, vol. 20, 179-179
- **PENNINAGA et al.** *Biochemistry,* 1995, 3368-3376
- **KAWAMURA ; DOI.** *J. of Bacteriology,* October 1984, 442-444
- Molecular Biological Methods for Bacillus. John Wiley & Sons Ltd, 1990
- SAS/STAT® User's Guide, Version 6. proc LOGISTIC in SAS Institute Inc. SAS Institute Inc, 1989, vol. 2

SEQUENCE LISTING

<110> Danisco A/S

<120> Proteins

<130> P020608EPA

<150> GB0330016.7
<151> 2003-12-24

<150> PCT/IB04/000655
<151> 2004-01-15

<150> GB0415999.2
<151> 2004-07-16

<150> US 10/911, 160
<151> 2004-08-02

<160> 78

<170> PatentIn version 3.5

<210> 1
<211> 361
<212> PRT
<213> Artificial

<220>
<223> pfam00567 consensus sequence

<400> 1

```
Ile Val Ala Phe Gly Asp Ser Leu Thr Asp Gly Glu Ala Tyr Tyr Gly
1               5                   10                  15

Asp Ser Asp Gly Gly Gly Trp Gly Ala Gly Leu Ala Asp Arg Leu Thr
            20                  25                  30

Ala Leu Leu Arg Leu Arg Ala Arg Pro Arg Gly Val Asp Val Phe Asn
        35                  40                  45

Arg Gly Ile Ser Gly Arg Thr Ser Asp Gly Arg Leu Ile Val Asp Ala
    50                  55                  60

Leu Val Ala Leu Leu Phe Leu Ala Gln Ser Leu Gly Leu Pro Asn Leu
65                  70                  75                  80

Pro Pro Tyr Leu Ser Gly Asp Phe Leu Arg Gly Ala Asn Phe Ala Ser
                85                  90                  95

Ala Gly Ala Thr Ile Leu Pro Thr Ser Gly Pro Phe Leu Ile Gln Val
            100                 105                 110

Gln Phe Lys Asp Phe Lys Ser Gln Val Leu Glu Leu Arg Gln Ala Leu
        115                 120                 125
```

```
Gly Leu Leu Gln Glu Leu Leu Arg Leu Leu Pro Val Leu Asp Ala Lys
    130                 135                 140

Ser Pro Asp Leu Val Thr Ile Met Ile Gly Thr Asn Asp Leu Ile Thr
145                 150                 155                 160

Ser Ala Phe Phe Gly Pro Lys Ser Thr Glu Ser Asp Arg Asn Val Ser
                165                 170                 175

Val Pro Glu Phe Lys Asp Asn Leu Arg Gln Leu Ile Lys Arg Leu Arg
            180                 185                 190

Ser Asn Asn Gly Ala Arg Ile Ile Val Leu Ile Thr Leu Val Ile Leu
        195                 200                 205

Asn Leu Gly Pro Leu Gly Cys Leu Pro Leu Lys Leu Ala Leu Ala Leu
    210                 215                 220

Ala Ser Ser Lys Asn Val Asp Ala Ser Gly Cys Leu Glu Arg Leu Asn
225                 230                 235                 240

Glu Ala Val Ala Asp Phe Asn Glu Ala Leu Arg Glu Leu Ala Ile Ser
                245                 250                 255

Lys Leu Glu Asp Gln Leu Arg Lys Asp Gly Leu Pro Asp Val Lys Gly
            260                 265                 270

Ala Asp Val Pro Tyr Val Asp Leu Tyr Ser Ile Phe Gln Asp Leu Asp
        275                 280                 285

Gly Ile Gln Asn Pro Ser Ala Tyr Val Tyr Gly Phe Glu Thr Thr Lys
    290                 295                 300

Ala Cys Cys Gly Tyr Gly Gly Arg Tyr Asn Tyr Asn Arg Val Cys Gly
305                 310                 315                 320

Asn Ala Gly Leu Cys Asn Val Thr Ala Lys Ala Cys Asn Pro Ser Ser
                325                 330                 335

Tyr Leu Leu Ser Phe Leu Phe Trp Asp Gly Phe His Pro Ser Glu Lys
            340                 345                 350

Gly Tyr Lys Ala Val Ala Glu Ala Leu
        355                 360
```

<210> 2
<211> 317
<212> PRT
<213> Aeromonas hydrophila

<400> 2

```
Ala Asp Ser Arg Pro Ala Phe Ser Arg Ile Val Met Phe Gly Asp Ser
1               5                   10                  15

Leu Ser Asp Thr Gly Lys Met Tyr Ser Lys Met Arg Gly Tyr Leu Pro
            20                  25                  30

Ser Ser Pro Pro Tyr Tyr Glu Gly Arg Phe Ser Asn Gly Pro Val Trp
        35                  40                  45

Leu Glu Gln Leu Thr Asn Glu Phe Pro Gly Leu Thr Ile Ala Asn Glu
    50                  55                  60

Ala Glu Gly Gly Pro Thr Ala Val Ala Tyr Asn Lys Ile Ser Trp Asn
65                  70                  75                  80

Pro Lys Tyr Gln Val Ile Asn Asn Leu Asp Tyr Glu Val Thr Gln Phe
                85                  90                  95

Leu Gln Lys Asp Ser Phe Lys Pro Asp Asp Leu Val Ile Leu Trp Val
            100                 105                 110

Gly Ala Asn Asp Tyr Leu Ala Tyr Gly Trp Asn Thr Glu Gln Asp Ala
        115                 120                 125

Lys Arg Val Arg Asp Ala Ile Ser Asp Ala Ala Asn Arg Met Val Leu
    130                 135                 140

Asn Gly Ala Lys Glu Ile Leu Leu Phe Asn Leu Pro Asp Leu Gly Gln
145                 150                 155                 160

Asn Pro Ser Ala Arg Ser Gln Lys Val Val Glu Ala Ala Ser His Val
                165                 170                 175

Ser Ala Tyr His Asn Gln Leu Leu Leu Asn Leu Ala Arg Gln Leu Ala
            180                 185                 190

Pro Thr Gly Met Val Lys Leu Phe Glu Ile Asp Lys Gln Phe Ala Glu
        195                 200                 205

Met Leu Arg Asp Pro Gln Asn Phe Gly Leu Ser Asp Gln Arg Asn Ala
    210                 215                 220

Cys Tyr Gly Gly Ser Tyr Val Trp Lys Pro Phe Ala Ser Arg Ser Ala
225                 230                 235                 240
```

Ser Thr Asp Ser Gln Leu Ser Ala Phe Asn Pro Gln Glu Arg Leu Ala
245 250 255

Ile Ala Gly Asn Pro Leu Leu Ala Gln Ala Val Ala Ser Pro Met Ala
260 265 270

Ala Arg Ser Ala Ser Thr Leu Asn Cys Glu Gly Lys Met Phe Trp Asp
275 280 285

Gln Val His Pro Thr Thr Val Val His Ala Ala Leu Ser Glu Pro Ala
290 295 300

Ala Thr Phe Ile Glu Ser Gln Tyr Glu Phe Leu Ala His
305 310 315

<210> 3
<211> 336
<212> PRT
<213> Aeromonas salmonicida

<400> 3

Met Lys Lys Trp Phe Val Cys Leu Leu Gly Leu Ile Ala Leu Thr Val
1 5 10 15

Gln Ala Ala Asp Thr Arg Pro Ala Phe Ser Arg Ile Val Met Phe Gly
20 25 30

Asp Ser Leu Ser Asp Thr Gly Lys Met Tyr Ser Lys Met Arg Gly Tyr
35 40 45

Leu Pro Ser Ser Pro Pro Tyr Tyr Glu Gly Arg Phe Ser Asn Gly Pro
50 55 60

Val Trp Leu Glu Gln Leu Thr Lys Gln Phe Pro Gly Leu Thr Ile Ala
65 70 75 80

Asn Glu Ala Glu Gly Gly Ala Thr Ala Val Ala Tyr Asn Lys Ile Ser
85 90 95

Trp Asn Pro Lys Tyr Gln Val Tyr Asn Asn Leu Asp Tyr Glu Val Thr
100 105 110

Gln Phe Leu Gln Lys Asp Ser Phe Lys Pro Asp Asp Leu Val Ile Leu
115 120 125

Trp Val Gly Ala Asn Asp Tyr Leu Ala Tyr Gly Trp Asn Thr Glu Gln
130 135 140

```
Asp Ala Lys Arg Val Arg Asp Ala Ile Ser Asp Ala Ala Asn Arg Met
145                 150                 155                 160

Val Leu Asn Gly Ala Lys Gln Ile Leu Leu Phe Asn Leu Pro Asp Leu
                165                 170                 175

Gly Gln Asn Pro Ser Ala Arg Ser Gln Lys Val Val Glu Ala Val Ser
            180                 185                 190

His Val Ser Ala Tyr His Asn Lys Leu Leu Leu Asn Leu Ala Arg Gln
        195                 200                 205

Leu Ala Pro Thr Gly Met Val Lys Leu Phe Glu Ile Asp Lys Gln Phe
    210                 215                 220

Ala Glu Met Leu Arg Asp Pro Gln Asn Phe Gly Leu Ser Asp Val Glu
225                 230                 235                 240

Asn Pro Cys Tyr Asp Gly Gly Tyr Val Trp Lys Pro Phe Ala Thr Arg
                245                 250                 255

Ser Val Ser Thr Asp Arg Gln Leu Ser Ala Phe Ser Pro Gln Glu Arg
            260                 265                 270

Leu Ala Ile Ala Gly Asn Pro Leu Leu Ala Gln Ala Val Ala Ser Pro
        275                 280                 285

Met Ala Arg Arg Ser Ala Ser Pro Leu Asn Cys Glu Gly Lys Met Phe
    290                 295                 300

Trp Asp Gln Val His Pro Thr Thr Val Val His Ala Ala Leu Ser Glu
305                 310                 315                 320

Arg Ala Ala Thr Phe Ile Glu Thr Gln Tyr Glu Phe Leu Ala His Gly
                325                 330                 335
```

<210> 4
<211> 295
<212> PRT
<213> Streptomyces coelicolor

<400> 4

```
Met Pro Lys Pro Ala Leu Arg Arg Val Met Thr Ala Thr Val Ala Ala
1               5                   10                  15

Val Gly Thr Leu Ala Leu Gly Leu Thr Asp Ala Thr Ala His Ala Ala
            20                  25                  30

Pro Ala Gln Ala Thr Pro Thr Leu Asp Tyr Val Ala Leu Gly Asp Ser
```

```
            35                  40                  45

Tyr Ser Ala Gly Ser Gly Val Leu Pro Val Asp Pro Ala Asn Leu Leu
    50                  55                  60

Cys Leu Arg Ser Thr Ala Asn Tyr Pro His Val Ile Ala Asp Thr Thr
65                  70                  75                  80

Gly Ala Arg Leu Thr Asp Val Thr Cys Gly Ala Ala Gln Thr Ala Asp
                85                  90                  95

Phe Thr Arg Ala Gln Tyr Pro Gly Val Ala Pro Gln Leu Asp Ala Leu
            100                 105                 110

Gly Thr Gly Thr Asp Leu Val Thr Leu Thr Ile Gly Gly Asn Asp Asn
        115                 120                 125

Ser Thr Phe Ile Asn Ala Ile Thr Ala Cys Gly Thr Ala Gly Val Leu
    130                 135                 140

Ser Gly Gly Lys Gly Ser Pro Cys Lys Asp Arg His Gly Thr Ser Phe
145                 150                 155                 160

Asp Asp Glu Ile Glu Ala Asn Thr Tyr Pro Ala Leu Lys Glu Ala Leu
                165                 170                 175

Leu Gly Val Arg Ala Arg Ala Pro His Ala Arg Val Ala Ala Leu Gly
            180                 185                 190

Tyr Pro Trp Ile Thr Pro Ala Thr Ala Asp Pro Ser Cys Phe Leu Lys
        195                 200                 205

Leu Pro Leu Ala Ala Gly Asp Val Pro Tyr Leu Arg Ala Ile Gln Ala
    210                 215                 220

His Leu Asn Asp Ala Val Arg Arg Ala Ala Glu Glu Thr Gly Ala Thr
225                 230                 235                 240

Tyr Val Asp Phe Ser Gly Val Ser Asp Gly His Asp Ala Cys Glu Ala
                245                 250                 255

Pro Gly Thr Arg Trp Ile Glu Pro Leu Leu Phe Gly His Ser Leu Val
            260                 265                 270

Pro Val His Pro Asn Ala Leu Gly Glu Arg Arg Met Ala Glu His Thr
        275                 280                 285

Met Asp Val Leu Gly Leu Asp
```

290 295

<210> 5
<211> 295
<212> PRT
<213> Streptomyces coelicolor

<400> 5

```
Met Pro Lys Pro Ala Leu Arg Arg Val Met Thr Ala Thr Val Ala Ala
1               5                   10                  15

Val Gly Thr Leu Ala Leu Gly Leu Thr Asp Ala Thr Ala His Ala Ala
            20                  25                  30

Pro Ala Gln Ala Thr Pro Thr Leu Asp Tyr Val Ala Leu Gly Asp Ser
        35                  40                  45

Tyr Ser Ala Gly Ser Gly Val Leu Pro Val Asp Pro Ala Asn Leu Leu
    50                  55                  60

Cys Leu Arg Ser Thr Ala Asn Tyr Pro His Val Ile Ala Asp Thr Thr
65                  70                  75                  80

Gly Ala Arg Leu Thr Asp Val Thr Cys Gly Ala Ala Gln Thr Ala Asp
                85                  90                  95

Phe Thr Arg Ala Gln Tyr Pro Gly Val Ala Pro Gln Leu Asp Ala Leu
            100                 105                 110

Gly Thr Gly Thr Asp Leu Val Thr Leu Thr Ile Gly Gly Asn Asp Asn
        115                 120                 125

Ser Thr Phe Ile Asn Ala Ile Thr Ala Cys Gly Thr Ala Gly Val Leu
    130                 135                 140

Ser Gly Gly Lys Gly Ser Pro Cys Lys Asp Arg His Gly Thr Ser Phe
145                 150                 155                 160

Asp Asp Glu Ile Glu Ala Asn Thr Tyr Pro Ala Leu Lys Glu Ala Leu
                165                 170                 175

Leu Gly Val Arg Ala Arg Ala Pro His Ala Arg Val Ala Ala Leu Gly
            180                 185                 190

Tyr Pro Trp Ile Thr Pro Ala Thr Ala Asp Pro Ser Cys Phe Leu Lys
        195                 200                 205

Leu Pro Leu Ala Ala Gly Asp Val Pro Tyr Leu Arg Ala Ile Gln Ala
    210                 215                 220
```

```
His Leu Asn Asp Ala Val Arg Arg Ala Ala Glu Glu Thr Gly Ala Thr
225                 230                 235                 240

Tyr Val Asp Phe Ser Gly Val Ser Asp Gly His Asp Ala Cys Glu Ala
                245                 250                 255

Pro Gly Thr Arg Trp Ile Glu Pro Leu Leu Phe Gly His Ser Leu Val
            260                 265                 270

Pro Val His Pro Asn Ala Leu Gly Glu Arg Arg Met Ala Glu His Thr
        275                 280                 285

Met Asp Val Leu Gly Leu Asp
    290                 295
```

<210> 6
<211> 238
<212> PRT
<213> Saccharomyces cerevisiae

<400> 6

```
Met Asp Tyr Glu Lys Phe Leu Leu Phe Gly Asp Ser Ile Thr Glu Phe
1               5                   10                  15

Ala Phe Asn Thr Arg Pro Ile Glu Asp Gly Lys Asp Gln Tyr Ala Leu
            20                  25                  30

Gly Ala Ala Leu Val Asn Glu Tyr Thr Arg Lys Met Asp Ile Leu Gln
        35                  40                  45

Arg Gly Phe Lys Gly Tyr Thr Ser Arg Trp Ala Leu Lys Ile Leu Pro
    50                  55                  60

Glu Ile Leu Lys His Glu Ser Asn Ile Val Met Ala Thr Ile Phe Leu
65                  70                  75                  80

Gly Ala Asn Asp Ala Cys Ser Ala Gly Pro Gln Ser Val Pro Leu Pro
                85                  90                  95

Glu Phe Ile Asp Asn Ile Arg Gln Met Val Ser Leu Met Lys Ser Tyr
            100                 105                 110

His Ile Arg Pro Ile Ile Ile Gly Pro Gly Leu Val Asp Arg Glu Lys
        115                 120                 125

Trp Glu Lys Glu Lys Ser Glu Glu Ile Ala Leu Gly Tyr Phe Arg Thr
    130                 135                 140
```

```
Asn Glu Asn Phe Ala Ile Tyr Ser Asp Ala Leu Ala Lys Leu Ala Asn
145                 150                 155                 160

Glu Glu Lys Val Pro Phe Val Ala Leu Asn Lys Ala Phe Gln Gln Glu
                165                 170                 175

Gly Gly Asp Ala Trp Gln Gln Leu Leu Thr Asp Gly Leu His Phe Ser
            180                 185                 190

Gly Lys Gly Tyr Lys Ile Phe His Asp Glu Leu Leu Lys Val Ile Glu
        195                 200                 205

Thr Phe Tyr Pro Gln Tyr His Pro Lys Asn Met Gln Tyr Lys Leu Lys
    210                 215                 220

Asp Trp Arg Asp Val Leu Asp Asp Gly Ser Asn Ile Met Ser
225                 230                 235
```

<210> 7
<211> 1005
<212> DNA
<213> Aeromonas hydrophila

<400> 7

```
atgaaaaaat ggtttgtgtg tttattggga ttggtcgcgc tgacagttca ggcagccgac      60
agccgtcccg ccttctcccg gatcgtgatg tttggcgaca gcctctccga taccggcaag     120
atgtacagca agatgcgcgg ttacctcccc tccagccccc cctactatga gggccgcttc     180
tccaacgggc ccgtctggct ggagcagctg accaacgagt tcccgggcct gaccatagcc     240
aacgaggcgg aaggcggacc gaccgccgtg gcttacaaca agatctcctg gaatcccaag     300
tatcaggtca tcaacaacct ggactacgag gtcacccagt tcctgcaaaa agacagcttc     360
aagccggacg atctggtgat cctctgggtc ggcgccaacg actatctggc ctatggctgg     420
aacacagagc aggatgccaa gcgggtgcgc gacgccatca gcgatgcggc caaccgcatg     480
gtgctgaacg gcgccaagga gatactgctg ttcaacctgc cggatctggg ccagaacccc     540
tcggcccgca gccagaaggt ggtcgaggcg gccagccatg tctccgccta ccacaaccag     600
ctgctgctga acctggcacg ccagctggct cccaccggca tggtgaagct gttcgagatc     660
gacaagcagt ttgccgagat gctgcgtgat ccgcagaact tcggcctgag cgaccagagg     720
aacgcctgct acggtggcag ctatgtatgg aagccgtttg cctcccgcag cgccagcacc     780
gacagccagc tctccgcctt caacccgcag gagcgcctcg ccatcgccgg caacccgctg     840
ctggcccagg ccgtcgccag ccccatggct gcccgcagcg ccagcaccct caactgtgag     900
ggcaagatgt tctgggatca ggtccacccc accactgtcg tgcacgccgc cctgagcgag     960
cccgccgcca ccttcatcga gagccagtac gagttcctcg cccac                    1005
```

<210> 8
<211> 1011
<212> DNA
<213> Aeromonas salmonicida

<400> 8

```
atgaaaaaat ggtttgtttg tttattgggg ttgatcgcgc tgacagttca ggcagccgac      60
actcgccccg ccttctcccg gatcgtgatg ttcggcgaca gcctctccga taccggcaaa     120
atgtacagca agatgcgcgg ttacctcccc tccagcccgc cctactatga gggccgtttc     180
tccaacggac ccgtctggct ggagcagctg accaagcagt tcccgggtct gaccatcgcc     240
aacgaagcgg aaggcggtgc cactgccgtg gcttacaaca agatctcctg gaatcccaag     300
tatcaggtct acaacaacct ggactacgag gtcacccagt tcttgcagaa agacagcttc     360
aagccggacg atctggtgat cctctgggtc ggtgccaatg actatctggc atatggctgg     420
aatacggagc aggatgccaa gcgagttcgc gatgccatca gcgatgcggc caaccgcatg     480
gtactgaacg gtgccaagca gatactgctg ttcaacctgc cggatctggg ccagaacccg     540
tcagcccgca gtcagaaggt ggtcgaggcg gtcagccatg tctccgccta tcacaacaag     600
ctgctgctga acctggcacg ccagctggcc cccaccggca tggtaaagct gttcgagatc     660
gacaagcaat ttgccgagat gctgcgtgat ccgcagaact tcggcctgag cgacgtcgag     720
aacccctgct acgacggcgg ctatgtgtgg aagccgtttg ccacccgcag cgtcagcacc     780
gaccgccagc tctccgcctt cagtccgcag gaacgcctcg ccatcgccgg caacccgctg     840
ctggcacagg ccgttgccag tcctatggcc cgccgcagcg ccagccccct caactgtgag     900
ggcaagatgt tctgggatca ggtacacccg accactgtcg tgcacgcagc cctgagcgag     960
cgcgccgcca ccttcatcga gacccagtac gagttcctcg cccacggatg a             1011
```

<210> 9
<211> 888
<212> DNA
<213> Streptomyces coelicolor

<400> 9

```
atgccgaagc ctgcccttcg ccgtgtcatg accgcgacag tcgccgccgt cggcacgctc      60
gccctcggcc tcaccgacgc caccgcccac gccgcgcccg cccaggccac tccgaccctg     120
gactacgtcg ccctcggcga cagctacagc gccggctccg gcgtcctgcc cgtcgacccc     180
gccaacctgc tctgtctgcg ctcgacggcc aactaccccc acgtcatcgc ggacacgacg     240
ggcgcccgcc tcacggacgt cacctgcggc gccgcgcaga ccgccgactt cacgcgggcc     300
cagtacccgg gcgtcgcacc ccagttggac gcgctcggca ccggcacgga cctggtcacg     360
ctcaccatcg gcggcaacga caacagcacc ttcatcaacg ccatcacggc ctgcggcacg     420
gcgggtgtcc tcagcggcgg caagggcagc ccctgcaagg acaggcacgg cacctccttc     480
gacgacgaga tcgaggccaa cacgtacccc gcgctcaagg aggcgctgct cggcgtccgc     540
gccagggctc cccacgccag ggtggcggct ctcggctacc cgtggatcac cccggccacc     600
gccgacccgt cctgcttcct gaagctcccc ctcgccgccg gtgacgtgcc ctacctgcgg     660
gccatccagg cacacctcaa cgacgcggtc cggcgggccg ccgaggagac cggagccacc     720
tacgtggact tctccggggt gtccgacggc cacgacgcct gcgaggcccc cggcacccgc     780
tggatcgaac cgctgctctt cgggcacagc ctcgttcccg tccaccccaa cgccctgggc     840
gagcggcgca tggccgagca cacgatggac gtcctcggcc tggactga                  888
```

<210> 10
<211> 888
<212> DNA
<213> Streptomyces coelicolor

<400> 10

tcagtccagg ccgaggacgt ccatcgtgtg ctcggccatg cgccgctcgc ccagggcgtt 60
ggggtggacg ggaacgaggc tgtgcccgaa gagcagcggt tcgatccagc gggtgccggg 120
ggcctcgcag gcgtcgtggc cgtcggacac cccggagaag tccacgtagg tggctccggt 180
ctcctcggcg gcccgccgga ccgcgtcgtt gaggtgtgcc tggatggccc gcaggtaggg 240
cacgtcaccg gcggcgaggg ggagcttcag gaagcaggac gggtcggcgg tggccggggt 300
gatccacggg tagccgagag ccgccaccct ggcgtgggga gccctggcgc ggacgccgag 360
cagcgcctcc ttgagcgcgg ggtacgtgtt ggcctcgatc tcgtcgtcga aggaggtgcc 420
gtgcctgtcc ttgcaggggc tgcccttgcc gccgctgagg acacccgccg tgccgcaggc 480
cgtgatggcg ttgatgaagg tgctgttgtc gttgccgccg atggtgagcg tgaccaggtc 540
cgtgccggtg ccgagcgcgt ccaactgggg tgcgacgccc gggtactggg cccgcgtgaa 600
gtcggcggtc tgcgcggcgc cgcaggtgac gtccgtgagg cgggcgcccg tcgtgtccgc 660
gatgacgtgg gggtagttgg ccgtcgagcg cagacagagc aggttggcgg ggtcgacggg 720
caggacgccg gagccggcgc tgtagctgtc gccgagggcg acgtagtcca gggtcggagt 780
ggcctgggcg ggcgcggcgt gggcggtggc gtcggtgagg ccgagggcga gcgtgccgac 840
ggcggcgact gtcgcggtca tgacacggcg aagggcaggc ttcggcat 888

<210> 11
<211> 717
<212> DNA
<213> Saccharomyces cerevisiae

<400> 11

atggattacg agaagtttct gttatttggg gattccatta ctgaatttgc ttttaatact 60
aggcccattg aagatggcaa agatcagtat gctcttggag ccgcattagt caacgaatat 120
acgagaaaaa tggatattct tcaaagaggg ttcaaagggt acacttctag atgggcgttg 180
aaaatacttc ctgagatttt aaagcatgaa tccaatattg tcatggccac aatatttttg 240
ggtgccaacg atgcatgctc agcaggtccc caaagtgtcc ccctccccga atttatcgat 300
aatattcgtc aaatggtatc tttgatgaag tcttaccata tccgtcctat tataatagga 360
ccggggctag tagatagaga gaagtgggaa aaagaaaaat ctgaagaaat agctctcgga 420
tacttccgta ccaacgagaa ctttgccatt tattccgatg ccttagcaaa actagccaat 480
gaggaaaaag ttcccttcgt ggctttgaat aaggcgtttc aacaggaagg tggtgatgct 540
tggcaacaac tgctaacaga tggactgcac ttttccggaa aagggtacaa aatttttcat 600
gacgaattat tgaaggtcat tgagacattc taccccccaat atcatcccaa aaacatgcag 660
tacaaactga aagattggag agatgtgcta gatgatggat ctaacataat gtcttga 717

<210> 12
<211> 347
<212> PRT
<213> Ralstonia sp.

<400> 12

```
Met Asn Leu Arg Gln Trp Met Gly Ala Ala Thr Ala Ala Leu Ala Leu
1               5                   10                  15

Gly Leu Ala Ala Cys Gly Gly Gly Gly Thr Asp Gln Ser Gly Asn Pro
            20                  25                  30

Asn Val Ala Lys Val Gln Arg Met Val Val Phe Gly Asp Ser Leu Ser
        35                  40                  45

Asp Ile Gly Thr Tyr Thr Pro Val Ala Gln Ala Val Gly Gly Gly Lys
    50                  55                  60

Phe Thr Thr Asn Pro Gly Pro Ile Trp Ala Glu Thr Val Ala Ala Gln
65                  70                  75                  80

Leu Gly Val Thr Leu Thr Pro Ala Val Met Gly Tyr Ala Thr Ser Val
                85                  90                  95

Gln Asn Cys Pro Lys Ala Gly Cys Phe Asp Tyr Ala Gln Gly Gly Ser
            100                 105                 110

Arg Val Thr Asp Pro Asn Gly Ile Gly His Asn Gly Gly Ala Gly Ala
        115                 120                 125

Leu Thr Tyr Pro Val Gln Gln Gln Leu Ala Asn Phe Tyr Ala Ala Ser
    130                 135                 140

Asn Asn Thr Phe Asn Gly Asn Asn Asp Val Val Phe Val Leu Ala Gly
```

```
145                 150                 155                 160

Ser Asn Asp Ile Phe Phe Trp Thr Thr Ala Ala Ala Thr Ser Gly Ser
                165                 170                 175

Gly Val Thr Pro Ala Ile Ala Thr Ala Gln Val Gln Gln Ala Ala Thr
            180                 185                 190

Asp Leu Val Gly Tyr Val Lys Asp Met Ile Ala Lys Gly Ala Thr Gln
        195                 200                 205

Val Tyr Val Phe Asn Leu Pro Asp Ser Ser Leu Thr Pro Asp Gly Val
    210                 215                 220

Ala Ser Gly Thr Thr Gly Gln Ala Leu Leu His Ala Leu Val Gly Thr
225                 230                 235                 240

Phe Asn Thr Thr Leu Gln Ser Gly Leu Ala Gly Thr Ser Ala Arg Ile
                245                 250                 255

Ile Asp Phe Asn Ala Gln Leu Thr Ala Ala Ile Gln Asn Gly Ala Ser
            260                 265                 270

Phe Gly Phe Ala Asn Thr Ser Ala Arg Ala Cys Asp Ala Thr Lys Ile
        275                 280                 285

Asn Ala Leu Val Pro Ser Ala Gly Gly Ser Ser Leu Phe Cys Ser Ala
    290                 295                 300

Asn Thr Leu Val Ala Ser Gly Ala Asp Gln Ser Tyr Leu Phe Ala Asp
305                 310                 315                 320

Gly Val His Pro Thr Thr Ala Gly His Arg Leu Ile Ala Ser Asn Val
                325                 330                 335

Leu Ala Arg Leu Leu Ala Asp Asn Val Ala His
            340                 345
```

<210> 13
<211> 1044
<212> DNA
<213> Ralstonia sp.

<400> 13

```
atgaacctgc gtcaatggat gggcgccgcc acggctgccc ttgccttggg cttggccgcg      60
tgcgggggcg gtgggaccga ccagagcggc aatcccaatg tcgccaaggt gcagcgcatg     120
gtggtgttcg gcgacagcct gagcgatatc ggcacctaca cccccgtcgc gcaggcggtg     180
ggcggcggca agttcaccac caacccgggc ccgatctggg ccgagaccgt ggccgcgcaa     240
ctgggcgtga cgctcacgcc ggcggtgatg ggctacgcca cctccgtgca gaattgcccc     300
aaggccggct gcttcgacta tgcgcagggc ggctcgcgcg tgaccgatcc gaacggcatc     360
ggccacaacg gcggcgcggg ggcgctgacc tacccggttc agcagcagct cgccaacttc     420
tacgcggcca gcaacaacac attcaacggc aataacgatg tcgtcttcgt gctggccggc     480
agcaacgaca ttttcttctg gaccactgcg gcggccacca gcggctccgg cgtgacgccc     540
gccattgcca cggcccaggt gcagcaggcc gcgacggacc tggtcggcta tgtcaaggac     600
atgatcgcca agggtgcgac gcaggtctac gtgttcaacc tgcccgacag cagcctgacg     660
ccggacggcg tggcaagcgg cacgaccggc caggcgctgc tgcacgcgct ggtgggcacg     720
ttcaacacga cgctgcaaag cgggctggcc ggcacctcgg cgcgcatcat cgacttcaac     780
gcacaactga ccgcggcgat ccagaatggc gcctcgttcg gcttcgccaa caccagcgcc     840
cgggcctgcg acgccaccaa gatcaatgcc ctggtgccga gcgccggcgg cagctcgctg     900
ttctgctcgg ccaacacgct ggtggcttcc ggtgcggacc agagctacct gttcgccgac     960
ggcgtgcacc cgaccacggc cggccatcgc ctgatcgcca gcaacgtgct ggcgcgcctg    1020
ctggcggata acgtcgcgca ctga                                          1044
```

<210> 14
<211> 261
<212> PRT
<213> Streptomyces coelicolor

<400> 14

```
Met Ile Gly Ser Tyr Val Ala Val Gly Asp Ser Phe Thr Glu Gly Val
1               5                   10                  15

Gly Asp Pro Gly Pro Asp Gly Ala Phe Val Gly Trp Ala Asp Arg Leu
            20                  25                  30

Ala Val Leu Leu Ala Asp Arg Arg Pro Glu Gly Asp Phe Thr Tyr Thr
        35                  40                  45

Asn Leu Ala Val Arg Gly Arg Leu Leu Asp Gln Ile Val Ala Glu Gln
    50                  55                  60

Val Pro Arg Val Val Gly Leu Ala Pro Asp Leu Val Ser Phe Ala Ala
65                  70                  75                  80

Gly Gly Asn Asp Ile Ile Arg Pro Gly Thr Asp Pro Asp Glu Val Ala
                85                  90                  95

Glu Arg Phe Glu Leu Ala Val Ala Ala Leu Thr Ala Ala Ala Gly Thr
            100                 105                 110
```

```
Val Leu Val Thr Thr Gly Phe Asp Thr Arg Gly Val Pro Val Leu Lys
        115                 120                 125

His Leu Arg Gly Lys Ile Ala Thr Tyr Asn Gly His Val Arg Ala Ile
    130                 135                 140

Ala Asp Arg Tyr Gly Cys Pro Val Leu Asp Leu Trp Ser Leu Arg Ser
145                 150                 155                 160

Val Gln Asp Arg Arg Ala Trp Asp Ala Asp Arg Leu His Leu Ser Pro
                165                 170                 175

Glu Gly His Thr Arg Val Ala Leu Arg Ala Gly Gln Ala Leu Gly Leu
            180                 185                 190

Arg Val Pro Ala Asp Pro Asp Gln Pro Trp Pro Pro Leu Pro Pro Arg
        195                 200                 205

Gly Thr Leu Asp Val Arg Arg Asp Asp Val His Trp Ala Arg Glu Tyr
    210                 215                 220

Leu Val Pro Trp Ile Gly Arg Arg Leu Arg Gly Glu Ser Ser Gly Asp
225                 230                 235                 240

His Val Thr Ala Lys Gly Thr Leu Ser Pro Asp Ala Ile Lys Thr Arg
                245                 250                 255

Ile Ala Ala Val Ala
            260
```

<210> 15
<211> 786
<212> DNA
<213> Streptomyces coelicolor

<400> 15

```
gtgatcgggt cgtacgtggc ggtgggggac agcttcaccg agggcgtcgg cgaccccggc      60
cccgacgggg cgttcgtcgg ctgggccgac cggctcgccg tactgctcgc ggaccggcgc     120
cccgagggcg acttcacgta cacgaacctc gccgtgcgcg gcaggctcct cgaccagatc     180
gtggcggaac aggtcccgcg ggtcgtcgga ctcgcgcccg acctcgtctc gttcgcggcg     240
ggcggcaacg acatcatccg gcccggcacc gatcccgacg aggtcgccga gcggttcgag     300
ctggcggtgg ccgcgctgac cgccgcggcc ggaaccgtcc tggtgaccac cgggttcgac     360
acccgggggg tgcccgtcct caagcacctg cgcggcaaga tcgccacgta caacgggcac     420
gtccgcgcca tcgccgaccg ctacggctgc ccggtgctcg acctgtggtc gctgcggagc     480
gtccaggacc gcagggcgtg ggacgccgac cggctgcacc tgtcgccgga ggggcacacc     540

cgggtggcgc tgcgcgcggg gcaggccctg ggcctgcgcg tcccggccga ccctgaccag     600
ccctggccgc ccctgccgcc gcgcggcacg ctcgacgtcc ggcgcgacga cgtgcactgg     660
gcgcgcgagt acctggtgcc gtggatcggg cgccggctgc ggggcgagtc gtcgggcgac     720
cacgtgacgg ccaaggggac gctgtcgccg gacgccatca agacgcggat cgccgcggtg     780
gcctga                                                                786
```

<210> 16
<211> 260
<212> PRT
<213> Streptomyces coelicolor

<400> 16

```
Met Gln Thr Asn Pro Ala Tyr Thr Ser Leu Val Ala Val Gly Asp Ser
1               5                   10                  15

Phe Thr Glu Gly Met Ser Asp Leu Leu Pro Asp Gly Ser Tyr Arg Gly
            20                  25                  30

Trp Ala Asp Leu Leu Ala Thr Arg Met Ala Ala Arg Ser Pro Gly Phe
        35                  40                  45

Arg Tyr Ala Asn Leu Ala Val Arg Gly Lys Leu Ile Gly Gln Ile Val
    50                  55                  60

Asp Glu Gln Val Asp Val Ala Ala Ala Met Gly Ala Asp Val Ile Thr
65                  70                  75                  80

Leu Val Gly Gly Leu Asn Asp Thr Leu Arg Pro Lys Cys Asp Met Ala
                85                  90                  95

Arg Val Arg Asp Leu Leu Thr Gln Ala Val Glu Arg Leu Ala Pro His
            100                 105                 110

Cys Glu Gln Leu Val Leu Met Arg Ser Pro Gly Arg Gln Gly Pro Val
        115                 120                 125

Leu Glu Arg Phe Arg Pro Arg Met Glu Ala Leu Phe Ala Val Ile Asp
    130                 135                 140

Asp Leu Ala Gly Arg His Gly Ala Val Val Val Asp Leu Tyr Gly Ala
145                 150                 155                 160

Gln Ser Leu Ala Asp Pro Arg Met Trp Asp Val Asp Arg Leu His Leu
                165                 170                 175

Thr Ala Glu Gly His Arg Arg Val Ala Glu Ala Val Trp Gln Ser Leu
```

180 185 190

Gly His Glu Pro Glu Asp Pro Glu Trp His Ala Pro Ile Pro Ala Thr
195 200 205

Pro Pro Pro Gly Trp Val Thr Arg Arg Thr Ala Asp Val Arg Phe Ala
210 215 220

Arg Gln His Leu Leu Pro Trp Ile Gly Arg Arg Leu Thr Gly Arg Ser
225 230 235 240

Ser Gly Asp Gly Leu Pro Ala Lys Arg Pro Asp Leu Leu Pro Tyr Glu
245 250 255

Asp Pro Ala Arg
260

<210> 17
<211> 783
<212> DNA
<213> Streptomyces coelicolor

<400> 17

```
atgcagacga accccgcgta caccagtctc gtcgccgtcg gcgactcctt caccgagggc      60
atgtcggacc tgctgcccga cggctcctac cgtggctggg ccgacctcct cgccacccgg     120
atggcggccc gctcccccgg cttccggtac gccaacctgg cggtgcgcgg gaagctgatc     180
ggacagatcg tcgacgagca ggtggacgtg gccgccgcca tgggagccga cgtgatcacg     240
ctggtcggcg ggctcaacga cacgctgcgg cccaagtgcg acatggcccg ggtgcgggac     300
ctgctgaccc aggccgtgga acggctcgcc ccgcactgcg agcagctggt gctgatgcgc     360
agtcccggtc gccagggtcc ggtgctggag cgcttccggc cccgcatgga ggccctgttc     420
gccgtgatcg acgacctggc cgggcggcac ggcgccgtgg tcgtcgacct gtacggggcc     480
cagtcgctgg ccgaccctcg gatgtgggac gtggaccggc tgcacctgac cgccgagggc     540
caccgccggg tcgcggaggc ggtgtggcag tcgctcggcc acgagcccga ggaccccgag     600
tggcacgcgc cgatcccggc gacgccgccg ccggggtggg tgacgcgcag gaccgcggac     660
gtccggttcg cccggcagca cctgctgccc tggataggcc gcaggctgac cgggcgctcg     720
tccggggacg gcctgccggc caagcgcccg gacctgctgc cctacgagga ccccgcacgg     780
tga                                                                   783
```

<210> 18
<211> 454
<212> PRT
<213> Streptomyces coelicolor

<400> 18

```
Met Thr Arg Gly Arg Asp Gly Gly Ala Gly Ala Pro Pro Thr Lys His
1               5                   10                  15

Arg Ala Leu Leu Ala Ala Ile Val Thr Leu Ile Val Ala Ile Ser Ala
            20                  25                  30

Ala Ile Tyr Ala Gly Ala Ser Ala Asp Asp Gly Ser Arg Asp His Ala
        35                  40                  45

Leu Gln Ala Gly Gly Arg Leu Pro Arg Gly Asp Ala Ala Pro Ala Ser
    50                  55                  60

Thr Gly Ala Trp Val Gly Ala Trp Ala Thr Ala Pro Ala Ala Ala Glu
65                  70                  75                  80

Pro Gly Thr Glu Thr Thr Gly Leu Ala Gly Arg Ser Val Arg Asn Val
                85                  90                  95

Val His Thr Ser Val Gly Gly Thr Gly Ala Arg Ile Thr Leu Ser Asn
            100                 105                 110

Leu Tyr Gly Gln Ser Pro Leu Thr Val Thr His Ala Ser Ile Ala Leu
        115                 120                 125

Ala Ala Gly Pro Asp Thr Ala Ala Ala Ile Ala Asp Thr Met Arg Arg
    130                 135                 140

Leu Thr Phe Gly Gly Ser Ala Arg Val Ile Ile Pro Ala Gly Gly Gln
145                 150                 155                 160

Val Met Ser Asp Thr Ala Arg Leu Ala Ile Pro Tyr Gly Ala Asn Val
                165                 170                 175

Leu Val Thr Thr Tyr Ser Pro Ile Pro Ser Gly Pro Val Thr Tyr His
            180                 185                 190

Pro Gln Ala Arg Gln Thr Ser Tyr Leu Ala Asp Gly Asp Arg Thr Ala
        195                 200                 205

Asp Val Thr Ala Val Ala Tyr Thr Thr Pro Thr Pro Tyr Trp Arg Tyr
    210                 215                 220

Leu Thr Ala Leu Asp Val Leu Ser His Glu Ala Asp Gly Thr Val Val
225                 230                 235                 240

Ala Phe Gly Asp Ser Ile Thr Asp Gly Ala Arg Ser Gln Ser Asp Ala
                245                 250                 255
```

```
Asn His Arg Trp Thr Asp Val Leu Ala Ala Arg Leu His Glu Ala Ala
            260                 265                 270

Gly Asp Gly Arg Asp Thr Pro Arg Tyr Ser Val Val Asn Glu Gly Ile
        275                 280                 285

Ser Gly Asn Arg Leu Leu Thr Ser Arg Pro Gly Arg Pro Ala Asp Asn
    290                 295                 300

Pro Ser Gly Leu Ser Arg Phe Gln Arg Asp Val Leu Glu Arg Thr Asn
305                 310                 315                 320

Val Lys Ala Val Val Val Val Leu Gly Val Asn Asp Val Leu Asn Ser
                325                 330                 335

Pro Glu Leu Ala Asp Arg Asp Ala Ile Leu Thr Gly Leu Arg Thr Leu
            340                 345                 350

Val Asp Arg Ala His Ala Arg Gly Leu Arg Val Val Gly Ala Thr Ile
        355                 360                 365

Thr Pro Phe Gly Gly Tyr Gly Gly Tyr Thr Glu Ala Arg Glu Thr Met
    370                 375                 380

Arg Gln Glu Val Asn Glu Glu Ile Arg Ser Gly Arg Val Phe Asp Thr
385                 390                 395                 400

Val Val Asp Phe Asp Lys Ala Leu Arg Asp Pro Tyr Asp Pro Arg Arg
                405                 410                 415

Met Arg Ser Asp Tyr Asp Ser Gly Asp His Leu His Pro Gly Asp Lys
            420                 425                 430

Gly Tyr Ala Arg Met Gly Ala Val Ile Asp Leu Ala Ala Leu Lys Gly
        435                 440                 445

Ala Ala Pro Val Lys Ala
    450
```

<210> 19
<211> 1365
<212> DNA
<213> Streptomyces coelicolor

<400> 19

```
atgacccggg gtcgtgacgg gggtgcgggg gcgcccccca ccaagcaccg tgccctgctc      60
gcggcgatcg tcaccctgat agtggcgatc tccgcggcca tatacgccgg agcgtccgcg     120
gacgacggca gcagggacca cgcgctgcag gccggaggcc gtctcccacg aggagacgcc     180
```

gcccccgcgt ccaccggtgc ctgggtgggc gcctgggcca ccgcaccggc cgcggccgag 240

ccgggcaccg agacgaccgg cctggcgggc cgctccgtgc gcaacgtcgt gcacacctcg 300

gtcggcggca ccggcgcgcg gatcaccctc tcgaacctgt acgggcagtc gccgctgacc 360

gtcacacacg cctcgatcgc cctggccgcc gggcccgaca ccgccgccgc gatcgccgac 420

accatgcgcc ggctcacctt cggcggcagc gcccgggtga tcatcccggc gggcggccag 480

gtgatgagcg acaccgcccg cctcgccatc ccctacgggg cgaacgtcct ggtcaccacg 540

tactccccca tcccgtccgg gccggtgacc taccatccgc aggcccggca gaccagctac 600

ctggccgacg gcgaccgcac ggcggacgtc accgccgtcg cgtacaccac ccccacgccc 660

tactggcgct acctgaccgc cctcgacgtg ctgagccacg aggccgacgg cacggtcgtg 720

gcgttcggcg actccatcac cgacggcgcc cgctcgcaga gcgacgccaa ccaccgctgg 780

accgacgtcc tcgccgcacg cctgcacgag gcggcgggcg acggccggga cacgccccgc 840

tacagcgtcg tcaacgaggg catcagcggc aaccggctcc tgaccagcag gccggggcgg 900

ccggccgaca acccgagcgg actgagccgg ttccagcggg acgtgctgga acgcaccaac 960

gtcaaggccg tcgtcgtcgt cctcggcgtc aacgacgtcc tgaacagccc ggaactcgcc 1020

gaccgcgacg ccatcctgac cggcctgcgc accctcgtcg accgggcgca cgcccgggga 1080

ctgcgggtcg tcggcgccac gatcacgccg ttcggcggct acggcggcta caccgaggcc 1140

cgcgagacga tgcggcagga ggtcaacgag gagatccgct ccggccgggt cttcgacacg 1200

gtcgtcgact tcgacaaggc cctgcgcgac ccgtacgacc cgcgccggat gcgctccgac 1260

tacgacagcg gcgaccacct gcaccccggc gacaaggggt acgcgcgcat gggcgcggtc 1320

atcgacctgg ccgcgctgaa gggcgcggcg ccggtcaagg cgtag 1365

<210> 20
<211> 340
<212> PRT
<213> Streptomyces coelicolor

<400> 20

Met Thr Ser Met Ser Arg Ala Arg Val Ala Arg Arg Ile Ala Ala Gly
1               5                   10                  15

Ala Ala Tyr Gly Gly Gly Gly Ile Gly Leu Ala Gly Ala Ala Ala Val
            20                  25                  30

Gly Leu Val Val Ala Glu Val Gln Leu Ala Arg Arg Arg Val Gly Val
        35                  40                  45

Gly Thr Pro Thr Arg Val Pro Asn Ala Gln Gly Leu Tyr Gly Gly Thr
    50                  55                  60

```
Leu Pro Thr Ala Gly Asp Pro Pro Leu Arg Leu Met Met Leu Gly Asp
65                  70                  75                  80

Ser Thr Ala Ala Gly Gln Gly Val His Arg Ala Gly Gln Thr Pro Gly
                85                  90                  95

Ala Leu Leu Ala Ser Gly Leu Ala Ala Val Ala Glu Arg Pro Val Arg
            100                 105                 110

Leu Gly Ser Val Ala Gln Pro Gly Ala Cys Ser Asp Asp Leu Asp Arg
        115                 120                 125

Gln Val Ala Leu Val Leu Ala Glu Pro Asp Arg Val Pro Asp Ile Cys
    130                 135                 140

Val Ile Met Val Gly Ala Asn Asp Val Thr His Arg Met Pro Ala Thr
145                 150                 155                 160

Arg Ser Val Arg His Leu Ser Ser Ala Val Arg Arg Leu Arg Thr Ala
                165                 170                 175

Gly Ala Glu Val Val Val Gly Thr Cys Pro Asp Leu Gly Thr Ile Glu
            180                 185                 190

Arg Val Arg Gln Pro Leu Arg Trp Leu Ala Arg Arg Ala Ser Arg Gln
        195                 200                 205

Leu Ala Ala Ala Gln Thr Ile Gly Ala Val Glu Gln Gly Gly Arg Thr
    210                 215                 220

Val Ser Leu Gly Asp Leu Leu Gly Pro Glu Phe Ala Gln Asn Pro Arg
225                 230                 235                 240

Glu Leu Phe Gly Pro Asp Asn Tyr His Pro Ser Ala Glu Gly Tyr Ala
                245                 250                 255

Thr Ala Ala Met Ala Val Leu Pro Ser Val Cys Ala Ala Leu Gly Leu
            260                 265                 270

Trp Pro Ala Asp Glu Glu His Pro Asp Ala Leu Arg Arg Glu Gly Phe
        275                 280                 285

Leu Pro Val Ala Arg Ala Ala Ala Glu Ala Ala Ser Glu Ala Gly Thr
    290                 295                 300

Glu Val Ala Ala Ala Met Pro Thr Gly Pro Arg Gly Pro Trp Ala Leu
305                 310                 315                 320
```

```
Leu Lys Arg Arg Arg Arg Arg Arg Val Ser Glu Ala Glu Pro Ser Ser
                325                     330                 335

Pro Ser Gly Val
            340
```

<210> 21
<211> 1023
<212> DNA
<213> Streptomyces coelicolor

<400> 21

```
atgacgagca tgtcgagggc gagggtggcg cggcggatcg cggccggcgc ggcgtacggc      60
ggcggcggca tcggcctggc gggagcggcg gcggtcggtc tggtggtggc cgaggtgcag     120
ctggccagac gcagggtggg ggtgggcacg ccgacccggg tgccgaacgc gcagggactg     180
tacggcggca ccctgcccac ggccggcgac ccgccgctgc ggctgatgat gctgggcgac     240
tccacggccg ccgggcaggg cgtgcaccgg gccgggcaga cgccgggcgc gctgctggcg     300
tccgggctcg cggcggtggc ggagcggccg gtgcggctgg ggtcggtcgc ccagccgggg     360
gcgtgctcgg acgacctgga ccggcaggtg gcgctggtgc tcgccgagcc ggaccgggtg     420
cccgacatct gcgtgatcat ggtcggcgcc aacgacgtca cccaccggat gccggcgacc     480
cgctcggtgc ggcacctgtc ctcggcggta cggcggctgc gcacggccgg tgcggaggtg     540
gtggtcggca cctgtccgga cctgggcacg atcgagcggg tgcggcagcc gctgcgctgg     600
ctggcccggc gggcctcacg gcagctcgcg gcggcacaga ccatcggcgc cgtcgagcag     660
ggcgggcgca cggtgtcgct gggcgacctg ctgggtccgg agttcgcgca gaacccgcgg     720
gagctcttcg gccccgacaa ctaccacccc tccgccgagg ggtacgccac ggccgcgatg     780
gcggtactgc cctcggtgtg cgccgcgctc ggcctgtggc cggccgacga ggagcacccg     840
gacgcgctgc gccgcgaggg cttcctgccg gtggcgcgcg cggcggcgga ggcggcgtcc     900
gaggcgggta cggaggtcgc cgccgccatg cctacggggc ctcgggggcc ctgggcgctg     960
ctgaagcgcc ggagacggcg tcgggtgtcg gaggcggaac cgtccagccc gtccggcgtt    1020
tga                                                                  1023
```

<210> 22
<211> 305
<212> PRT
<213> Streptomyces coelicolor

<400> 22

```
Met Gly Arg Gly Thr Asp Gln Arg Thr Arg Tyr Gly Arg Arg Arg Ala
1               5                   10                  15

Arg Val Ala Leu Ala Ala Leu Thr Ala Ala Val Leu Gly Val Gly Val
```

```
            20                  25                  30

Ala Gly Cys Asp Ser Val Gly Gly Asp Ser Pro Ala Pro Ser Gly Ser
        35                  40                  45

Pro Ser Lys Arg Thr Arg Thr Ala Pro Ala Trp Asp Thr Ser Pro Ala
    50                  55                  60

Ser Val Ala Ala Val Gly Asp Ser Ile Thr Arg Gly Phe Asp Ala Cys
65                  70                  75                  80

Ala Val Leu Ser Asp Cys Pro Glu Val Ser Trp Ala Thr Gly Ser Ser
                85                  90                  95

Ala Lys Val Asp Ser Leu Ala Val Arg Leu Leu Gly Lys Ala Asp Ala
            100                 105                 110

Ala Glu His Ser Trp Asn Tyr Ala Val Thr Gly Ala Arg Met Ala Asp
        115                 120                 125

Leu Thr Ala Gln Val Thr Arg Ala Ala Gln Arg Glu Pro Glu Leu Val
    130                 135                 140

Ala Val Met Ala Gly Ala Asn Asp Ala Cys Arg Ser Thr Thr Ser Ala
145                 150                 155                 160

Met Thr Pro Val Ala Asp Phe Arg Ala Gln Phe Glu Glu Ala Met Ala
                165                 170                 175

Thr Leu Arg Lys Lys Leu Pro Lys Ala Gln Val Tyr Val Ser Ser Ile
            180                 185                 190

Pro Asp Leu Lys Arg Leu Trp Ser Gln Gly Arg Thr Asn Pro Leu Gly
        195                 200                 205

Lys Gln Val Trp Lys Leu Gly Leu Cys Pro Ser Met Leu Gly Asp Ala
    210                 215                 220

Asp Ser Leu Asp Ser Ala Ala Thr Leu Arg Arg Asn Thr Val Arg Asp
225                 230                 235                 240

Arg Val Ala Asp Tyr Asn Glu Val Leu Arg Glu Val Cys Ala Lys Asp
                245                 250                 255

Arg Arg Cys Arg Ser Asp Asp Gly Ala Val His Glu Phe Arg Phe Gly
            260                 265                 270

Thr Asp Gln Leu Ser His Trp Asp Trp Phe His Pro Ser Val Asp Gly
```

```
            275                 280                 285

Gln Ala Arg Leu Ala Glu Ile Ala Tyr Arg Ala Val Thr Ala Lys Asn
    290                 295                 300

Pro
305
```

<210> 23
<211> 918
<212> DNA
<213> Streptomyces coelicolor

<400> 23

```
atgggtcgag ggacggacca gcggacgcgg tacggccgtc gccgggcgcg tgtcgcgctc      60
gccgccctga ccgccgccgt cctgggcgtg ggcgtggcgg gctgcgactc cgtgggcggc     120
gactcacccg ctccttccgg cagcccgtcg aagcggacga ggacggcgcc cgcctgggac     180
accagcccgg cgtccgtcgc cgccgtgggc gactccatca cgcgcggctt cgacgcctgt     240
gcggtgctgt cggactgccc ggaggtgtcg tgggcgaccg gcagcagcgc gaaggtcgac     300
tcgctggccg tacggctgct ggggaaggcg gacgcggccg agcacagctg gaactacgcg     360
gtcaccgggg cccggatggc ggacctgacc gctcaggtga cgcgggcggc gcagcgcgag     420
ccggagctgg tggcggtgat ggccggggcg aacgacgcgt gccggtccac gacctcggcg     480
atgacgccgg tggcggactt ccgggcgcag ttcgaggagg cgatggccac cctgcgcaag     540
aagctcccca aggcgcaggt gtacgtgtcg agcatcccgg acctcaagcg gctctggtcc     600
cagggccgca ccaacccgct gggcaagcag gtgtggaagc tcggcctgtg cccgtcgatg     660
ctgggcgacg cggactccct ggactcggcg gcgaccctgc ggcgcaacac ggtgcgcgac     720
cgggtggcgg actacaacga ggtgctgcgg gaggtctgcg cgaaggaccg gcggtgccgc     780
agcgacgacg gcgcggtgca cgagttccgg ttcggcacgg accagttgag ccactgggac     840
tggttccacc cgagtgtgga cggccaggcc cggctggcgg agatcgccta ccgcgcggtc     900
accgcgaaga atccctga                                                   918
```

<210> 24
<211> 268
<212> PRT
<213> Streptomyces rimosus

<400> 24

```
Met Arg Leu Ser Arg Arg Ala Ala Thr Ala Ser Ala Leu Leu Leu Thr
1               5                   10                  15

Pro Ala Leu Ala Leu Phe Gly Ala Ser Ala Ala Val Ser Ala Pro Arg
            20                  25                  30
```

```
Ile Gln Ala Thr Asp Tyr Val Ala Leu Gly Asp Ser Tyr Ser Ser Gly
        35                  40                  45

Val Gly Ala Gly Ser Tyr Asp Ser Ser Ser Gly Ser Cys Lys Arg Ser
    50                  55                  60

Thr Lys Ser Tyr Pro Ala Leu Trp Ala Ala Ser His Thr Gly Thr Arg
65                  70                  75                  80

Phe Asn Phe Thr Ala Cys Ser Gly Ala Arg Thr Gly Asp Val Leu Ala
                85                  90                  95

Lys Gln Leu Thr Pro Val Asn Ser Gly Thr Asp Leu Val Ser Ile Thr
            100                 105                 110

Ile Gly Gly Asn Asp Ala Gly Phe Ala Asp Thr Met Thr Thr Cys Asn
        115                 120                 125

Leu Gln Gly Glu Ser Ala Cys Leu Ala Arg Ile Ala Lys Ala Arg Ala
    130                 135                 140

Tyr Ile Gln Gln Thr Leu Pro Ala Gln Leu Asp Gln Val Tyr Asp Ala
145                 150                 155                 160

Ile Asp Ser Arg Ala Pro Ala Ala Gln Val Val Val Leu Gly Tyr Pro
                165                 170                 175

Arg Phe Tyr Lys Leu Gly Gly Ser Cys Ala Val Gly Leu Ser Glu Lys
            180                 185                 190

Ser Arg Ala Ala Ile Asn Ala Ala Ala Asp Asp Ile Asn Ala Val Thr
        195                 200                 205

Ala Lys Arg Ala Ala Asp His Gly Phe Ala Phe Gly Asp Val Asn Thr
    210                 215                 220

Thr Phe Ala Gly His Glu Leu Cys Ser Gly Ala Pro Trp Leu His Ser
225                 230                 235                 240

Val Thr Leu Pro Val Glu Asn Ser Tyr His Pro Thr Ala Asn Gly Gln
                245                 250                 255

Ser Lys Gly Tyr Leu Pro Val Leu Asn Ser Ala Thr
            260                 265
```

<210> 25
<211> 1068
<212> DNA
<213> Streptomyces rimosus

<400> 25

```
ttcatcacaa cgatgtcaca acaccggcca tccgggtcat ccctgatcgt gggaatgggt      60
gacaagcctt cccgtgacga aagggtcctg ctacatcaga aatgacagaa atcctgctca     120
gggaggttcc atgagactgt cccgacgcgc ggccacggcg tccgcgctcc tcctcacccc     180
ggcgctcgcg ctcttcggcg cgagcgccgc cgtgtccgcg ccgcgaatcc aggccaccga     240
ctacgtggcc ctcggcgact cctactcctc gggggtcggc gcgggcagct acgacagcag     300
cagtggctcc tgtaagcgca gcaccaagtc ctacccggcc ctgtgggccg cctcgcacac     360
cggtacgcgg ttcaacttca ccgcctgttc gggcgcccgc acaggagacg tgctggccaa     420
gcagctgacc ccggtcaact ccggcaccga cctggtcagc attaccatcg gcggcaacga     480
cgcgggcttc gccgacacca tgaccacctg caacctccag ggcgagagcg cgtgcctggc     540
gcggatcgcc aaggcgcgcg cctacatcca gcagacgctg cccgcccagc tggaccaggt     600
ctacgacgcc atcgacagcc gggcccccgc agcccaggtc gtcgtcctgg gctacccgcg     660
cttctacaag ctgggcggca gctgcgccgt cggtctctcg gagaagtccc gcgcggccat     720
caacgccgcc gccgacgaca tcaacgccgt caccgccaag cgcgccgccg accacggctt     780
cgccttcggg gacgtcaaca cgaccttcgc cgggcacgag ctgtgctccg gcgccccctg     840
gctgcacagc gtcacccttc ccgtggagaa ctcctaccac cccacggcca acggacagtc     900
caagggctac ctgcccgtcc tgaactccgc cacctgatct cgcggctact ccgcccctga     960
cgaagtcccg cccccgggcg gggcttcgcc gtaggtgcgc gtaccgccgt cgcccgtcgc    1020
gccggtggcc ccgccgtacg tgccgccgcc cccggacgcg gtcggttc                 1068
```

<210> 26
<211> 335
<212> PRT
<213> Aeromonas hydrophila

<400> 26

```
Met Lys Lys Trp Phe Val Cys Leu Leu Gly Leu Val Ala Leu Thr Val
1               5                   10                  15

Gln Ala Ala Asp Ser Arg Pro Ala Phe Ser Arg Ile Val Met Phe Gly
            20                  25                  30

Asp Ser Leu Ser Asp Thr Gly Lys Met Tyr Ser Lys Met Arg Gly Tyr
        35                  40                  45

Leu Pro Ser Ser Pro Pro Tyr Tyr Glu Gly Arg Phe Ser Asn Gly Pro
    50                  55                  60

Val Trp Leu Glu Gln Leu Thr Lys Gln Phe Pro Gly Leu Thr Ile Ala
```

65 70 75 80

Asn Glu Ala Glu Gly Gly Ala Thr Ala Val Ala Tyr Asn Lys Ile Ser
85 90 95

Trp Asn Pro Lys Tyr Gln Val Ile Asn Asn Leu Asp Tyr Glu Val Thr
100 105 110

Gln Phe Leu Gln Lys Asp Ser Phe Lys Pro Asp Asp Leu Val Ile Leu
115 120 125

Trp Val Gly Ala Asn Asp Tyr Leu Ala Tyr Gly Trp Asn Thr Glu Gln
130 135 140

Asp Ala Lys Arg Val Arg Asp Ala Ile Ser Asp Ala Ala Asn Arg Met
145 150 155 160

Val Leu Asn Gly Ala Lys Gln Ile Leu Leu Phe Asn Leu Pro Asp Leu
165 170 175

Gly Gln Asn Pro Ser Ala Arg Ser Gln Lys Val Val Glu Ala Val Ser
180 185 190

His Val Ser Ala Tyr His Asn Gln Leu Leu Leu Asn Leu Ala Arg Gln
195 200 205

Leu Ala Pro Thr Gly Met Val Lys Leu Phe Glu Ile Asp Lys Gln Phe
210 215 220

Ala Glu Met Leu Arg Asp Pro Gln Asn Phe Gly Leu Ser Asp Val Glu
225 230 235 240

Asn Pro Cys Tyr Asp Gly Gly Tyr Val Trp Lys Pro Phe Ala Thr Arg
245 250 255

Ser Val Ser Thr Asp Arg Gln Leu Ser Ala Phe Ser Pro Gln Glu Arg
260 265 270

Leu Ala Ile Ala Gly Asn Pro Leu Leu Ala Gln Ala Val Ala Ser Pro
275 280 285

Met Ala Arg Arg Ser Ala Ser Pro Leu Asn Cys Glu Gly Lys Met Phe
290 295 300

Trp Asp Gln Val His Pro Thr Thr Val Val His Ala Ala Leu Ser Glu
305 310 315 320

Arg Ala Ala Thr Phe Ile Ala Asn Gln Tyr Glu Phe Leu Ala His

325 330 335

<210> 27
<211> 1008
<212> DNA
<213> Aeromonas hydrophila

<400> 27

```
atgaaaaaat ggtttgtgtg tttattggga ttggtcgcgc tgacagttca ggcagccgac      60
agtcgccccg ccttttcccg gatcgtgatg ttcggcgaca gcctctccga taccggcaaa     120
atgtacagca agatgcgcgg ttacctcccc tccagcccgc cctactatga gggccgtttc     180
tccaacggac ccgtctggct ggagcagctg accaaacagt tcccgggtct gaccatcgcc     240
aacgaagcgg aaggcggtgc cactgccgtg gcttacaaca agatctcctg gaatcccaag     300
tatcaggtca tcaacaacct ggactacgag gtcacccagt tcttgcagaa agacagcttc     360
aagccggacg atctggtgat cctctgggtc ggtgccaatg actatctggc ctatggctgg     420
aacacggagc aggatgccaa gcgggttcgc gatgccatca gcgatgcggc caaccgcatg     480
gtactgaacg gtgccaagca gatactgctg ttcaacctgc cggatctggg ccagaacccg     540
tcagctcgca gtcagaaggt ggtcgaggcg gtcagccatg tctccgccta tcacaaccag     600
ctgctgctga acctggcacg ccagctggcc cccaccggca tggtaaagct gttcgagatc     660
gacaagcaat ttgccgagat gctgcgtgat ccgcagaact tcggcctgag cgacgtcgag     720
aacccctgct acgacggcgg ctatgtgtgg aagccgtttg ccacccgcag cgtcagcacc     780
gaccgccagc tctccgcctt cagtccgcag gaacgcctcg ccatcgccgg caacccgctg     840
ctggcacagg ccgttgccag tcctatggcc cgccgcagcg ccagccccct caactgtgag     900
ggcaagatgt tctgggatca ggtacacccg accactgtcg tgcacgcagc cctgagcgag     960
cgcgccgcca ccttcatcgc gaaccagtac gagttcctcg cccactga                 1008
```

<210> 28
<211> 336
<212> PRT
<213> Aeromonas salmonicida subsp. Salmonicida

<400> 28

```
Met Lys Lys Trp Phe Val Cys Leu Leu Gly Leu Ile Ala Leu Thr Val
1               5                   10                  15

Gln Ala Ala Asp Thr Arg Pro Ala Phe Ser Arg Ile Val Met Phe Gly
            20                  25                  30

Asp Ser Leu Ser Asp Thr Gly Lys Met Tyr Ser Lys Met Arg Gly Tyr
        35                  40                  45

Leu Pro Ser Ser Pro Pro Tyr Tyr Glu Gly Arg Phe Ser Asn Gly Pro
```

```
    50                  55                  60

Val Trp Leu Glu Gln Leu Thr Lys Gln Phe Pro Gly Leu Thr Ile Ala
65                  70                  75                  80

Asn Glu Ala Glu Gly Gly Ala Thr Ala Val Ala Tyr Asn Lys Ile Ser
                85                  90                  95

Trp Asn Pro Lys Tyr Gln Val Ile Asn Asn Leu Asp Tyr Glu Val Thr
            100                 105                 110

Gln Phe Leu Gln Lys Asp Ser Phe Lys Pro Asp Asp Leu Val Ile Leu
        115                 120                 125

Trp Val Gly Ala Asn Asp Tyr Leu Ala Tyr Gly Trp Asn Thr Glu Gln
    130                 135                 140

Asp Ala Lys Arg Val Arg Asp Ala Ile Ser Asp Ala Ala Asn Arg Met
145                 150                 155                 160

Val Leu Asn Gly Ala Lys Gln Ile Leu Leu Phe Asn Leu Pro Asp Leu
                165                 170                 175

Gly Gln Asn Pro Ser Ala Arg Ser Gln Lys Val Val Glu Ala Val Ser
            180                 185                 190

His Val Ser Ala Tyr His Asn Lys Leu Leu Leu Asn Leu Ala Arg Gln
        195                 200                 205

Leu Ala Pro Thr Gly Met Val Lys Leu Phe Glu Ile Asp Lys Gln Phe
    210                 215                 220

Ala Glu Met Leu Arg Asp Pro Gln Asn Phe Gly Leu Ser Asp Val Glu
225                 230                 235                 240

Asn Pro Cys Tyr Asp Gly Gly Tyr Val Trp Lys Pro Phe Ala Thr Arg
                245                 250                 255

Ser Val Ser Thr Asp Arg Gln Leu Ser Ala Phe Ser Pro Gln Glu Arg
            260                 265                 270

Leu Ala Ile Ala Gly Asn Pro Leu Leu Ala Gln Ala Val Ala Ser Pro
        275                 280                 285

Met Ala Arg Arg Ser Ala Ser Pro Leu Asn Cys Glu Gly Lys Met Phe
    290                 295                 300

Trp Asp Gln Val His Pro Thr Thr Val Val His Ala Ala Leu Ser Glu
```

305 310 315 320

Arg Ala Ala Thr Phe Ile Glu Thr Gln Tyr Glu Phe Leu Ala His Gly
325 330 335

<210> 29
<211> 1011
<212> DNA
<213> Aeromonas salmonicida subsp. Salmonicida

<400> 29

```
atgaaaaaat ggtttgtttg tttattgggg ttgatcgcgc tgacagttca ggcagccgac      60
actcgccccg ccttctcccg gatcgtgatg ttcggcgaca gcctctccga taccggcaaa     120
atgtacagca agatgcgcgg ttacctcccc tccagcccgc cctactatga gggccgtttc     180
tccaacggac ccgtctggct ggagcagctg accaagcagt tcccgggtct gaccatcgcc     240
aacgaagcgg aaggcggtgc cactgccgtg gcttacaaca agatctcctg gaatcccaag     300
tatcaggtca tcaacaacct ggactacgag gtcacccagt tcttgcagaa agacagcttc     360
aagccggacg atctggtgat cctctgggtc ggtgccaatg actatctggc atatggctgg     420
aatacggagc aggatgccaa gcgagttcgc gatgccatca gcgatgcggc caaccgcatg     480
gtactgaacg gtgccaagca gatactgctg ttcaacctgc cggatctggg ccagaacccg     540
tcagcccgca gtcagaaggt ggtcgaggcg gtcagccatg tctccgccta tcacaacaag     600
ctgctgctga acctggcacg ccagctggcc cccaccggca tggtaaagct gttcgagatc     660
gacaagcaat ttgccgagat gctgcgtgat ccgcagaact tcggcctgag cgacgtcgag     720
aacccctgct acgacggcgg ctatgtgtgg aagccgtttg ccacccgcag cgtcagcacc     780
gaccgccagc tctccgcctt cagtccgcag gaacgcctcg ccatcgccgg caacccgctg     840
ctggcacagg ccgttgccag tcctatggcc cgccgcagcg ccagccccct caactgtgag     900
ggcaagatgt tctgggatca ggtacacccg accactgtcg tgcacgcagc cctgagcgag     960
cgcgccgcca ccttcatcga gacccagtac gagttcctcg cccacggatg a             1011
```

<210> 30
<211> 347
<212> PRT
<213> Artificial Sequence

<220>
<223> Fusion construct

<400> 30

```
Met Phe Lys Phe Lys Lys Asn Phe Leu Val Gly Leu Ser Ala Ala Leu
1               5                   10                  15

Met Ser Ile Ser Leu Phe Ser Ala Thr Ala Ser Ala Ala Ser Ala Asp
            20                  25                  30
```

```
Ser Arg Pro Ala Phe Ser Arg Ile Val Met Phe Gly Asp Ser Leu Ser
        35                  40                  45

Asp Thr Gly Lys Met Tyr Ser Lys Met Arg Gly Tyr Leu Pro Ser Ser
    50                  55                  60

Pro Pro Tyr Tyr Glu Gly Arg Phe Ser Asn Gly Pro Val Trp Leu Glu
65                  70                  75                  80

Gln Leu Thr Lys Gln Phe Pro Gly Leu Thr Ile Ala Asn Glu Ala Glu
                85                  90                  95

Gly Gly Ala Thr Ala Val Ala Tyr Asn Lys Ile Ser Trp Asn Pro Lys
            100                 105                 110

Tyr Gln Val Ile Asn Asn Leu Asp Tyr Glu Val Thr Gln Phe Leu Gln
        115                 120                 125

Lys Asp Ser Phe Lys Pro Asp Asp Leu Val Ile Leu Trp Val Gly Ala
    130                 135                 140

Asn Asp Tyr Leu Ala Tyr Gly Trp Asn Thr Glu Gln Asp Ala Lys Arg
145                 150                 155                 160

Val Arg Asp Ala Ile Ser Asp Ala Ala Asn Arg Met Val Leu Asn Gly
                165                 170                 175

Ala Lys Gln Ile Leu Leu Phe Asn Leu Pro Asp Leu Gly Gln Asn Pro
            180                 185                 190

Ser Ala Arg Ser Gln Lys Val Val Glu Ala Val Ser His Val Ser Ala
        195                 200                 205

Tyr His Asn Gln Leu Leu Leu Asn Leu Ala Arg Gln Leu Ala Pro Thr
    210                 215                 220

Gly Met Val Lys Leu Phe Glu Ile Asp Lys Gln Phe Ala Glu Met Leu
225                 230                 235                 240

Arg Asp Pro Gln Asn Phe Gly Leu Ser Asp Val Glu Asn Pro Cys Tyr
                245                 250                 255

Asp Gly Gly Tyr Val Trp Lys Pro Phe Ala Thr Arg Ser Val Ser Thr
            260                 265                 270

Asp Arg Gln Leu Ser Ala Phe Ser Pro Gln Glu Arg Leu Ala Ile Ala
        275                 280                 285
```

```
Gly Asn Pro Leu Leu Ala Gln Ala Val Ala Ser Pro Met Ala Arg Arg
    290                 295                 300

Ser Ala Ser Pro Leu Asn Cys Glu Gly Lys Met Phe Trp Asp Gln Val
305                 310                 315                 320

His Pro Thr Thr Val Val His Ala Ala Leu Ser Glu Arg Ala Ala Thr
                325                 330                 335

Phe Ile Ala Asn Gln Tyr Glu Phe Leu Ala His
            340                 345
```

<210> 31
<211> 1047
<212> DNA
<213> Aeromonas hydrophila

<400> 31

```
atgtttaagt ttaaaaagaa tttcttagtt ggattatcgg cagctttaat gagtattagc      60
ttgttttcgg caaccgcctc tgcagctagc gccgacagcc gtcccgcctt ttcccggatc     120
gtgatgttcg gcgacagcct ctccgatacc ggcaaaatgt acagcaagat gcgcggttac     180
ctcccctcca gcccgcccta ctatgagggc cgtttctcca acggacccgt ctggctggag     240
cagctgacca aacagttccc gggtctgacc atcgccaacg aagcggaagg cggtgccact     300
gccgtggctt acaacaagat ctcctggaat cccaagtatc aggtcatcaa caacctggac     360
tacgaggtca cccagttctt gcagaaagac agcttcaagc cggacgatct ggtgatcctc     420
tgggtcggtg ccaatgacta tctggcctat ggctggaaca cggagcagga tgccaagcgg     480
gttcgcgatg ccatcagcga tgcggccaac cgcatggtac tgaacggtgc caagcagata     540
ctgctgttca acctgccgga tctgggccag aacccgtcag ctcgcagtca gaaggtggtc     600
gaggcggtca gccatgtctc cgcctatcac aaccagctgc tgctgaacct ggcacgccag     660
ctggccccca ccggcatggt aaagctgttc gagatcgaca agcaatttgc cgagatgctg     720
cgtgatccgc agaacttcgg cctgagcgac gtcgagaacc cctgctacga cggcggctat     780
gtgtggaagc cgtttgccac ccgcagcgtc agcaccgacc gccagctctc cgccttcagt     840
ccgcaggaac gcctcgccat cgccggcaac ccgctgctgg cacaggccgt tgccagtcct     900
atggcccgcc gcagcgccag ccccctcaac tgtgagggca agatgttctg ggatcaggta     960
cacccgacca ctgtcgtgca cgcagccctg agcgagcgcg ccgccacctt catcgcgaac    1020
cagtacgagt tcctcgccca ctgatga                                        1047
```

<210> 32
<211> 1371
<212> DNA
<213> Streptomyces sp.

<400> 32

```
acaggccgat gcacggaacc gtacctttcc gcagtgaagc gctctccccc catcgttcgc      60
cgggacttca tccgcgattt tggcatgaac acttccttca acgcgcgtag cttgctacaa     120
gtgcggcagc agacccgctc gttggaggct cagtgagatt gacccgatcc ctgtcggccg     180
catccgtcat cgtcttcgcc ctgctgctcg cgctgctggg catcagcccg gcccaggcag     240
ccggcccggc ctatgtggcc ctgggggatt cctattcctc gggcaacggc gccggaagtt     300
acatcgattc gagcggtgac tgtcaccgca gcaacaacgc gtaccccgcc cgctgggcgg     360
cggccaacgc accgtcctcc ttcaccttcg cggcctgctc gggagcggtg accacggatg     420
tgatcaacaa tcagctgggc gccctcaacg cgtccaccgg cctggtgagc atcaccatcg     480
gcggcaatga cgcgggcttc gcggacgcga tgaccacctg cgtcaccagc tcggacagca     540
cctgcctcaa ccggctggcc accgccacca actacatcaa caccaccctg ctcgcccggc     600
tcgacgcggt ctacagccag atcaaggccc gtgcccccaa cgcccgcgtg gtcgtcctcg     660
gctacccgcg catgtacctg gcctcgaacc cctggtactg cctgggcctg agcaacacca     720
agcgcgcggc catcaacacc accgccgaca ccctcaactc ggtgatctcc tcccgggcca     780
ccgcccacgg attccgattc ggcgatgtcc gcccgacctt caacaaccac gaactgttct     840
tcggcaacga ctggctgcac tcactcaccc tgccggtgtg ggagtcgtac caccccacca     900
gcacgggcca tcagagcggc tatctgccgg tcctcaacgc caacagctcg acctgatcaa     960
cgcacggccg tgcccgcccc gcgcgtcacg ctcggcgcgg gcgccgcagc gcgttgatca    1020
gcccacagtg ccggtgacgg tcccaccgtc acggtcgagg gtgtacgtca cggtggcgcc    1080
gctccagaag tggaacgtca gcaggaccgt ggagccgtcc ctgacctcgt cgaagaactc    1140
cggggtcagc gtgatcaccc ctcccccgta gccgggggcg aaggcggcgc cgaactcctt    1200
gtaggacgtc cagtcgtgcg gcccggcgtt gccaccgtcc gcgtagaccg cttccatggt    1260
cgccagccgg tccccgcgga actcggtggg gatgtccgtg cccaaggtgg tcccggtggt    1320
gtccgagagc accgggggct cgtaccggat gatgtgcaga tccaaagaat t              1371
```

<210> 33
<211> 267
<212> PRT
<213> Streptomyces sp.

<400> 33

```
Met Arg Leu Thr Arg Ser Leu Ser Ala Ala Ser Val Ile Val Phe Ala
1               5                   10                  15

Leu Leu Leu Ala Leu Leu Gly Ile Ser Pro Ala Gln Ala Ala Gly Pro
            20                  25                  30
```

```
Ala Tyr Val Ala Leu Gly Asp Ser Tyr Ser Ser Gly Asn Gly Ala Gly
        35                  40                  45

Ser Tyr Ile Asp Ser Ser Gly Asp Cys His Arg Ser Asn Asn Ala Tyr
    50                  55                  60

Pro Ala Arg Trp Ala Ala Ala Asn Ala Pro Ser Ser Phe Thr Phe Ala
65                  70                  75                  80

Ala Cys Ser Gly Ala Val Thr Thr Asp Val Ile Asn Asn Gln Leu Gly
                85                  90                  95

Ala Leu Asn Ala Ser Thr Gly Leu Val Ser Ile Thr Ile Gly Gly Asn
            100                 105                 110

Asp Ala Gly Phe Ala Asp Ala Met Thr Thr Cys Val Thr Ser Ser Asp
        115                 120                 125

Ser Thr Cys Leu Asn Arg Leu Ala Thr Ala Thr Asn Tyr Ile Asn Thr
    130                 135                 140

Thr Leu Leu Ala Arg Leu Asp Ala Val Tyr Ser Gln Ile Lys Ala Arg
145                 150                 155                 160

Ala Pro Asn Ala Arg Val Val Val Leu Gly Tyr Pro Arg Met Tyr Leu
                165                 170                 175

Ala Ser Asn Pro Trp Tyr Cys Leu Gly Leu Ser Asn Thr Lys Arg Ala
            180                 185                 190

Ala Ile Asn Thr Thr Ala Asp Thr Leu Asn Ser Val Ile Ser Ser Arg
        195                 200                 205

Ala Thr Ala His Gly Phe Arg Phe Gly Asp Val Arg Pro Thr Phe Asn
    210                 215                 220

Asn His Glu Leu Phe Phe Gly Asn Asp Trp Leu His Ser Leu Thr Leu
225                 230                 235                 240

Pro Val Trp Glu Ser Tyr His Pro Thr Ser Thr Gly His Gln Ser Gly
                245                 250                 255

Tyr Leu Pro Val Leu Asn Ala Asn Ser Ser Thr
            260                 265
```

<210> 34
<211> 548
<212> PRT
<213> Thermobifida sp.

<400> 34

```
Met Leu Pro His Pro Ala Gly Glu Arg Gly Glu Val Gly Ala Phe Phe
1               5                   10                  15

Ala Leu Leu Val Gly Thr Pro Gln Asp Arg Arg Leu Arg Leu Glu Cys
            20                  25                  30

His Glu Thr Arg Pro Leu Arg Gly Arg Cys Gly Cys Gly Glu Arg Arg
        35                  40                  45

Val Pro Pro Leu Thr Leu Pro Gly Asp Gly Val Leu Cys Thr Thr Ser
    50                  55                  60

Ser Thr Arg Asp Ala Glu Thr Val Trp Arg Lys His Leu Gln Pro Arg
65                  70                  75                  80

Pro Asp Gly Gly Phe Arg Pro His Leu Gly Val Gly Cys Leu Leu Ala
                85                  90                  95

Gly Gln Gly Ser Pro Gly Val Leu Trp Cys Gly Arg Glu Gly Cys Arg
            100                 105                 110

Phe Glu Val Cys Arg Arg Asp Thr Pro Gly Leu Ser Arg Thr Arg Asn
        115                 120                 125

Gly Asp Ser Ser Pro Pro Phe Arg Ala Gly Trp Ser Leu Pro Pro Lys
    130                 135                 140

Cys Gly Glu Ile Ser Gln Ser Ala Arg Lys Thr Pro Ala Val Pro Arg
145                 150                 155                 160

Tyr Ser Leu Leu Arg Thr Asp Arg Pro Asp Gly Pro Arg Gly Arg Phe
                165                 170                 175

Val Gly Ser Gly Pro Arg Ala Ala Thr Arg Arg Arg Leu Phe Leu Gly
            180                 185                 190

Ile Pro Ala Leu Val Leu Val Thr Ala Leu Thr Leu Val Leu Ala Val
        195                 200                 205

Pro Thr Gly Arg Glu Thr Leu Trp Arg Met Trp Cys Glu Ala Thr Gln
    210                 215                 220

Asp Trp Cys Leu Gly Val Pro Val Asp Ser Arg Gly Gln Pro Ala Glu
225                 230                 235                 240
```

```
Asp Gly Glu Phe Leu Leu Leu Ser Pro Val Gln Ala Ala Thr Trp Gly
                245                 250                 255

Asn Tyr Tyr Ala Leu Gly Asp Ser Tyr Ser Ser Gly Asp Gly Ala Arg
            260                 265                 270

Asp Tyr Tyr Pro Gly Thr Ala Val Lys Gly Gly Cys Trp Arg Ser Ala
        275                 280                 285

Asn Ala Tyr Pro Glu Leu Val Ala Glu Ala Tyr Asp Phe Ala Gly His
    290                 295                 300

Leu Ser Phe Leu Ala Cys Ser Gly Gln Arg Gly Tyr Ala Met Leu Asp
305                 310                 315                 320

Ala Ile Asp Glu Val Gly Ser Gln Leu Asp Trp Asn Ser Pro His Thr
                325                 330                 335

Ser Leu Val Thr Ile Gly Ile Gly Gly Asn Asp Leu Gly Phe Ser Thr
            340                 345                 350

Val Leu Lys Thr Cys Met Val Arg Val Pro Leu Leu Asp Ser Lys Ala
        355                 360                 365

Cys Thr Asp Gln Glu Asp Ala Ile Arg Lys Arg Met Ala Lys Phe Glu
    370                 375                 380

Thr Thr Phe Glu Glu Leu Ile Ser Glu Val Arg Thr Arg Ala Pro Asp
385                 390                 395                 400

Ala Arg Ile Leu Val Val Gly Tyr Pro Arg Ile Phe Pro Glu Glu Pro
                405                 410                 415

Thr Gly Ala Tyr Tyr Thr Leu Thr Ala Ser Asn Gln Arg Trp Leu Asn
            420                 425                 430

Glu Thr Ile Gln Glu Phe Asn Gln Gln Leu Ala Glu Ala Val Ala Val
        435                 440                 445

His Asp Glu Glu Ile Ala Ala Ser Gly Gly Val Gly Ser Val Glu Phe
    450                 455                 460

Val Asp Val Tyr His Ala Leu Asp Gly His Glu Ile Gly Ser Asp Glu
465                 470                 475                 480

Pro Trp Val Asn Gly Val Gln Leu Arg Asp Leu Ala Thr Gly Val Thr
                485                 490                 495
```

```
Val Asp Arg Ser Thr Phe His Pro Asn Ala Ala Gly His Arg Ala Val
            500                 505                 510

Gly Glu Arg Val Ile Glu Gln Ile Glu Thr Gly Pro Gly Arg Pro Leu
        515                 520                 525

Tyr Ala Thr Phe Ala Val Val Ala Gly Ala Thr Val Asp Thr Leu Ala
    530                 535                 540

Gly Glu Val Gly
545
```

<210> 35
<211> 3000
<212> DNA
<213> Thermobifida sp.

<400> 35

ggtggtgaac cagaacaccc ggtcgtcggc gtgggcgtcc aggtgcaggt gcaggttctt 60
caactgctcc agcaggatgc cgccgtggcc gtgcacgatg gccttgggca ggcctgtggt 120
ccccgacgag tacagcaccc atagcggatg gtcgaacggc agcggggtga actccagttc 180
cgcgccttcg cccgcggctt cgaactccgc ccaggacagg gtgtcggcga cagggccgca 240
gcccaggtac ggcaggacga cggtgtgctg caggctgggc atgccgtcgc gcagggcttt 300
gagcacgtca cggcggtcga agtccttacc gccgtagcgg tagccgtcca cggccagcag 360
cactttcggt tcgatctgcg cgaaccggtc gaggacgctg cgcaccccga agtcggggga 420
acaggacgac caggtcgcac cgatcgcggc gcaggcgagg aatgcggccg tcgcctcggc 480
gatgttcggc aggtaggcca cgacccggtc gccggggccc accccgaggc tgcggagggc 540
cgcagcgatc gcggcggtgc gggtccgcag ttctccccag gtccactcgg tcaacggccg 600
gagttcggac gcgtgccgga tcgccacggc tgatgggtca cggtcgcgga agatgtgctc 660
ggcgtagttg agggtggcgc cggggaacca gacggcgccg ggcatggcgt cggaggcgag 720
cactgtggtg tacggggtgg cggcgcgcac ccggtagtac tcccagatcg cggaccagaa 780
tccttcgagg tcggttaccg accagcgcca cagtgcctcg tagtccggtg cgtccacacc 840
gcggtgctcc cgcacccagc gggtgaacgc ggtgaggttg gcgcgttctt tgcgctcctc 900
gtcgggactc cacaggatcg gcggctgcgg cttgagtgtc atgaaacgcg accccttcgt 960
ggacggtgcg gatgcggtga gcgtcgggtg cctcccctaa cgctccccgg tgacggagtg 1020
ttgtgcacca catctagcac gcgggacgcg gaaaccgtat ggagaaaaca cctacaaccc 1080
cggccggacg gtgggtttcg gccacactta ggggtcgggt gcctgcttgc cgggcagggc 1140
agtcccgggg tgctgtggtg cgggcgggag ggctgtcgct tcgaggtgtg ccggcgggac 1200
actccgggcc tcagccgtac ccgcaacggg gacagttctc ctcccttccg ggctggatgg 1260

tcccttcccc cgaaatgcgg cgagatctcc cagtcagccc ggaaaacacc cgctgtgccc 1320
aggtactctt tgcttcgaac agacaggccg gacggtccac gggggaggtt tgtgggcagc 1380
ggaccacgtg cggcgaccag acgacggttg ttcctcggta tccccgctct tgtacttgtg 1440
acagcgctca cgctggtctt ggctgtcccg acggggcgcg agacgctgtg gcgcatgtgg 1500
tgtgaggcca cccaggactg gtgcctgggg gtgccggtcg actcccgcgg acagcctgcg 1560
gaggacggcg agtttctgct gctttctccg gtccaggcag cgacctgggg gaactattac 1620
gcgctcgggg attcgtactc ttcgggggac ggggcccgcg actactatcc cggcaccgcg 1680
gtgaagggcg gttgctggcg gtccgctaac gcctatccgg agctggtcgc cgaagcctac 1740
gacttcgccg gacacttgtc gttcctggcc tgcagcggcc agcgcggcta cgccatgctt 1800
gacgctatcg acgaggtcgg ctcgcagctg gactggaact cccctcacac gtcgctggtg 1860
acgatcggga tcggcggcaa cgatctgggg ttctccacgg ttttgaagac ctgcatggtg 1920
cgggtgccgc tgctggacag caaggcgtgc acggaccagg aggacgctat ccgcaagcgg 1980
atggcgaaat tcgagacgac gtttgaagag ctcatcagcg aagtgcgcac ccgcgcgccg 2040
gacgcccgga tccttgtcgt gggctacccc cggatttttc cggaggaacc gaccggcgcc 2100
tactacacgc tgaccgcgag caaccagcgg tggctcaacg aaaccattca ggagttcaac 2160
cagcagctcg ccgaggctgt cgcggtccac gacgaggaga ttgccgcgtc gggcggggtg 2220
ggcagcgtgg agttcgtgga cgtctaccac gcgttggacg gccacgagat cggctcggac 2280
gagccgtggg tgaacggggt gcagttgcgg gacctcgcca ccggggtgac tgtggaccgc 2340
agtaccttcc accccaacgc cgctgggcac cgggcggtcg gtgagcgggt catcgagcag 2400
atcgaaaccg gcccgggccg tccgctctat gccactttcg cggtggtggc gggggcgacc 2460
gtggacactc tcgcgggcga ggtggggtga cccggcttac cgtccggccc gcaggtctgc 2520
gagcactgcg gcgatctggt ccactgccca gtgcagttcg tcttcggtga tgaccagcgg 2580
cggggagagc cggatcgttg agccgtgcgt gtctttgacg agcacacccc gctgcaggag 2640
ccgttcgcac agttctcttc cggtggccag agtcgggtcg acgtcgatcc cagcccacag 2700
gccgatgctg cgggccgcga ccacgccgtt gccgaccagt tggtcgaggc gggcgcgcag 2760
cacgggggcg agggcgcgga catggtccag gtaagggccg tcgcggacga ggctcaccac 2820
ggcagtgccg accgcgcagg cgagggcgtt gccgccgaag gtgctgccgt gctggccggg 2880
gcggatcacg tcgaagactt ccgcgtcgcc taccgccgcc gccacgggca ggatgccgcc 2940
gcccagcgct ttgccgaaca ggtagatatc ggcgtcgact ccgctgtggt cgcaggcccg 3000

<210> 36
<211> 372
<212> PRT
<213> Thermobifida sp.

<400> 36

```
Val Gly Ser Gly Pro Arg Ala Ala Thr Arg Arg Arg Leu Phe Leu Gly
1               5                   10                  15

Ile Pro Ala Leu Val Leu Val Thr Ala Leu Thr Leu Val Leu Ala Val
            20                  25                  30

Pro Thr Gly Arg Glu Thr Leu Trp Arg Met Trp Cys Glu Ala Thr Gln
        35                  40                  45

Asp Trp Cys Leu Gly Val Pro Val Asp Ser Arg Gly Gln Pro Ala Glu
    50                  55                  60

Asp Gly Glu Phe Leu Leu Leu Ser Pro Val Gln Ala Ala Thr Trp Gly
65                  70                  75                  80

Asn Tyr Tyr Ala Leu Gly Asp Ser Tyr Ser Ser Gly Asp Gly Ala Arg
                85                  90                  95

Asp Tyr Tyr Pro Gly Thr Ala Val Lys Gly Gly Cys Trp Arg Ser Ala
            100                 105                 110

Asn Ala Tyr Pro Glu Leu Val Ala Glu Ala Tyr Asp Phe Ala Gly His
        115                 120                 125

Leu Ser Phe Leu Ala Cys Ser Gly Gln Arg Gly Tyr Ala Met Leu Asp
    130                 135                 140

Ala Ile Asp Glu Val Gly Ser Gln Leu Asp Trp Asn Ser Pro His Thr
145                 150                 155                 160

Ser Leu Val Thr Ile Gly Ile Gly Gly Asn Asp Leu Gly Phe Ser Thr
                165                 170                 175

Val Leu Lys Thr Cys Met Val Arg Val Pro Leu Leu Asp Ser Lys Ala
            180                 185                 190

Cys Thr Asp Gln Glu Asp Ala Ile Arg Lys Arg Met Ala Lys Phe Glu
        195                 200                 205

Thr Thr Phe Glu Glu Leu Ile Ser Glu Val Arg Thr Arg Ala Pro Asp
    210                 215                 220

Ala Arg Ile Leu Val Val Gly Tyr Pro Arg Ile Phe Pro Glu Glu Pro
225                 230                 235                 240

Thr Gly Ala Tyr Tyr Thr Leu Thr Ala Ser Asn Gln Arg Trp Leu Asn
                245                 250                 255
```

Glu Thr Ile Gln Glu Phe Asn Gln Gln Leu Ala Glu Ala Val Ala Val
260 265 270

His Asp Glu Glu Ile Ala Ala Ser Gly Gly Val Gly Ser Val Glu Phe
275 280 285

Val Asp Val Tyr His Ala Leu Asp Gly His Glu Ile Gly Ser Asp Glu
290 295 300

Pro Trp Val Asn Gly Val Gln Leu Arg Asp Leu Ala Thr Gly Val Thr
305 310 315 320

Val Asp Arg Ser Thr Phe His Pro Asn Ala Ala Gly His Arg Ala Val
325 330 335

Gly Glu Arg Val Ile Glu Gln Ile Glu Thr Gly Pro Gly Arg Pro Leu
340 345 350

Tyr Ala Thr Phe Ala Val Val Ala Gly Ala Thr Val Asp Thr Leu Ala
355 360 365

Gly Glu Val Gly
370

<210> 37
<211> 300
<212> PRT
<213> Corynebacterium efficiens

<400> 37

Met Arg Thr Thr Val Ile Ala Ala Ser Ala Leu Leu Leu Leu Ala Gly
1 5 10 15

Cys Ala Asp Gly Ala Arg Glu Glu Thr Ala Gly Ala Pro Pro Gly Glu
20 25 30

Ser Ser Gly Gly Ile Arg Glu Glu Gly Ala Glu Ala Ser Thr Ser Ile
35 40 45

Thr Asp Val Tyr Ile Ala Leu Gly Asp Ser Tyr Ala Ala Met Gly Gly
50 55 60

Arg Asp Gln Pro Leu Arg Gly Glu Pro Phe Cys Leu Arg Ser Ser Gly
65 70 75 80

Asn Tyr Pro Glu Leu Leu His Ala Glu Val Thr Asp Leu Thr Cys Gln
85 90 95

```
Gly Ala Val Thr Gly Asp Leu Leu Glu Pro Arg Thr Leu Gly Glu Arg
            100                 105                 110

Thr Leu Pro Ala Gln Val Asp Ala Leu Thr Glu Asp Thr Thr Leu Val
        115                 120                 125

Thr Leu Ser Ile Gly Gly Asn Asp Leu Gly Phe Gly Glu Val Ala Gly
    130                 135                 140

Cys Ile Arg Glu Arg Ile Ala Gly Glu Asn Ala Asp Asp Cys Val Asp
145                 150                 155                 160

Leu Leu Gly Glu Thr Ile Gly Glu Gln Leu Asp Gln Leu Pro Pro Gln
                165                 170                 175

Leu Asp Arg Val His Glu Ala Ile Arg Asp Arg Ala Gly Asp Ala Gln
            180                 185                 190

Val Val Val Thr Gly Tyr Leu Pro Leu Val Ser Ala Gly Asp Cys Pro
        195                 200                 205

Glu Leu Gly Asp Val Ser Glu Ala Asp Arg Arg Trp Ala Val Glu Leu
    210                 215                 220

Thr Gly Gln Ile Asn Glu Thr Val Arg Glu Ala Ala Glu Arg His Asp
225                 230                 235                 240

Ala Leu Phe Val Leu Pro Asp Asp Ala Asp Glu His Thr Ser Cys Ala
                245                 250                 255

Pro Pro Gln Gln Arg Trp Ala Asp Ile Gln Gly Gln Gln Thr Asp Ala
            260                 265                 270

Tyr Pro Leu His Pro Thr Ser Ala Gly His Glu Ala Met Ala Ala Ala
        275                 280                 285

Val Arg Asp Ala Leu Gly Leu Glu Pro Val Gln Pro
    290                 295                 300
```

<210> 38
<211> 3000
<212> DNA
<213> Corynebacterium efficiens

<400> 38

```
ttctggggtg ttatggggtt gttatcggct cgtcctgggt ggatcccgcc aggtggggta      60
ttcacggggg acttttgtgt ccaacagccg agaatgagtg ccctgagcgg tgggaatgag     120
gtgggcgggg ctgtgtcgcc atgagggggc ggcgggctct gtggtgcccc gcgacccccg     180
```

gccccggtga gcggtgaatg aaatccggct gtaatcagca tcccgtgccc accccgtcgg 240
ggaggtcagc gcccggagtg tctacgcagt cggatcctct cggactcggc catgctgtcg 300
gcagcatcgc gctcccgggt cttggcgtcc ctcggctgtt ctgcctgctg tccctggaag 360
gcgaaatgat caccggggag tgatacaccg gtggtctcat cccggatgcc cacttcggcg 420
ccatccggca attcgggcag ctccgggtgg aagtaggtgg catccgatgc gtcggtgacg 480
ccatagtggg cgaagatctc atcctgctcg agggtgctca ggccactctc cggatcgata 540
tcgggggcgt ccttgatggc gtccttgctg aaaccgaggt gcagcttgtg ggcttccaat 600
ttcgcaccac ggagcgggac gaggctggaa tgacggccga agagcccgtg gtggacctca 660
acgaaggtgg gtagtcccgt gtcatcattg aggaacacgc cctccaccgc acccagcttg 720
tggccggagt tgtcgtaggc gctggcatcc agaagggaaa cgatctcata tttgtcggtg 780
tgctcagaca tgatcttcct ttgctgtcgg tgtctggtac taccacggta gggctgaatg 840
caactgttat ttttctgtta ttttaggaat tggtccatat cccacaggct ggctgtggtc 900
aaatcgtcat caagtaatcc ctgtcacaca aaatgggtgg tgggagccct ggtcgcggtt 960
ccgtgggagg cgccgtgccc cgcaggatcg tcggcatcgg cggatctggc cggtaccccg 1020
cggtgaataa aatcattctg taaccttcat cacggttggt tttaggtatc cgcccctttc 1080
gtcctgaccc cgtccccggc gcgcgggagc ccgcgggttg cggtagacag gggagacgtg 1140
gacaccatga ggacaacggt catcgcagca agcgcattac tccttctcgc cggatgcgcg 1200
gatggggccc gggaggagac cgccggtgca ccgccgggtg agtcctccgg gggcatccgg 1260
gaggaggggg cggaggcgtc gacaagcatc accgacgtct acatcgccct cggggattcc 1320
tatgcggcga tgggcgggcg ggatcagccg ttacggggtg agccgttctg cctgcgctcg 1380
tccggtaatt acccggaact cctccacgca gaggtcaccg atctcacctg ccaggggggcg 1440
gtgaccgggg atctgctcga acccaggacg ctgggggagc gcacgctgcc ggcgcaggtg 1500
gatgcgctga cggaggacac caccctggtc accctctcca tcgggggcaa tgacctcgga 1560
ttcggggagg tggcgggatg catccgggaa cggatcgccg gggagaacgc tgatgattgc 1620
gtggacctgc tgggggaaac catcggggag cagctcgatc agcttccccc gcagctggac 1680
cgcgtgcacg aggctatccg ggaccgcgcc ggggacgcgc aggttgtggt caccggttac 1740
ctgccgctcg tgtctgccgg ggactgcccc gaactggggg atgtctccga ggcggatcgt 1800
cgttgggcgg ttgagctgac cgggcagatc aacgagaccg tgcgcgaggc ggccgaacga 1860
cacgatgccc tctttgtcct gcccgacgat gccgatgagc acaccagttg tgcaccccca 1920
cagcagcgct gggcggatat ccagggccaa cagaccgatg cctatccgct gcacccgacc 1980
tccgccggcc atgaggcgat ggccgccgcc gtccgggacg cgctgggcct ggaaccggtc 2040
cagccgtagc gccgggcgcg cgcttgtcga cgaccaaccc atgccaggct gcagtcacat 2100

```
ccgcacatag cgcgcgcggg cgatggagta cgcaccatag aggatgagcc cgatgccgac     2160
gatgatgagc agcacactgc cgaagggttg ttccccgagg gtgcgcagag ccgagtccag     2220
acctgcggcc tgctccggat catgggccca accggcgatg acgatcaaca cccccaggat     2280
cccgaaggcg ataccacggg cgacataacc ggctgttccg gtgatgatga tcgcggtccc     2340
gacctgccct gaccccgcac ccgcctccag atcctcccgg aaatcccggg tggccccctt     2400
ccagaggttg tagacacccg cccccagtac caccagcccg gcgaccacaa ccagcaccac     2460
accccagggt tgggatagga cggtggcggt gacatcggtg gcggtctccc catcggaggt     2520
gctgccgccc cgggcgaagg tggaggtggt caccgccagg gagaagtaga ccatggccat     2580
gaccgccccc ttggcccttt ccttgaggtc ctcgcccgcc agcagctggc tcaattgcca     2640
gagtcccagg gccgccaggg cgatgacggc aacccacagg aggaactgcc cacccggagc     2700
ctccgcgatg gtggccaggg cacctgaatt cgaggcctca tcacccgaac cgccggatcc     2760
agtggcgatg cgcaccgcga tccacccgat gaggatgtgc agtatgccca ggacaatgaa     2820
accacctctg gccagggtgg tcagcgcggg gtggtcctcg gcctggtcgg cagcccgttc     2880
gatcgtccgt ttcgcggatc tggtgtcgcc cttatccata gctcccattg aaccgccttg     2940
aggggtgggc ggccactgtc agggcggatt gtgatctgaa ctgtgatgtt ccatcaaccc     3000
```

<210> 39
<211> 284
<212> PRT
<213> Novosphingobium aromaticovorans

<400> 39

```
Met Gly Gln Val Lys Leu Phe Ala Arg Arg Cys Ala Pro Val Leu Leu
1               5                   10                  15

Ala Leu Ala Gly Leu Ala Pro Ala Ala Thr Val Ala Arg Glu Ala Pro
            20                  25                  30

Leu Ala Glu Gly Ala Arg Tyr Val Ala Leu Gly Ser Ser Phe Ala Ala
        35                  40                  45

Gly Pro Gly Val Gly Pro Asn Ala Pro Gly Ser Pro Glu Arg Cys Gly
    50                  55                  60

Arg Gly Thr Leu Asn Tyr Pro His Leu Leu Ala Glu Ala Leu Lys Leu
65                  70                  75                  80

Asp Leu Val Asp Ala Thr Cys Ser Gly Ala Thr Thr His His Val Leu
                85                  90                  95

Gly Pro Trp Asn Glu Val Pro Pro Gln Ile Asp Ser Val Asn Gly Asp
```

100 105 110

Thr Arg Leu Val Thr Leu Thr Ile Gly Gly Asn Asp Val Ser Phe Val
115 120 125

Gly Asn Ile Phe Ala Ala Ala Cys Glu Lys Met Ala Ser Pro Asp Pro
130 135 140

Arg Cys Gly Lys Trp Arg Glu Ile Thr Glu Glu Glu Trp Gln Ala Asp
145 150 155 160

Glu Glu Arg Met Arg Ser Ile Val Arg Gln Ile His Ala Arg Ala Pro
165 170 175

Leu Ala Arg Val Val Val Val Asp Tyr Ile Thr Val Leu Pro Pro Ser
180 185 190

Gly Thr Cys Ala Ala Met Ala Ile Ser Pro Asp Arg Leu Ala Gln Ser
195 200 205

Arg Ser Ala Ala Lys Arg Leu Ala Arg Ile Thr Ala Arg Val Ala Arg
210 215 220

Glu Glu Gly Ala Ser Leu Leu Lys Phe Ser His Ile Ser Arg Arg His
225 230 235 240

His Pro Cys Ser Ala Lys Pro Trp Ser Asn Gly Leu Ser Ala Pro Ala
245 250 255

Asp Asp Gly Ile Pro Val His Pro Asn Arg Leu Gly His Ala Glu Ala
260 265 270

Ala Ala Ala Leu Val Lys Leu Val Lys Leu Met Lys
275 280

<210> 40
<211> 1500
<212> DNA
<213> Novosphingobium aromaticovorans

<400> 40

```
tgccggaact caagcggcgt ctagccgaac tcatgcccga aagcgcgtgg cactatcccg      60
aagaccaggt ctcggacgcc agcgagcgcc tgatggccgc cgaaatcacg cgcgaacagc     120
tctaccgcca gctccacgac gagctgccct atgacagtac cgtacgtccc gagaagtacc     180
tccatcgcaa ggacggttcg atcgagatcc accagcagat cgtgattgcc cgcgagacac     240
agcgtccgat cgtgctgggc aagggtggcg cgaagatcaa ggcgatcgga gaggccgcac     300
gcaaggaact ttcgcaattg ctcgacacca aggtgcacct gttcctgcat gtgaaggtcg     360

acgagcgctg ggccgacgcc aaggaaatct acgaggaaat cggcctcgaa tgggtcaagt     420
gaagctcttc gcgcgccgct gcgccccagt acttctcgcc cttgccgggc tggctccggc     480
ggctacggtc gcgcgggaag caccgctggc cgaaggcgcg cgttacgttg cgctgggaag     540
ctccttcgcc gcaggtccgg gcgtggggcc caacgcgccc ggatcgcccg aacgctgcgg     600
ccggggcacg ctcaactacc cgcacctgct cgccgaggcg ctcaagctcg atctcgtcga     660
tgcgacctgc agcggcgcga cgacccacca cgtgctgggc ccctggaacg aggttccccc     720
tcagatcgac agcgtgaatg gcgacacccg cctcgtcacc ctgaccatcg gcggaaacga     780
tgtgtcgttc gtcggcaaca tcttcgccgc cgcttgcgag aagatggcgt cgcccgatcc     840
gcgctgcggc aagtggcggg agatcaccga ggaagagtgg caggccgacg aggagcggat     900
gcgctccatc gtacgccaga tccacgcccg cgcgcctctc gcccgggtgg tggtggtcga     960
ttacatcacg gtcctgccgc catcaggcac ttgcgctgcc atggcgattt cgccggaccg    1020
gctggcccag agccgcagcg ccgcgaaacg gcttgcccgg attaccgcac gggtcgcgcg    1080
agaagagggt gcatcgctgc tcaagttctc gcatatctcg cgccggcacc atccatgctc    1140
tgccaagccc tggagcaacg gcctttccgc cccggccgac gacggcatcc cggtccatcc    1200
gaaccggctc ggacatgctg aagcggcagc ggcgctggtc aagcttgtga aattgatgaa    1260
gtagctactg cactgatttc aaatagtatt gcctgtcagc tttccagccc ggattgttgc    1320
agcgcaacag aaacttgtcc gtaatggatt gatggtttat gtcgctcgca aattgccgtc    1380
gaagggaacg ggcgcgtcgc tcgttaacgt cctgggtgca gcagtgacgg agcgcgtgga    1440
tgagtgatac tggcggtgtc atcggtgtac gcgccgccat tcccatgcct gtacgcgccg    1500
```

<210> 41
<211> 268
<212> PRT
<213> Streptomyces coelicolor

<400> 41

```
Met Arg Arg Phe Arg Leu Val Gly Phe Leu Ser Ser Leu Val Leu Ala
1               5                   10                  15

Ala Gly Ala Ala Leu Thr Gly Ala Ala Thr Ala Gln Ala Ala Gln Pro
            20                  25                  30

Ala Ala Ala Asp Gly Tyr Val Ala Leu Gly Asp Ser Tyr Ser Ser Gly
        35                  40                  45

Val Gly Ala Gly Ser Tyr Ile Ser Ser Ser Gly Asp Cys Lys Arg Ser
    50                  55                  60

Thr Lys Ala His Pro Tyr Leu Trp Ala Ala Ala His Ser Pro Ser Thr
```

```
65                  70                  75                  80

Phe Asp Phe Thr Ala Cys Ser Gly Ala Arg Thr Gly Asp Val Leu Ser
                85                  90                  95

Gly Gln Leu Gly Pro Leu Ser Ser Gly Thr Gly Leu Val Ser Ile Ser
            100                 105                 110

Ile Gly Gly Asn Asp Ala Gly Phe Ala Asp Thr Met Thr Thr Cys Val
        115                 120                 125

Leu Gln Ser Glu Ser Ser Cys Leu Ser Arg Ile Ala Thr Ala Glu Ala
    130                 135                 140

Tyr Val Asp Ser Thr Leu Pro Gly Lys Leu Asp Gly Val Tyr Ser Ala
145                 150                 155                 160

Ile Ser Asp Lys Ala Pro Asn Ala His Val Val Val Ile Gly Tyr Pro
                165                 170                 175

Arg Phe Tyr Lys Leu Gly Thr Thr Cys Ile Gly Leu Ser Glu Thr Lys
            180                 185                 190

Arg Thr Ala Ile Asn Lys Ala Ser Asp His Leu Asn Thr Val Leu Ala
        195                 200                 205

Gln Arg Ala Ala Ala His Gly Phe Thr Phe Gly Asp Val Arg Thr Thr
    210                 215                 220

Phe Thr Gly His Glu Leu Cys Ser Gly Ser Pro Trp Leu His Ser Val
225                 230                 235                 240

Asn Trp Leu Asn Ile Gly Glu Ser Tyr His Pro Thr Ala Ala Gly Gln
                245                 250                 255

Ser Gly Gly Tyr Leu Pro Val Leu Asn Gly Ala Ala
            260                 265
```

<210> 42
<211> 2000
<212> DNA
<213> Streptomyces coelicolor

<400> 42

```
cccggcggcc cgtgcaggag cagcagccgg cccgcgatgt cctcgggcgt cgtcttcatc        60
aggccgtcca tcgcgtcggc gaccggcgcc gtgtagttgg cccggacctc gtcccaggtg       120
cccgcggcga tctggcgggt ggtgcggtgc gggccgcgcc gaggggagac gtaccagaag       180
cccatcgtca cgttctccgg ctgcggttcg ggctcgtccg ccgctccgtc cgtcgcctcg       240
```

```
ccgagcacct tctcggcgag gtcggcgctg gtcgccgtca ccgtgacgtc ggcgccccgg    300
ctccagcgcg agatcagcag cgtccagccg tcgccctccg ccagcgtcgc gctgcggtcg    360
tcgtcgcggg cgatccgcag cacgcgcgcg ccgggcggca gcagcgtggc gccggaccgt    420
acgcggtcga tgttcgccgc gtgcgagtac ggctgctcac ccgtggcgaa acggccgagg    480
aacagcgcgt cgacgacgtc ggacggggag tcgctgtcgt ccacgttgag ccggatcggc    540
agggcttcgt gcgggttcac ggacatgtcg ccatgatcgg gcacccggcc gccgcgtgca    600
cccgctttcc cgggcacgca cgacaggggc tttctcgccg tcttccgtcc gaacttgaac    660
gagtgtcagc catttcttgg catggacact tccagtcaac gcgcgtagct gctaccacgg    720
ttgtggcagc aatcctgcta agggaggttc catgagacgt ttccgacttg tcggcttcct    780
gagttcgctc gtcctcgccg ccggcgccgc cctcaccggg gcagcgaccg cccaggcggc    840
ccaacccgcc gccgccgacg gctatgtggc cctcggcgac tcctactcct ccggggtcgg    900
agcgggcagc tacatcagct cgagcggcga ctgcaagcgc agcacgaagg cccatcccta    960
cctgtgggcg gccgcccact cgccctccac gttcgacttc accgcctgtt ccggcgcccg   1020
tacgggtgat gttctctccg gacagctcgg cccgctcagc tccggcaccg gcctcgtctc   1080
gatcagcatc ggcggcaacg acgccggttt cgccgacacc atgacgacct gtgtgctcca   1140
gtccgagagc tcctgcctgt cgcggatcgc caccgccgag gcgtacgtcg actcgacgct   1200
gcccggcaag ctcgacggcg tctactcggc aatcagcgac aaggcgccga acgcccacgt   1260
cgtcgtcatc ggctacccgc gcttctacaa gctcggcacc acctgcatcg gcctgtccga   1320
gaccaagcgg acggcgatca acaaggcctc cgaccacctc aacaccgtcc tcgcccagcg   1380
cgccgccgcc cacggcttca ccttcggcga cgtacgcacc accttcaccg gccacgagct   1440
gtgctccggc agcccctggc tgcacagcgt caactggctg aacatcggcg agtcgtacca   1500
ccccaccgcg gccggccagt ccggtggcta cctgccggtc ctcaacggcg ccgcctgacc   1560
tcaggcggaa ggagaagaag aaggagcgga gggagacgag gagtgggagg ccccgcccga   1620
cggggtcccc gtccccgtct ccgtctccgt cccggtcccg caagtcaccg agaacgccac   1680
cgcgtcggac gtggcccgca ccggactccg cacctccacg cgcacggcac tctcgaacgc   1740
gccggtgtcg tcgtgcgtcg tcaccaccac gccgtcctgg cgcgagcgct cgccgcccga   1800
cgggaaggac agcgtccgcc accccggatc ggagaccgac ccgtccgcgg tcacccaccg   1860
gtagccgacc tccgcgggca gccgcccgac cgtgaacgtc gccgtgaacg cgggtgcccg   1920
gtcgtgcggc ggcggacagg cccccgagta gtgggtgcgc gagcccacca cggtcacctc   1980
caccgactgc gctgcggggc                                               2000
```

<210> 43
<211> 269
<212> PRT

<213> Streptomyces avermitilis

<400> 43

```
Met Arg Arg Ser Arg Ile Thr Ala Tyr Val Thr Ser Leu Leu Leu Ala
1               5                   10                  15

Val Gly Cys Ala Leu Thr Gly Ala Ala Thr Ala Gln Ala Ser Pro Ala
            20                  25                  30

Ala Ala Ala Thr Gly Tyr Val Ala Leu Gly Asp Ser Tyr Ser Ser Gly
        35                  40                  45

Val Gly Ala Gly Ser Tyr Leu Ser Ser Ser Gly Asp Cys Lys Arg Ser
    50                  55                  60

Ser Lys Ala Tyr Pro Tyr Leu Trp Gln Ala Ala His Ser Pro Ser Ser
65                  70                  75                  80

Phe Ser Phe Met Ala Cys Ser Gly Ala Arg Thr Gly Asp Val Leu Ala
                85                  90                  95

Asn Gln Leu Gly Thr Leu Asn Ser Ser Thr Gly Leu Val Ser Leu Thr
            100                 105                 110

Ile Gly Gly Asn Asp Ala Gly Phe Ser Asp Val Met Thr Thr Cys Val
        115                 120                 125

Leu Gln Ser Asp Ser Ala Cys Leu Ser Arg Ile Asn Thr Ala Lys Ala
    130                 135                 140

Tyr Val Asp Ser Thr Leu Pro Gly Gln Leu Asp Ser Val Tyr Thr Ala
145                 150                 155                 160

Ile Ser Thr Lys Ala Pro Ser Ala His Val Ala Val Leu Gly Tyr Pro
                165                 170                 175

Arg Phe Tyr Lys Leu Gly Gly Ser Cys Leu Ala Gly Leu Ser Glu Thr
            180                 185                 190

Lys Arg Ser Ala Ile Asn Asp Ala Ala Asp Tyr Leu Asn Ser Ala Ile
        195                 200                 205

Ala Lys Arg Ala Ala Asp His Gly Phe Thr Phe Gly Asp Val Lys Ser
    210                 215                 220

Thr Phe Thr Gly His Glu Ile Cys Ser Ser Ser Thr Trp Leu His Ser
225                 230                 235                 240
```

Leu Asp Leu Leu Asn Ile Gly Gln Ser Tyr His Pro Thr Ala Ala Gly
245 250 255

Gln Ser Gly Gly Tyr Leu Pro Val Met Asn Ser Val Ala
260 265

<210> 44
<211> 1980
<212> DNA
<213> Steptomyces avermitilis

<400> 44

ccaccgccgg gtcggcggcg agtctcctgg cctcggtcgc ggagaggttg gccgtgtagc 60
cgttcagcgc ggcgccgaac gtcttcttca ccgtgccgcc gtactcgttg atcaggccct 120
tgcccttgct cgacgcggcc ttgaagccgg tgcccttctt gagcgtgacg atgtagctgc 180
ccttgatcgc ggtgggggag ccggcggcga gcaccgtgcc ctcggccggg gtggcctggg 240
cgggcagtgc ggtgaatccg cccacgaggg cgccggtcgc cacggcggtt atcgcggcga 300
tccggatctt cttgctacgc agctgtgcca tacgagggag tcctcctctg ggcagcggcg 360
cgcctgggtg gggcgcacgg ctgtgggggg tgcgcgcgtc atcacgcaca cggccctgga 420
gcgtcgtgtt ccgccctggg ttgagtaaag cctcggccat ctacgggggt ggctcaaggg 480
agttgagacc ctgtcatgag tctgacatga gcacgcaatc aacggggccg tgagcacccc 540
ggggcgaccc cggaaagtgc cgagaagtct tggcatggac acttcctgtc aacacgcgta 600
gctggtacga cggttacggc agagatcctg ctaaagggag gttccatgag acgttcccga 660
attacggcat acgtgacctc actcctcctc gccgtcggct gcgccctcac cggggcagcg 720
acggcgcagg cgtccccagc cgccgcggcc acgggctatg tggccctcgg cgactcgtac 780
tcgtccggtg tcggcgccgg cagctacctc agctccagcg gcgactgcaa gcgcagttcg 840
aaggcctatc cgtacctctg gcaggccgcg cattcaccct cgtcgttcag tttcatggct 900
tgctcgggcg ctcgtacggg tgatgtcctg gccaatcagc tcggcaccct gaactcgtcc 960
accggcctgg tctccctcac catcggaggc aacgacgcgg gcttctccga cgtcatgacg 1020
acctgtgtgc tccagtccga cagcgcctgc ctctcccgca tcaacacggc gaaggcgtac 1080
gtcgactcca ccctgcccgg ccaactcgac agcgtgtaca cggcgatcag cacgaaggcc 1140
ccgtcggccc atgtggccgt gctgggctac ccccgcttct acaaactggg cggctcctgc 1200
ctcgcgggcc tctcggagac caagcggtcc gccatcaacg acgcggccga ctatctgaac 1260
agcgccatcg ccaagcgcgc cgccgaccac ggcttcacct tcggcgacgt caagagcacc 1320
ttcaccggcc atgagatctg ctccagcagc acctggctgc acagtctcga cctgctgaac 1380
atcggccagt cctaccaccc gaccgcggcc ggccagtccg gcggctatct gccggtcatg 1440
aacagcgtgg cctgagctcc cacggcctga atttttaagg cctgaatttt taaggcgaag 1500

```
gtgaaccgga agcggaggcc ccgtccgtcg gggtctccgt cgcacaggtc accgagaacg     1560
gcacggagtt ggacgtcgtg cgcaccgggt cgcgcacctc gacggcgatc tcgttcgaga     1620
tcgttccgct cgtgtcgtac gtggtgacga acacctgctt ctgctgggtc tttccgccgc     1680
tcgccgggaa ggacagcgtc ttccagcccg gatccgggac ctcgcccttc ttggtcaccc     1740
agcggtactc cacctcgacc ggcacccggc ccaccgtgaa ggtcgccgtg aacgtgggcg     1800
cctgggcggt gggcggcggg caggcaccgg agtagtcggt gtgcacgccg gtgaccgtca     1860
ccttcacgga ctgggccggc ggggtcgtcg taccgccgcc gccaccgccg cctcccggag     1920
tggagcccga gctgtggtcg cccccgccgt cggcgttgtc gtcctcgggg gttttcgaac     1980
```

<210> 45
<211> 267
<212> PRT
<213> Streptomyces sp.

<400> 45

```
Met Arg Leu Thr Arg Ser Leu Ser Ala Ala Ser Val Ile Val Phe Ala
1               5                   10                  15

Leu Leu Leu Ala Leu Leu Gly Ile Ser Pro Ala Gln Ala Ala Gly Pro
            20                  25                  30

Ala Tyr Val Ala Leu Gly Asp Ser Tyr Ser Ser Gly Asn Gly Ala Gly
        35                  40                  45

Ser Tyr Ile Asp Ser Ser Gly Asp Cys His Arg Ser Asn Asn Ala Tyr
    50                  55                  60

Pro Ala Arg Trp Ala Ala Ala Asn Ala Pro Ser Ser Phe Thr Phe Ala
65                  70                  75                  80

Ala Cys Ser Gly Ala Val Thr Thr Asp Val Ile Asn Asn Gln Leu Gly
                85                  90                  95

Ala Leu Asn Ala Ser Thr Gly Leu Val Ser Ile Thr Ile Gly Gly Asn
            100                 105                 110

Asp Ala Gly Phe Ala Asp Ala Met Thr Thr Cys Val Thr Ser Ser Asp
        115                 120                 125

Ser Thr Cys Leu Asn Arg Leu Ala Thr Ala Thr Asn Tyr Ile Asn Thr
    130                 135                 140

Thr Leu Leu Ala Arg Leu Asp Ala Val Tyr Ser Gln Ile Lys Ala Arg
145                 150                 155                 160
```

```
Ala Pro Asn Ala Arg Val Val Val Leu Gly Tyr Pro Arg Met Tyr Leu
                165                 170                 175

Ala Ser Asn Pro Trp Tyr Cys Leu Gly Leu Ser Asn Thr Lys Arg Ala
            180                 185                 190

Ala Ile Asn Thr Thr Ala Asp Thr Leu Asn Ser Val Ile Ser Ser Arg
        195                 200                 205

Ala Thr Ala His Gly Phe Arg Phe Gly Asp Val Arg Pro Thr Phe Asn
    210                 215                 220

Asn His Glu Leu Phe Phe Gly Asn Asp Trp Leu His Ser Leu Thr Leu
225                 230                 235                 240

Pro Val Trp Glu Ser Tyr His Pro Thr Ser Thr Gly His Gln Ser Gly
                245                 250                 255

Tyr Leu Pro Val Leu Asn Ala Asn Ser Ser Thr
            260                 265
```

<210> 46
<211> 1371
<212> DNA
<213> Streptomyces sp.

<400> 46

```
acaggccgat gcacggaacc gtacctttcc gcagtgaagc gctctccccc catcgttcgc      60
cgggacttca tccgcgattt tggcatgaac acttccttca acgcgcgtag cttgctacaa     120
gtgcggcagc agacccgctc gttggaggct cagtgagatt gacccgatcc ctgtcggccg     180
catccgtcat cgtcttcgcc ctgctgctcg cgctgctggg catcagcccg gcccaggcag     240
ccggcccggc ctatgtggcc ctgggggatt cctattcctc gggcaacggc gccggaagtt     300
acatcgattc gagcggtgac tgtcaccgca gcaacaacgc gtaccccgcc cgctgggcgg     360
cggccaacgc accgtcctcc ttcaccttcg cggcctgctc gggagcggtg accacggatg     420
tgatcaacaa tcagctgggc gccctcaacg cgtccaccgg cctggtgagc atcaccatcg     480
gcggcaatga cgcgggcttc gcggacgcga tgaccacctg cgtcaccagc tcggacagca     540
cctgcctcaa ccggctggcc accgccacca actacatcaa caccaccctg ctcgcccggc     600
tcgacgcggt ctacagccag atcaaggccc gtgcccccaa cgcccgcgtg gtcgtcctcg     660
gctacccgcg catgtacctg gcctcgaacc cctggtactg cctgggcctg agcaacacca     720
agcgcgcggc catcaacacc accgccgaca ccctcaactc ggtgatctcc tcccgggcca     780
ccgcccacgg attccgattc ggcgatgtcc gcccgacctt caacaaccac gaactgttct     840
tcggcaacga ctggctgcac tcactcaccc tgccggtgtg ggagtcgtac caccccacca     900

gcacgggcca tcagagcggc tatctgccgg tcctcaacgc caacagctcg acctgatcaa     960
cgcacggccg tgcccgcccc gcgcgtcacg ctcggcgcgg gcgccgcagc gcgttgatca    1020
gcccacagtg ccggtgacgg tcccaccgtc acggtcgagg gtgtacgtca cggtggcgcc    1080
gctccagaag tggaacgtca gcaggaccgt ggagccgtcc ctgacctcgt cgaagaactc    1140
cggggtcagc gtgatcaccc ctcccccgta gccgggggcg aaggcggcgc cgaactcctt    1200
gtaggacgtc cagtcgtgcg gcccggcgtt gccaccgtcc gcgtagaccg cttccatggt    1260
cgccagccgg tccccgcgga actcggtggg gatgtccgtg cccaaggtgg tcccggtggt    1320
gtccgagagc accgggggct cgtaccggat gatgtgcaga tccaaagaat t             1371
```

<210> 47
<211> 295
<212> PRT
<213> Aeromonas hydrophila

<400> 47

```
Ile Val Met Phe Gly Asp Ser Leu Ser Asp Thr Gly Lys Met Tyr Ser
1               5                   10                  15

Lys Met Arg Gly Tyr Leu Pro Ser Ser Pro Pro Tyr Tyr Glu Gly Arg
            20                  25                  30

Phe Ser Asn Gly Pro Val Trp Leu Glu Gln Leu Thr Asn Glu Phe Pro
        35                  40                  45

Gly Leu Thr Ile Ala Asn Glu Ala Glu Gly Gly Pro Thr Ala Val Ala
    50                  55                  60

Tyr Asn Lys Ile Ser Trp Asn Pro Lys Tyr Gln Val Ile Asn Asn Leu
65                  70                  75                  80

Asp Tyr Glu Val Thr Gln Phe Leu Gln Lys Asp Ser Phe Lys Pro Asp
                85                  90                  95

Asp Leu Val Ile Leu Trp Val Gly Ala Asn Asp Tyr Leu Ala Tyr Gly
            100                 105                 110

Trp Asn Thr Glu Gln Asp Ala Lys Arg Val Arg Asp Ala Ile Ser Asp
        115                 120                 125

Ala Ala Asn Arg Met Val Leu Asn Gly Ala Lys Glu Ile Leu Leu Phe
    130                 135                 140

Asn Leu Pro Asp Leu Gly Gln Asn Pro Ser Ala Arg Ser Gln Lys Val
145                 150                 155                 160
```

```
Val Glu Ala Ala Ser His Val Ser Ala Tyr His Asn Gln Leu Leu Leu
                165                 170                 175

Asn Leu Ala Arg Gln Leu Ala Pro Thr Gly Met Val Lys Leu Phe Glu
            180                 185                 190

Ile Asp Lys Gln Phe Ala Glu Met Leu Arg Asp Pro Gln Asn Phe Gly
        195                 200                 205

Leu Ser Asp Gln Arg Asn Ala Cys Tyr Gly Gly Ser Tyr Val Trp Lys
    210                 215                 220

Pro Phe Ala Ser Arg Ser Ala Ser Thr Asp Ser Gln Leu Ser Ala Phe
225                 230                 235                 240

Asn Pro Gln Glu Arg Leu Ala Ile Ala Gly Asn Pro Leu Leu Ala Gln
                245                 250                 255

Ala Val Ala Ser Pro Met Ala Ala Arg Ser Ala Ser Thr Leu Asn Cys
            260                 265                 270

Glu Gly Lys Met Phe Trp Asp Gln Val His Pro Thr Thr Val Val His
        275                 280                 285

Ala Ala Leu Ser Glu Pro Ala
    290                 295
```

<210> 48
<211> 295
<212> PRT
<213> Aeromonas salmonicida

<400> 48

```
Ile Val Met Phe Gly Asp Ser Leu Ser Asp Thr Gly Lys Met Tyr Ser
1               5                   10                  15

Lys Met Arg Gly Tyr Leu Pro Ser Ser Pro Pro Tyr Tyr Glu Gly Arg
            20                  25                  30

Phe Ser Asn Gly Pro Val Trp Leu Glu Gln Leu Thr Lys Gln Phe Pro
        35                  40                  45

Gly Leu Thr Ile Ala Asn Glu Ala Glu Gly Gly Ala Thr Ala Val Ala
    50                  55                  60

Tyr Asn Lys Ile Ser Trp Asn Pro Lys Tyr Gln Val Tyr Asn Asn Leu
65                  70                  75                  80
```

```
Asp Tyr Glu Val Thr Gln Phe Leu Gln Lys Asp Ser Phe Lys Pro Asp
                85                  90                  95

Asp Leu Val Ile Leu Trp Val Gly Ala Asn Asp Tyr Leu Ala Tyr Gly
            100                 105                 110

Trp Asn Thr Glu Gln Asp Ala Lys Arg Val Arg Asp Ala Ile Ser Asp
        115                 120                 125

Ala Ala Asn Arg Met Val Leu Asn Gly Ala Lys Gln Ile Leu Leu Phe
    130                 135                 140

Asn Leu Pro Asp Leu Gly Gln Asn Pro Ser Ala Arg Ser Gln Lys Val
145                 150                 155                 160

Val Glu Ala Val Ser His Val Ser Ala Tyr His Asn Lys Leu Leu Leu
                165                 170                 175

Asn Leu Ala Arg Gln Leu Ala Pro Thr Gly Met Val Lys Leu Phe Glu
            180                 185                 190

Ile Asp Lys Gln Phe Ala Glu Met Leu Arg Asp Pro Gln Asn Phe Gly
        195                 200                 205

Leu Ser Asp Val Glu Asn Pro Cys Tyr Asp Gly Gly Tyr Val Trp Lys
    210                 215                 220

Pro Phe Ala Thr Arg Ser Val Ser Thr Asp Arg Gln Leu Ser Ala Phe
225                 230                 235                 240

Ser Pro Gln Glu Arg Leu Ala Ile Ala Gly Asn Pro Leu Leu Ala Gln
                245                 250                 255

Ala Val Ala Ser Pro Met Ala Arg Arg Ser Ala Ser Pro Leu Asn Cys
            260                 265                 270

Glu Gly Lys Met Phe Trp Asp Gln Val His Pro Thr Thr Val Val His
        275                 280                 285

Ala Ala Leu Ser Glu Arg Ala
    290                 295
```

<210> 49
<211> 247
<212> PRT
<213> Streptomyces coelicolor

<400> 49

```
Tyr Val Ala Leu Gly Asp Ser Tyr Ser Ala Gly Ser Gly Val Leu Pro
1               5                   10                  15

Val Asp Pro Ala Asn Leu Leu Cys Leu Arg Ser Thr Ala Asn Tyr Pro
            20                  25                  30

His Val Ile Ala Asp Thr Thr Gly Ala Arg Leu Thr Asp Val Thr Cys
        35                  40                  45

Gly Ala Ala Gln Thr Ala Asp Phe Thr Arg Ala Gln Tyr Pro Gly Val
    50                  55                  60

Ala Pro Gln Leu Asp Ala Leu Gly Thr Gly Thr Asp Leu Val Thr Leu
65                  70                  75                  80

Thr Ile Gly Gly Asn Asp Asn Ser Thr Phe Ile Asn Ala Ile Thr Ala
                85                  90                  95

Cys Gly Thr Ala Gly Val Leu Ser Gly Gly Lys Gly Ser Pro Cys Lys
            100                 105                 110

Asp Arg His Gly Thr Ser Phe Asp Asp Glu Ile Glu Ala Asn Thr Tyr
        115                 120                 125

Pro Ala Leu Lys Glu Ala Leu Leu Gly Val Arg Ala Arg Ala Pro His
    130                 135                 140

Ala Arg Val Ala Ala Leu Gly Tyr Pro Trp Ile Thr Pro Ala Thr Ala
145                 150                 155                 160

Asp Pro Ser Cys Phe Leu Lys Leu Pro Leu Ala Ala Gly Asp Val Pro
                165                 170                 175

Tyr Leu Arg Ala Ile Gln Ala His Leu Asn Asp Ala Val Arg Arg Ala
            180                 185                 190

Ala Glu Glu Thr Gly Ala Thr Tyr Val Asp Phe Ser Gly Val Ser Asp
        195                 200                 205

Gly His Asp Ala Cys Glu Ala Pro Gly Thr Arg Trp Ile Glu Pro Leu
    210                 215                 220

Leu Phe Gly His Ser Leu Val Pro Val His Pro Asn Ala Leu Gly Glu
225                 230                 235                 240

Arg Arg Met Ala Glu His Thr
                245
```

<210> 50
<211> 247
<212> PRT
<213> Streptomyces coelicolor

<400> 50

```
Tyr Val Ala Leu Gly Asp Ser Tyr Ser Ala Gly Ser Gly Val Leu Pro
1               5                   10                  15

Val Asp Pro Ala Asn Leu Leu Cys Leu Arg Ser Thr Ala Asn Tyr Pro
            20                  25                  30

His Val Ile Ala Asp Thr Thr Gly Ala Arg Leu Thr Asp Val Thr Cys
        35                  40                  45

Gly Ala Ala Gln Thr Ala Asp Phe Thr Arg Ala Gln Tyr Pro Gly Val
    50                  55                  60

Ala Pro Gln Leu Asp Ala Leu Gly Thr Gly Thr Asp Leu Val Thr Leu
65                  70                  75                  80

Thr Ile Gly Gly Asn Asp Asn Ser Thr Phe Ile Asn Ala Ile Thr Ala
                85                  90                  95

Cys Gly Thr Ala Gly Val Leu Ser Gly Gly Lys Gly Ser Pro Cys Lys
            100                 105                 110

Asp Arg His Gly Thr Ser Phe Asp Asp Glu Ile Glu Ala Asn Thr Tyr
        115                 120                 125

Pro Ala Leu Lys Glu Ala Leu Leu Gly Val Arg Ala Arg Ala Pro His
    130                 135                 140

Ala Arg Val Ala Ala Leu Gly Tyr Pro Trp Ile Thr Pro Ala Thr Ala
145                 150                 155                 160

Asp Pro Ser Cys Phe Leu Lys Leu Pro Leu Ala Ala Gly Asp Val Pro
                165                 170                 175

Tyr Leu Arg Ala Ile Gln Ala His Leu Asn Asp Ala Val Arg Arg Ala
            180                 185                 190

Ala Glu Glu Thr Gly Ala Thr Tyr Val Asp Phe Ser Gly Val Ser Asp
        195                 200                 205

Gly His Asp Ala Cys Glu Ala Pro Gly Thr Arg Trp Ile Glu Pro Leu
    210                 215                 220

Leu Phe Gly His Ser Leu Val Pro Val His Pro Asn Ala Leu Gly Glu
225                 230                 235                 240

Arg Arg Met Ala Glu His Thr
                245
```

<210> 51
<211> 198
<212> PRT
<213> Saccharomyces cerevisiae

<400> 51

```
Phe Leu Leu Phe Gly Asp Ser Ile Thr Glu Phe Ala Phe Asn Thr Arg
1               5                   10                  15

Pro Ile Glu Asp Gly Lys Asp Gln Tyr Ala Leu Gly Ala Ala Leu Val
            20                  25                  30

Asn Glu Tyr Thr Arg Lys Met Asp Ile Leu Gln Arg Gly Phe Lys Gly
        35                  40                  45

Tyr Thr Ser Arg Trp Ala Leu Lys Ile Leu Pro Glu Ile Leu Lys His
    50                  55                  60

Glu Ser Asn Ile Val Met Ala Thr Ile Phe Leu Gly Ala Asn Asp Ala
65                  70                  75                  80

Cys Ser Ala Gly Pro Gln Ser Val Pro Leu Pro Glu Phe Ile Asp Asn
                85                  90                  95

Ile Arg Gln Met Val Ser Leu Met Lys Ser Tyr His Ile Arg Pro Ile
            100                 105                 110

Ile Ile Gly Pro Gly Leu Val Asp Arg Glu Lys Trp Glu Lys Glu Lys
        115                 120                 125

Ser Glu Glu Ile Ala Leu Gly Tyr Phe Arg Thr Asn Glu Asn Phe Ala
    130                 135                 140

Ile Tyr Ser Asp Ala Leu Ala Lys Leu Ala Asn Glu Glu Lys Val Pro
145                 150                 155                 160

Phe Val Ala Leu Asn Lys Ala Phe Gln Gln Glu Gly Gly Asp Ala Trp
                165                 170                 175

Gln Gln Leu Leu Thr Asp Gly Leu His Phe Ser Gly Lys Gly Tyr Lys
            180                 185                 190

Ile Phe His Asp Glu Leu
        195
```

<210> 52
<211> 317
<212> PRT
<213> Aeromonas salmonicida

<400> 52

```
Ala Asp Thr Arg Pro Ala Phe Ser Arg Ile Val Met Phe Gly Asp Ser
1               5                   10                  15

Leu Ser Asp Thr Gly Lys Met Tyr Ser Lys Met Arg Gly Tyr Leu Pro
            20                  25                  30

Ser Ser Pro Pro Tyr Tyr Glu Gly Arg Phe Ser Asn Gly Pro Val Trp
        35                  40                  45

Leu Glu Gln Leu Thr Lys Gln Phe Pro Gly Leu Thr Ile Ala Asn Glu
    50                  55                  60

Ala Glu Gly Gly Ala Thr Ala Val Ala Tyr Asn Lys Ile Ser Trp Asn
65                  70                  75                  80

Pro Lys Tyr Gln Val Tyr Asn Asn Leu Asp Tyr Glu Val Thr Gln Phe
                85                  90                  95

Leu Gln Lys Asp Ser Phe Lys Pro Asp Asp Leu Val Ile Leu Trp Val
            100                 105                 110

Gly Ala Asn Asp Tyr Leu Ala Tyr Gly Trp Asn Thr Glu Gln Asp Ala
        115                 120                 125

Lys Arg Val Arg Asp Ala Ile Ser Asp Ala Ala Asn Arg Met Val Leu
    130                 135                 140

Asn Gly Ala Lys Gln Ile Leu Leu Phe Asn Leu Pro Asp Leu Gly Gln
145                 150                 155                 160

Asn Pro Ser Ala Arg Ser Gln Lys Val Val Glu Ala Val Ser His Val
                165                 170                 175

Ser Ala Tyr His Asn Lys Leu Leu Leu Asn Leu Ala Arg Gln Leu Ala
            180                 185                 190

Pro Thr Gly Met Val Lys Leu Phe Glu Ile Asp Lys Gln Phe Ala Glu
        195                 200                 205

Met Leu Arg Asp Pro Gln Asn Phe Gly Leu Ser Asp Val Glu Asn Pro
    210                 215                 220
```

Cys Tyr Asp Gly Gly Tyr Val Trp Lys Pro Phe Ala Thr Arg Ser Val
225 230 235 240

Ser Thr Asp Arg Gln Leu Ser Ala Phe Ser Pro Gln Glu Arg Leu Ala
245 250 255

Ile Ala Gly Asn Pro Leu Leu Ala Gln Ala Val Ala Ser Pro Met Ala
260 265 270

Arg Arg Ser Ala Ser Pro Leu Asn Cys Glu Gly Lys Met Phe Trp Asp
275 280 285

Gln Val His Pro Thr Thr Val Val His Ala Ala Leu Ser Glu Arg Ala
290 295 300

Ala Thr Phe Ile Glu Thr Gln Tyr Glu Phe Leu Ala His
305 310 315

<210> 53
<211> 320
<212> PRT
<213> Ralstonia solanacearum

<400> 53

Gln Ser Gly Asn Pro Asn Val Ala Lys Val Gln Arg Met Val Val Phe
1 5 10 15

Gly Asp Ser Leu Ser Asp Ile Gly Thr Tyr Thr Pro Val Ala Gln Ala
20 25 30

Val Gly Gly Gly Lys Phe Thr Thr Asn Pro Gly Pro Ile Trp Ala Glu
35 40 45

Thr Val Ala Ala Gln Leu Gly Val Thr Leu Thr Pro Ala Val Met Gly
50 55 60

Tyr Ala Thr Ser Val Gln Asn Cys Pro Lys Ala Gly Cys Phe Asp Tyr
65 70 75 80

Ala Gln Gly Gly Ser Arg Val Thr Asp Pro Asn Gly Ile Gly His Asn
85 90 95

Gly Gly Ala Gly Ala Leu Thr Tyr Pro Val Gln Gln Gln Leu Ala Asn
100 105 110

Phe Tyr Ala Ala Ser Asn Asn Thr Phe Asn Gly Asn Asn Asp Val Val
115 120 125

Phe Val Leu Ala Gly Ser Asn Asp Ile Phe Phe Trp Thr Thr Ala Ala

130 135 140

Ala Thr Ser Gly Ser Gly Val Thr Pro Ala Ile Ala Thr Ala Gln Val
145 150 155 160

Gln Gln Ala Ala Thr Asp Leu Val Gly Tyr Val Lys Asp Met Ile Ala
165 170 175

Lys Gly Ala Thr Gln Val Tyr Val Phe Asn Leu Pro Asp Ser Ser Leu
180 185 190

Thr Pro Asp Gly Val Ala Ser Gly Thr Thr Gly Gln Ala Leu Leu His
195 200 205

Ala Leu Val Gly Thr Phe Asn Thr Thr Leu Gln Ser Gly Leu Ala Gly
210 215 220

Thr Ser Ala Arg Ile Ile Asp Phe Asn Ala Gln Leu Thr Ala Ala Ile
225 230 235 240

Gln Asn Gly Ala Ser Phe Gly Phe Ala Asn Thr Ser Ala Arg Ala Cys
245 250 255

Asp Ala Thr Lys Ile Asn Ala Leu Val Pro Ser Ala Gly Gly Ser Ser
260 265 270

Leu Phe Cys Ser Ala Asn Thr Leu Val Ala Ser Gly Ala Asp Gln Ser
275 280 285

Tyr Leu Phe Ala Asp Gly Val His Pro Thr Thr Ala Gly His Arg Leu
290 295 300

Ile Ala Ser Asn Val Leu Ala Arg Leu Leu Ala Asp Asn Val Ala His
305 310 315 320

<210> 54
<211> 230
<212> PRT
<213> Aspergillus aculeatus

<400> 54

```
Thr Thr Val Tyr Leu Ala Gly Asp Ser Thr Met Ala Lys Asn Gly Gly
1               5                   10                  15

Gly Ser Gly Thr Asn Gly Trp Gly Glu Tyr Leu Ala Ser Tyr Leu Ser
            20                  25                  30

Ala Thr Val Val Asn Asp Ala Val Ala Gly Arg Ser Ala Arg Ser Tyr
        35                  40                  45

Thr Arg Glu Gly Arg Phe Glu Asn Ile Ala Asp Val Val Thr Ala Gly
    50                  55                  60

Asp Tyr Val Ile Val Glu Phe Gly His Asn Asp Gly Gly Ser Leu Ser
65                  70                  75                  80

Thr Asp Asn Gly Arg Thr Asp Cys Ser Gly Thr Gly Ala Glu Val Cys
                85                  90                  95

Tyr Ser Val Tyr Asp Gly Val Asn Glu Thr Ile Leu Thr Phe Pro Ala
            100                 105                 110

Tyr Leu Glu Asn Ala Ala Lys Leu Phe Thr Ala Lys Gly Ala Lys Val
        115                 120                 125

Ile Leu Ser Ser Gln Thr Pro Asn Asn Pro Trp Glu Thr Gly Thr Phe
    130                 135                 140

Val Asn Ser Pro Thr Arg Phe Val Glu Tyr Ala Glu Leu Ala Ala Glu
145                 150                 155                 160

Val Ala Gly Val Glu Tyr Val Asp His Trp Ser Tyr Val Asp Ser Ile
                165                 170                 175

Tyr Glu Thr Leu Gly Asn Ala Thr Val Asn Ser Tyr Phe Pro Ile Asp
            180                 185                 190

His Thr His Thr Ser Pro Ala Gly Ala Glu Val Val Ala Glu Ala Phe
        195                 200                 205

Leu Lys Ala Val Val Cys Thr Gly Thr Ser Leu Lys Ser Val Leu Thr
    210                 215                 220

Thr Thr Ser Phe Glu Gly
225                 230
```

<210> 55
<211> 184
<212> PRT
<213> Escherichia coli

<400> 55

```
Ala Asp Thr Leu Leu Ile Leu Gly Asp Ser Leu Ser Ala Gly Tyr Arg
1               5                   10                  15

Met Ser Ala Ser Ala Ala Trp Pro Ala Leu Leu Asn Asp Lys Trp Gln
            20                  25                  30

Ser Lys Thr Ser Val Val Asn Ala Ser Ile Ser Gly Asp Thr Ser Gln
        35                  40                  45

Gln Gly Leu Ala Arg Leu Pro Ala Leu Leu Lys Gln His Gln Pro Arg
    50                  55                  60

Trp Val Leu Val Glu Leu Gly Gly Asn Asp Gly Leu Arg Gly Phe Gln
65                  70                  75                  80

Pro Gln Gln Thr Glu Gln Thr Leu Arg Gln Ile Leu Gln Asp Val Lys
                85                  90                  95

Ala Ala Asn Ala Glu Pro Leu Leu Met Gln Ile Arg Leu Pro Ala Asn
            100                 105                 110

Tyr Gly Arg Arg Tyr Asn Glu Ala Phe Ser Ala Ile Tyr Pro Lys Leu
        115                 120                 125

Ala Lys Glu Phe Asp Val Pro Leu Leu Pro Phe Phe Met Glu Glu Val
    130                 135                 140

Tyr Leu Lys Pro Gln Trp Met Gln Asp Asp Gly Ile His Pro Asn Arg
145                 150                 155                 160

Asp Ala Gln Pro Phe Ile Ala Asp Trp Met Ala Lys Gln Leu Gln Pro
                165                 170                 175

Leu Val Asn His Asp Ser Leu Glu
            180
```

<210> 56
<211> 308
<212> PRT
<213> Aeromonas hydrophila

<400> 56

```
Ile Val Met Phe Gly Asp Ser Leu Ser Asp Thr Gly Lys Met Tyr Ser
1               5                   10                  15

Lys Met Arg Gly Tyr Leu Pro Ser Ser Pro Pro Tyr Tyr Glu Gly Arg
            20                  25                  30

Phe Ser Asn Gly Pro Val Trp Leu Glu Gln Leu Thr Asn Glu Phe Pro
        35                  40                  45

Gly Leu Thr Ile Ala Asn Glu Ala Glu Gly Gly Pro Thr Ala Val Ala
    50                  55                  60
```

```
Tyr Asn Lys Ile Ser Trp Asn Pro Lys Tyr Gln Val Ile Asn Asn Leu
65                  70                  75                  80

Asp Tyr Glu Val Thr Gln Phe Leu Gln Lys Asp Ser Phe Lys Pro Asp
                85                  90                  95

Asp Leu Val Ile Leu Trp Val Gly Ala Asn Asp Tyr Leu Ala Tyr Gly
            100                 105                 110

Trp Asn Thr Glu Gln Asp Ala Lys Arg Val Arg Asp Ala Ile Ser Asp
        115                 120                 125

Ala Ala Asn Arg Met Val Leu Asn Gly Ala Lys Glu Ile Leu Leu Phe
    130                 135                 140

Asn Leu Pro Asp Leu Gly Gln Asn Pro Ser Ala Arg Ser Gln Lys Val
145                 150                 155                 160

Val Glu Ala Ala Ser His Val Ser Ala Tyr His Asn Gln Leu Leu Leu
                165                 170                 175

Asn Leu Ala Arg Gln Leu Ala Pro Thr Gly Met Val Lys Leu Phe Glu
            180                 185                 190

Ile Asp Lys Gln Phe Ala Glu Met Leu Arg Asp Pro Gln Asn Phe Gly
        195                 200                 205

Leu Ser Asp Gln Arg Asn Ala Cys Tyr Gly Gly Ser Tyr Val Trp Lys
    210                 215                 220

Pro Phe Ala Ser Arg Ser Ala Ser Thr Asp Ser Gln Leu Ser Ala Phe
225                 230                 235                 240

Asn Pro Gln Glu Arg Leu Ala Ile Ala Gly Asn Pro Leu Leu Ala Gln
                245                 250                 255

Ala Val Ala Ser Pro Met Ala Ala Arg Ser Ala Ser Thr Leu Asn Cys
            260                 265                 270

Glu Gly Lys Met Phe Trp Asp Gln Val His Pro Thr Thr Val Val His
        275                 280                 285

Ala Ala Leu Ser Glu Pro Ala Ala Thr Phe Ile Glu Ser Gln Tyr Glu
    290                 295                 300

Phe Leu Ala His
305
```

<210> 57

<211> 232
<212> PRT
<213> Aspergillus aculeatus

<400> 57

```
Thr Thr Val Tyr Leu Ala Gly Asp Ser Thr Met Ala Lys Asn Gly Gly
1               5                   10                  15

Gly Ser Gly Thr Asn Gly Trp Gly Glu Tyr Leu Ala Ser Tyr Leu Ser
            20                  25                  30

Ala Thr Val Val Asn Asp Ala Val Ala Gly Arg Ser Ala Arg Ser Tyr
        35                  40                  45

Thr Arg Glu Gly Arg Phe Glu Asn Ile Ala Asp Val Val Thr Ala Gly
    50                  55                  60

Asp Tyr Val Ile Val Glu Phe Gly His Asn Asp Gly Gly Ser Leu Ser
65                  70                  75                  80

Thr Asp Asn Gly Arg Thr Asp Cys Ser Gly Thr Gly Ala Glu Val Cys
                85                  90                  95

Tyr Ser Val Tyr Asp Gly Val Asn Glu Thr Ile Leu Thr Phe Pro Ala
            100                 105                 110

Tyr Leu Glu Asn Ala Ala Lys Leu Phe Thr Ala Lys Gly Ala Lys Val
        115                 120                 125

Ile Leu Ser Ser Gln Thr Pro Asn Asn Pro Trp Glu Thr Gly Thr Phe
    130                 135                 140

Val Asn Ser Pro Thr Arg Phe Val Glu Tyr Ala Glu Leu Ala Ala Glu
145                 150                 155                 160

Val Ala Gly Val Glu Tyr Val Asp His Trp Ser Tyr Val Asp Ser Ile
                165                 170                 175

Tyr Glu Thr Leu Gly Asn Ala Thr Val Asn Ser Tyr Phe Pro Ile Asp
            180                 185                 190

His Thr His Thr Ser Pro Ala Gly Ala Glu Val Val Ala Glu Ala Phe
        195                 200                 205

Leu Lys Ala Val Val Cys Thr Gly Thr Ser Leu Lys Ser Val Leu Thr
    210                 215                 220

Thr Thr Ser Phe Glu Gly Thr Cys
```

225 230

<210> 58
<211> 167
<212> PRT
<213> Escherichia coli

<400> 58

Leu Leu Ile Leu Gly Asp Ser Leu Ser Ala Gly Tyr Arg Met Ser Ala
1 5 10 15

Ser Ala Ala Trp Pro Ala Leu Leu Asn Asp Lys Trp Gln Ser Lys Thr
20 25 30

Ser Val Val Asn Ala Ser Ile Ser Gly Asp Thr Ser Gln Gln Gly Leu
35 40 45

Ala Arg Leu Pro Ala Leu Leu Lys Gln His Gln Pro Arg Trp Val Leu
50 55 60

Val Glu Leu Gly Gly Asn Asp Gly Leu Arg Gly Phe Gln Pro Gln Gln
65 70 75 80

Thr Glu Gln Thr Leu Arg Gln Ile Leu Gln Asp Val Lys Ala Ala Asn
85 90 95

Ala Glu Pro Leu Leu Met Gln Ile Arg Leu Pro Ala Asn Tyr Gly Arg
100 105 110

Arg Tyr Asn Glu Ala Phe Ser Ala Ile Tyr Pro Lys Leu Ala Lys Glu
115 120 125

Phe Asp Val Pro Leu Leu Pro Phe Phe Met Glu Glu Val Tyr Leu Lys
130 135 140

Pro Gln Trp Met Gln Asp Asp Gly Ile His Pro Asn Arg Asp Ala Gln
145 150 155 160

Pro Phe Ile Ala Asp Trp Met
165

<210> 59
<211> 295
<212> PRT
<213> Aeromonas hydrophila

<400> 59

Ile Val Met Phe Gly Asp Ser Leu Ser Asp Thr Gly Lys Met Tyr Ser
1 5 10 15

```
Lys Met Arg Gly Tyr Leu Pro Ser Ser Pro Pro Tyr Tyr Glu Gly Arg
            20                  25                  30

Phe Ser Asn Gly Pro Val Trp Leu Glu Gln Leu Thr Asn Glu Phe Pro
        35                  40                  45

Gly Leu Thr Ile Ala Asn Glu Ala Glu Gly Gly Pro Thr Ala Val Ala
    50                  55                  60

Tyr Asn Lys Ile Ser Trp Asn Pro Lys Tyr Gln Val Ile Asn Asn Leu
65                  70                  75                  80

Asp Tyr Glu Val Thr Gln Phe Leu Gln Lys Asp Ser Phe Lys Pro Asp
                85                  90                  95

Asp Leu Val Ile Leu Trp Val Gly Ala Asn Asp Tyr Leu Ala Tyr Gly
            100                 105                 110

Trp Asn Thr Glu Gln Asp Ala Lys Arg Val Arg Asp Ala Ile Ser Asp
        115                 120                 125

Ala Ala Asn Arg Met Val Leu Asn Gly Ala Lys Glu Ile Leu Leu Phe
    130                 135                 140

Asn Leu Pro Asp Leu Gly Gln Asn Pro Ser Ala Arg Ser Gln Lys Val
145                 150                 155                 160

Val Glu Ala Ala Ser His Val Ser Ala Tyr His Asn Gln Leu Leu Leu
                165                 170                 175

Asn Leu Ala Arg Gln Leu Ala Pro Thr Gly Met Val Lys Leu Phe Glu
            180                 185                 190

Ile Asp Lys Gln Phe Ala Glu Met Leu Arg Asp Pro Gln Asn Phe Gly
        195                 200                 205

Leu Ser Asp Gln Arg Asn Ala Cys Tyr Gly Gly Ser Tyr Val Trp Lys
    210                 215                 220

Pro Phe Ala Ser Arg Ser Ala Ser Thr Asp Ser Gln Leu Ser Ala Phe
225                 230                 235                 240

Asn Pro Gln Glu Arg Leu Ala Ile Ala Gly Asn Pro Leu Leu Ala Gln
                245                 250                 255

Ala Val Ala Ser Pro Met Ala Ala Arg Ser Ala Ser Thr Leu Asn Cys
            260                 265                 270
```

Glu Gly Lys Met Phe Trp Asp Gln Val His Pro Thr Thr Val Val His
275 280 285

Ala Ala Leu Ser Glu Pro Ala
290 295

<210> 60
<211> 335
<212> PRT
<213> Aeromonas hydrophila

<400> 60

Met Lys Lys Trp Phe Val Cys Leu Leu Gly Leu Val Ala Leu Thr Val
1 5 10 15

Gln Ala Ala Asp Ser Arg Pro Ala Phe Ser Arg Ile Val Met Phe Gly
20 25 30

Asp Ser Leu Ser Asp Thr Gly Lys Met Tyr Ser Lys Met Arg Gly Tyr
35 40 45

Leu Pro Ser Ser Pro Pro Tyr Tyr Glu Gly Arg Phe Ser Asn Gly Pro
50 55 60

Val Trp Leu Glu Gln Leu Thr Asn Glu Phe Pro Gly Leu Thr Ile Ala
65 70 75 80

Asn Glu Ala Glu Gly Gly Pro Thr Ala Val Ala Tyr Asn Lys Ile Ser
85 90 95

Trp Asn Pro Lys Tyr Gln Val Ile Asn Asn Leu Asp Tyr Glu Val Thr
100 105 110

Gln Phe Leu Gln Lys Asp Ser Phe Lys Pro Asp Asp Leu Val Ile Leu
115 120 125

Trp Val Gly Ala Asn Asp Tyr Leu Ala Tyr Gly Trp Asn Thr Glu Gln
130 135 140

Asp Ala Lys Arg Val Arg Asp Ala Ile Ser Asp Ala Ala Asn Arg Met
145 150 155 160

Val Leu Asn Gly Ala Lys Glu Ile Leu Leu Phe Asn Leu Pro Asp Leu
165 170 175

Gly Gln Asn Pro Ser Ala Arg Ser Gln Lys Val Val Glu Ala Ala Ser
180 185 190

```
His Val Ser Ala Tyr His Asn Gln Leu Leu Leu Asn Leu Ala Arg Gln
        195                 200                 205

Leu Ala Pro Thr Gly Met Val Lys Leu Phe Glu Ile Asp Lys Gln Phe
    210                 215                 220

Ala Glu Met Leu Arg Asp Pro Gln Asn Phe Gly Leu Ser Asp Gln Arg
225                 230                 235                 240

Asn Ala Cys Tyr Gly Gly Ser Tyr Val Trp Lys Pro Phe Ala Ser Arg
                245                 250                 255

Ser Ala Ser Thr Asp Ser Gln Leu Ser Ala Phe Asn Pro Gln Glu Arg
            260                 265                 270

Leu Ala Ile Ala Gly Asn Pro Leu Leu Ala Gln Ala Val Ala Ser Pro
        275                 280                 285

Met Ala Ala Arg Ser Ala Ser Thr Leu Asn Cys Glu Gly Lys Met Phe
    290                 295                 300

Trp Asp Gln Val His Pro Thr Thr Val Val His Ala Ala Leu Ser Glu
305                 310                 315                 320

Pro Ala Ala Thr Phe Ile Glu Ser Gln Tyr Glu Phe Leu Ala His
                325                 330                 335
```

<210> 61
<211> 318
<212> PRT
<213> Aeromonas salmonicida

<400> 61

```
Ala Asp Thr Arg Pro Ala Phe Ser Arg Ile Val Met Phe Gly Asp Ser
1               5                   10                  15

Leu Ser Asp Thr Gly Lys Met Tyr Ser Lys Met Arg Gly Tyr Leu Pro
            20                  25                  30

Ser Ser Pro Pro Tyr Tyr Glu Gly Arg Phe Ser Asn Gly Pro Val Trp
        35                  40                  45

Leu Glu Gln Leu Thr Lys Gln Phe Pro Gly Leu Thr Ile Ala Asn Glu
    50                  55                  60

Ala Glu Gly Gly Ala Thr Ala Val Ala Tyr Asn Lys Ile Ser Trp Asn
65                  70                  75                  80

Pro Lys Tyr Gln Val Tyr Asn Asn Leu Asp Tyr Glu Val Thr Gln Phe
```

```
                85                  90                  95

Leu Gln Lys Asp Ser Phe Lys Pro Asp Asp Leu Val Ile Leu Trp Val
            100                 105                 110

Gly Ala Asn Asp Tyr Leu Ala Tyr Gly Trp Asn Thr Glu Gln Asp Ala
        115                 120                 125

Lys Arg Val Arg Asp Ala Ile Ser Asp Ala Ala Asn Arg Met Val Leu
    130                 135                 140

Asn Gly Ala Lys Gln Ile Leu Leu Phe Asn Leu Pro Asp Leu Gly Gln
145                 150                 155                 160

Asn Pro Ser Ala Arg Ser Gln Lys Val Val Glu Ala Val Ser His Val
                165                 170                 175

Ser Ala Tyr His Asn Lys Leu Leu Leu Asn Leu Ala Arg Gln Leu Ala
            180                 185                 190

Pro Thr Gly Met Val Lys Leu Phe Glu Ile Asp Lys Gln Phe Ala Glu
        195                 200                 205

Met Leu Arg Asp Pro Gln Asn Phe Gly Leu Ser Asp Val Glu Asn Pro
    210                 215                 220

Cys Tyr Asp Gly Gly Tyr Val Trp Lys Pro Phe Ala Thr Arg Ser Val
225                 230                 235                 240

Ser Thr Asp Arg Gln Leu Ser Ala Phe Ser Pro Gln Glu Arg Leu Ala
                245                 250                 255

Ile Ala Gly Asn Pro Leu Leu Ala Gln Ala Val Ala Ser Pro Met Ala
            260                 265                 270

Arg Arg Ser Ala Ser Pro Leu Asn Cys Glu Gly Lys Met Phe Trp Asp
        275                 280                 285

Gln Val His Pro Thr Thr Val Val His Ala Ala Leu Ser Glu Arg Ala
    290                 295                 300

Ala Thr Phe Ile Glu Thr Gln Tyr Glu Phe Leu Ala His Gly
305                 310                 315
```

<210> 62
<211> 50
<212> PRT
<213> Artificial Sequence

<220>
<223> Consensus sequence

<400> 62

```
Arg Pro Ala Phe Ser Arg Ile Val Met Phe Gly Asp Ser Leu Ser Asp
1               5                   10                  15

Thr Gly Lys Met Tyr Ser Lys Met Arg Gly Tyr Leu Pro Ser Ser Pro
            20                  25                  30

Pro Tyr Tyr Glu Gly Arg Phe Ser Asn Gly Pro Val Trp Leu Glu Gln
        35                  40                  45

Leu Thr
    50
```

<210> 63
<211> 13
<212> PRT
<213> Artificial Sequence

<220>
<223> Consensus sequence

<400> 63

```
Phe Pro Gly Leu Thr Ile Ala Asn Glu Ala Glu Gly Gly
1               5                   10
```

<210> 64
<211> 79
<212> PRT
<213> Artificial Sequence

<220>
<223> Consensus sequence

<400> 64

```
Thr Ala Val Ala Tyr Asn Lys Ile Ser Trp Asn Pro Lys Tyr Gln Val
1               5                   10                  15

Ile Asn Asn Leu Asp Tyr Glu Val Thr Gln Phe Leu Gln Lys Asp Ser
            20                  25                  30

Phe Lys Pro Asp Asp Leu Val Ile Leu Trp Val Gly Ala Asn Asp Tyr
        35                  40                  45

Leu Ala Tyr Gly Trp Asn Thr Glu Gln Asp Ala Lys Arg Val Arg Asp
    50                  55                  60

Ala Ile Ser Asp Ala Ala Asn Arg Met Val Leu Asn Gly Ala Lys
65                  70                  75
```

<210> 65
<211> 23
<212> PRT
<213> Artificial Sequence

<220>
<223> Consensus sequence

<400> 65

```
Ile Leu Leu Phe Asn Leu Pro Asp Leu Gly Gln Asn Pro Ser Ala Arg
1               5                   10                  15

Ser Gln Lys Val Val Glu Ala
            20
```

<210> 66
<211> 8
<212> PRT
<213> Artificial Sequence

<220>
<223> Consensus sequence

<400> 66

```
Ser His Val Ser Ala Tyr His Asn
1               5
```

<210> 67
<211> 38
<212> PRT
<213> Artificial Sequence

<220>
<223> Consensus sequence

<400> 67

Leu Leu Leu Asn Leu Ala Arg Gln Leu Ala Pro Thr Gly Met Val Lys
1 5 10 15

Leu Phe Glu Ile Asp Lys Gln Phe Ala Glu Met Leu Arg Asp Pro Gln
20 25 30

Asn Phe Gly Leu Ser Asp
35

<210> 68
<211> 7
<212> PRT
<213> Artificial Sequence

<220>
<223> Consensus sequence

<400> 68

Tyr Val Trp Lys Pro Phe Ala
1 5

<210> 69
<211> 5
<212> PRT
<213> Artificial Sequence

<220>
<223> Consensus sequence

<400> 69

Gln Leu Ser Ala Phe
1 5

<210> 70
<211> 22
<212> PRT
<213> Artificial Sequence

<220>
<223> Consensus sequence

<400> 70

Pro Gln Glu Arg Leu Ala Ile Ala Gly Asn Pro Leu Leu Ala Gln Ala
1 5 10 15

Val Ala Ser Pro Met Ala
20

<210> 71
<211> 4
<212> PRT

<213> Artificial Sequence

<220>
<223> Consensus sequence

<400> 71

Arg Ser Ala Ser
1

<210> 72
<211> 24
<212> PRT
<213> Artificial Sequence

<220>
<223> Consensus sequence

<400> 72

Leu Asn Cys Glu Gly Lys Met Phe Trp Asp Gln Val His Pro Thr Thr
1 5 10 15

Val Val His Ala Ala Leu Ser Glu
20

<210> 73
<211> 5
<212> PRT
<213> Artificial Sequence

<220>
<223> Consensus sequence

<400> 73

Ala Ala Thr Phe Ile
1 5

<210> 74
<211> 7
<212> PRT
<213> Artificial Sequence

<220>
<223> Consensus sequence

<400> 74

Gln Tyr Glu Phe Leu Ala His
1 5

<210> 75
<211> 4
<212> PRT
<213> Artificial Sequence

<220>
<223> Sequence motif

<220>
<221> MISC_FEATURE
<222> (4)..(4)
<223> Xaa may be one or more of the following amino acid residues Leu, Ala, Val, Ile, Phe, Tyr, His, Gln, Thr, Asn, Met or Ser.

<400> 75

```
Gly Asp Ser Xaa
1
```

<210> 76
<211> 5
<212> PRT
<213> Artificial Sequence

<220>
<223> Sequence motif

<220>
<221> MISC_FEATURE
<222> (2)..(2)
<223> Xaa may be Ala or Gly

<220>
<221> MISC_FEATURE
<222> (5)..(5)
<223> Xaa may be Tyr, Ala or Leu

<400> 76

```
Gly Ala Asn Asp Tyr
1               5
```

<210> 77
<211> 8
<212> PRT
<213> Artificial Sequence

<220>
<223> Block 1 GDSX block

<220>
<221> MISC_FEATURE
<222> (1)..(1)
<223> Xaa is a hydrophobic residue selected from Met, Ile, Leu, Val, Ala, Gly, Cys, His, Lys, Trp, Tyr or Phe

<220>
<221> misc_feature
<222> (2)..(4)
<223> Xaa can be any naturally occurring amino acid

<220>
<221> MISC_FEATURE
<222> (8)..(8)

<223> Xaa is a hydrophobic residue selected from Met, Ile, Leu, Val, Ala, Gly, Cys, His, Lys, Trp, Tyr or Phe

<400> 77

```
Xaa Xaa Xaa Xaa Gly Asp Ser Xaa
1               5
```

<210> 78
<211> 6
<212> PRT
<213> Artificial Sequence

<220>
<223> Block 2 GANDY block

<220>
<221> MISC_FEATURE
<222> (1)..(1)
<223> Xaa is a hydrophobic residue selected from Met, Ile, Leu, Val, Ala, Gly, Cys, His, Lys, Trp, Tyr or Phe

<220>
<221> MISC_FEATURE
<222> (3)..(3)
<223> Xaa is a hydrophobic residue selected from Met, Ile, Leu, Val, Ala, Gly, Cys, His, Lys, Trp, Tyr or Phe

<220>
<221> MISC_FEATURE
<222> (6)..(6)
<223> Xaa is a hydrophobic residue selected from Met, Ile, Leu, Val, Ala, Gly, Cys, His, Lys, Trp, Tyr or Phe

<400> 78

```
Xaa Gly Xaa Asn Asp Xaa
1               5
```